(19) Europäisches Patentamt / European Patent Office / Office européen des brevets

(11) **EP 3 957 325 A1**

(12) **EUROPEAN PATENT APPLICATION**
published in accordance with Art. 153(4) EPC

(43) Date of publication:
**23.02.2022 Bulletin 2022/08**

(21) Application number: **20791023.3**

(22) Date of filing: **17.04.2020**

(51) International Patent Classification (IPC):
*A61K 39/395* (2006.01)   *A61P 35/00* (2006.01)
*C12N 5/10* (2006.01)   *C12N 1/15* (2006.01)
*C12N 1/19* (2006.01)   *C12N 1/21* (2006.01)
*C12N 15/13* (2006.01)   *C12N 15/62* (2006.01)
*C12N 15/63* (2006.01)   *A61K 38/16* (2006.01)
*A61K 35/17* (2015.01)

(52) Cooperative Patent Classification (CPC):
**A61K 35/17; A61K 38/16; A61K 39/395;**
**A61P 35/00; C12N 5/10; C12N 15/62; C12N 15/63**

(86) International application number:
**PCT/JP2020/016915**

(87) International publication number:
**WO 2020/213724 (22.10.2020 Gazette 2020/43)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **19.04.2019   JP 2019080566**

(71) Applicants:
• **CHUGAI SEIYAKU KABUSHIKI KAISHA**
  **Tokyo 115-8543 (JP)**
• **Yamaguchi University**
  **Yamaguchi 753-8511 (JP)**

(72) Inventors:
• **SAKURAI, Mika**
  **Gotemba-shi, Shizuoka 412-8513 (JP)**
• **IGAWA, Tomoyuki**
  **Synapse,138623 (SG)**
• **TAMADA, Koji**
  **Ube-shi, Yamaguchi 755-8505 (JP)**
• **SAKODA, Yukimi**
  **Ube-shi, Yamaguchi 755-8505 (JP)**

(74) Representative: **Vossius & Partner**
**Patentanwälte Rechtsanwälte mbB**
**Siebertstraße 3**
**81675 München (DE)**

(54) **CHIMERIC RECEPTOR RECOGNIZING MODIFICATION SITE OF ANTIBODY**

(57)   The present disclosure provides a pharmaceutical composition for use in combination with administration of a mutated antibody having a mutation, including substitution, deletion, addition or modification, of at least one amino acid in a CH1 region, a CH2 region, a CH3 region, a CL region, or a framework region, wherein the pharmaceutical composition comprises a cell expressing a chimeric receptor, the mutated antibody is capable of binding to the extracellular binding domain of the chimeric receptor via a moiety having the mutation, and the extracellular binding domain does not bind to an antibody free of the mutation.

Figure 1

SEQUENCE LISTING

<110>   CHUGAI SEIYAKU KABUSHIKI KAISHA
        YAMAGUCHI UNIVERSITY

<120>   Chimeric receptor recognizing an altered part of antibody

<130>   FA0001-20059

<150>   JP 2019-080566
<151>   2019-04-19

<160>   25

<170>   PatentIn version 3.5

<210>   1
<211>   115
<212>   PRT
<213>   Artificial Sequence

<220>
<223>   VH

<400>   1

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Asp Tyr
            20                  25                  30


Glu Met His Trp Ile Arg Gln Pro Pro Gly Gln Gly Leu Glu Trp Ile
            35                  40                  45


Gly Ala Ile Asp Pro Lys Thr Gly Asp Thr Ala Tyr Ser Gln Lys Phe
        50                  55                  60


Lys Gly Arg Val Thr Leu Thr Ala Asp Lys Ser Thr Ser Thr Ala Tyr
65                  70                  75                  80


Met Glu Leu Ser Ser Leu Thr Ser Glu Asp Thr Ala Val Tyr Tyr Cys
                85                  90                  95


Thr Arg Phe Tyr Ser Tyr Thr Tyr Trp Gly Gln Gly Thr Leu Val Thr
            100                 105                 110


Val Ser Ser
            115


<210>   2
<211>   112
<212>   PRT
<213>   Artificial Sequence

```
<220>
<223>   VL

<400>   2

Asp Ile Val Met Thr Gln Ser Pro Leu Ser Leu Pro Val Thr Pro Gly
1               5                   10                  15


Glu Pro Ala Ser Ile Ser Cys Arg Ser Ser Gln Ser Leu Val His Ser
            20                  25                  30


Asn Arg Asn Thr Tyr Leu His Trp Tyr Gln Gln Lys Pro Gly Gln Ala
        35                  40                  45


Pro Arg Leu Leu Ile Tyr Lys Val Ser Asn Arg Phe Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Val Gly Val Tyr Tyr Cys Ser Gln Asn
                85                  90                  95


Thr His Val Pro Pro Thr Phe Gly Gln Gly Thr Lys Leu Glu Ile Lys
                100                 105                 110


<210>   3
<211>   328
<212>   PRT
<213>   Artificial Sequence

<220>
<223>   CH

<400>   3

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
            35                  40                  45


Gly Val His Thr Phe Pro Ala Val Leu Gln Ser Ser Gly Leu Tyr Ser
        50                  55                  60


Leu Ser Ser Val Val Thr Val Pro Ser Ser Ser Leu Gly Thr Gln Thr
65                  70                  75                  80
```

```
Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Arg Arg Gly Pro Lys Val Phe Leu Phe Pro Pro
            115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
            195                 200                 205

Lys Gly Leu Pro Ser Ser Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
                260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
        290                 295                 300

Val Phe Ser Cys Ser Val Leu His Glu Ala Leu His Ala His Tyr Thr
305                 310                 315                 320

Arg Lys Glu Leu Ser Leu Ser Pro
                325
```

98

```
<210>    4
<211>    107
<212>    PRT
<213>    Artificial Sequence

<220>
<223>    CL

<400>    4

Arg Thr Val Ala Ala Pro Ser Val Phe Ile Phe Pro Pro Ser Asp Glu
1               5                   10                  15


Gln Leu Lys Ser Gly Thr Ala Ser Val Val Cys Leu Leu Asn Asn Phe
            20                  25                  30


Tyr Pro Arg Glu Ala Lys Val Gln Trp Lys Val Asp Asn Ala Leu Gln
        35                  40                  45


Ser Gly Asn Ser Gln Glu Ser Val Thr Glu Gln Asp Ser Lys Asp Ser
    50                  55                  60


Thr Tyr Ser Leu Ser Ser Thr Leu Thr Leu Ser Lys Ala Asp Tyr Glu
65                  70                  75                  80


Lys His Lys Val Tyr Ala Cys Glu Val Thr His Gln Gly Leu Ser Ser
                85                  90                  95


Pro Val Thr Lys Ser Phe Asn Arg Gly Glu Cys
            100                 105


<210>    5
<211>    330
<212>    PRT
<213>    Artificial Sequence

<220>
<223>    CH

<400>    5

Ala Ser Thr Lys Gly Pro Ser Val Phe Pro Leu Ala Pro Ser Ser Lys
1               5                   10                  15


Ser Thr Ser Gly Gly Thr Ala Ala Leu Gly Cys Leu Val Lys Asp Tyr
            20                  25                  30


Phe Pro Glu Pro Val Thr Val Ser Trp Asn Ser Gly Ala Leu Thr Ser
        35                  40                  45


Gly Val His Thr Phe Pro Ala Val Leu Gln Ser Ser Gly Leu Tyr Ser
    50                  55                  60
```

```
Leu Ser Ser Val Val Thr Val Pro Ser Ser Ser Leu Gly Thr Gln Thr
65              70          75              80

Tyr Ile Cys Asn Val Asn His Lys Pro Ser Asn Thr Lys Val Asp Lys
            85              90              95

Arg Val Glu Pro Lys Ser Cys Asp Lys Thr His Thr Cys Pro Pro Cys
        100             105             110

Pro Ala Pro Glu Leu Leu Gly Gly Pro Ser Val Phe Leu Phe Pro Pro
        115             120             125

Lys Pro Lys Asp Thr Leu Met Ile Ser Arg Thr Pro Glu Val Thr Cys
    130             135             140

Val Val Val Asp Val Ser His Glu Asp Pro Glu Val Lys Phe Asn Trp
145             150             155             160

Tyr Val Asp Gly Val Glu Val His Asn Ala Lys Thr Lys Pro Arg Glu
            165             170             175

Glu Gln Tyr Asn Ser Thr Tyr Arg Val Val Ser Val Leu Thr Val Leu
        180             185             190

His Gln Asp Trp Leu Asn Gly Lys Glu Tyr Lys Cys Lys Val Ser Asn
        195             200             205

Lys Ala Leu Pro Ala Pro Ile Glu Lys Thr Ile Ser Lys Ala Lys Gly
    210             215             220

Gln Pro Arg Glu Pro Gln Val Tyr Thr Leu Pro Pro Ser Arg Glu Glu
225             230             235             240

Met Thr Lys Asn Gln Val Ser Leu Thr Cys Leu Val Lys Gly Phe Tyr
            245             250             255

Pro Ser Asp Ile Ala Val Glu Trp Glu Ser Asn Gly Gln Pro Glu Asn
            260             265             270

Asn Tyr Lys Thr Thr Pro Pro Val Leu Asp Ser Asp Gly Ser Phe Phe
        275             280             285

Leu Tyr Ser Lys Leu Thr Val Asp Lys Ser Arg Trp Gln Glu Gly Asn
    290             295             300

Val Phe Ser Cys Ser Val Met His Glu Ala Leu His Asn His Tyr Thr
```

100

305                   310                   315                   320

Gln Lys Ser Leu Ser Leu Ser Pro Gly Lys
                325                   330


<210>  6
<211>  115
<212>  PRT
<213>  Artificial Sequence

<220>
<223>  VH

<400>  6

Gln Ser Val Glu Glu Ser Gly Gly Arg Leu Val Thr Pro Gly Thr Ser
1                   5                   10                  15


Leu Thr Leu Thr Cys Thr Val Ser Gly Ile Asp Leu Ser Ser Tyr Gly
                20                  25                  30


Met Gly Trp Val Arg Gln Ala Pro Gly Met Gly Leu Glu Tyr Ile Gly
            35                  40                  45


Ile Ile Tyr Ala Ala Gly Arg Thr Tyr Tyr Ala Ser Trp Val Lys Gly
        50                  55                  60


Arg Phe Ile Ile Ser Lys Thr Ser Thr Thr Val Asp Leu Glu Met Thr
65                  70                  75                  80


Ser Leu Thr Thr Glu Asp Thr Ala Thr Tyr Phe Cys Ala Arg His Gly
                85                  90                  95


Ser Trp Tyr Ala Gly Met Asp Leu Trp Gly Pro Gly Thr Leu Val Thr
            100                 105                 110


Val Ser Ser
            115


<210>  7
<211>  110
<212>  PRT
<213>  Artificial Sequence

<220>
<223>  VL

<400>  7

Ala Ile Glu Met Thr Gln Thr Pro Ser Pro Val Ser Ala Ala Val Gly
1                   5                   10                  15

Gly Thr Val Ser Ile Asn Cys Gln Ala Ser Glu Asp Ile Glu Asn Tyr
20                      25                      30

Phe Ala Trp Tyr Gln Gln Lys Pro Gly Gln Pro Pro Lys Leu Leu Ile
35                      40                      45

Tyr Asp Ala Ser Glu Leu Ala Ser Gly Val Pro Ser Arg Phe Ser Gly
50                      55                      60

Ser Gly Ser Gly Thr Asp Phe Thr Leu Thr Ile Ser Gly Val Gln Ser
65                      70                      75                      80

Asp Asp Ala Ala Thr Tyr Tyr Cys Gln His Ala Asp Tyr Ala Ala Ser
85                      90                      95

Ser Glu Asn Thr Phe Gly Gly Gly Thr Glu Val Val Val Lys
100                     105                     110


<210>  8
<211>  122
<212>  PRT
<213>  Artificial Sequence

<220>
<223>  VH

<400>  8

Gln Val Gln Leu Val Glu Ser Gly Gly Gly Val Val Gln Pro Gly Gly
1                   5                   10                      15

Ser Leu Arg Leu Ser Cys Ala Ala Ser Gly Phe Thr Phe Ser Asn Ala
20                      25                      30

Trp Met His Trp Val Arg Gln Ala Pro Gly Lys Gly Leu Glu Trp Val
35                      40                      45

Ala Gln Ile Lys Asp Lys Ser Gln Asn Tyr Ala Thr Tyr Val Ala Glu
50                      55                      60

Ser Val Lys Gly Arg Phe Thr Ile Ser Arg Ala Asp Ser Lys Asn Ser
65                      70                      75                      80

Ile Tyr Leu Gln Met Asn Ser Leu Lys Thr Glu Asp Thr Ala Val Tyr
85                      90                      95

Tyr Cys Arg Tyr Val His Tyr Ala Ala Gly Tyr Gly Val Asp Ile Trp
100                     105                     110

Gly Gln Gly Thr Thr Val Thr Val Ser Ser

115                          120

<210>   9
<211>   112
<212>   PRT
<213>   Artificial Sequence

<220>
<223>   VL

<400>   9

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Pro Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Gly Gln Gly
            85                  90                  95

Thr Gln Val Pro Tyr Thr Phe Gly Gln Gly Thr Lys Leu Glu Ile Lys
            100                 105                 110

<210>   10
<211>   114
<212>   PRT
<213>   Artificial Sequence

<220>
<223>   VH

<400>   10

Gln Ser Leu Glu Glu Ser Gly Gly Arg Leu Val Thr Pro Gly Gly Ser
1               5                   10                  15

Leu Thr Leu Thr Cys Thr Val Ser Gly Ile Asp Leu Ser Asn Tyr Ala
            20                  25                  30

Val Gly Trp Val Arg Gln Ala Pro Gly Lys Gly Leu Glu Tyr Ile Gly
        35                  40                  45

103

```
        Ile Ile Tyr Ala Ser Gly Ser Ala Tyr Tyr Ala Ser Trp Ala Lys Gly
            50                  55                  60

        Arg Phe Thr Ile Ser Lys Thr Ser Ser Thr Thr Val Asp Leu Lys Val
        65                  70                  75                  80

        Thr Ser Leu Thr Thr Glu Asp Thr Ala Thr Tyr Phe Cys Ala Arg Gly
                            85                  90                  95

        Tyr Gly Arg Ala Phe Arg Ile Trp Gly Pro Gly Thr Leu Val Thr Val
                        100                 105                 110

        Ser Ser


        <210>  11
        <211>  109
        <212>  PRT
        <213>  Artificial Sequence

        <220>
        <223>  VL

        <400>  11

        Ala Val Leu Thr Gln Thr Pro Ser Pro Val Ser Ala Ala Val Gly Gly
        1               5                   10                  15

        Thr Val Thr Ile Ser Cys Gln Ala Ser Gln Ser Val Tyr Asn Asn Asn
                        20                  25                  30

        Phe Leu Ser Trp Tyr Gln Gln Lys Pro Gly Gln Arg Pro Lys Leu Leu
                    35                  40                  45

        Ile Tyr Asp Ala Ser Thr Leu Glu Ser Gly Val Pro Ser Arg Phe Ser
                50                  55                  60

        Gly Ser Gly Ser Gly Thr Gln Phe Thr Leu Thr Ile Ser Ser Val Gln
        65                  70                  75                  80

        Cys Asp Asp Ala Ala Thr Tyr Tyr Cys Leu Gly Gly Tyr Asp Asp His
                            85                  90                  95

        Asp Asn Ala Phe Gly Gly Gly Thr Glu Val Val Val Lys
                        100                 105


        <210>  12
        <211>  328
        <212>  PRT
        <213>  Artificial Sequence
```

<220>
<223>  CH

<400>  12

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Arg Gly Gly Pro Lys Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
            245                      250                      255

Pro Ser Asp Ile Ala Val Glu Trp Glu Ser Asn Gly Gln Pro Glu Asn
            260                      265                      270

Asn Tyr Lys Thr Thr Pro Pro Tyr Leu Asp Ser Asp Gly Ser Phe Phe
        275                    280                  285

Leu Tyr Ser Lys Leu Thr Val Asp Lys Ser Arg Trp Gln Gln Gly Asn
    290                295                300

Val Phe Ser Cys Ser Val Met His Glu Ala Leu His Asn His Tyr Thr
305                  310              315              320

Gln Glu Ser Leu Ser Leu Ser Pro
            325

<210> 13
<211> 330
<212> PRT
<213> Artificial Sequence

<220>
<223> CH

<400> 13

Ala Ser Thr Lys Gly Pro Ser Val Phe Pro Leu Ala Pro Ser Ser Lys
1                5                    10                  15

Ser Thr Ser Gly Gly Thr Ala Ala Leu Gly Cys Leu Val Lys Asp Tyr
        20                    25                  30

Phe Pro Glu Pro Val Thr Val Ser Trp Asn Ser Gly Ala Leu Thr Ser
        35                    40                  45

Gly Val His Thr Phe Pro Ala Val Leu Gln Ser Ser Gly Leu Tyr Ser
    50                55                60

Leu Ser Ser Val Val Thr Val Pro Ser Ser Ser Leu Gly Thr Gln Thr
65                70              75              80

Tyr Ile Cys Asn Val Asn His Lys Pro Ser Asn Thr Lys Val Asp Lys
            85                90              95

Lys Val Glu Pro Lys Ser Cys Asp Lys Thr His Thr Cys Pro Pro Cys
        100                105              110

```
Pro Ala Pro Glu Leu Arg Gly Gly Pro Lys Val Phe Leu Phe Pro Pro
        115                 120             125

Lys Pro Lys Asp Thr Leu Met Ile Ser Arg Thr Pro Glu Val Thr Cys
        130                 135             140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
            195                 200                 205

Lys Ala Leu Pro Ala Pro Ile Glu Lys Thr Ile Ser Lys Ala Lys Gly
        210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Tyr Leu Asp Ser Asp Gly Ser Phe Phe
            275                 280                 285

Leu Tyr Ser Lys Leu Thr Val Asp Lys Ser Arg Trp Gln Gln Gly Asn
        290                 295                 300

Val Phe Ser Cys Ser Val Met His Glu Ala Leu His Asn His Tyr Thr
305                 310                 315                 320

Gln Glu Ser Leu Ser Leu Ser Pro Gly Lys
                325                 330
```

```
<210>  14
<211>  328
<212>  PRT
<213>  Artificial Sequence

<220>
```

<223> CH

<400> 14

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
            35                  40                  45

Gly Val His Thr Phe Pro Ala Val Leu Gln Ser Ser Gly Leu Tyr Ser
            50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Arg Gly Gly Pro Lys Val Phe Leu Phe Pro Pro
            115                 120                 125

Lys Pro Lys Asp Thr Leu Met Ile Ser Arg Thr Pro Glu Val Thr Cys
            130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Ala Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
            195                 200                 205

Lys Ala Leu Pro Ala Pro Ile Glu Lys Thr Ile Ser Lys Ala Lys Gly
            210                 215                 220

Gln Pro Arg Glu Pro Gln Val Tyr Thr Leu Pro Pro Ser Arg Lys Glu
225                 230                 235                 240

```
Met Thr Lys Asn Gln Val Ser Leu Thr Cys Leu Val Lys Gly Phe Tyr
              245                 250                 255

Pro Ser Asp Ile Ala Val Glu Trp Glu Ser Asn Gly Gln Pro Glu Asn
              260                 265                 270

Asn Tyr Lys Thr Thr Pro Pro Tyr Leu Asp Ser Asp Gly Ser Phe Phe
          275                 280                 285

Leu Tyr Ser Lys Leu Thr Val Asp Lys Ser Arg Trp Gln Gln Gly Asn
      290                 295                 300

Val Phe Ser Cys Ser Val Met His Glu Ala Leu His Asn His Tyr Thr
305                 310                 315                 320

Gln Lys Ser Leu Ser Leu Ser Pro
              325
```

```
<210>  15
<211>  330
<212>  PRT
<213>  Artificial Sequence

<220>
<223>  CH

<400>  15
```

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
          20                  25                  30

Phe Pro Glu Pro Val Thr Val Ser Trp Asn Ser Gly Ala Leu Thr Ser
          35                  40                  45

Gly Val His Thr Phe Pro Ala Val Leu Gln Ser Ser Gly Leu Tyr Ser
      50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
              85                  90                  95

Lys Val Glu Pro Lys Ser Cys Asp Lys Thr His Thr Cys Pro Pro Cys
              100                 105                 110
```

```
Pro Ala Pro Glu Leu Arg Gly Gly Pro Lys Val Phe Leu Phe Pro Pro
        115                 120             125

Lys Pro Lys Asp Thr Leu Met Ile Ser Arg Thr Pro Glu Val Thr Cys
        130                 135             140

Val Val Val Asp Val Ser His Glu Asp Pro Glu Val Lys Phe Asn Trp
145                 150             155                 160

Tyr Val Asp Gly Val Glu Val His Asn Ala Lys Thr Lys Pro Arg Glu
                165                 170             175

Glu Gln Tyr Ala Ser Thr Tyr Arg Val Val Ser Val Leu Thr Val Leu
            180                 185             190

His Gln Asp Trp Leu Asn Gly Lys Glu Tyr Lys Cys Lys Val Ser Asn
            195                 200             205

Lys Ala Leu Pro Ala Pro Ile Glu Lys Thr Ile Ser Lys Ala Lys Gly
        210                 215             220

Gln Pro Arg Glu Pro Gln Val Tyr Thr Leu Pro Pro Ser Arg Lys Glu
225                 230             235                 240

Met Thr Lys Asn Gln Val Ser Leu Thr Cys Leu Val Lys Gly Phe Tyr
                245                 250             255

Pro Ser Asp Ile Ala Val Glu Trp Glu Ser Asn Gly Gln Pro Glu Asn
            260                 265             270

Asn Tyr Lys Thr Thr Pro Pro Tyr Leu Asp Ser Asp Gly Ser Phe Phe
        275                 280             285

Leu Tyr Ser Lys Leu Thr Val Asp Lys Ser Arg Trp Gln Gln Gly Asn
    290                 295             300

Val Phe Ser Cys Ser Val Met His Glu Ala Leu His Asn His Tyr Thr
305                 310             315                 320

Gln Lys Ser Leu Ser Leu Ser Pro Gly Lys
                325                 330
```

```
<210>   16
<211>   545
<212>   PRT
<213>   Homo sapiens

<400>   16
```

```
Gln Pro Pro Pro Pro Pro Pro Asp Ala Thr Cys His Gln Val Arg Ser
1               5               10              15

Phe Phe Gln Arg Leu Gln Pro Gly Leu Lys Trp Val Pro Glu Thr Pro
            20              25              30

Val Pro Gly Ser Asp Leu Gln Val Cys Leu Pro Lys Gly Pro Thr Cys
        35              40              45

Cys Ser Arg Lys Met Glu Glu Lys Tyr Gln Leu Thr Ala Arg Leu Asn
    50              55              60

Met Glu Gln Leu Leu Gln Ser Ala Ser Met Glu Leu Lys Phe Leu Ile
65              70              75              80

Ile Gln Asn Ala Ala Val Phe Gln Glu Ala Phe Glu Ile Phe Val Arg
            85              90              95

His Ala Lys Asn Tyr Thr Asn Ala Met Phe Lys Asn Asn Tyr Pro Ser
        100             105             110

Leu Thr Pro Gln Ala Phe Glu Phe Val Gly Glu Phe Phe Thr Asp Val
        115             120             125

Ser Leu Tyr Ile Leu Gly Ser Asp Ile Asn Val Asp Asp Met Val Asn
130             135             140

Glu Leu Phe Asp Ser Leu Phe Pro Val Ile Tyr Thr Gln Leu Met Asn
145             150             155             160

Pro Gly Leu Pro Asp Ser Ala Leu Asp Ile Asn Glu Cys Leu Arg Gly
            165             170             175

Ala Arg Arg Asp Leu Lys Val Phe Gly Asn Phe Pro Lys Leu Ile Met
            180             185             190

Thr Gln Val Ser Lys Ser Leu Gln Val Thr Arg Ile Phe Leu Gln Ala
        195             200             205

Leu Asn Leu Gly Ile Glu Val Ile Asn Thr Thr Asp His Leu Lys Phe
        210             215             220

Ser Lys Asp Cys Gly Arg Met Leu Thr Arg Met Trp Tyr Cys Ser Tyr
225             230             235             240

Cys Gln Gly Leu Met Met Val Lys Pro Cys Gly Gly Tyr Cys Asn Val
            245             250             255
```

Val Met Gln Gly Cys Met Ala Gly Val Val Glu Ile Asp Lys Tyr Trp
              260                   265                   270

Arg Glu Tyr Ile Leu Ser Leu Glu Glu Leu Val Asn Gly Met Tyr Arg
              275                   280                   285

Ile Tyr Asp Met Glu Asn Val Leu Leu Gly Leu Phe Ser Thr Ile His
              290                   295                   300

Asp Ser Ile Gln Tyr Val Gln Lys Asn Ala Gly Lys Leu Thr Thr Thr
305                   310                   315                   320

Ile Gly Lys Leu Cys Ala His Ser Gln Gln Arg Gln Tyr Arg Ser Ala
              325                   330                   335

Tyr Tyr Pro Glu Asp Leu Phe Ile Asp Lys Lys Val Leu Lys Val Ala
              340                   345                   350

His Val Glu His Glu Glu Thr Leu Ser Ser Arg Arg Arg Glu Leu Ile
              355                   360                   365

Gln Lys Leu Lys Ser Phe Ile Ser Phe Tyr Ser Ala Leu Pro Gly Tyr
              370                   375                   380

Ile Cys Ser His Ser Pro Val Ala Glu Asn Asp Thr Leu Cys Trp Asn
385                   390                   395                   400

Gly Gln Glu Leu Val Glu Arg Tyr Ser Gln Lys Ala Ala Arg Asn Gly
              405                   410                   415

Met Lys Asn Gln Phe Asn Leu His Glu Leu Lys Met Lys Gly Pro Glu
              420                   425                   430

Pro Val Val Ser Gln Ile Ile Asp Lys Leu Lys His Ile Asn Gln Leu
              435                   440                   445

Leu Arg Thr Met Ser Met Pro Thr Gly Arg Val Leu Asp Lys Asn Leu
              450                   455                   460

Asp Glu Glu Gly Phe Glu Ala Gly Asp Cys Gly Asp Asp Glu Asp Glu
465                   470                   475                   480

Cys Ile Gly Gly Ala Gly Asp Gly Met Ile Lys Val Lys Asn Gln Leu
              485                   490                   495

Arg Phe Leu Ala Glu Leu Ala Tyr Asp Leu Asp Val Asp Asp Ala Pro
              500                   505                   510

```
Gly Asn Ser Gln Gln Ala Thr Pro Lys Asp Asn Glu Ile Ser Thr Phe
        515             520             525

His Asn Leu Gly Asn Val His Ser Pro Leu Lys His His His His His
        530             535             540

His
545


<210>  17
<211>  822
<212>  PRT
<213>  Artificial Sequence

<220>
<223>  Anti-CH2 Chimeric Receptor Construct

<400>  17

Met Asp Trp Thr Trp Arg Ile Leu Phe Leu Val Ala Ala Ala Thr Gly
1               5               10              15

Ala His Ser Ala Ile Glu Met Thr Gln Thr Pro Ser Pro Val Ser Ala
        20              25              30

Ala Val Gly Gly Thr Val Ser Ile Asn Cys Gln Ala Ser Glu Asp Ile
        35              40              45

Glu Asn Tyr Phe Ala Trp Tyr Gln Gln Lys Pro Gly Gln Pro Pro Lys
    50              55              60

Leu Leu Ile Tyr Asp Ala Ser Glu Leu Ala Ser Gly Val Pro Ser Arg
65              70              75              80

Phe Ser Gly Ser Gly Ser Gly Thr Asp Phe Thr Leu Thr Ile Ser Gly
            85              90              95

Val Gln Ser Asp Asp Ala Ala Thr Tyr Tyr Cys Gln His Ala Asp Tyr
        100             105             110

Ala Ala Ser Ser Glu Asn Thr Phe Gly Gly Gly Thr Glu Val Val Val
        115             120             125

Lys Ser Ser Ala Asp Asp Ala Lys Lys Asp Ala Ala Lys Lys Asp Asp
        130             135             140

Ala Lys Lys Asp Asp Ala Lys Lys Asp Gly Gln Ser Val Glu Glu Ser
145             150             155             160
```

```
Gly Gly Arg Leu Val Thr Pro Gly Thr Ser Leu Thr Leu Thr Cys Thr
                165             170             175

Val Ser Gly Ile Asp Leu Ser Ser Tyr Gly Met Gly Trp Val Arg Gln
                180             185             190

Ala Pro Gly Met Gly Leu Glu Tyr Ile Gly Ile Ile Tyr Ala Ala Gly
                195             200             205

Arg Thr Tyr Tyr Ala Ser Trp Val Lys Gly Arg Phe Ile Ile Ser Lys
    210             215             220

Thr Ser Thr Thr Val Asp Leu Glu Met Thr Ser Leu Thr Thr Glu Asp
225             230             235             240

Thr Ala Thr Tyr Phe Cys Ala Arg His Gly Ser Trp Tyr Ala Gly Met
                245             250             255

Asp Leu Trp Gly Pro Gly Thr Leu Val Thr Val Ser Ser Ala Ala Ala
                260             265             270

Phe Val Pro Val Phe Leu Pro Ala Lys Pro Thr Thr Thr Pro Ala Pro
                275             280             285

Arg Pro Pro Thr Pro Ala Pro Thr Ile Ala Ser Gln Pro Leu Ser Leu
    290             295             300

Arg Pro Glu Ala Cys Arg Pro Ala Ala Gly Gly Ala Val His Thr Arg
305             310             315             320

Gly Leu Asp Phe Ala Cys Asp Ile Tyr Ile Trp Ala Pro Leu Ala Gly
                325             330             335

Thr Cys Gly Val Leu Leu Leu Ser Leu Val Ile Thr Leu Tyr Cys Asn
                340             345             350

His Arg Asn Arg Ser Lys Arg Ser Arg Leu Leu His Ser Asp Tyr Met
                355             360             365

Asn Met Thr Pro Arg Arg Pro Gly Pro Thr Arg Lys His Tyr Gln Pro
    370             375             380

Tyr Ala Pro Pro Arg Asp Phe Ala Ala Tyr Arg Ser Arg Phe Ser Val
385             390             395             400

Val Lys Arg Gly Arg Lys Lys Leu Leu Tyr Ile Phe Lys Gln Pro Phe
                405             410             415
```

```
Met Arg Pro Val Gln Thr Thr Gln Glu Glu Asp Gly Cys Ser Cys Arg
        420             425         430

Phe Pro Glu Glu Glu Glu Gly Gly Cys Glu Leu Arg Val Lys Phe Ser
        435             440         445

Arg Ser Ala Asp Ala Pro Ala Tyr Gln Gln Gly Gln Asn Gln Leu Tyr
        450             455         460

Asn Glu Leu Asn Leu Gly Arg Arg Glu Glu Tyr Asp Val Leu Asp Lys
465             470         475             480

Arg Arg Gly Arg Asp Pro Glu Met Gly Gly Lys Pro Arg Arg Lys Asn
            485         490             495

Pro Gln Glu Gly Leu Tyr Asn Glu Leu Gln Lys Asp Lys Met Ala Glu
        500             505         510

Ala Tyr Ser Glu Ile Gly Met Lys Gly Glu Arg Arg Arg Gly Lys Gly
        515             520         525

His Asp Gly Leu Tyr Gln Gly Leu Ser Thr Ala Thr Lys Asp Thr Tyr
        530             535         540

Asp Ala Leu His Met Gln Ala Leu Pro Pro Arg Glu Phe Gly Ser Gly
545             550         555             560

Val Lys Gln Thr Leu Asn Phe Asp Leu Leu Lys Leu Ala Gly Asp Val
            565         570             575

Glu Ser Asn Pro Gly Pro Cys Met Val Ser Lys Gly Glu Glu Leu Phe
        580             585         590

Thr Gly Val Val Pro Ile Leu Val Glu Leu Asp Gly Asp Val Asn Gly
        595             600         605

His Lys Phe Ser Val Ser Gly Glu Gly Glu Gly Asp Ala Thr Tyr Gly
        610             615         620

Lys Leu Thr Leu Lys Phe Ile Cys Thr Thr Gly Lys Leu Pro Val Pro
625             630             635             640

Trp Pro Thr Leu Val Thr Thr Leu Thr Tyr Gly Val Gln Cys Phe Ser
            645             650         655

Arg Tyr Pro Asp His Met Lys Gln His Asp Phe Phe Lys Ser Ala Met
```

115

```
                   660                      665                          670
```

Pro Glu Gly Tyr Val Gln Glu Arg Thr Ile Phe Phe Lys Asp Asp Gly
        675                 680                 685

Asn Tyr Lys Thr Arg Ala Glu Val Lys Phe Glu Gly Asp Thr Leu Val
    690                 695                 700

Asn Arg Ile Glu Leu Lys Gly Ile Asp Phe Lys Glu Asp Gly Asn Ile
705                 710                 715                 720

Leu Gly His Lys Leu Glu Tyr Asn Tyr Asn Ser His Asn Val Tyr Ile
                725                 730                 735

Met Ala Asp Lys Gln Lys Asn Gly Ile Lys Val Asn Phe Lys Ile Arg
        740                 745                 750

His Asn Ile Glu Asp Gly Ser Val Gln Leu Ala Asp His Tyr Gln Gln
    755                 760                 765

Asn Thr Pro Ile Gly Asp Gly Pro Val Leu Leu Pro Asp Asn His Tyr
    770                 775                 780

Leu Ser Thr Gln Ser Ala Leu Ser Lys Asp Pro Asn Glu Lys Arg Asp
785                 790                 795                 800

His Met Val Leu Leu Glu Phe Val Thr Ala Ala Gly Ile Thr Leu Gly
                805                 810                 815

Met Asp Glu Leu Tyr Lys
                820

```
<210>  18
<211>  19
<212>  PRT
<213>  Artificial Sequence

<220>
<223>  Leader Sequence Domain

<400>  18
```

Met Asp Trp Thr Trp Arg Ile Leu Phe Leu Val Ala Ala Ala Thr Gly
1                   5                   10                  15

Ala His Ser

```
<210>  19
<211>  110
```

```
<212>   PRT
<213>   Artificial Sequence

<220>
<223>   Anti CH2 scFv VL Domain

<400>   19

Ala Ile Glu Met Thr Gln Thr Pro Ser Pro Val Ser Ala Ala Val Gly
1               5               10              15

Gly Thr Val Ser Ile Asn Cys Gln Ala Ser Glu Asp Ile Glu Asn Tyr
            20              25              30

Phe Ala Trp Tyr Gln Gln Lys Pro Gly Gln Pro Pro Lys Leu Leu Ile
        35              40              45

Tyr Asp Ala Ser Glu Leu Ala Ser Gly Val Pro Ser Arg Phe Ser Gly
    50              55              60

Ser Gly Ser Gly Thr Asp Phe Thr Leu Thr Ile Ser Gly Val Gln Ser
65              70              75              80

Asp Asp Ala Ala Thr Tyr Tyr Cys Gln His Ala Asp Tyr Ala Ala Ser
            85              90              95

Ser Glu Asn Thr Phe Gly Gly Gly Thr Glu Val Val Val Lys
            100             105             110


<210>   20
<211>   83
<212>   PRT
<213>   Artificial Sequence

<220>
<223>   CD8 Domain

<400>   20

Phe Val Pro Val Phe Leu Pro Ala Lys Pro Thr Thr Thr Pro Ala Pro
1               5               10              15

Arg Pro Pro Thr Pro Ala Pro Thr Ile Ala Ser Gln Pro Leu Ser Leu
            20              25              30

Arg Pro Glu Ala Cys Arg Pro Ala Ala Gly Gly Ala Val His Thr Arg
            35              40              45

Gly Leu Asp Phe Ala Cys Asp Ile Tyr Ile Trp Ala Pro Leu Ala Gly
        50              55              60

Thr Cys Gly Val Leu Leu Leu Ser Leu Val Ile Thr Leu Tyr Cys Asn
```

65                              70                              75                              80


His Arg Asn


<210>    21
<211>    41
<212>    PRT
<213>    Artificial Sequence

<220>
<223>    CD28 Domain

<400>    21

Arg Ser Lys Arg Ser Arg Leu Leu His Ser Asp Tyr Met Asn Met Thr
1                   5                   10                  15


Pro Arg Arg Pro Gly Pro Thr Arg Lys His Tyr Gln Pro Tyr Ala Pro
            20                  25                  30


Pro Arg Asp Phe Ala Ala Tyr Arg Ser
            35                  40


<210>    22
<211>    47
<212>    PRT
<213>    Artificial Sequence

<220>
<223>    4-1BB Domain

<400>    22

Arg Phe Ser Val Val Lys Arg Gly Arg Lys Lys Leu Leu Tyr Ile Phe
1                   5                   10                  15


Lys Gln Pro Phe Met Arg Pro Val Gln Thr Thr Gln Glu Glu Asp Gly
            20                  25                  30


Cys Ser Cys Arg Phe Pro Glu Glu Glu Glu Gly Gly Cys Glu Leu
            35                  40                  45


<210>    23
<211>    112
<212>    PRT
<213>    Artificial Sequence

<220>
<223>    CD3 zeta Domain

<400>    23

Arg Val Lys Phe Ser Arg Ser Ala Asp Ala Pro Ala Tyr Gln Gln Gly
1                   5                   10                  15

```
Gln Asn Gln Leu Tyr Asn Glu Leu Asn Leu Gly Arg Arg Glu Glu Tyr
            20                  25                  30
```

```
Asp Val Leu Asp Lys Arg Arg Gly Arg Asp Pro Glu Met Gly Gly Lys
            35                  40                  45
```

```
Pro Arg Arg Lys Asn Pro Gln Glu Gly Leu Tyr Asn Glu Leu Gln Lys
        50                  55                  60
```

```
Asp Lys Met Ala Glu Ala Tyr Ser Glu Ile Gly Met Lys Gly Glu Arg
65                  70                  75                  80
```

```
Arg Arg Gly Lys Gly His Asp Gly Leu Tyr Gln Gly Leu Ser Thr Ala
                85                  90                  95
```

```
Thr Lys Asp Thr Tyr Asp Ala Leu His Met Gln Ala Leu Pro Pro Arg
                100                 105                 110
```

```
<210>  24
<211>  22
<212>  PRT
<213>  Artificial Sequence

<220>
<223>  F2A Domain

<400>  24
```

```
Val Lys Gln Thr Leu Asn Phe Asp Leu Leu Lys Leu Ala Gly Asp Val
1               5                   10                  15
```

```
Glu Ser Asn Pro Gly Pro
                20
```

```
<210>  25
<211>  239
<212>  PRT
<213>  Artificial Sequence

<220>
<223>  eGFP Domain

<400>  25
```

```
Met Val Ser Lys Gly Glu Glu Leu Phe Thr Gly Val Val Pro Ile Leu
1               5                   10                  15
```

```
Val Glu Leu Asp Gly Asp Val Asn Gly His Lys Phe Ser Val Ser Gly
                20                  25                  30
```

```
Glu Gly Glu Gly Asp Ala Thr Tyr Gly Lys Leu Thr Leu Lys Phe Ile
```

```
                35                      40                      45

        Cys Thr Thr Gly Lys Leu Pro Val Pro Trp Pro Thr Leu Val Thr Thr
            50                  55                  60

        Leu Thr Tyr Gly Val Gln Cys Phe Ser Arg Tyr Pro Asp His Met Lys
        65                  70                  75                  80

        Gln His Asp Phe Phe Lys Ser Ala Met Pro Glu Gly Tyr Val Gln Glu
                        85                  90                  95

        Arg Thr Ile Phe Phe Lys Asp Asp Gly Asn Tyr Lys Thr Arg Ala Glu
                        100                 105                 110

        Val Lys Phe Glu Gly Asp Thr Leu Val Asn Arg Ile Glu Leu Lys Gly
                115                 120                 125

        Ile Asp Phe Lys Glu Asp Gly Asn Ile Leu Gly His Lys Leu Glu Tyr
            130                 135                 140

        Asn Tyr Asn Ser His Asn Val Tyr Ile Met Ala Asp Lys Gln Lys Asn
        145                 150                 155                 160

        Gly Ile Lys Val Asn Phe Lys Ile Arg His Asn Ile Glu Asp Gly Ser
                        165                 170                 175

        Val Gln Leu Ala Asp His Tyr Gln Gln Asn Thr Pro Ile Gly Asp Gly
                        180                 185                 190

        Pro Val Leu Leu Pro Asp Asn His Tyr Leu Ser Thr Gln Ser Ala Leu
                195                 200                 205

        Ser Lys Asp Pro Asn Glu Lys Arg Asp His Met Val Leu Leu Glu Phe
            210                 215                 220

        Val Thr Ala Ala Gly Ile Thr Leu Gly Met Asp Glu Leu Tyr Lys
        225                 230                 235
```

**Description**

TECHNICAL FIELD

The present disclosure relates to a chimeric receptor, a cell expressing a chimeric receptor, and a method for treating a disease using the cell, particularly, adoptive cell immunotherapy, adoptive T cell immunotherapy, or CAR-T therapy using the cell, and a T cell-redirecting antibody.

BACKGROUND ART

Chimeric antigen receptors (hereinafter, also referred to as "CARs") are chimeric proteins prepared by artificially fusing an antibody that recognizes a cell surface antigen of cancer cells or the like with a signaling region that induces the activation of T cells. CAR-expressing T cells (hereinafter, also simply referred to as "CAR-T cells") are prepared by introducing a gene encoding CAR to normal peripheral blood T cells (peripheral blood T lymphocyte) having no antigen reactivity. The CAR-expressing T cells prepared by such an approach are used in the treatment of diseases such as cancers by adoptive immunotherapy. These CAR-T cells have reactivity with target cells expressing the antigen and become capable of damaging the target cells without depending on interaction with major histocompatibility complex (MHC).

Clinical trials are ongoing worldwide on cancer immunotherapy involving the administration of CAR-T cells, more specifically, therapy which involves collecting T cells from patients, and introducing a gene encoding CAR to these T cells, which are then cultured and expanded, and transferred to the patients again (Non Patent Literature 1). The cancer immunotherapy involving the administration of CAR-T cells has obtained results indicating efficacy on, for example, hematopoietic malignant tumor such as leukemia or lymphoma. In 2017, Kymriah(R) (Novartis International AG, tisagenlecleucel, CTL-019, CD3 zeta-CD137) and Yescarta(R) (KiTE, axicabtagene ciloleucel, CD3 zeta-CD28), which are CAR-T against CD 19 as an antigen, were approved as drugs in the USA.

The establishment of techniques for preparing universal T cells that recognize antigens that can be changed has a significant clinical relevance in making treatment methods using T cells widely available. Some reports have been made on such research (Patent Literatures 1 to 6 and Non Patent Literatures 2 to 5).

Also, antibodies, each molecule of which binds to two or more types of antigens (bispecific antibodies), have been studied as molecules that bind to a plurality of targets. The modification of a natural IgG antibody is capable of conferring binding activity against two different antigens (first antigen and second antigen) (Non Patent Literature 5). Hence, in addition to the effect of each molecule binding to two or more types of antigens, antitumor activity is enhanced by cross-linking a cell having cytotoxic activity to a cancer cell.

T cell-redirecting antibodies, which are antibodies having an antitumor effect based on a cytotoxic mechanism through which T cells are recruited as effector cells, have been known as one of the bispecific antibodies since 1980s (Patent Literature 7 and Non Patent Literatures 6, 7 and 8). Unlike antibodies having an antitumor effect based on an ADCC mechanism through which NK cells or macrophages are recruited as effector cells, the T cell-redirecting antibodies are bispecific antibodies comprising a binding domain for any constituent subunit of a T cell receptor (TCR) complex on T cells, particularly, a domain that binds to a CD3 epsilon chain, and a domain that binds to an antigen on targeted cancer cells. The T cell-redirecting antibody binds to the CD3 epsilon chain and the tumor antigen at the same time so that the T cells approach the cancer cells. As a result, the cytotoxicity effect of the T cells exerts an antitumor effect on the cancer cells.

The preparation of antibodies highly selective for target tissues has also been reported from the viewpoint of the alleviation of adverse reactions, etc. (Patent Literature 8).

CITATION LIST

PATENT LITERATURE

[Patent Literature 1] WO2012/082841
[Patent Literature 2] WO2015/058081
[Patent Literature 3] WO2016/040441
[Patent Literature 4] WO2017/161333
[Patent Literature 5] WO2018/177966
[Patent Literature 6] WO2018/189611
[Patent Literature 7] WO2012/073985

[Patent Literature 8] WO2013/180200

NON PATENT LITERATURE

[Non Patent Literature 1] Grupp et al. 2013 N Engl J Med 368(16): 1509- 18
[Non Patent Literature 2] Maude et al. 2014 2014 N Engl J Med 371(16): 1507-17
[Non Patent Literature 3] Kim et al. J Am Chem Soc 2015; 137: 2832-2835
[Non Patent Literature 4] Tamada et al. Clin Cancer Res 2012; 18(23): 6436-6445
[Non Patent Literature 5] Kontermann, mAbs 2012; 4: 182-197.
[Non Patent Literature 6] Mezzanzanica et al., International journal of cancer 1988; 41: 609-615.
[Non Patent Literature 7] Staerz and Bevan, Proceedings of the National Academy of Sciences of the United States of America 1986; 83: 1453-1457.
[Non Patent Literature 8] Staerz et al., Nature 1985; 314: 628-631.

SUMMARY OF INVENTION

TECHNICAL PROBLEM

In spite of therapy using CAR-T cells regarded as promising treatment of cancer patients, there are some limitations on the expansion of the clinical application of the CAR-T cells. First of all, a single tumor antigen is not universally expressed in every cancer. Therefore, the antigen recognition site of CAR needs to be constructed for each targeted tumor antigen. Secondly, economical cost and labor associated with operations for identifying antigen recognition sites for various tumor antigens and newly establishing CAR-T cells harboring these sites are major problems. Thirdly, tumor antigens targeted by CAR may cause tumor immune escape in such a way that treatment decreases their expression levels or mutates the tumor antigens. Particularly, existing CAR-T cells recognize only one target antigen. Therefore, the tumor immune escape causes reduction or disappearance of therapeutic effects.

When a tumor antigen for a T cell-redirecting antibody is expressed in normal tissues, the normal tissues are damaged, inducing strong adverse reactions. Therefore, the exertion of activity selective only for target tissues is important.

Although research has been made on CAR-T cells and T cell-redirecting antibodies that recognize tumor antigens that can be changed according to the phase of treatment, and treatment methods using them, inexpensive treatment has been needed to be realized by the development of treatment methods and generally-applicable techniques having sufficient therapeutic effects upon administration to patients and having high safety.

SOLUTION TO PROBLEM

The inventors have conducted studies to solve the technical problems described above and consequently completed the present disclosure by finding that a CAR-T cell or a T cell-redirecting antibody using a chimeric receptor that recognizes an engineered site in an antibody is effective for treatment. One aspect of the present disclosure provides the following invention.

[1] A pharmaceutical composition for use in combination with administration of a mutated antibody having a mutation, including substitution, deletion, addition or modification, of at least one amino acid in a CH1 region, a CH2 region, a CH3 region, a CL region, or a framework region, wherein

the pharmaceutical composition comprises a cell expressing a chimeric receptor,
the chimeric receptor comprises an extracellular binding domain, a transmembrane domain and an intracellular signaling domain,
the mutated antibody is capable of binding to the extracellular binding domain of the chimeric receptor via a moiety having the mutation, and
the extracellular binding domain does not specifically bind to an antibody free of the mutation.

[2] A pharmaceutical composition for use in combination with administration of a cell expressing a chimeric receptor, wherein

the pharmaceutical composition comprises a mutated antibody having a mutation, including substitution, deletion, addition or modification, of at least one amino acid in a CH1 region, a CH2 region, a CH3 region, a CL region, or a framework region,

the chimeric receptor comprises an extracellular binding domain, a transmembrane domain and an intracellular signaling domain,

the mutated antibody is capable of binding to the extracellular binding domain of the chimeric receptor via a moiety having the mutation, and

the extracellular binding domain does not specifically bind to an antibody free of the mutation.

[3] A pharmaceutical composition for use in combination with administration of a mutated antibody having a mutation, including substitution, deletion, addition or modification, of at least one amino acid in a CH1 region, a CH2 region, a CH3 region, a CL region, or a framework region, wherein

the pharmaceutical composition comprises a bispecific antibody, and

the bispecific antibody comprises (1) a domain comprising antibody variable regions that specifically bind to the mutated antibody via a moiety having the mutation, and (2) a domain comprising antibody variable regions having binding activity against a molecule expressed on T cell surface, and does not specifically bind to an antibody free of the mutation.

[4] A pharmaceutical composition for use in combination with administration of a bispecific antibody, wherein

the pharmaceutical composition comprises a mutated antibody having a mutation, including substitution, deletion, addition or modification, of at least one amino acid in a CH1 region, a CH2 region, a CH3 region, a CL region, or a framework region, and

the bispecific antibody comprises (1) a domain comprising antibody variable regions that specifically bind to the mutated antibody via a moiety having the mutation, and (2) a domain comprising antibody variable regions having binding activity against a molecule expressed on T cell surface, and does not specifically bind to an antibody free of the mutation.

[5] The pharmaceutical composition according to any of [1] to [4], wherein the mutated antibody has the mutation in a CH2 region, and the mutated antibody has reduced binding activity against Fc gamma receptor and C1q compared with a corresponding non-mutated antibody.

[6] The pharmaceutical composition according to any of [1] to [5], wherein the mutated antibody has a CH2 region mutation at any of positions 234, 235, 236, 237, 238, 265, 266, 267, 268, 269, 270, 271, 295, 296, 298, 300, 324, 325, 326, 327, 328, 329, 330, 331, 332, 333, 334, 335, 336, and 337 according to the EU numbering, and the mutated antibody binds to the extracellular binding domain via a moiety having the mutation.

[7] The pharmaceutical composition according to any of [1] to [6], wherein

the CH2 region of the mutated antibody has a mutation selected from the group of

a mutation of an amino acid at position 235 to arginine,

a mutation of an amino acid at position 236 to arginine,

a mutation of an amino acid at position 239 to lysine,

a mutation of an amino acid at position 250 to valine,

a mutation of an amino acid at position 252 to tyrosine,

a mutation of an amino acid at position 297 to alanine,

a mutation of an amino acid at position 307 to glutamine,

a mutation of an amino acid at position 308 to proline,

a mutation of an amino acid at position 311 to alanine,

a mutation of an amino acid at position 434 to tyrosine, and

a mutation of an amino acid at position 436 to valine, according to the EU numbering, and

the mutated antibody binds to the extracellular binding domain via a moiety having the mutation.

[8] An isolated nucleic acid encoding a chimeric receptor or a bispecific antibody contained in a pharmaceutical composition according to any of [1] to [7].

[9] A vector comprising an isolated nucleic acid according to [8].

[10] The vector according to [9], wherein the vector is operably linkable to at least one regulatory element for the expression of the chimeric receptor or the bispecific antibody.

[11] A cell transformed or transduced with an isolated nucleic acid according to [8] or a vector according to [9] or [10].

[12] The pharmaceutical composition according to any of [1] to [7], wherein the mutated antibody binds to a tumor antigen.

[A1-1] A chimeric receptor comprising an extracellular binding domain, a transmembrane domain and an intracellular signaling domain, wherein

the extracellular binding domain is capable of specifically binding to a mutated antibody having a mutation, including substitution, deletion, addition or modification, of at least one amino acid in a CH2 region, via a moiety having the mutation, and does not specifically bind to an antibody free of the mutation.

[A1-2] The chimeric receptor according to [A1-1], wherein the mutated antibody does not increase the occurrence of intercellular bridge with other immunocytes, compared with a corresponding non-mutated antibody.

[A1-3] The chimeric receptor according to [A1-1] or [A1-2], wherein the mutated antibody is an antibody having reduced binding activity against any Fcγ receptor of Fcγl, FcγIIA, FcγIIB, FcγIIIA and FcγIIIB as compared with a corresponding non-mutated antibody.

[A1-3-1] The chimeric receptor according to any of [A1-1] to [A1-3], wherein the mutation has mutations at one or more positions selected from the group of 231, 232, 233, 234, 235, 236, 237, 238, 239, 240, 264, 265, 266, 267, 269, 270, 295, 296, 297, 298, 299, 300, 325, 327, 328, 329, 330, 331 and 332, each represented by its position according to the EU numbering, and the extracellular binding domain is capable of binding to the mutated antibody via a moiety having the mutations.

[A1-3-2] The chimeric receptor according to any of [A1-1] to [A1-3], wherein the mutation has one or more mutations selected from the group of 234A, 235A, and 297A, each represented by its position according to the EU numbering and the amino acid introduced by the mutation, and the extracellular binding domain is capable of binding to the mutated antibody via a moiety having the mutations.

[A1-3-3] The chimeric receptor according to any of [A1-1] to [A1-3], wherein the mutation further has one or more mutations selected from the group of 349C, 356C, 366W or S, 368A, and 407V, each represented by its position according to the EU numbering and the amino acid introduced by the mutation, and the extracellular binding domain is capable of binding to the mutated antibody via a moiety having the mutations.

[A1-3-4] The chimeric receptor according to any of [A1-1] to [A1-3], wherein the mutation is one or more combinations selected from the following combinations:

(1) 235R and 239K;
(2) 235R and 236R;
(3) 235R, 239K and 297A;
(4) 235R, 236R and 239K;
(5) 252Y and 434Y;
(6) 235R, 239K, 252Y and 434Y;
(7) 252Y, 434Y and 436V;
(8) 235R, 239K, 252Y, 434Y and 436V;
(9) 250V, 252Y, 307Q, 308P, 311A, 434Y and 436V; and
(10) 235R, 239K, 250V, 252Y, 307Q, 308P, 311A, 434Y and 436V

each represented by its position according to the EU numbering and the amino acid introduced by the mutation, and the extracellular binding domain is capable of binding to the mutated antibody via a moiety having the mutations.

[A1-3-5] The chimeric receptor according to any of [A1-1] to [A1-3], wherein the mutation has mutations at one or more positions selected from the group of 235, 236, and 239, each represented by its position according to the EU numbering, and the extracellular binding domain binds to the mutated antibody via a moiety having the mutations.

[A1-4] The chimeric receptor according to [A1-1], wherein the mutated antibody is an antibody having enhanced binding activity against FcγRIa as compared with a corresponding non-mutated antibody.

[A1-4-1] The chimeric receptor according to [A1-1] or [A1-4], wherein the mutation has mutations at one or more positions selected from the group of 234, 235, 236, 237, 238, 265, 266, 267, 268, 269, 270, 271, 295, 296, 298, 300, 324, 325, 326, 327, 328, 329, 330, 331, 332, 333, 334, 335, 336 and 337, each represented by its position according to the EU numbering, and the extracellular binding domain is capable of binding to the mutated antibody via a moiety having the mutations.

[A1-5] The chimeric receptor according to [A1-1], wherein the mutated antibody is an antibody having enhanced binding activity against any Fcγ receptor of Fcγl, FcγIIA, FcγIIB, FcγIIIA and FcγIIIB as compared with a corresponding non-mutated antibody.

[A1-5-1] The chimeric receptor according to [A1-1] or [A1-5], wherein the mutation has mutations at one or more positions selected from the group of 231, 232, 233, 234, 235, 236, 237, 238, 239, 240, 265, 266, 267, 268, 269, 270, 271, 295, 296, 298, 300, 324, 325, 326, 327, 328, 329, 330, 331, 332, 333, 334, 335, 336 and 337, each represented by its position according to the EU numbering, and the extracellular binding domain is capable of binding to the mutated antibody via a moiety having the mutations.

[A1-5-2] The chimeric receptor according to [A1-1] or [A1-5], wherein the mutation is one or more combinations selected from the following combinations:

(1) 234Y, 235Y, 236W, 268D, 270E and 298A;
(2) 234Y, 235Q, 236W, 239M, 268D, 270E and 298A;
(3) 234Y, 235Q, 236W, 239M, 268D, 270E and 298A;
(4) 234Y, 235Y, 236W, 268D, 298A and 327D;
(5) 234Y, 235Y, 236W, 239M, 268D, 298A and 327D;
(6) 234Y, 235Y, 236W, 239M, 268D, 298A, 327D, 328W and 334L;
(7) 326D, 330M and 334E;
(8) 270E, 326D, 330M and 334E; and
(9) 270E, 326D, 330K and 334E

each represented by its position according to the EU numbering and the amino acid introduced by the mutation, and the extracellular binding domain is capable of binding to the mutated antibody via a moiety having the mutations.

[A1-5-3] The chimeric receptor according to [A1-1] or [A1-5], wherein the mutation is one or more combinations selected from the following combinations:

(1) 234L, S, F, E, V, D, Q, I, M, T, A, G or H, 235Y or Q, 236W, 239M or I, 268D, and 298A;
(2) 234L, S, F, E, V, D, Q, I, M, T, A, G or H, 235Y or Q, 236W, 239M or I, 268D, 298A and 327D;
(3) 234F, E, D, S or L, 235Y or Q, 236W, 239M or I, 268D, 298A and 327D;
(4) 270E, 326D, 330A, K, M, F, I, Y or H, and 334E;
(5) 270E, 326D, 330A, K, M, F, I, Y or H, and 334E;
(6) 270E, 326D, 330A, F or K, and 334E; and
(7) 234L, S, F, E, V, D, Q, I, M, T, A, G or H, 235Y or Q, 236W, 239M or I, 268D, 270E, and, 298A

each represented by its position according to the EU numbering and the amino acid introduced by the mutation, and the extracellular binding domain is capable of binding to the mutated antibody via a moiety having the mutations.

[A1-6] The chimeric receptor according to [A1-1], wherein the mutated antibody is an antibody having maintained or decreased binding activity against both H and R forms which are gene polymorphisms of FcγRIIa, and enhanced binding activity against FcγRIIb as compared with a corresponding non-mutated antibody.

[A1-6-1] The chimeric receptor according to [A1-1] or [A1-6], wherein the mutation has mutations at one or more positions selected from the group of 233, 234, 237, 238, 239, 267, 268, 296, 271, 323, 326, and 330, each represented by its position according to the EU numbering, and the extracellular binding domain is capable of binding to the mutated antibody via a moiety having the mutations.

[A1-6-2] The chimeric receptor according to [A1-1] or [A1-6], wherein the mutation has one or more mutations selected from the group of 238D, 328E, 237W, 267V, 267Q, 268N, 271G, 326M, 239D, 267A, 234W, 237A, 237D, 237E, 237L, 237M, 237Y, 330K, 330R, 233D, 268D, 268E, 326D, 326S, 326T, 3231, 323L, 323M, 296D, 326A, 326N, and 330M, each represented by its position according to the EU numbering and the amino acid introduced by the mutation, and the extracellular binding domain is capable of binding to the mutated antibody via a moiety having the mutations.

[A1-6-3] The chimeric receptor according to [A1-1] or [A1-6], wherein the mutation has mutations at positions of one or more combinations selected from the following combinations:

(1) 238, 233, 237, 268, 271, 296 and 330;
(2) 238, 237, 268, 271, 296 and 330;
(3) 238, 233, 237, 268, 271, 296, 330 and 332;
(4) 238, 233, 237, 264, 267, 268, 271 and 330;
(5) 238, 233, 237, 267, 268, 271, 296, 330 and 332;
(6) 238, 237, 267, 268, 271, 296, 330 and 332;
(7) 238, 233, 237, 268, 271, 296, 327 and 330;
(8) 238, 233, 237, 264, 267, 268 and 271;
(9) 238, 233, 237, 264, 267, 268, 271, 296 and 330;
(10) 238, 233, 237, 264, 267, 268, 271, 296, 330 and 396;
(11) 238, 237, 264, 267, 268, 271 and 330;
(12) 238, 237, 264, 267, 268, 271, 296 and 330;
(13) 238, 264, 267, 268 and 271;
(14) 238, 264, 267, 268, 271 and 296;

(15) 238, 237, 267, 268, 271, 296 and 330;
(16) 238, 233, 237, 264, 267, 268, 271, 330 and 396;
(17) 238, 233, 237, 264, 267, 268, 271, 296, 327, 330 and 396;
(18) 238, 233, 237, 264, 267, 268, 271, 272 and 296;
(19) 238, 237, 264, 267, 268, 271, 272 and 330;
(20) 238, 237, 264, 267, 268, 271, 272, 296 and 330;
(21) 238, 233, 264, 267, 268 and 271;
(21) 238, 237, 267, 268, 271, 296 and 330;
(22) 238, 264, 267, 268, 271, 272 and 296; and
(22) 238, 233, 264, 267, 268, 271 and 296

each represented by its position according to the EU numbering, and the extracellular binding domain is capable of binding to the mutated antibody via a moiety having the mutations.

[A1-6-4] The chimeric receptor according to [A1-1] or [A1-6], wherein the mutation comprises a mutation at position 238 and comprises mutations at one or more positions selected from 235, 237, 241, 268, 295, 296, 298, 323, 324 and 330, each represented by its position according to the EU numbering, wherein the mutations decrease binding activity against every active FcγR, and wherein the extracellular binding domain is capable of binding to the mutated antibody via a moiety having the mutations.

[A1-6-5] The chimeric receptor according to [A1-1] or [A1-6], wherein the mutation comprises a mutation at position 238 and comprises mutations at positions of a combination selected from the group consisting of (1) 241, 268, 296 and 324; (2) 237, 241, 296 and 330; and (3) 235, 237, 241 and 296, each represented by its position according to the EU numbering, wherein the mutations decrease binding activity against every active FcγR, and wherein the extracellular binding domain is capable of binding to the mutated antibody via a moiety having the mutations.

[A1-6-6] The chimeric receptor according to [A1-1] or [A1-6], wherein the mutation comprises mutations at positions 238 and 271 and comprises mutations at one or more positions selected from 234, 235, 236, 237, 239, 265, 267 and 297, each represented by its position according to the EU numbering, wherein the mutations decrease binding activity against every active FcγR, and wherein the extracellular binding domain is capable of binding to the mutated antibody via a moiety having the mutations.

[A1-6-7] The chimeric receptor according to [A1-1] or [A1-6], wherein the mutation comprises mutations at positions of a combination selected from the group consisting of (1) 233, 238, 264, 267, 268 and 271; (2) 233, 237, 238, 264, 267, 268, 271, 296, 297, 330 and 396; and (3) 233, 238, 264, 267, 268, 271 and 296, each represented by its position according to the EU numbering, wherein the mutations decrease binding activity against every active FcγR, and wherein the extracellular binding domain is capable of binding to the mutated antibody via a moiety having the mutations.

[A1-6-8] The chimeric receptor according to [A1-1] or [A1-6], wherein the mutation comprises 238D and comprises one or more mutations selected from 235F, 237Q or D, 241M or L, 268P, 295M or V, 296E, H, N or D, 298A or M, 323I, 324N or H, and 330H or Y, each represented by its position according to the EU numbering and the amino acid introduced by the mutation, wherein the mutations decrease binding activity against every active FcγR, and wherein the extracellular binding domain is capable of binding to the mutated antibody via a moiety having the mutations.

[A1-6-9] The chimeric receptor according to [A1-1] or [A1-6], wherein the mutation comprises 238D and comprises mutations of a combination selected from the group consisting of (1) 241M, 268P, 296E and 324H; (2) 237Q or D, 241M, 296E and 330H; and (3) 235F, 237Q or D, 241M and 296E, each represented by its position according to the EU numbering and the amino acid introduced by the mutation, wherein the mutations decrease binding activity against every active FcγR, and wherein the extracellular binding domain is capable of binding to the mutated antibody via a moiety having the mutations.

[A1-6-10] The chimeric receptor according to [A1-1] or [A1-6], wherein the mutation comprises 238D and 271G and comprises two or more mutations selected from 234A, H, N, K or R, 235A, 236Q, 237R or K, 239K, 265K, N, R, S or V, 267K, R or Y, and 297A, each represented by its position according to the EU numbering and the amino acid introduced by the mutation, wherein the mutations decrease binding activity against every active FcγR, and wherein the extracellular binding domain is capable of binding to the mutated antibody via a moiety having the mutations.

[A1-6-11] The chimeric receptor according to [A1-1] or [A1-6], wherein the mutation comprises 238D and 271G and comprises mutations of a combination selected from the group of (1) 233D, 238D, 264I, 267R, 268E and 271G; (2) 233D, 237D, 238D, 264I, 267A, 268E, 271G, 296D, 297A, 330R and 396M; (3) 233D, 238D, 264I, 267R, 268P, 271G and 296E, each represented by its position according to the EU numbering and the amino acid introduced by the mutation, wherein the mutations decrease binding activity against every active FcγR, and wherein the extracellular binding domain is capable of binding to the mutated antibody via a moiety having the mutations.

[A1-6-12] The chimeric receptor according to any of [A1-6-1] to [A1-6-11], wherein the mutated antibody is an antibody further having decreased binding to a complement.

[A1-6-13] The chimeric receptor according to [A1-1] or [A1-2], wherein the mutation comprises mutations at one or more positions selected from the group of 322, 327, 330 and 331, each represented by its position according to the EU numbering, wherein the mutations decrease binding to a complement, and wherein the extracellular binding domain is capable of binding to the mutated antibody via a moiety having the mutations.

[A1-6-14] The chimeric receptor according to [A1-1] or [A1-2], wherein the mutation comprises one or more mutations selected from the group of 322A or E, 327G, 330S and 331S, each represented by its position according to the EU numbering and the amino acid introduced by the mutation, and the extracellular binding domain is capable of binding to the mutated antibody via a moiety having the mutations.

[A1-6-15] The chimeric receptor according to [A1-1] or [A1-2], wherein the mutation comprises mutations at one or more positions selected from the group of 238, 271, 327, 330, and 331, each represented by its position according to the EU numbering, wherein the mutated antibody maintains binding activity against FcγRIIb as compared with a non-mutated antibody having a natural IgG Fc region and the mutations decrease binding activity against every active FcγR, and wherein the extracellular binding domain is capable of binding to the mutated antibody via a moiety having the mutations.

[A1-6-16] The chimeric receptor according to [A1-1] or [A1-6], wherein the mutation has one or more mutations selected from the group of 238D, 271G, 327G, 330S and 331S, each represented by its position according to the EU numbering and the amino acid introduced by the mutation, and the extracellular binding domain is capable of binding to the mutated antibody via a moiety having the mutations.

[A1-6-17] The chimeric receptor according to [A1-6-14], wherein the mutated antibody further has one or more mutations selected from the group of 233D, 237D, 264I, 267A, and 268D or E, and the extracellular binding domain is capable of binding to the mutated antibody via a moiety having the mutations.

[A1-6-18] The chimeric receptor according to [A1-1] or [A1-6], wherein the mutation comprises 238D and 271G and comprises mutations at positions of a combination selected from the group consisting of (1) 237D, 238D, 268D or E, 271G, 327G, 330S and 331S; (2) 233D, 237D, 238D, 268D, 271G, 327G, 330S and 331S; (3) 238D, 267A, 268E, 271G, 327G, 330S and 331S; (4) 238D, 264I, 267A, 271G, 327G, 330S and 331S, each represented by its position according to the EU numbering and the amino acid introduced by the mutation, and the extracellular binding domain is capable of binding to the mutated antibody via a moiety having the mutations.

[A1-6-19] The chimeric receptor according to [A1-1] or [A1-6], wherein the mutation comprises one or more mutations selected from the group of 233, 238, 264, 267, 268, 271, 327, 330 and 331, each represented by its position according to the EU numbering, and the extracellular binding domain is capable of binding to the mutated antibody via a moiety having the mutations.

[A1-6-20] The chimeric receptor according to [A1-1] or [A1-6], wherein the mutation comprises mutations at positions of one or more combinations selected from (1) 237, 238, 268, 271, 327, 330 and 331; (2) 233, 237, 238, 268, 271, 327, 330 and 331; (3) 238, 267, 268, 271, 327, 330 and 331; (4) 238, 264, 267, 271, 327, 330 and 331, each represented by its position according to the EU numbering, and the extracellular binding domain is capable of binding to the mutated antibody via a moiety having the mutations.

[A1-7] The chimeric receptor according to [A1-1], wherein the mutated antibody is an antibody whose binding activity against an antigen varies depending on ion concentration conditions as compared with a corresponding non-mutated antibody, wherein the antibody has binding activity against FcRn under pH neutral conditions, but does not form a heterocomplex comprising two molecules of FcRn and one molecule of active Fcγ receptor under pH neutral conditions.

[A1-7-1] The chimeric receptor according to [A1-1] or [A1-7], wherein the mutation comprises mutations at one or more positions selected from the group of 235, 237, 238, 239, 270, 298, 325 and 329, each represented by its position according to the EU numbering, and the extracellular binding domain is capable of binding to the mutated antibody via a moiety having the mutations.

[A1-7-2] The chimeric receptor according to [A1-1] or [A1-7], wherein the mutation comprises one or more mutations selected from the group of 235K or R, 237K or R, 238K or R, 239L or R, 270F, 298G, 325G, and 329K or R, each represented by its position according to the EU numbering and the amino acid introduced by the mutation, and the extracellular binding domain is capable of binding to the mutated antibody via a moiety having the mutations.

[A2-1] A chimeric receptor comprising an extracellular binding domain, a transmembrane domain and an intracellular signaling domain, wherein the extracellular binding domain is capable of specifically binding to a mutated antibody having a mutation, including substitution, deletion, addition or modification, of at least one amino acid in a CH3 region, via a moiety having the mutation, and does not specifically bind to an antibody free of the mutation.

[A2-2] The chimeric receptor according to [A2-1], wherein the mutated antibody improves the stability, homogeneity, immunogenicity, safety, production efficiency and/or circulation time in plasma of the mutated antibody, compared with a corresponding non-mutated antibody.

[A2-3] The chimeric receptor according to [A2-1] or [A2-2], wherein the mutated antibody is an antibody lacking an amino acid at any of positions 446 and 447 according to the EU numbering.

[A2-3-1] The chimeric receptor according to any of [A2-1] to [A2-3], wherein the mutation further comprises a mutation at position 434 represented by its position according to the EU numbering, and the extracellular binding domain is capable of binding to the mutated antibody via the mutation.

[A2-3-2] The chimeric receptor according to any of [A2-1] to [A2-3], wherein the mutation further comprises a mutation at a position selected from the group of 131, 133, 137, 138, 219, 268, 330, 331, 335, 339, 397, and 419, each represented by its position according to the EU numbering, and the extracellular binding domain is capable of binding to the mutated antibody via a moiety having the mutation.

[A2-3-3] The chimeric receptor according to any of [A2-1] to [A2-3], wherein the mutation further comprises a mutation at an EU numbering position selected from the group of 131, 133, 137, 138, 214, 217, 219, 220, 221, 222, 233, 234, 235, 236, and 409, and the extracellular binding domain is capable of binding to the mutated antibody via a moiety having the mutation.

[A2-4] The chimeric receptor according to [A2-1] or [A2-2], wherein the mutated antibody is an antibody having a mutation in an amino acid residue at an interface such that two or more amino acid residues forming the interface have the same charge.

[A2-4-1] The chimeric receptor according to [A2-1], [A2-2] or [A2-4], wherein the mutated antibody is an antibody comprising two or more heavy chain CH3 regions and having a mutation such that amino acid residues of the first heavy chain have the same charge, wherein the mutation comprises a mutation at any one of positions of each of one or more combinations selected from the following combinations:

(1) 356 and 439;
(2) 357 and 370; and
(3) 399 and 409

each represented by its position according to the EU numbering, and wherein the extracellular binding domain is capable of binding to the mutated antibody via a moiety having the mutation.

[A2-4-2] The chimeric receptor according to [A2-1], [A2-2] or [A2-4], wherein the mutation has mutations at one or more positions selected from the group of 356, 357, 370, 399, 409, and 439, each represented by its position according to the EU numbering, and the extracellular binding domain is capable of binding to the mutated antibody via a moiety having the mutations.

[A2-4-3] The chimeric receptor according to [A2-4-1] or [A2-4-2], wherein the mutation further comprises mutations at one or more positions selected from the group of amino acid residues 10, 12, 23, 39, 43 and 105 according to the Kabat numbering in the heavy chain variable region or amino acid residues 137, 196, 203, 214, 217, 233, 268, 274, 276 and 297 according to the EU numbering in the heavy chain constant region, and the extracellular binding domain is capable of binding to the mutated antibody via a moiety having the mutations.

[A2-5] The chimeric receptor according to [A2-1] or [A2-2], wherein the mutated antibody is an antibody promoting polypeptide heteromerization under reductive conditions as compared with a corresponding non-mutated antibody.

[A2-5-1] The chimeric receptor according to [A2-1], [A2-2] or [A2-5], wherein the mutated antibody is an antibody comprising two or more heavy chain CH3 regions and having a mutation such that amino acid residues of the first heavy chain have the same charge, wherein the mutation has a mutation at any one of positions of each of one or more combinations selected from the following combinations:

(1) 356 and 439;
(2) 357 and 370;
(3) 399 and 409; and
(4) 399, 409, 356 and 439

each represented by its position according to the EU numbering, and wherein the extracellular binding domain is capable of binding to the mutated antibody via a moiety having the mutation.

[A2-5-2] The chimeric receptor according to [A2-1], [A2-2] or [A2-5], wherein the mutation has one or more mutations selected from the group of 397M, F or Y, 392D, E, T, V or I, 356K, 397F or Y, and 439E, each represented by its position according to the EU numbering and the amino acid introduced by the mutation, and the extracellular binding domain is capable of binding to the mutated antibody via a moiety having the mutations.

[A2-5-3] The chimeric receptor according to [A2-1], [A2-2], [A2-5] or [A2-5-1], wherein the mutated antibody is an antibody comprising two or more heavy chain CH3 regions, wherein at least one amino acid residue at any of positions 349, 351, 354, 356, 394 and 407 (according to the EU numbering) is cysteine, and wherein the extracellular binding domain is capable of binding to the mutated antibody via a moiety having the mutation.

[A2-5-4] The chimeric receptor according to [A2-1], [A2-2] or [A2-5], wherein the mutated antibody has a mutation at EU numbering position 226 and/or 229 or lacks a core hinge region, and the extracellular binding domain is capable of binding to the mutated antibody via a moiety having the mutation.

[A2-5-5] The chimeric receptor according to [A2-5-4], wherein the mutated antibody is an antibody further having the substitution of positions 220 to 225 (according to the EU numbering) by Y-G-P-P or lacking positions 219 to 229 (according to the EU numbering).

[A2-6] The chimeric receptor according to [A2-1] or [A2-2], wherein the mutated antibody is an antibody comprising a first polypeptide and a second polypeptide in contact at their boundary moieties, wherein the first polypeptide has, at the boundary moiety, a knob capable of being positioned in a hole of the boundary moiety of the second polypeptide.

[A2-6-1] The chimeric receptor according to [A2-1], [A2-2] or [A2-6], wherein the mutated antibody is an antibody comprising two or more heavy chain CH3 regions and has mutations at one or more positions selected from the group of 366, 394, 405, and 407 according to the EU numbering, and the extracellular binding domain is capable of binding to the mutated antibody via a moiety having the mutations.

[A2-6-2] The chimeric receptor according to [A2-1], [A2-2] or [A2-6], wherein the mutated antibody is an antibody comprising two or more heavy chain CH3 regions, wherein mutations in the first and second heavy chain CH3 regions have mutations of one or more combinations selected from the following combinations:

(1) 366Y in the first heavy chain CH3 region and 407T in the second heavy chain CH3 region;
(2) 366W in the first heavy chain CH3 region and 497A in the second heavy chain CH3 region;
(3) 405A in the first heavy chain CH3 region and 394W in the second heavy chain CH3 region;
(4) 407T in the first heavy chain CH3 region and 366Y in the second heavy chain CH3 region;
(5) 366Y and 405A in the first heavy chain CH3 region, and 394W and 407T in the second heavy chain CH3 region;
(6) 366W and 405W in the first heavy chain CH3 region, and 394S and 407A in the second heavy chain CH3 region;
(7) 405W and 407A in the first heavy chain CH3 region, and 366W and 394S in the second heavy chain CH3 region; and
(8) 405W in the first heavy chain CH3 region and 394S in the second heavy chain CH3 region,

each represented by its position according to the EU numbering and the amino acid introduced by the mutation, and wherein the extracellular binding domain is capable of binding to the mutated antibody via a moiety having the mutations.

[A3-1] A chimeric receptor comprising an extracellular binding domain, a transmembrane domain and an intracellular signaling domain, wherein
the extracellular binding domain is capable of specifically binding to a mutated antibody having a mutation, including substitution, deletion, addition or modification, of at least one amino acid in a CH1 region, via a moiety having the mutation, and does not specifically bind to an antibody free of the mutation.

[A3-2] The chimeric receptor according to [A3-1], wherein the mutated antibody is an antibody having improved pharmacokinetics and/or hinge region heterogeneity as compared with a corresponding non-mutated antibody.

[A3-2-1] The chimeric receptor according to [A3-1] or [A3-2], wherein the mutation has mutations at one or more positions selected from the group of 131, 133, 137, and 138, each represented by its position according to the EU numbering, and the extracellular binding domain is capable of binding to the mutated antibody via a moiety having the mutations.

[A3-2-2] The chimeric receptor according to [A3-1] or [A3-2], wherein the mutation comprises one or more mutations selected from the group of 131S, 133K, 220S, 137G, 138G, 268Q, 355Q and 419E, each represented by its position according to the EU numbering and the amino acid introduced by the mutation, and the extracellular binding domain is capable of binding to the mutated antibody via a moiety having the mutations.

[A3-2-3] The chimeric receptor according to [A3-2-1] or [A3-2-2], wherein the mutation further comprises mutations at one or more positions selected from the group of 220, 268, 330, 331, 339, 355, 419, 446, and 447, each represented by its position according to the EU numbering, and the extracellular binding domain is capable of binding to the mutated antibody via a moiety having the mutations.

[A4-1] A chimeric receptor comprising an extracellular binding domain, a transmembrane domain and an intracellular signaling domain, wherein
the extracellular binding domain is capable of specifically binding to a mutated antibody having a mutation, including substitution, deletion, addition or modification, of at least one amino acid in a hinge region, via a moiety having the mutation, and does not specifically bind to an antibody free of the mutation.

[A4-2] The chimeric receptor according to [A4-1], wherein the mutated antibody is an antibody having enhanced binding activity against any Fcγ receptor of FcγI, FcγIIA, FcγIIB, FcγIIIA and FcγIIIB as compared with a corresponding non-mutated antibody.

[A4-2-1] The chimeric receptor according to [A4-1] or [A4-2], wherein the mutation comprises mutations at one or more positions selected from the group of 220, 221, 222, 223, 224, 225, 226, 227, 228, 229, and 230, each represented by its position according to the EU numbering, and the extracellular binding domain is capable of binding to the mutated antibody via a moiety having the mutations.

[A4-2-2] The chimeric receptor according to [A4-2-1], wherein the mutation further comprises mutations at one or more positions selected from the group of 231, 232, 233, 234, 235, 236, 237, 238, 239, 240, 264, 265, 266, 267, 269, 270, 295, 296, 297, 298, 299, 300, 325, 327, 328, 329, 330, 331, and 332, each represented by its position according to the EU numbering, and the extracellular binding domain is capable of binding to the mutated antibody via a moiety having the mutations.

[A4-2-3] The chimeric receptor according to [A4-1] or [A4-2], wherein the mutation comprises one or more mutations selected from the group of 235R, 239K and 297A, each represented by its position according to the EU numbering and the amino acid introduced by the mutation, and the extracellular binding domain is capable of binding to the mutated antibody via a moiety having the mutations.

[A4-2-4] The chimeric receptor according to any of [A4-2-1] to [A4-2-3], wherein the mutation further has mutations at one or more positions selected from the group of 231, 232, 233, 234, 235, 236, 237, 238, 239, 240, 241, 243, 244, 245, 246, 247, 249, 250, 251, 252, 254, 255, 256, 257, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 307, 308, 309, 311, 312, 313, 314, 315, 316, 317, 318, 320, 322, 323, 324, 325, 326, 327, 328, 329, 330, 331, 332, 333, 334, 335, 336, 337, 339, 341, 343, 375, 376, 377, 378, 379, 380, 382, 385, 386, 387, 389, 392, 396, 421, 423, 427, 428, 429, 430, 431, 433, 434, 436, 438, 440 and 442, each represented by its position according to the EU numbering, and the extracellular binding domain is capable of binding to the mutated antibody via a moiety having the mutations.

[A4-2-5] The chimeric receptor according to [A4-1] or [A4-2], wherein the mutation comprises one or more mutations selected from the group of 221K or Y, 222F, W, E or Y, 223F, W, E or K, 224F, W, E or Y, 225E, K or W, 227E, G, K or Y, 228E, G, K or Y, and 230A, E, G or Y, each represented by its position according to the EU numbering and the amino acid introduced by the mutation, and the extracellular binding domain is capable of binding to the mutated antibody via a moiety having the mutations.

[A4-3] The chimeric receptor according to [A4-1], wherein the mutated antibody is an antibody having reduced binding activity against any Fcγ receptor of FcγI, FcγIIA, FcγIIB, FcγIIIA and FcγIIIB as compared with a corresponding non-mutated antibody.

[A4-3-1] The chimeric receptor according to [A4-1] or [A4-3], wherein the mutation comprises mutations at one or more positions selected from the group of 220, 226, and 229, each represented by its position according to the EU numbering, and the extracellular binding domain is capable of binding to the mutated antibody via a moiety having the mutations.

[A5-1] A chimeric receptor comprising an extracellular binding domain, a transmembrane domain and an intracellular signaling domain, wherein
the extracellular binding domain is capable of specifically binding to a mutated antibody having a mutation, including substitution, deletion, addition or modification, of at least one amino acid in a CH2 region and a CH3 region, via a moiety having the mutation, and does not specifically bind to an antibody free of the mutation.

[A5-2] The chimeric receptor according to [A5-1], wherein the mutated antibody is an antibody having enhanced binding activity against FcRn at acidic pH as compared with a corresponding non-mutated antibody.

[A5-2-1] The chimeric receptor according to [A5-1] or [A5-2], wherein the mutation comprises mutations at one or more positions selected from the group of 235, 236, 239, 327, 330, 331, 428, 434, 436, 438, and 440, each represented by its position according to the EU numbering, and the extracellular binding domain is capable of binding to the mutated antibody via a moiety having the mutations.

[A5-2-2] The chimeric receptor according to [A5-1] or [A5-2], wherein the mutation has one or more mutations selected from the group of 235R, 236R, 239K, 327G, 330S, 331S, 428L, 434A, 436T, 438R, and 440E, each represented by its position according to the EU numbering and the amino acid introduced by the mutation, and the extracellular binding domain is capable of binding to the mutated antibody via a moiety having the mutations.

[A5-2-2-1] The chimeric receptor according to [A5-1] or [A5-2], wherein the mutation has mutations at one or more positions selected from the group of 214, 235, 236, 239, 327, 330, 331, 446, and 447, each represented by its position according to the EU numbering, and the extracellular binding domain binds to the mutated antibody via a moiety having the mutations.

[A5-2-3] The chimeric receptor according to [A5-1] or [A5-2], wherein the mutation has mutations at one or more positions selected from the group of 238, 244, 245, 249, 250, 251, 252, 253, 254, 255, 256, 257, 258, 260, 262, 265, 270, 272, 279, 283, 285, 286, 288, 293, 303, 305, 307, 308, 309, 311, 312, 314, 316, 317, 318, 332, 339, 340, 341, 343, 356, 360, 362, 375, 376, 377, 378, 380, 382, 385, 386, 387, 388, 389, 400, 413, 415, 423, 424, 427, 428, 430, 431, 433, 434, 435, 436, 438, 439, 440, 442 and 447, each represented by its position according to the EU numbering, and the extracellular binding domain is capable of binding to the mutated antibody via a moiety having the mutations.
[A5-2-4] The chimeric receptor according to [A5-1] or [A5-2], wherein the mutation comprises one or more mutations selected from

238L,
244L,
245R,
249P, and
250Q or E,
or comprises one or more mutations selected from
251R, D, E, or L,
252F, S, T, or Y,
254S or T,
255R, G, I, or L,
256A, R, N, D, Q, E, P, or T,
257A, I, M, N, S, or V,
258D,
260S,
262L,
270K,
272L, or R,
279A, D, G, H, M, N, Q, R, S, T, W, or Y,
283A, D, F, G, H, I, K, L, N, P, Q, R, S, T, W, or Y,
285N,
286F,
288N, or P,
293V,
307A, E, Q, or M,
311A, E, I, K, L, M, S, V, or W,
309P,
312A, D, or P,
314A or L,
316K,
317P,
318N, or T,
332F, H, K, L, M, R, S, or W,
339N, T, or W,
341P,
343E, H, K, Q, R, T, or Y,
375R,
376G, I, M, P, T, or V,
377K,
378D, N, or V,
380A, N, S, or T,
382F, H, I, K, L, M, N, Q, R, S, T, V, W, or Y,
385A, R, D, G, H, K, S, or T,
386R, D, I, K, M, P, S, or T,
387A, R, H, P, S, or T,
389N, P, or S,
423N,
427N,
428L, M, F, S, or T,
430A, F, G, H, I, K, L, M, N, Q, R, S, T, V, or Y,
431H, or N,
433R, Q, H, I, K, P, or S,
434A, G, H, F, S, W, or Y,
436R, N, H, I, L, K, M, or T,
438K, L, T, or W,

440K, and,

442K, 3081, P, or T,

each represented by its position according to the EU numbering and the amino acid introduced by the mutation, and the extracellular binding domain is capable of binding to the mutated antibody via a moiety having the mutations.

[A5-3] The chimeric receptor according to [A5-1], wherein the mutated antibody is an antibody having enhanced binding activity against FcRn at neutral pH as compared with a corresponding non-mutated antibody.

[A5-3-1] The chimeric receptor according to [A5-1] or [A5-3], wherein the mutation comprises mutations at one or more positions selected from the group of 248, 250, 252, 254, 255, 256, 257, 258, 265, 286, 289, 297, 303, 305, 307, 308, 309, 311, 312, 314, 315, 317, 332, 334, 360, 376, 380, 382, 384, 385, 386, 387, 389, 424, 428, 433, 434, and 436, each represented by its position according to the EU numbering, and the extracellular binding domain is capable of binding to the mutated antibody via a moiety having the mutations.

[A5-3-2] The chimeric receptor according to [A5-1] or [A5-3], wherein the mutation comprises one or more mutations selected from the group of 237M, 2481, 250A, F, I, M, Q, S, V, W or Y, 252F, W or Y, 254T, 255E, 256D, E or Q, 257A, G, I, L, M, N, S, T or Y, 258H, 265A, 286A or E, 289H, 297A, 303A, 305A, 307A, D, F, G, H, I, K, L, M, N, P, Q, R, S, V, W or Y, 308A, F, I, L, M, P, Q or T, 309A, D, E, P or R, 311A, H or I, 312A or H, 314K or R, 315A, D or H, 317A, 332V, 334L, 360H, 376A, 380A, 382A, 384A, 385D or H, 386P, 387E, 389A or S, 424A, 428A, D, F, G, H, I, K, L, N, P, Q, S, T, V, W or Y, and 436H, I, L, or V, each represented by its position according to the EU numbering and the amino acid introduced by the mutation, and the extracellular binding domain is capable of binding to the mutated antibody via a moiety having the mutations.

[A5-3-3] The chimeric receptor according to [A5-1] or [A5-3], wherein the mutation comprises mutations at one or more positions selected from the group of 238, 250, 252, 254, 255, 258, 286, 307, 308, 309, 311, 315, 428, 433, 434, and 436, each represented by its position according to the EU numbering, and the extracellular binding domain is capable of binding to the mutated antibody via a moiety having the mutations.

[A5-3-4] The chimeric receptor according to [A5-1] or [A5-3], wherein the mutation comprises one or more mutations selected from the group of 238D, 250V, 252Y, 254T, 255L, 256E, 258D or I, 286E, 307Q, 308P, 309E, 311A or H, 315D, 4281, 433A, K, P, R or S, 434Y or W, and 4361, L, V, T or F, each represented by its position according to the EU numbering and the amino acid introduced by the mutation, and the extracellular binding domain is capable of binding to the mutated antibody via a moiety having the mutations.

[A5-4] The chimeric receptor according to [A5-1], wherein the mutated antibody is an antibody having higher binding activity against an antigen at neutral pH than that against the antigen at acidic pH as compared with a corresponding non-mutated antibody.

[A5-4-1] The chimeric receptor according to [A5-1] or [A5-4], wherein the mutation comprises mutations at one or more positions selected from the group of 257, 308, 428 and 434, each represented by its position according to the EU numbering, and the extracellular binding domain is capable of binding to the mutated antibody via a moiety having the mutations.

[A5-4-2] The chimeric receptor according to [A5-1] or [A5-4], wherein the mutation comprises one or more mutations selected from the group of 257A, 308P, 428L, and 434Y, each represented by its position according to the EU numbering and the amino acid introduced by the mutation, and the extracellular binding domain is capable of binding to the mutated antibody via a moiety having the mutations.

[A5-5] The chimeric receptor according to [A5-1], wherein the mutated antibody is an antibody having enhanced binding activity against FcγRIIb as compared with a corresponding non-mutated antibody.

[A5-5-1] The chimeric receptor according to [A5-1] or [A5-5], wherein the mutation comprises mutations at one or more positions selected from the group of 231, 232, 233, 234, 235, 236, 237, 238, 239, 264, 266, 267, 268, 271, 295, 298, 325, 326, 327, 328, 330, 331, 332, 334, and 396, each represented by its position according to the EU numbering, and the extracellular binding domain is capable of binding to the mutated antibody via a moiety having the mutations.

[A5-5-2] The chimeric receptor according to [A5-1] or [A5-5], wherein the mutation comprises a mutation at position 236 and comprises mutations at positions of one or more combinations selected from (1) 231, 232, 233, 234, 235, 237, 238, 239, 264, 266, 267, 268, 271, 295, 298, 325, 326, 327, 328, 330, 331, 332, 334, and 396; (2) 231, 232, 235, 239, 268, 295, 298, 326, 330, and 396; or (3) 268, 295, 326, and 330, each represented by its position according to the EU numbering, and the extracellular binding domain is capable of binding to the mutated antibody via a moiety having the mutations.

[A5-5-3] The chimeric receptor according to [A5-1] or [A5-5], wherein the mutation comprises mutations at one or more positions selected from the group of 285, 311, 312, 315, 318, 333, 335, 337, 341, 342, 343, 384, 385, 388, 390, 399, 400, 401, 402, 413, 420, 422, and 431, each represented by its position according to the EU numbering, and the extracellular binding domain is capable of binding to the mutated antibody via a moiety having the mutations.

[A5-5-4] The chimeric receptor according to [A5-1] or [A5-5], wherein the mutation comprises mutations of one or more combinations selected from the following combinations:

(1) 231D, E, F, G, H, I, K, L, M, N, P, Q, R, S, T, V, W, or Y,
(2) 232A, D, E, F, G, H, I, K, L, M, N, Q, R, S, T, V, W, or Y,
(3) 233D,
(4) 234W, or Y,
(5) 235W,
(6) 236A, D, E, H, I, L, M, N, Q, S, T, or V,
(7) 237D, or Y,
(8) 238E, I, M, Q, or Y,
(9) 239I, L, N, P, or V,
(10) 264I,
(11) 266F,
(12) 267A, H, or L,
(13) 268D, or E,
(14) 271D, E, or G,
(15) 295L,
(16) 298L,
(17) 325E, F, I, or L,
(18) 326T,
(19) 327I, or N,
(20) 328T,
(21) 330K, or R,
(22) 331E,
(23) 332D,
(24) 334D, I, M, V, or Y, and
(25) 396A, D, E, F, G, H, I, K, L, M, N, Q, R, S, T, V, W, or Y

each represented by its position according to the EU numbering and the amino acid introduced by the mutation, and the extracellular binding domain is capable of binding to the mutated antibody via a moiety having the mutations.

[A5-5-5] The chimeric receptor according to [A5-1] or [A5-5], wherein the mutation comprises (1) mutations at one or more positions selected from 234, 238, 250, 264, 267, 307 and 330 and comprises (2) mutations at two or more positions selected from 285, 311, 312, 315, 318, 333, 335, 337, 341, 342, 343, 384, 385, 388, 390, 399, 400, 401, 402, 413, 420, 422 and 431, each represented by its position according to the EU numbering, and the extracellular binding domain is capable of binding to the mutated antibody via a moiety having the mutations.

[A5-5-6] The chimeric receptor according to [A5-1] or [A5-5], wherein the mutation comprises mutations at positions of one or more combinations selected from the following combinations:

(1) 234, 238, 250, 264, 307, 311, 330 and 343;
(2) 234, 238, 250, 264, 307, 311, 330 or 413;
(3) 234, 238, 250, 264, 267, 307, 311 or 343;
(4) 234, 238, 250, 264, 267, 307, 311, 330 and 413;
(5) 234, 238, 250, 267, 307, 311, 330 and 343;
(6) 234, 238, 250, 267, 307, 311, 330 and 413;
(7) 234, 238, 250, 307, 311, 330 and 343;
(8) 234, 238, 250, 307, 311, 330 and 413;
(9) 238, 250, 264, 267, 307, 311, 330 and 343;
(10) 238, 250, 264, 267, 307, 311, 330 and 413;
(11) 238, 250, 264, 307, 311, 330 and 343;
(12) 238, 250, 264, 307, 311, 330 and 413;
(13) 238, 250, 267, 307, 311, 330 and 343;
(14) 238, 250, 267, 307, 311, 330 and 413;
(15) 238, 250, 307, 311, 330 and 343; and
(16) 238, 250, 307, 311, 330, and 413

each represented by its position according to the EU numbering and the amino acid introduced by the mutation, and the extracellular binding domain is capable of binding to the mutated antibody via a moiety having the mutations.

[A5-6] The chimeric receptor according to [A5-1], wherein the mutated antibody is an antibody having improved stability through a mutation at a loop site of an Fc region.

[A5-6-1] The chimeric receptor according to [A5-1] or [A5-6], wherein the mutation comprises mutations at one or more positions selected from the group of 234, 235, 236, 237, 238, 239, 247, 250, 265, 266, 267, 268, 269, 270, 271, 295, 296, 298, 300, 307, 309, 315, 324, 325, 326, 327, 329, 330, 333, 335, 337, 360, 385, 386, 387, 389, 428, and 433, each represented by its position according to the EU numbering, and the extracellular binding domain is capable of binding to the mutated antibody via a moiety having the mutations.

[A6-1] A chimeric receptor comprising an extracellular binding domain, a transmembrane domain and an intracellular signaling domain, wherein

the extracellular binding domain is capable of specifically binding to a mutated antibody having a mutation, including substitution, deletion, addition or modification, of at least one amino acid in a framework region, and does not specifically bind to an antibody free of the mutation.

[A6-2] The chimeric receptor according to [A6-1], wherein the mutated antibody is an antibody having enhanced binding activity against any Fcγ receptor of FcγI, FcγIIA, FcγIIB, FcγIIIA and FcγIIIB as compared with a corresponding non-mutated antibody.

[A6-2-1] The chimeric receptor according to [A6-1] or [A6-2], wherein the mutation comprises mutations at one or more positions selected from the group of 10, 12, 23, 39, 43 and 105, each represented by its position according to the Kabat numbering, and the extracellular binding domain is capable of binding to the mutated antibody via a moiety having the mutations.

[A6-2-2] The chimeric receptor according to [A6-1] or [A6-2], wherein the mutation has mutations at one or more positions selected from the group of 10, 12, 23, 39, 43 and 105, each represented by its position according to the Kabat numbering, or 137, 196, 203, 214, 217, 233, 268, 274, 276, 297, 355, 392, 419, and 435 (according to the EU numbering), and the extracellular binding domain is capable of binding to the mutated antibody via a moiety having the mutations.

[A6-3] The chimeric receptor according to [A6-1], wherein the mutated antibody is an antibody capable of binding to an antigen in a pH-dependent manner as compared with a corresponding non-mutated antibody.

[A6-3-1] The chimeric receptor according to [A6-1] or [A6-3], wherein the mutation comprises (1) mutations at one or more positions selected from 1, 3, 5, 8, 10, 12, 13, 15, 16, 18, 19, 23, 25, 26, 39, 41, 42, 43, 44, 46, 68, 71, 72, 73, 75, 76, 77, 81, 82, 82a, 82b, 83, 84, 85, 86, 105, 108, 110, and 112 in a heavy chain;

> and comprises (2) mutations at one or more positions selected from 1, 3, 7, 8, 9, 11, 12, 16, 17, 18, 20, 22, 37, 38, 39, 41, 42, 43, 45, 46, 49, 57, 60, 63, 65, 66, 68, 69, 70, 74, 76, 77, 79, 80, 81, 85, 100, 103, 105, 106, 107, and 108 in a light chain,
> each represented by its position according to the Kabat numbering, and the extracellular binding domain is capable of binding to the mutated antibody via a moiety having the mutations.

[A6-3-2] The chimeric receptor according to [A6-1] or [A6-3], wherein the mutation further comprises mutations at one or more positions selected from the group of 196, 253, 254, 256, 258, 278, 280, 281, 282, 285, 286, 307, 309, 311, 315, 327, 330, 342, 343, 345, 356, 358, 359, 361, 362, 373, 382, 384, 385, 386, 387, 389, 399, 400, 401, 402, 413, 415, 418, 419, 421, 424, 430, 433, 434, and 443, each represented by its position according to the EU numbering, and the extracellular binding domain is capable of binding to the mutated antibody via a moiety having the mutations.

[A7-1] A chimeric receptor comprising an extracellular binding domain, a transmembrane domain and an intracellular signaling domain, wherein

the extracellular binding domain is capable of specifically binding to a mutated antibody having a mutation, including substitution, deletion, addition or modification, of at least one amino acid in a CL region, and does not specifically bind to an antibody free of the mutation.

[A7-2] The chimeric receptor according to [A7-1], wherein the mutated antibody is an antibody inhibiting the association between CH1 and CL as compared with a corresponding non-mutated antibody.

[A7-2-1] The chimeric receptor according to [A7-1] or [A7-2], wherein the mutation has mutations at one or more positions selected from the group of 123, 131, 160, and 180, each represented by its position according to the EU numbering, and the extracellular binding domain is capable of binding to the mutated antibody via a moiety having the mutations.

[A7-2-2] The chimeric receptor according to [A7-1] or [A7-2], wherein the mutation comprises a mutation at any one of positions of a combination selected from the following combinations:

> (1) amino acid 147 contained in CH1 and amino acid 180 contained in CL;
> (2) amino acid 147 contained in CH1 and amino acid 131 contained in CL;
> (3) amino acid 175 contained in CH1 and amino acid 160 contained in CL; and

(4) amino acid 213 contained in CH1 and amino acid 123 contained in CL,

each represented by its position according to the EU numbering, wherein the mutated antibody is an antibody having an amino acid mutation such that the amino acid residues of the combination have charges that repel each other, and wherein the extracellular binding domain is capable of binding to the mutated antibody via a moiety having the mutations.

[A1-3-5] The chimeric receptor according to any of [A1-1] to [A1-3], wherein the mutation has mutations at one or more positions selected from the group of 235, 236, and 239, each represented by its position according to the EU numbering, and the extracellular binding domain binds to the mutated antibody via a moiety having the mutations.

[A8-1] The chimeric receptor according to any of [A1-1] to [A1-7-2], [A2-1] to [A2-6-2], [A3-1] to [A3-2-3], [A4-1] to [A4-3-1], [A5-1] to [A5-6-1], [A6-1] to [A6-3-2], and [A7-1] to [A7-2-2] wherein the extracellular binding domain comprises an antibody single chain Fv fragment, a CrossMab fragment, or an antibody single chain Fab fragment.

[A8-2] The chimeric receptor according to [A8-1], wherein the extracellular binding domain comprises a single chain Fv fragment.

[A8-3] The chimeric receptor according to [A8-1], wherein the extracellular binding domain comprises a CrossMab fragment.

[A8-4] The chimeric receptor according to [A8-1], wherein the extracellular binding domain comprises an antibody single chain Fab fragment.

[A8-5] The chimeric receptor according to any of [A1-1] to [A1-7-2], [A2-1] to [A2-6-2], [A3-1] to [A3-2-3], [A4-1] to [A4-3-1], [A5-1] to [A5-6-1], [A6-1] to [A6-3-2], and [A7-1] to [A7-2-2] wherein the intracellular signaling domain comprises a stimulatory molecule signaling domain derived from a stimulatory molecule.

[A8-6] The chimeric receptor according to [A8-5], wherein the intracellular signaling domain comprises a costimulatory molecule signaling domain derived from a costimulatory molecule and a functional signaling domain derived from a stimulatory molecule.

[A8-7] The chimeric receptor according to [A8-5] or [A8-6], wherein the intracellular signaling domain comprises one or more costimulatory molecule signaling domains derived from costimulatory molecule(s) and a stimulatory molecule signaling domain derived from a stimulatory molecule.

[A8-8] The chimeric receptor according to [A8-5], wherein the intracellular signaling domain comprises one or more costimulatory molecule signaling domains derived from costimulatory molecule(s), a stimulatory molecule signaling domain derived from a stimulatory molecule, and an additional functional domain and/or motif.

[A8-9] The chimeric receptor according to any of [A8-1] to [A8-8], wherein the transmembrane domain is selected from the group consisting of a T cell receptor alpha chain, beta chain or zeta chain, CD28, CD3 epsilon, CD45, CD4, CD5, CD8, CD9, CD16, CD22, CD33, CD37, CD64, CD80, CD86, CD134, CD137 and CD154.

[A8-10] The chimeric receptor according to [A8-1], comprising an extracellular binding domain capable of recognizing a predetermined antigen via an antibody having a mutation in an Fc gamma receptor binding domain of CH2, the extracellular binding domain optionally comprising the amino acid sequences of SEQ ID NOs: 19 and 21.

[A8-11] The chimeric receptor according to [A8-1] or [A8-10], comprising a hinge domain and a transmembrane domain of the CD8 alpha of SEQ ID NO: 22.

[A8-12] The chimeric receptor according to any of [A8-1], [A8-10], and [A8-11], comprising the CD28 molecule of SEQ ID NO: 23.

[A8-13] The chimeric receptor according to any of [A8-1] and [A8-10] to [A8-12], comprising a costimulatory molecule signaling domain derived from the 4-1BB molecule of SEQ ID NO: 24.

[A8-14] The chimeric receptor according to any of [A8-1] and [A8-10] to [A8-13], comprising a stimulatory molecule signaling domain derived from the CD3 zeta molecule of SEQ ID NO: 25.

[A8-15] The chimeric receptor according to any of [A8-9], [A8-10], and [A8-12] to [A8-14], wherein the hinge domain and the transmembrane domain of the CD8 alpha comprises a CD8 alpha molecule amino acid sequence encoded by a sequence from nucleotides 1271 to 1519 (GenBank NM001768.6).

[A8-16] The chimeric receptor according to [A8-1], wherein the intracellular signaling domain comprises one or more costimulatory molecule signaling domains selected from the group consisting of cytoplasmic domains of CD28, CD2, CD4, CD5, CD8 alpha, CD8 beta, CD134, CD137, IL-2Rb, OX40, CD27, CDS, ICAM-1, LFA-1 (CD11a/CD18) and 4-1BB (CD137), and a binding domain of STAT3.

[A8-17] The chimeric receptor according to [A8-1], wherein the intracellular signaling domain comprises a human CD28 molecule amino acid sequence encoded by a sequence from nucleotides 760 to 882 (GenBank NM006139.2) and/or a 4-1BB molecule amino acid sequence encoded by a sequence from nucleotides 760 to 882 (GenBank NM006139.2).

[A8-18] The chimeric receptor according to [A8-1], wherein the intracellular signaling domain comprises one or more stimulatory molecule signaling domains selected from the group consisting of CD3 zeta, common FcR gamma (FCER1G), Fc gamma RIIa, FcR beta (Fc epsilon R1b), CD3 gamma, CD3 delta, CD3 epsilon, CD79a, CD79b, DAP10 and DAP12.

[A8-19] The chimeric receptor according to [A8-1], wherein the intracellular signaling domain comprises a CD3 zeta molecule amino acid sequence encoded by a sequence from nucleotides 299 to 637 (GenBank NM000734.3), a 2A peptide (F2A), and an eGFP molecule, and is positioned at the C terminus of the chimeric receptor.

[A8-20] The chimeric receptor according to [A8-1], comprising a portion or the whole of the amino acid sequence of SEQ ID NO: 17.

[A9-1] An isolated nucleic acid encoding a chimeric receptor according to any of [A9-1][A1-1] to [A1-7-2], [A2-1] to [A2-6-2], [A3-1] to [A3-2-3], [A4-1] to [A4-3-1], [A5-1] to [A5-6-1], [A6-1] to [A6-3-2], [A7-1] to [A7-2-2], and [A8-1] to [A8-20].

[A9-2] A vector comprising an isolated nucleic acid according to [A9-1].

[A9-3] The vector according to [A9-2], wherein the vector is operably linkable to at least one regulatory element for the expression of the chimeric receptor.

[A9-4] The vector according to [A9-2] or [A9-3], wherein the vector is selected from the group consisting of DNA, RNA, a plasmid, a lentivirus vector, an adenovirus vector, and a retrovirus vector.

[A9-5] A cell transformed or transduced with an isolated nucleic acid according to [A9-1] or a vector according to any of [A9-2] to [A9-4].

[A9-6] The cell according to [A9-5], wherein the cell is a T lymphocyte, an NK cell or a macrophage.

[A9-7] The cell according to [A9-5] or [A9-6], wherein the cell is a T lymphocyte whose expression of an endogenous T cell receptor is blocked or eliminated.

[A9-8] The cell according to any of [A9-5] to [A9-7], wherein the cell is a cell activated and/or grown *ex vivo.*

[A9-9] The cell according to any of [A9-5] to [A9-8], wherein the cell is a cell genetically engineered by retroviral transduction, lentiviral transduction, DNA electroporation and RNA electroporation, DNA or RNA transfection, or gene editing.

[A9-10] The chimeric receptor according to any of [A1-1] to [A1-7-2], [A2-1] to [A2-6-2], [A3-1] to [A3-2-3], [A4-1] to [A4-3-1], [A5-1] to [A5-6-1], [A6-1] to [A6-3-2], [A7-1] to [A7-2-2], and [A8-1] to [A8-20] wherein the mutated antibody is capable of binding to a tumor antigen.

[A10-1] A pharmaceutical composition for use in combination with administration of a mutated antibody having a mutation, including substitution, deletion, addition or modification, of at least one amino acid in a CH1 region, a CH2 region, a CH3 region, a CL region, or a framework region, wherein

the pharmaceutical composition comprises a cell expressing a chimeric receptor,
the chimeric receptor comprises an extracellular binding domain, a transmembrane domain and an intracellular signaling domain,
the mutated antibody is capable of binding to the extracellular binding domain of the chimeric receptor via a moiety having the mutation, and
the extracellular binding domain does not specifically bind to an antibody free of the mutation.

[A10-2] A pharmaceutical composition for use in combination with administration of a cell expressing a chimeric receptor, wherein

the pharmaceutical composition comprises a mutated antibody having a mutation, including substitution, deletion, addition or modification, of at least one amino acid in a CH1 region, a CH2 region, a CH3 region, a CL region, or a framework region,
the chimeric receptor comprises an extracellular binding domain, a transmembrane domain and an intracellular signaling domain,
the extracellular binding domain is capable of binding to the mutated antibody via a moiety having the mutation, and
the extracellular binding domain does not specifically bind to an antibody free of the mutation.

[A10-3] The pharmaceutical composition according to [A10-1] or [A10-2], wherein the chimeric receptor is a chimeric receptor according to any of [A1-1] to [A1-7-2], [A2-1] to [A2-6-2], [A3-1] to [A3-2-3], [A4-1] to [A4-3-1], [A5-1] to [A5-6-1], [A6-1] to [A6-3-2], [A7-1] to [A7-2-2], and [A8-1] to [A8-20].

[A10-4] The pharmaceutical composition according to any of [A10-1] to [A10-3], wherein the cell is a cell according to any of [A9-5] to [A9-9].

[A10-5] The pharmaceutical composition according to any of [A10-1] to [A10-4], wherein the mutated antibody has a prolonged half-life in blood or a high isoelectric point compared with a corresponding non-mutated antibody.

[A10-6] The pharmaceutical composition according to any of [A10-1] to [A10-5], wherein the mutated antibody has a mutation, including substitution, deletion, addition or modification, of at least one amino acid in a CH2 region, and the extracellular binding domain is capable of binding to the mutated antibody via a moiety having the mutation.

[A10-7] The pharmaceutical composition according to [A10-6], wherein the mutation in the CH2 region is a mutation at any of positions 231, 232, 233, 234, 235, 236, 237, 238, 239, 240, 264, 265, 266, 267, 269, 270, 295, 296, 297, 298, 299, 300, 325, 327, 328, 329, 330, 331 and 332 according to the EU numbering.

[A10-8] The pharmaceutical composition according to [A10-6] or [A10-7], wherein the CH2 region of the mutated antibody has a mutation selected from the group of 234A, 235A, and/or 297A, and the mutation positions are numbered according to the EU numbering.

[A10-9] The pharmaceutical composition according to any of [A10-6] or [A10-7], wherein the CH2 region of the mutated antibody has mutations of a combination selected from the group of

(1) 235R and 239K;
(2) 235R and 236R;
(3) 235R, 239K and 297A;
(4) 235R, 236R and 239K;
(5) 252Y and 434Y;
(6) 235R, 239K, 252Y and 434Y;
(7) 252Y, 434Y and 436V;
(8) 235R, 239K, 252Y, 434Y and 436V;
(9) 250V, 252Y, 307Q, 308P, 311A, 434Y and 436V; and
(10) 235R, 239K, 250V, 252Y, 307Q, 308P, 311A, 434Y and 436V

and the mutation positions are numbered according to the EU numbering.

[A10-10] The pharmaceutical composition according to any of [A10-6] to [A10-9], wherein the CH2 region of the mutated antibody comprises the amino acid sequence of SEQ ID NO: 3.

[A10-11] The pharmaceutical composition according to any of [A10-1] to [A10-5], wherein the mutated antibody has a mutation, including substitution, deletion, addition or modification, of at least one amino acid in a CH1 region, and the extracellular binding domain is capable of binding to the mutated antibody via a moiety having the mutation.

[A10-12] The pharmaceutical composition according to [A10-11], wherein the mutated antibody is a full-length antibody, Fab, or F(ab')$_2$.

[A10-13] The pharmaceutical composition according to any of [A10-10] to [A10-12], wherein the mutation in the CH1 region is a mutation at any of positions 131, 133, 137 and 138 according to the EU numbering.

[A10-14] The pharmaceutical composition according to any of [A10-10] to [A10-13], wherein the CH1 region of the mutated antibody has a mutation selected from the group of 131S, 133K, 220S, 137G and 138G, and the mutation positions are numbered according to the EU numbering.

[A10-16] The pharmaceutical composition according to any of [A10-1] to [A10-5], wherein the mutated antibody has a mutation, including substitution, deletion, addition or modification, of at least one amino acid in a CH3 region, and the extracellular binding domain is capable of binding to the mutated antibody via a moiety having the mutation.

[A10-17] The pharmaceutical composition according to [A10-16], wherein the mutation in the CH3 region is a mutation at any of positions 349, 351, 354, 356, 357, 366, 370, 394, 399, 405, 407, 409, 439, 446 and 447 according to the EU numbering.

[A10-18] The pharmaceutical composition according to [A10-16] or [A10-17], wherein the CH3 region of the mutated antibody has a mutation selected from the group of 397M, F or Y, 392D, E, T, V or I, 356K, 397F or Y, 439E, 366Y, 366W, 394S, 394W, 405A, 405W, 407T, and 407A, and the mutation positions are numbered according to the EU numbering.

[A10-19] The pharmaceutical composition according to [A10-16] or [A10-17], wherein the CH3 region of the mutated antibody has a mutation at any one of positions of a combination selected from the group of

(1) 356 and 439;
(2) 357 and 370;
(3) 399 and 409; and
(4) 399, 409, 356 and 439,

and the mutation positions are numbered according to the EU numbering.

[A10-20] The pharmaceutical composition according to any of [A10-16] to [A10-19], wherein terminal GK of CH3 of the mutated antibody is deleted.

[A10-21] The pharmaceutical composition according to any of [A10-1] to [A10-5], wherein the mutated antibody has a mutation, including substitution, deletion, addition or modification, of at least one amino acid in a CL region, and the extracellular binding domain is capable of binding to the mutated antibody via a moiety having the mutation.

[A10-22] The pharmaceutical composition according to [A10-21], wherein the mutated antibody is a full-length antibody, Fab, or F(ab')$_2$.

[A10-23] The pharmaceutical composition according to [A10-21] or [A10-22], wherein the mutation in the CL region is a mutation at any of positions 123, 131, 160 and 180 according to the EU numbering.

[A10-24] The pharmaceutical composition according to any of [A10-1] to [A10-5], wherein the mutated antibody has a mutation, including substitution, deletion, addition or modification, of at least one amino acid in CH2 and CH3 regions, and the extracellular binding domain is capable of binding to the mutated antibody via a moiety having the mutation.

[A10-25] The pharmaceutical composition according to [A10-24], wherein the CH2 and CH3 regions of the mutated antibody have a mutation selected from the group of 397M, F or Y, 392D, E, T, V or I, 356K, 397F or Y, 439E, 366Y, 366W, 394S, 394W, 405A, 405W, 407T, and 407A, and the mutation positions are numbered according to the EU numbering.

[A10-26] The pharmaceutical composition according to any of [A10-1] to [A10-5], wherein the mutated antibody has a mutation, including substitution, deletion, addition or modification, of at least one amino acid in a framework region, and the extracellular binding domain is capable of binding to the mutated antibody via a moiety having the mutation.

[A10-27] The pharmaceutical composition according to [A10-26], wherein the mutation in the framework region is a mutation at any of positions 10, 12, 23, 39, 43 and 105, each represented by its position according to the Kabat numbering.

[A10-28] The pharmaceutical composition according to any of [A10-1] to [A10-27], wherein the mutated antibody has a mutation in a hinge, and the position of the mutation is any of positions 220, 221, 222, 223, 224, 225, 226, 227, 228, 229, and 230 according to the EU numbering.

[A10-29] The pharmaceutical composition according to any of [A10-1] to [A10-28], wherein the hinge region of the mutated antibody comprises any mutation represented by 221K or Y, 222F, W, E or Y, 223F, W, E or K, 224F, W, E or Y, 225E, K or W, 227E, G, K or Y, 228E, G, K or Y, or 230A, E, G or Y.

[A10-30] The pharmaceutical composition according to any of [A10-1] to [A10-29], wherein the cell is derived from an allogeneic T lymphocyte, an allogeneic NK cell, or an allogeneic macrophage.

[A10-31] The pharmaceutical composition according to any of [A10-1] to [A10-30], wherein the cell is derived from an autologous T lymphocyte, an autologous NK cell or an autologous macrophage isolated from a recipient.

[A10-32] The pharmaceutical composition according to any of [A10-1] to [A10-31], wherein the mutated antibody further has a second mutation other than the mutation involved in binding to the extracellular binding domain.

[A10-33] The pharmaceutical composition according to [A10-32], wherein the mutated antibody having the second mutation, compared with a corresponding antibody free of the mutation has a prolonged half-life in blood or a high isoelectric point.

[A10-34] The pharmaceutical composition according to any of [A10-1] to [A10-33] for use in treatment or prevention through the antibody-dependent cellular cytotoxicity (ADCC) of a T lymphocyte or an NK cell or the antibody-dependent cellular phagocytosis (ADCP) of a macrophage in a recipient.

[A10-35] The pharmaceutical composition according to any of [A10-1] to [A10-34], wherein the mutated antibody is capable of binding to a tumor antigen.

[A10-36] The pharmaceutical composition according to any of [A10-1] to [A10-35] for use in the treatment or prevention of a cancer.

[A10-37] The pharmaceutical composition according to [A10-36], wherein the cancer is selected from the group consisting of carcinoma, lymphoma, sarcoma, blastoma and leukemia.

[A10-38] The pharmaceutical composition according to [A10-36], wherein the cancer is selected from the group consisting of B-lineage acute lymphoblastic leukemia, B-cell chronic lymphocytic leukemia, B-cell non-Hodgkin's lymphoma, breast cancer, stomach cancer, neuroblastoma, osteosarcoma, lung cancer, melanoma, prostate cancer, colon cancer, renal cell cancer, ovary cancer, rhabdomyosarcoma, leukemia and Hodgkin's lymphoma.

[A10-39] The pharmaceutical composition according to any of [A10-1] to [A10-37] for use in adoptive cell immunotherapy, adoptive T cell immunotherapy, or CAR-T therapy.

[A11-1] The pharmaceutical composition according to any of [A10-1] to [A10-39], wherein the extracellular binding domain of the chimeric receptor is an extracellular binding domain whose binding activity against the mutated antibody varies according to a concentration of a compound specific for a target tissue.

[A11-2] The pharmaceutical composition according to [A11-1], wherein the target tissue is a cancer tissue.

[A11-3] The pharmaceutical composition according to [A11-2], wherein the compound specific for the cancer tissue is a cancer cell-specific metabolite, a metabolite specific to immunocytes infiltrating into the cancer tissue, or a metabolite specific to stromal cells of the cancer tissue.

[A11-4] The pharmaceutical composition according to [A11-1], wherein the target tissue is an inflammatory tissue.

[A11-5] The pharmaceutical composition according to [A11-4], wherein the compound specific for the inflammatory tissue is a metabolite specific to immunocytes infiltrating into the inflammatory tissue, or a metabolite specific to normal cells damaged in the inflammatory tissue.

[A11-6] The pharmaceutical composition according to any of [A11-1] to [A11-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[A11-7] The pharmaceutical composition according to any of [A11-1] to [A11-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[A12-1] The chimeric receptor according to any of [A1-1] to [A1-7-2], [A2-1] to [A2-6-2], [A3-1] to [A3-2-3], [A4-1] to [A4-3-1], [A5-1] to [A5-6-1], [A6-1] to [A6-3-2], [A7-1] to [A7-2-2], and [A8-1] to [A8-20] wherein the extracellular binding domain is an extracellular binding domain whose binding activity against the mutated antibody varies according to a concentration of a compound specific for a target tissue.

[A12-2] The chimeric receptor according to [A12-1], wherein the target tissue is a cancer tissue.

[A12-3] The chimeric receptor according to [A12-2], wherein the compound specific for the cancer tissue is a cancer cell-specific metabolite, a metabolite specific to immunocytes infiltrating into the cancer tissue, or a metabolite specific to stromal cells of the cancer tissue.

[A12-4] The chimeric receptor according to [A12-1], wherein the target tissue is an inflammatory tissue.

[A12-5] The chimeric receptor according to [A12-4], wherein the compound specific for the inflammatory tissue is a metabolite specific to immunocytes infiltrating into the inflammatory tissue, or a metabolite specific to normal cells damaged in the inflammatory tissue.

[A12-6] The chimeric receptor according to any of [A12-1] to [A12-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[A12-7] The chimeric receptor according to any of [A12-1] to [A12-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[A13-1] The pharmaceutical composition according to any of [A10-1] to [A10-39], wherein the mutated antibody is an antibody whose binding activity against an antigen varies according to a concentration of a compound specific for a target tissue.

[A13-2] The pharmaceutical composition according to [A13-1], wherein the target tissue is a cancer tissue.

[A13-3] The pharmaceutical composition according to [A13-2], wherein the compound specific for the cancer tissue is a cancer cell-specific metabolite, a metabolite specific to immunocytes infiltrating into the cancer tissue, or a metabolite specific to stromal cells of the cancer tissue.

[A13-4] The pharmaceutical composition according to [A13-1], wherein the target tissue is an inflammatory tissue.

[A13-5] The pharmaceutical composition according to [A13-4], wherein the compound specific for the inflammatory tissue is a metabolite specific to immunocytes infiltrating into the inflammatory tissue, or a metabolite specific to normal cells damaged in the inflammatory tissue.

[A13-6] The pharmaceutical composition according to any of [A13-1] to [A13-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[A13-7] The pharmaceutical composition according to any of [A13-1] to [A13-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[A14-1] The chimeric receptor according to any of [A1-1] to [A1-7-2], [A2-1] to [A2-6-2], [A3-1] to [A3-2-3], [A4-1] to [A4-3-1], [A5-1] to [A5-6-1], [A6-1] to [A6-3-2], [A7-1] to [A7-2-2], and [A8-1] to [A8-20] wherein the mutated antibody is an antibody whose binding activity against an antigen varies according to a concentration of a compound specific for a target tissue.

[A14-2] The chimeric receptor according to [A14-1], wherein the target tissue is a cancer tissue.

[A14-3] The chimeric receptor according to [A14-2], wherein the compound specific for the cancer tissue is a cancer cell-specific metabolite, a metabolite specific to immunocytes infiltrating into the cancer tissue, or a metabolite specific to stromal cells of the cancer tissue.

[A14-4] The chimeric receptor according to [A14-1], wherein the target tissue is an inflammatory tissue.

[A14-5] The chimeric receptor according to [A14-4], wherein the compound specific for the inflammatory tissue is a metabolite specific to immunocytes infiltrating into the inflammatory tissue, or a metabolite specific to normal cells damaged in the inflammatory tissue.

[A14-6] The chimeric receptor according to any of [A14-1] to [A14-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[A14-7] The chimeric receptor according to any of [A14-1] to [A14-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[B1-1] A bispecific antibody comprising (1) a domain comprising antibody variable regions capable of specifically binding to a mutated antibody via a moiety having a mutation, and (2) a domain comprising antibody variable regions having binding activity against a molecule expressed on T cell surface, wherein the domain (1) is capable of specifically binding to a mutated antibody having a mutation, including substitution, deletion, addition or modification, of at least one amino acid in a CH2 region, via a moiety having the mutation, and does not specifically bind to an antibody free of the mutation.

[B1-2] The bispecific antibody according to [B1-1], wherein the mutated antibody does not increase the occurrence of intercellular bridge with other immunocytes compared with a corresponding non-mutated antibody.

[B1-3] The bispecific antibody according to [B1-1] or [B1-2], wherein the mutated antibody is an antibody having reduced binding activity against any Fcγ receptor of FcγI, FcγIIA, FcγIIB, FcγIIIA and FcγIIIB as compared with a corresponding non-mutated antibody.

[B1-3-1] The bispecific antibody according to any of [B1-1] to [B1-3], wherein the mutation comprises mutations at one or more positions selected from the group of 231, 232, 233, 234, 235, 236, 237, 238, 239, 240, 264, 265, 266, 267, 269, 270, 295, 296, 297, 298, 299, 300, 325, 327, 328, 329, 330, 331 and 332, each represented by its position according to the EU numbering, and the domain (1) is capable of binding to the mutated antibody via a moiety having the mutations.

[B1-3-2] The bispecific antibody according to any of [B1-1] to [B1-3], wherein the mutation has one or more mutations selected from the group of 234A, 235A, and/or 297A, each represented by its position according to the EU numbering and the amino acid introduced by the mutation, and the domain (1) is capable of binding to the mutated antibody via a moiety having the mutations.

[B1-3-3] The bispecific antibody according to any of [B1-1] to [B1-3], wherein the mutation comprises one or more mutations selected from the group of 349C, 356C, 366W or S, 368A, and 407V, each represented by its position according to the EU numbering and the amino acid introduced by the mutation, and the domain (1) is capable of binding to the mutated antibody via a moiety having the mutations.

[B1-3-4] The bispecific antibody according to any of [B1-1] to [B1-3], wherein the mutation comprises mutations of one or more combinations selected from the following combinations:

(1) 235R and 239K;
(2) 235R and 236R;
(3) 235R, 239K and 297A;
(4) 235R, 236R and 239K;
(5) 252Y and 434Y;
(6) 235R, 239K, 252Y and 434Y;
(7) 252Y, 434Y and 436V;
(8) 235R, 239K, 252Y, 434Y and 436V;
(9) 250V, 252Y, 307Q, 308P, 311A, 434Y and 436V; and
(10) 235R, 239K, 250V, 252Y, 307Q, 308P, 311A, 434Y and 436V

each represented by its position according to the EU numbering and the amino acid introduced by the mutation, and the domain (1) is capable of binding to the mutated antibody via a moiety having the mutations.

[B1-3-5] The bispecific antibody according to any of [B1-1] to [B1-3], wherein the mutation has mutations at one or more positions selected from the group of 235, 236, and 239, each represented by its position according to the EU numbering, and the domain (1) is capable of binding to the mutated antibody via a moiety having the mutations.

[B1-4] The bispecific antibody according to [B1-1], wherein the mutated antibody is an antibody having enhanced binding activity against FcγRIa as compared with a corresponding non-mutated antibody.

[B1-4-1] The bispecific antibody according to [B1-1] or [B1-4], wherein the mutation comprises mutations at one or more positions selected from the group of 234, 235, 236, 237, 238, 265, 266, 267, 268, 269, 270, 271, 295, 296, 298, 300, 324, 325, 326, 327, 328, 329, 330, 331, 332, 333, 334, 335, 336 and 337, each represented by its position according to the EU numbering, and the domain (1) is capable of binding to the mutated antibody via a moiety having the mutations.

[B1-5] The bispecific antibody according to [B1-1], wherein the mutated antibody is an antibody having enhanced binding activity against any Fcγ receptor of FcγI, FcγIIA, FcγIIB, FcγIIIA and FcγIIIB as compared with a corresponding non-mutated antibody.

[B1-5-1] The bispecific antibody according to [B1-1] or [B1-5], wherein the mutation comprises mutations at one or more positions selected from the group of 231, 232, 233, 234, 235, 236, 237, 238, 239, 240, 265, 266, 267, 268, 269, 270, 271, 295, 296, 298, 300, 324, 325, 326, 327, 328, 329, 330, 331, 332, 333, 334, 335, 336 and 337, each represented by its position according to the EU numbering, and the domain (1) is capable of binding to the mutated antibody via a moiety having the mutations.

[B1-5-2] The bispecific antibody according to [B1-1] or [B1-5], wherein the mutation comprises mutations of one or more combinations selected from the following combinations:

    (1) 234Y, 235Y, 236W, 268D, 270E and 298A;
    (2) 234Y, 235Q, 236W, 239M, 268D, 270E and 298A; (3) 234Y, 235Q, 236W, 239M, 268D, 270E and 298A;
    (4) 234Y, 235Y, 236W, 268D, 298A and 327D;
    (5) 234Y, 235Y, 236W, 239M, 268D, 298A and 327D;
    (6) 234Y, 235Y, 236W, 239M, 268D, 298A, 327D, 328W and 334L;
    (7) 326D, 330M and 334E;
    (8) 270E, 326D, 330M and 334E; and
    (9) 270E, 326D, 330K and 334E

each represented by its position according to the EU numbering and the amino acid introduced by the mutation, and the domain (1) is capable of binding to the mutated antibody via a moiety having the mutations.

[B1-5-3] The bispecific antibody according to [B1-1] or [B1-5], wherein the mutation comprises mutations of one or more combinations selected from the following combinations:

    (1) 234L, S, F, E, V, D, Q, I, M, T, A, G or H, 235Y or Q, 236W, 239M or I, 268D, and 298A;
    (2) 234L, S, F, E, V, D, Q, I, M, T, A, G or H, 235Y or Q, 236W, 239M or I, 268D, 298A and 327D;
    (3) 234F, E, D, S or L, 235Y or Q, 236W, 239M or I, 268D, 298A and 327D;
    (4) 270E, 326D, 330A, K, M, F, I, Y or H, and 334E;
    (5) 270E, 326D, 330A, K, M, F, I, Y or H, and 334E;
    (6) 270E, 326D, 330A, F or K, and 334E; and
    (7) 234L, S, F, E, V, D, Q, I, M, T, A, G or H, 235Y or Q, 236W, 239M or I, 268D, 270E, and, 298A

each represented by its position according to the EU numbering and the amino acid introduced by the mutation, and the domain (1) is capable of binding to the mutated antibody via a moiety having the mutations.

[B1-6] The bispecific antibody according to [B1-1], wherein the mutated antibody is an antibody having maintained or decreased binding activity against both H and R forms which are gene polymorphisms of FcγRIIa, and enhanced binding activity against FcγRIIb as compared with a corresponding non-mutated antibody.

[B1-6-1] The bispecific antibody according to [B1-1] or [B1-6], wherein the mutation comprises mutations at one or more positions selected from the group of 233, 234, 237, 238, 239, 267, 268, 296, 271, 323, 326, and 330, each represented by its position according to the EU numbering, and the domain (1) is capable of binding to the mutated antibody via a moiety having the mutations.

[B1-6-2] The bispecific antibody according to [B1-1] or [B1-6], wherein the mutation comprises one or more mutations selected from the group of 238D, 328E, 237W, 267V, 267Q, 268N, 271G, 326M, 239D, 267A, 234W, 237A, 237D, 237E, 237L, 237M, 237Y, 330K, 330R, 233D, 268D, 268E, 326D, 326S, 326T, 3231, 323L, 323M, 296D, 326A, 326N, and 330M, each represented by its position according to the EU numbering and the amino acid introduced by the mutation, and the domain (1) is capable of binding to the mutated antibody via a moiety having the mutations.

[B1-6-3] The bispecific antibody according to [B1-1] or [B1-6], wherein the mutation comprises mutations at positions of one or more combinations selected from the following combinations:

    (1) 238, 233, 237, 268, 271, 296 and 330;
    (2) 238, 237, 268, 271, 296 and 330;
    (3) 238, 233, 237, 268, 271, 296, 330 and 332;
    (4) 238, 233, 237, 264, 267, 268, 271 and 330;
    (5) 238, 233, 237, 267, 268, 271, 296, 330 and 332;
    (6) 238, 237, 267, 268, 271, 296, 330 and 332;
    (7) 238, 233, 237, 268, 271, 296, 327 and 330;
    (8) 238, 233, 237, 264, 267, 268 and 271;
    (9) 238, 233, 237, 264, 267, 268, 271, 296 and 330;
    (10) 238, 233, 237, 264, 267, 268, 271, 296, 330 and 396;

(11) 238, 237, 264, 267, 268, 271 and 330;
(12) 238, 237, 264, 267, 268, 271, 296 and 330;
(13) 238, 264, 267, 268 and 271;
(14) 238, 264, 267, 268, 271 and 296;
(15) 238, 237, 267, 268, 271, 296 and 330;
(16) 238, 233, 237, 264, 267, 268, 271, 330 and 396;
(17) 238, 233, 237, 264, 267, 268, 271, 296, 327, 330 and 396;
(18) 238, 233, 237, 264, 267, 268, 271, 272 and 296;
(19) 238, 237, 264, 267, 268, 271, 272 and 330;
(20) 238, 237, 264, 267, 268, 271, 272, 296 and 330;
(21) 238, 233, 264, 267, 268 and 271;
(22) 238, 237, 267, 268, 271, 296 and 330;
(23) 238, 264, 267, 268, 271, 272 and 296; and
(24) 238, 233, 264, 267, 268, 271 and 296

each represented by its position according to the EU numbering, and the domain (1) is capable of binding to the mutated antibody via a moiety having the mutations.

[B1-6-4] The bispecific antibody according to [B1-1] or [B1-6], wherein the mutation comprises a mutation at position 238 and comprises mutations at one or more positions selected from 235, 237, 241, 268, 295, 296, 298, 323, 324 and 330, each represented by its position according to the EU numbering, wherein the mutations decrease binding activity against every active FcyR, and wherein the domain (1) is capable of binding to the mutated antibody via a moiety having the mutations.

[B1-6-5] The bispecific antibody according to [B1-1] or [B1-6], wherein the mutation comprises a mutation at position 238 and comprises mutations at positions of one or more combinations selected from the group consisting of (1) 241, 268, 296 and 324; (2) 237, 241, 296 and 330; and (3) 235, 237, 241 and 296, each represented by its position according to the EU numbering, wherein the mutations decrease binding activity against every active FcyR, and wherein the domain (1) is capable of binding to the mutated antibody via a moiety having the mutations.

[B1-6-6] The bispecific antibody according to [B1-1] or [B1-6], wherein the mutation comprises mutations at positions 238 and 271 and comprises mutations at two or more positions selected from 234, 235, 236, 237, 239, 265, 267 and 297, each represented by its position according to the EU numbering, wherein the mutations decrease binding activity against every active FcyR, and wherein the domain (1) is capable of binding to the mutated antibody via a moiety having the mutations.

[B1-6-7] The bispecific antibody according to [B1-1] or [B1-6], wherein the mutation comprises mutations at positions of any combination selected from the group consisting of (1) 233, 238, 264, 267, 268 and 271; (2) 233, 237, 238, 264, 267, 268, 271, 296, 297, 330 and 396; and (3) 233, 238, 264, 267, 268, 271 and 296, each represented by its position according to the EU numbering, wherein the mutations decrease binding activity against every active FcyR, and wherein the domain (1) is capable of binding to the mutated antibody via a moiety having the mutations.

[B1-6-8] The bispecific antibody according to [B1-1] or [B1-6], wherein the mutation comprises 238D and comprises one or more mutations selected from 235F, 237Q or D, 241M or L, 268P, 295M or V, 296E, H, N or D, 298A or M, 3231, 324N or H, and 330H or Y, each represented by its position according to the EU numbering and the amino acid introduced by the mutation, wherein the mutations decrease binding activity against every active FcyR, and wherein the domain (1) is capable of binding to the mutated antibody via a moiety having the mutations.

[B1-6-9] The bispecific antibody according to [B1-1] or [B1-6], wherein the mutation comprises mutation 238D and comprises mutations of one or more combinations selected from the group consisting of (1) 241M, 268P, 296E and 324H; (2) 237Q or D, 241M, 296E and 330H; and (3) 235F, 237Q or D, 241M and 296E, each represented by its position according to the EU numbering and the amino acid introduced by the mutation, wherein the mutations decrease binding activity against every active FcyR, and wherein the domain (1) is capable of binding to the mutated antibody via a moiety having the mutations.

[B1-6-10] The bispecific antibody according to [B1-1] or [B1-6], wherein the mutation comprises mutations 238D and 271G and comprises one or more mutations selected from 234A, H, N, K or R, 235A, 236Q, 237R or K, 239K, 265K, N, R, S or V, 267K, R or Y, and 297A, each represented by its position according to the EU numbering and the amino acid introduced by the mutation, wherein the mutations decrease binding activity against every active FcγR, and wherein the domain (1) is capable of binding to the mutated antibody via a moiety having the mutations.

[B1-6-11] The bispecific antibody according to [B1-1] or [B1-6], wherein the mutation comprises mutations 238D and 271G and comprises mutations of one or more combinations selected from the group of (1) 233D, 238D, 2641, 267R, 268E and 271G; (2) 233D, 237D, 238D, 2641, 267A, 268E, 271G, 296D, 297A, 330R and 396M; (3) 233D, 238D, 2641, 267R, 268P, 271G and 296E, each represented by its position according to the EU numbering and the amino acid introduced by the mutation, wherein the mutations decrease binding activity against every active FcyR, and wherein the domain (1) is capable of binding to the mutated antibody via a moiety having the mutations.

[B1-6-12] The bispecific antibody according to any of [B1-6-1] to [B1-6-11], wherein the mutated antibody is an antibody further having decreased binding to a complement.

[B1-6-13] The bispecific antibody according to [B1-1] or [B1-2], wherein the mutation comprises mutations at one or more positions selected from the group of 322, 327, 330 and 331, each represented by its position according to the EU numbering, wherein the mutations decrease binding to a complement, and wherein the domain (1) is capable of binding to the mutated antibody via a moiety having the mutations.

[B1-6-14] The bispecific antibody according to [B1-1] or [B1-2], wherein the mutation comprises one or more mutations selected from the group of 322A or E, 327G, 330S and 331S, each represented by its position according to the EU numbering and the amino acid introduced by the mutation, and the domain (1) is capable of binding to the mutated antibody via a moiety having the mutations.

[B1-6-15] The bispecific antibody according to [B1-1] or [B1-2], wherein the mutation comprises mutations at one or more positions selected from the group of 238, 271, 327, 330, and 331, each represented by its position according to the EU numbering, wherein the mutated antibody maintains binding activity against FcγRIIb as compared with a non-mutated antibody having a natural IgG Fc region and the mutations decrease binding activity against every active FcγR, and wherein the domain (1) is capable of binding to the mutated antibody via a moiety having the mutations.

[B1-6-16] The bispecific antibody according to [B1-1] or [B1-6], wherein the mutation comprises one or more mutations selected from the group of 238D, 271G, 327G, 330S and 331S, each represented by its position according to the EU numbering and the amino acid introduced by the mutation, and the domain (1) is capable of binding to the mutated antibody via a moiety having the mutations.

[B1-6-17] The bispecific antibody according to [B 1-6-14], wherein the mutated antibody further has one or more mutations selected from the group of 233D, 237D, 2641, 267A, and 268D or E, and the domain (1) is capable of binding to the mutated antibody via a moiety having the mutations.

[B1-6-18] The bispecific antibody according to [B1-1] or [B 1-6], wherein the mutation comprises mutations 238D and 271G and comprises mutations of one or more combinations selected from the group consisting of (1) 237D, 238D, 268D or E, 271G, 327G, 330S and 331S; (2) 233D, 237D, 238D, 268D, 271G, 327G, 330S and 331S; (3) 238D, 267A, 268E, 271G, 327G, 330S and 331S; (4) 238D, 2641, 267A, 271G, 327G, 330S and 331S, each represented by its position according to the EU numbering and the amino acid introduced by the mutation, and the domain (1) is capable of binding to the mutated antibody via a moiety having the mutations.

[B1-6-19] The bispecific antibody according to [B1-1] or [B 1-6], wherein the mutation comprises mutations at one or more positions selected from the group of 233, 238, 264, 267, 268, 271, 327, 330 and 331, each represented by its position according to the EU numbering, and the domain (1) is capable of binding to the mutated antibody via a moiety having the mutations.

[B 1-6-20] The bispecific antibody according to [B1-1] or [B 1-6], wherein the mutation comprises mutations at positions of one or more combinations selected from (1)237, 238, 268, 271, 327, 330 and 331; (2) 233, 237, 238, 268, 271, 327, 330 and 331; (3) 238, 267, 268, 271, 327, 330 and 331; (4) 238, 264, 267, 271, 327, 330 and 331, each represented by its position according to the EU numbering, and the domain (1) is capable of binding to the mutated antibody via a moiety having the mutations.

[B1-7] The bispecific antibody according to [B1-1], wherein the mutated antibody is an antibody whose binding activity against an antigen varies depending on ion concentration conditions as compared with a corresponding non-mutated antibody, wherein the antibody has binding activity against FcRn under pH neutral conditions, but does not form a heterocomplex comprising two molecules of FcRn and one molecule of active Fcγ receptor under pH neutral conditions.

[B1-7-1] The bispecific antibody according to [B1-1] or [B1-7], wherein the mutation comprises mutations at one or more positions selected from the group of 235, 237, 238, 239, 270, 298, 325 and 329, each represented by its position according to the EU numbering, and the domain (1) is capable of binding to the mutated antibody via a moiety having the mutations.

[B1-7-2] The bispecific antibody according to [B1-1] or [B1-7], wherein the mutation comprises one or more mutations selected from the group of 235K or R, 237K or R, 238K or R, 239L or R, 270F, 298G, 325G, and 329K or R, each represented by its position according to the EU numbering and the amino acid introduced by the mutation, and the domain (1) is capable of binding to the mutated antibody via a moiety having the mutations.

[B2-1] A bispecific antibody comprising (1) a domain comprising antibody variable regions capable of specifically binding to a mutated antibody via a moiety having a mutation, and (2) a domain comprising antibody variable regions having binding activity against a molecule expressed on T cell surface, wherein the domain (1) is capable of specifically binding to a mutated antibody having a mutation, including substitution, deletion, addition or modification, of at least one amino acid in a CH3 region, via a moiety having the mutation, and does not specifically bind to an antibody free of the mutation.

[B2-2] The bispecific antibody according to [B2-1], wherein the mutated antibody improves the stability, homogeneity, immunogenicity, safety, production efficiency and/or circulation time in plasma of the mutated antibody, compared with a corresponding non-mutated antibody.

[B2-3] The bispecific antibody according to [B2-1] or [B2-2], wherein the mutated antibody is an antibody lacking an amino acid at any of positions 446 and 447 according to the EU numbering.

[B2-3-1] The bispecific antibody according to any of [B2-1] to [B2-3], wherein the mutation further comprises a mutation at position 434 represented by its position according to the EU numbering, and the domain (1) is capable of binding to the mutated antibody via the mutation.

[B2-3-2] The bispecific antibody according to any of [B2-1] to [B2-3], wherein the mutation further comprises mutations at one or more positions selected from the group of 131, 133, 137, 138, 219, 268, 330, 331, 335, 339, 397, and 419, each represented by its position according to the EU numbering, and the domain (1) is capable of binding to the mutated antibody via a moiety having the mutations.

[B2-3-3] The bispecific antibody according to any of [B2-1] to [B2-3], wherein the mutation further comprises mutations at one or more positions selected from the group of 131, 133, 137, 138, 214, 217, 219, 220, 221, 222, 233, 234, 235, 236, and 409 according to the EU numbering, and the domain (1) is capable of binding to the mutated antibody via a moiety having the mutations.

[B2-4] The bispecific antibody according to [B2-1] or [B2-2], wherein the mutated antibody is an antibody having a mutation introduced in an amino acid residue at an interface such that two or more amino acid residues forming the interface have the same charge.

[B2-4-1] The bispecific antibody according to [B2-1], [B2-2] or [B2-4], wherein the mutated antibody is an antibody comprising two or more heavy chain CH3 regions and having a mutation such that amino acid residues of the first heavy chain have the same charge, wherein the mutation comprises a mutation at any one of positions of each of one or more combinations selected from the following combinations:

> (1) 356 and 439;
> (2) 357 and 370; and
> (3) 399 and 409

each represented by its position according to the EU numbering, and wherein the domain (1) is capable of binding to the mutated antibody via a moiety having the mutations.

[B2-4-2] The bispecific antibody according to [B2-1], [B2-2] or [B2-4], wherein the mutation comprises mutations at one or more positions selected from the group of 356, 357, 370, 399, 409, and 439, each represented by its position according to the EU numbering, and the domain (1) is capable of binding to the mutated antibody via a moiety having the mutations.

[B2-4-3] The bispecific antibody according to [B2-4-1] or [B2-4-2], wherein the mutation further comprises mutations at one or more positions selected from the group of amino acid residues 10, 12, 23, 39, 43 and 105 according to the Kabat numbering in the heavy chain variable region or amino acid residues 137, 196, 203, 214, 217, 233, 268, 274, 276 and 297 according to the EU numbering in the heavy chain constant region, and the domain (1) is capable of binding to the mutated antibody via a moiety having the mutations.

[B2-5] The bispecific antibody according to [B2-1] or [B2-2], wherein the mutated antibody is an antibody promoting polypeptide heteromerization under reductive conditions as compared with a corresponding non-mutated antibody.

[B2-5-1] The bispecific antibody according to [B2-1], [B2-2] or [B2-5], wherein the mutated antibody is an antibody comprising two or more heavy chain CH3 regions and having a mutation such that amino acid residues of the first heavy chain have the same charge, wherein the mutation comprises a mutation at any one of positions of each of one or more combinations selected from the following combinations:

> (1) 356 and 439; (2) 357 and 370; (3) 399 and 409; and
> (4) 399, 409, 356 and 439

each represented by its position according to the EU numbering, and wherein the domain (1) is capable of binding to the mutated antibody via a moiety having the mutations.

[B2-5-2] The bispecific antibody according to [B2-1], [B2-2] or [B2-5], wherein the mutation comprises one or more mutations selected from the group of 397M, F or Y, 392D, E, T, V or I, 356K, 397F or Y, and 439E, each represented by its position according to the EU numbering and the amino acid introduced by the mutation, and the domain (1) is capable of binding to the mutated antibody via a moiety having the mutations.

[B2-5-3] The bispecific antibody according to [B2-1], [B2-2], [B2-5] or [B2-5-1], wherein the mutated antibody is an antibody comprising two or more heavy chain CH3 regions, wherein at least one amino acid residue at any of positions 349, 351, 354, 356, 394 and 407 (according to the EU numbering) is cysteine, and wherein the domain (1) is capable of binding to the mutated antibody via a moiety having the mutations.

[B2-5-4] The bispecific antibody according to [B2-1], [B2-2] or [B2-5], wherein the mutated antibody has a mutation at EU numbering position 226 and/or 229 or lacks a core hinge region, and the domain (1) is capable of binding to the mutated antibody via a moiety having the mutations.

[B2-5-5] The bispecific antibody according to [B2-5-4], wherein the mutated antibody is an antibody further having the substitution of positions 220 to 225 (according to the EU numbering) by Y-G-P-P or lacking positions 219 to 229 (according to the EU numbering).

[B2-6] The bispecific antibody according to [B2-1] or [B2-2], wherein the mutated antibody is an antibody comprising a first polypeptide and a second polypeptide in contact at their boundary moieties, wherein the first polypeptide has, at the boundary moiety, a knob capable of being positioned in a hole of the boundary moiety of the second polypeptide.

[B2-6-1] The bispecific antibody according to [B2-1], [B2-2] or [B2-6], wherein the mutated antibody is an antibody comprising two or more heavy chain CH3 regions and has mutations at one or more positions selected from the group of 366, 394, 405, and 407 according to the EU numbering, and the domain (1) is capable of binding to the mutated antibody via a moiety having the mutations.

[B2-6-2] The bispecific antibody according to [B2-1], [B2-2] or [B2-6], wherein the mutated antibody is an antibody comprising two or more heavy chain CH3 regions, wherein mutations in the first and second heavy chain CH3 regions comprise mutations of one or more combinations selected from the following combinations:

(1) 366Y in the first heavy chain CH3 region and 407T in the second heavy chain CH3 region;
(2) 366W in the first heavy chain CH3 region and 497A in the second heavy chain CH3 region;
(3) 405A in the first heavy chain CH3 region and 394W in the second heavy chain CH3 region;
(4) 407T in the first heavy chain CH3 region and 366Y in the second heavy chain CH3 region;
(5) 366Y and 405A in the first heavy chain CH3 region, and 394W and 407T in the second heavy chain CH3 region;
(6) 366W and 405W in the first heavy chain CH3 region, and 394S and 407A in the second heavy chain CH3 region;
(7) 405W and 407A in the first heavy chain CH3 region, and 366W and 394S in the second heavy chain CH3 region; and
(8) 405W in the first heavy chain CH3 region and 394S in the second heavy chain CH3 region,

each represented by its position according to the EU numbering and the amino acid introduced by the mutation, and wherein the domain (1) is capable of binding to the mutated antibody via a moiety having the mutations.

[B3-1] A bispecific antibody comprising (1) a domain comprising antibody variable regions capable of specifically binding to a mutated antibody via a moiety having a mutation, and (2) a domain comprising antibody variable regions having binding activity against a molecule expressed on T cell surface, wherein the domain (1) is capable of specifically binding to a mutated antibody having a mutation, including substitution, deletion, addition or modification, of at least one amino acid in a CH1 region, via a moiety having the mutation, and does not specifically bind to an antibody free of the mutation.

[B3-2] The bispecific antibody according to [B3-1], wherein the mutated antibody is an antibody having improved pharmacokinetics and/or hinge region heterogeneity as compared with a corresponding non-mutated antibody.

[B3-2-1] The bispecific antibody according to [B3-1] or [B3-2], wherein the mutation comprises mutations at one or more positions selected from the group of 131, 133, 137, and 138, each represented by its position according to the EU numbering, and the domain (1) is capable of binding to the mutated antibody via a moiety having the mutations.

[B3-2-2] The bispecific antibody according to [B3-1] or [B3-2], wherein the mutation comprises one or more mutations selected from the group of 131S, 133K, 220S, 137G, 138G, 268Q, 355Q and 419E, each represented by its position according to the EU numbering and the amino acid introduced by the mutation, and the domain (1) is capable of binding to the mutated antibody via a moiety having the mutations.

[B3-2-3] The bispecific antibody according to [B3-2-1] or [B3-2-2], wherein the mutation further comprises mutations at one or more positions selected from the group of 220, 268, 330, 331, 339, 355, 419, 446, and 447, each represented by its position according to the EU numbering, and the domain (1) is capable of binding to the mutated antibody via a moiety having the mutations.

[B4-1] A bispecific antibody comprising (1) a domain comprising antibody variable regions capable of specifically binding to a mutated antibody via a moiety having a mutation, and (2) a domain comprising antibody variable regions having binding activity against a molecule expressed on T cell surface, wherein the domain (1) is capable of specifically binding to a mutated antibody having a mutation, including substitution, deletion, addition or modification, of at least one amino acid in a hinge region, via a moiety having the mutation, and does not specifically bind to an antibody free of the mutation.

[B4-2] The bispecific antibody according to [B4-1], wherein the mutated antibody is an antibody having enhanced binding activity against any Fcγ receptor of FcγI, FcγIIA, FcγIIB, FcγIIIA and FcγIIIB as compared with a corresponding non-mutated antibody.

[B4-2-1] The bispecific antibody according to [B4-1] or [B4-2], wherein the mutation comprises mutations at one or more positions selected from the group of 220, 221, 222, 223, 224, 225, 226, 227, 228, 229, and 230, each represented by its position according to the EU numbering, and the domain (1) is capable of binding to the mutated antibody via a moiety having the mutations.

[B4-2-2] The bispecific antibody according to [B4-2-1], wherein the mutation further comprises mutations at one or more positions selected from the group of 231, 232, 233, 234, 235, 236, 237, 238, 239, 240, 264, 265, 266, 267, 269, 270, 295, 296, 297, 298, 299, 300, 325, 327, 328, 329, 330, 331, and 332, each represented by its position according to the EU numbering, and the domain (1) is capable of binding to the mutated antibody via a moiety having the mutations.

[B4-2-3] The bispecific antibody according to [B4-1] or [B4-2], wherein the mutation comprises one or more mutations selected from the group of 235R, 239K and 297A, each represented by its position according to the EU numbering and the amino acid introduced by the mutation, and the domain (1) is capable of binding to the mutated antibody via a moiety having the mutations.

[B4-2-4] The bispecific antibody according to any of [B4-2-1] to [B4-2-3], wherein the mutation further comprises mutations at one or more positions selected from the group of 231, 232, 233, 234, 235, 236, 237, 238, 239, 240, 241, 243, 244, 245, 246, 247, 249, 250, 251, 252, 254, 255, 256, 257, 258, 260, 262, 263, 264, 265, 266, 267, 268, 269, 270, 271, 272, 273, 274, 275, 276, 278, 279, 280, 281, 282, 283, 284, 285, 286, 288, 290, 291, 292, 293, 294, 295, 296, 297, 298, 299, 300, 301, 302, 303, 304, 305, 307, 308, 309, 311, 312, 313, 314, 315, 316, 317, 318, 320, 322, 323, 324, 325, 326, 327, 328, 329, 330, 331, 332, 333, 334, 335, 336, 337, 339, 341, 343, 375, 376, 377, 378, 379, 380, 382, 385, 386, 387, 389, 392, 396, 421, 423, 427, 428, 429, 430, 431, 433, 434, 436, 438, 440 and 442, each represented by its position according to the EU numbering, and the domain (1) is capable of binding to the mutated antibody via a moiety having the mutations.

[B4-2-5] The bispecific antibody according to [B4-1] or [B4-2], wherein the mutation comprises one or more mutations selected from the group of 221K or Y, 222F, W, E or Y, 223F, W, E or K, 224F, W, E or Y, 225E, K or W, 227E, G, K or Y, 228E, G, K or Y, and 230A, E, G or Y, each represented by its position according to the EU numbering and the amino acid introduced by the mutation, and the domain (1) is capable of binding to the mutated antibody via a moiety having the mutations.

[B4-3] The bispecific antibody according to [B4-1], wherein the mutated antibody is an antibody having reduced binding activity against any Fcγ receptor of FcγI, FcγIIA, FcγIIB, FcγIIIA and FcγIIIB as compared with a corresponding non-mutated antibody.

[B4-3-1] The bispecific antibody according to [B4-1] or [B4-3], wherein the mutation comprises mutations at one or more positions selected from the group of 220, 226, and 229, each represented by its position according to the EU numbering, and the domain (1) is capable of binding to the mutated antibody via a moiety having the mutations.

[B5-1] A bispecific antibody comprising (1) a domain comprising antibody variable regions that specifically bind to a mutated antibody via a moiety having a mutation, and (2) a domain comprising antibody variable regions having binding activity against a molecule expressed on T cell surface, wherein

the domain (1) is capable of specifically binding to a mutated antibody having a mutation, including substitution, deletion, addition or modification, of at least one amino acid in a CH2 region and a CH3 region, via a moiety having the mutation, and does not specifically bind to an antibody free of the mutation.

[B5-2] The bispecific antibody according to [B5-1], wherein the mutated antibody is an antibody having enhanced binding activity against FcRn at acidic pH as compared with a corresponding non-mutated antibody.

[B5-2-1] The bispecific antibody according to [B5-1] or [B5-2], wherein the mutation comprises mutations at one or more positions selected from the group of 235, 236, 239, 327, 330, 331, 428, 434, 436, 438, and 440, each represented by its position according to the EU numbering, and the domain (1) is capable of binding to the mutated antibody via a moiety having the mutations.

[B5-2-2] The bispecific antibody according to [B5-1] or [B5-2], wherein the mutation comprises one or more mutations selected from the group of 235R, 236R, 239K, 327G, 330S, 331S, 428L, 434A, 436T, 438R, and 440E, each represented by its position according to the EU numbering and the amino acid introduced by the mutation, and the domain (1) is capable of binding to the mutated antibody via a moiety having the mutations.

[B5-2-2-1] The bispecific antibody according to [B5-1] or [B5-2], wherein the mutation has mutations at one or more positions selected from the group of 214, 235, 236, 239, 327, 330, 331, 446, and 447, each represented by its position according to the EU numbering, and the domain (1) is capable of binding to the mutated antibody via a moiety having the mutations.

[B5-2-3] The bispecific antibody according to [B5-1] or [B5-2], wherein the mutation comprises mutations at one or more positions selected from the group of 238, 244, 245, 249, 250, 251, 252, 253, 254, 255, 256, 257, 258, 260, 262, 265, 270, 272, 279, 283, 285, 286, 288, 293, 303, 305, 307, 308, 309, 311, 312, 314, 316, 317, 318, 332, 339, 340, 341, 343, 356, 360, 362, 375, 376, 377, 378, 380, 382, 385, 386, 387, 388, 389, 400, 413, 415, 423, 424, 427, 428, 430, 431, 433, 434, 435, 436, 438, 439, 440, 442 or 447, each represented by its position according to the EU numbering, and the domain (1) is capable of binding to the mutated antibody via a moiety having the mutations.

[B5-2-4] The bispecific antibody according to [B5-1] or [B5-2], wherein the mutation comprises one or more mutations selected from

238L,
244L,
245R,
249P, and
250Q or E

or comprises one or more mutations selected from

251R, D, E, or L,
252F, S, T, or Y,
254S or T,
255R, G, I, or L,
256A, R, N, D, Q, E, P, or T,
257A, I, M, N, S, or V,
258D,
260S,
262L,
270K,
272L, or R,
279A, D, G, H, M, N, Q, R, S, T, W, or Y,
283A, D, F, G, H, I, K, L, N, P, Q, R, S, T, W, or Y,
285N,
286F,
288N, or P,
293V,
307A, E, Q, or M,
311A, E, I, K, L, M, S, V, or W,
309P,
312A, D, or P,
314A or L,
316K,
317P,
318N, or T,
332F, H, K, L, M, R, S, or W,
339N, T, or W,
341P,
343E, H, K, Q, R, T, or Y,
375R,
376G, I, M, P, T, or V,
377K,
378D, N, or V,
380A, N, S, or T
382F, H, I, K, L, M, N, Q, R, S, T, V, W, or Y,
385A, R, D, G, H, K, S, or T,
386R, D, I, K, M, P, S, or T,
387A, R, H, P, S, or T,
389N, P, or S,
423N,
427N,
428L, M, F, S, or T,
430A, F, G, H, I, K, L, M, N, Q, R, S, T, V, or Y,
431H, or N,
433R, Q, H, I, K, P, or S,

434A, G, H, F, S, W, or Y,
436R, N, H, I, L, K, M, or T,
438K, L, T, or W,
440K, and,
442K, 3081, P, or T,

each represented by its position according to the EU numbering and the amino acid introduced by the mutation, and the domain (1) is capable of binding to the mutated antibody via a moiety having the mutations.

[B5-3] The bispecific antibody according to [B5-1], wherein the mutated antibody is an antibody having enhanced binding activity against FcRn at neutral pH as compared with a corresponding non-mutated antibody.

[B5-3-1] The bispecific antibody according to [B5-1] or [B5-3], wherein the mutation comprises mutations at one or more positions selected from the group of 248, 250, 252, 254, 255, 256, 257, 258, 265, 286, 289, 297, 303, 305, 307, 308, 309, 311, 312, 314, 315, 317, 332, 334, 360, 376, 380, 382, 384, 385, 386, 387, 389, 424, 428, 433, 434, and 436, each represented by its position according to the EU numbering, and the domain (1) is capable of binding to the mutated antibody via a moiety having the mutations.

[B5-3-2] The bispecific antibody according to [B5-1] or [B5-3], wherein the mutation comprises one or more mutations selected from the group of 237M, 2481, 250A, F, I, M, Q, S, V, W or Y, 252F, W or Y, 254T, 255E, 256D, E or Q, 257A, G, I, L, M, N, S, T or Y, 258H, 265A, 286A or E, 289H, 297A, 303A, 305A, 307A, D, F, G, H, I, K, L, M, N, P, Q, R, S, V, W or Y, 308A, F, I, L, M, P, Q or T, 309A, D, E, P or R, 311A, H or I, 312A or H, 314K or R, 315A, D or H, 317A, 332V, 334L, 360H, 376A, 380A, 382A, 384A, 385D or H, 386P, 387E, 389A or S, 424A, 428A, D, F, G, H, I, K, L, N, P, Q, S, T, V, W or Y, and 436H, I, L, V, each represented by its position according to the EU numbering and the amino acid introduced by the mutation, and the domain (1) is capable of binding to the mutated antibody via a moiety having the mutations.

[B5-3-3] The bispecific antibody according to [B5-1] or [B5-3], wherein the mutation comprises mutations at one or more positions selected from the group of 238, 250, 252, 254, 255, 258, 286, 307, 308, 309, 311, 315, 428, 433, 434, and 436, each represented by its position according to the EU numbering, and the domain (1) is capable of binding to the mutated antibody via a moiety having the mutations.

[B5-3-4] The bispecific antibody according to [B5-1] or [B5-3], wherein the mutation comprises one or more mutations selected from the group of 238D, 250V, 252Y, 254T, 255L, 256E, 258D or I, 286E, 307Q, 308P, 309E, 311A or H, 315D, 428I, 433A, K, P, R or S, 434Y or W, and 4361, L, V, T or F, each represented by its position according to the EU numbering and the amino acid introduced by the mutation, and the domain (1) is capable of binding to the mutated antibody via a moiety having the mutations.

[B5-4] The bispecific antibody according to [B5-1], wherein the mutated antibody is an antibody having higher binding activity against an antigen at neutral pH than that against the antigen at acidic pH as compared with a corresponding non-mutated antibody.

[B5-4-1] The bispecific antibody according to [B5-1] or [B5-4], wherein the mutation comprises mutations at one or more positions selected from the group of 257, 308, 428 and 434, each represented by its position according to the EU numbering, and the domain (1) is capable of binding to the mutated antibody via a moiety having the mutations.

[B5-4-2] The bispecific antibody according to [B5-1] or [B5-4], wherein the mutation comprises one or more mutations selected from the group of 257A, 308P, 428L, and 434Y, each represented by its position according to the EU numbering and the amino acid introduced by the mutation, and the domain (1) is capable of binding to the mutated antibody via a moiety having the mutations.

[B5-5] The bispecific antibody according to [B5-1], wherein the mutated antibody is an antibody having enhanced binding activity against FcγRIIb as compared with a corresponding non-mutated antibody.

[B5-5-1] The bispecific antibody according to [B5-1] or [B5-5], wherein the mutation comprises mutations at one or more positions selected from the group of 231, 232, 233, 234, 235, 236, 237, 238, 239, 264, 266, 267, 268, 271, 295, 298, 325, 326, 327, 328, 330, 331, 332, 334, and 396, each represented by its position according to the EU numbering, and the domain (1) is capable of binding to the mutated antibody via a moiety having the mutations.

[B5-5-2] The bispecific antibody according to [B5-1] or [B5-5], wherein the mutation comprises a mutation at position 236 and comprises mutations at positions of one or more combinations selected from (1) 231, 232, 233, 234, 235, 237, 238, 239, 264, 266, 267, 268, 271, 295, 298, 325, 326, 327, 328, 330, 331, 332, 334, and 396; (2) 231, 232, 235, 239, 268, 295, 298, 326, 330, and 396; or (3) 268, 295, 326, and 330 each represented by its position according to the EU numbering, and the domain (1) is capable of binding to the mutated antibody via a moiety having the mutations.

[B5-5-3] The bispecific antibody according to [B5-1] or [B5-5], wherein the mutation comprises mutations at one or more positions selected from the group of 285, 311, 312, 315, 318, 333, 335, 337, 341, 342, 343, 384, 385, 388, 390, 399, 400, 401, 402, 413, 420, 422, and 431, each represented by its position according to the EU numbering, and the domain (1) is capable of binding to the mutated antibody via a moiety having the mutations.

[B5-5-4] The bispecific antibody according to [B5-1] or [B5-5], wherein the mutation comprises mutations of one or more combinations selected from the following combinations:

(1) 231D, E, F, G, H, I, K, L, M, N, P, Q, R, S, T, V, W, or Y,
(2) 232A, D, E, F, G, H, I, K, L, M, N, Q, R, S, T, V, W, or Y,
(3) 233D,
(4) 234W, or Y,
(5) 235W,
(6) 236A, D, E, H, I, L, M, N, Q, S, T, or V,
(7) 237D, or Y,
(8) 238E, I, M, Q, or Y,
(9) 239I, L, N, P, or V,
(10) 264I,
(11) 266F,
(12) 267A, H, or L,
(13) 268D, or E,
(14) 271D, E, or G,
(15) 295L,
(16) 298L,
(17) 325E, F, I, or L,
(18) 326T,
(19) 327I, or N,
(20) 328T,
(21) 330K, or R,
(22) 331E,
(23) 332D,
(24) 334D, I, M, V, or Y, and
(25) 396A, D, E, F, G, H, I, K, L, M, N, Q, R, S, T, V, W, or Y

each represented by its position according to the EU numbering and the amino acid introduced by the mutation, and the domain (1) is capable of binding to the mutated antibody via a moiety having the mutations.

[B5-5-5] The bispecific antibody according to [B5-1] or [B5-5], wherein the mutation comprises (1) mutations at one or more positions selected from 234, 238, 250, 264, 267, 307 and 330 and comprises (2) mutations at two or more positions selected from 285, 311, 312, 315, 318, 333, 335, 337, 341, 342, 343, 384, 385, 388, 390, 399, 400, 401, 402, 413, 420, 422 and 431, each represented by its position according to the EU numbering, and the domain (1) is capable of binding to the mutated antibody via a moiety having the mutations.

[B5-5-6] The bispecific antibody according to [B5-1] or [B5-5], wherein the mutation comprises mutations at positions of one or more combinations selected from the following combinations:

(1) 234, 238, 250, 264, 307, 311, 330 and 343;
(2) 234, 238, 250, 264, 307, 311, 330 or 413;
(3) 234, 238, 250, 264, 267, 307, 311 or 343;
(4) 234, 238, 250, 264, 267, 307, 311, 330 and 413;
(5) 234, 238, 250, 267, 307, 311, 330 and 343;
(6) 234, 238, 250, 267, 307, 311, 330 and 413;
(7) 234, 238, 250, 307, 311, 330 and 343;
(8) 234, 238, 250, 307, 311, 330 and 413;
(9) 238, 250, 264, 267, 307, 311, 330 and 343;
(10) 238, 250, 264, 267, 307, 311, 330 and 413;
(11) 238, 250, 264, 307, 311, 330 and 343;
(12) 238, 250, 264, 307, 311, 330 and 413;
(13) 238, 250, 267, 307, 311, 330 and 343;
(14) 238, 250, 267, 307, 311, 330 and 413;
(15) 238, 250, 307, 311, 330 and 343; and
(16) 238, 250, 307, 311, 330, and 413

each represented by its position according to the EU numbering and the amino acid introduced by the mutation, and the domain (1) is capable of binding to the mutated antibody via a moiety having the mutations.

[B5-6] The bispecific antibody according to [B5-1], wherein the mutated antibody is an antibody having improved stability through a mutation at a loop site of an Fc region.

[B5-6-1] The bispecific antibody according to [B5-1] or [B5-6], wherein the mutation comprises mutations at one or more positions selected from the group of 234, 235, 236, 237, 238, 239, 247, 250, 265, 266, 267, 268, 269, 270, 271, 295, 296, 298, 300, 307, 309, 315, 324, 325, 326, 327, 329, 330, 333, 335, 337, 360, 385, 386, 387, 389, 428, and 433, each represented by its position according to the EU numbering, and the domain (1) is capable of binding to the mutated antibody via a moiety having the mutations.

[B6-1] A bispecific antibody comprising (1) a domain comprising antibody variable regions that specifically bind to a mutated antibody via a moiety having a mutation, and (2) a domain comprising antibody variable regions having binding activity against a molecule expressed on T cell surface, wherein

the domain (1) is capable of specifically binding to a mutated antibody having a mutation, including substitution, deletion, addition or modification, of at least one amino acid in a framework region, and does not specifically bind to an antibody free of the mutation.

[B6-2] The bispecific antibody according to [B6-1], wherein the mutated antibody is an antibody having enhanced binding activity against any Fcγ receptor of FcγI, FcγIIA, FcγIIB, FcγIIIA and FcγIIIB as compared with a corresponding non-mutated antibody.

[B6-2-1] The bispecific antibody according to [B6-1] or [B6-2], wherein the mutation has mutations at one or more positions selected from the group of 10, 12, 23, 39, 43 and 105, each represented by its position according to the Kabat numbering, and the domain (1) is capable of binding to the mutated antibody via a moiety having the mutations.

[B6-2-2] The bispecific antibody according to [B6-1] or [B6-2], wherein the mutation comprises mutations at one or more positions selected from the group of 10, 12, 23, 39, 43 and 105, each represented by its position according to the Kabat numbering, or 137, 196, 203, 214, 217, 233, 268, 274, 276, 297, 355, 392, 419, and 435 (according to the EU numbering), and the domain (1) is capable of binding to the mutated antibody via a moiety having the mutations.

[B6-3] The bispecific antibody according to [B6-1], wherein the mutated antibody is an antibody capable of binding to an antigen in a pH-dependent manner as compared with a corresponding non-mutated antibody.

[B6-3-1] The bispecific antibody according to [B6-1] or [B6-3], wherein the mutation comprises a mutation at any of positions:

> (1) 1, 3, 5, 8, 10, 12, 13, 15, 16, 18, 19, 23, 25, 26, 39, 41, 42, 43, 44, 46, 68, 71, 72, 73, 75, 76, 77, 81, 82, 82a, 82b, 83, 84, 85, 86, 105, 108, 110, and 112 in a heavy chain; and
> (2) 1, 3, 7, 8, 9, 11, 12, 16, 17, 18, 20, 22, 37, 38, 39, 41, 42, 43, 45, 46, 49, 57, 60, 63, 65, 66, 68, 69, 70, 74, 76, 77, 79, 80, 81, 85, 100, 103, 105, 106, 107, and 108 in a light chain, each represented by its position according to the Kabat numbering, and the domain (1) is capable of binding to the mutated antibody via a moiety having the mutation.

[B6-3-2] The bispecific antibody according to [B6-1] or [B6-3], wherein the mutation further comprises mutations at one or more positions selected from the group of 196, 253, 254, 256, 258, 278, 280, 281, 282, 285, 286, 307, 309, 311, 315, 327, 330, 342, 343, 345, 356, 358, 359, 361, 362, 373, 382, 384, 385, 386, 387, 389, 399, 400, 401, 402, 413, 415, 418, 419, 421, 424, 430, 433, 434, and 443, each represented by its position according to the EU numbering, and the domain (1) is capable of binding to the mutated antibody via a moiety having the mutations.

[B7-1] A bispecific antibody comprising (1) a domain comprising antibody variable regions that specifically bind to a mutated antibody via a moiety having a mutation, and (2) a domain comprising antibody variable regions having binding activity against a molecule expressed on T cell surface, wherein

the domain (1) is capable of specifically binding to a mutated antibody having a mutation, including substitution, deletion, addition or modification, of at least one amino acid in a CL region, and does not specifically bind to an antibody free of the mutation.

[B7-2] The bispecific antibody according to [B7-1], wherein the mutated antibody is an antibody inhibiting the association between CH1 and CL as compared with a corresponding non-mutated antibody.

[B7-2-1] The bispecific antibody according to [B7-1] or [B7-2], wherein the mutation comprises mutations at one or more positions selected from the group of 123, 131, 160, and 180, each represented by its position according to the EU numbering, and the domain (1) is capable of binding to the mutated antibody via a moiety having the mutations.

[B7-2-2] The bispecific antibody according to [B7-1] or [B7-2], wherein the mutation comprises a mutation at any one of positions of a combination selected from the following combinations:

> (1) amino acid 147 contained in CH1 and amino acid 180 contained in CL;
> (2) amino acid 147 contained in CH1 and amino acid 131 contained in CL;
> (3) amino acid 175 contained in CH1 and amino acid 160 contained in CL; and

(4) amino acid 213 contained in CH1 and amino acid 123 contained in CL,

each represented by its position according to the EU numbering, wherein the mutated antibody is an antibody having an amino acid mutation such that the amino acid residues of the combination have charges that repel each other, and wherein the domain (1) is capable of binding to the mutated antibody via a moiety having the mutations.

[B8-1] The bispecific antibody according to any of [B1-1] to [B1-7-2], [B2-1] to [B2-6-2], and [B3-1] to [B3-2-3], [B4-1] to [B4-3-1], [B5-1] to [B5-6-1], [B6-1] to [B6-3-2], and [B7-1] to [B7-2-2] wherein the molecule expressed on T cell surface is CD3, CD2, CD4, CD5, CD6, CD8, CD16, CD28 and/or CD44.

[B9-1] An isolated nucleic acid encoding a bispecific antibody according to any of [B9-1][B1-1] to [B1-7-2], [B2-1] to [B2-6-2], [B3-1] to [B3-2-3], [B4-1] to [B4-3-1], [B5-1] to [B5-6-1], [B6-1] to [B6-3-2], [B7-1] to [B7-2-2], and [B8-1].

[B9-2] A vector comprising an isolated nucleic acid according to [B9-1].

[B9-3] The vector according to [B9-2], wherein the vector is operably linkable to at least one regulatory element for the expression of the bispecific antibody.

[B9-4] The vector according to [B9-2] or [B9-3], wherein the vector is selected from the group consisting of DNA, RNA, a plasmid, a lentivirus vector, an adenovirus vector, and a retrovirus vector.

[B9-5] A cell transformed or transduced with an isolated nucleic acid according to [B9-1] or a vector according to any of [B9-2] to [B9-4].

[B9-6] The cell according to [B9-5], wherein the cell is a T lymphocyte, an NK cell or a macrophage.

[B9-7] The cell according to [B9-5] or [B9-6], wherein the cell is a T lymphocyte whose expression of an endogenous T cell receptor is blocked or eliminated.

[B9-8] The cell according to any of [B9-5] to [B9-7], wherein the cell is a cell activated and/or grown ex vivo.

[B9-9] The cell according to any of [B9-5] to [B9-8], wherein the cell is a cell genetically engineered by retroviral transduction, lentiviral transduction, DNA electroporation and RNA electroporation, DNA or RNA transfection, or gene editing.

[B9-10] The bispecific antibody according to any of [B1-1] to [B1-7-2], [B2-1] to [B2-6-2], [B3-1] to [B3-2-3], [B4-1] to [B4-3-1], [B5-1] to [B5-6-1], [B6-1] to [B6-3-2], [B7-1] to [B7-2-2], and [B8-1] wherein the mutated antibody is capable of binding to a tumor antigen.

[B10-1] A pharmaceutical composition for use in combination with administration of a mutated antibody having a mutation, including substitution, deletion, addition or modification, of at least one amino acid in a CH1 region, a CH2 region, a CH3 region, a CL region, or a framework region, wherein

the pharmaceutical composition comprises a bispecific antibody, and
the bispecific antibody comprises (1) a domain comprising antibody variable regions capable of specifically binding to the mutated antibody via a moiety having the mutation, and (2) a domain comprising antibody variable regions having binding activity against a molecule expressed on T cell surface, and does not specifically bind to an antibody free of the mutation.

[B10-2] A pharmaceutical composition for use in combination with administration of a bispecific antibody, wherein

the pharmaceutical composition comprises a mutated antibody having a mutation, including substitution, deletion, addition or modification, of at least one amino acid in a CH1 region, a CH2 region, a CH3 region, a CL region, or a framework region, and
the bispecific antibody comprises (1) a domain comprising antibody variable regions capable of specifically binding to the mutated antibody via a moiety having the mutation, and (2) a domain comprising antibody variable regions having binding activity against a molecule expressed on T cell surface, and does not specifically bind to an antibody free of the mutation.

[B10-3] The pharmaceutical composition according to [B10-1] or [B10-2], wherein the bispecific antibody is a bispecific antibody according to any of [B1-1] to [B1-7-2], [B2-1] to [B2-6-2], [B3-1] to [B3-2-3], [B4-1] to [B4-3-1], [B5-1] to [B5-6-1], [B6-1] to [B6-3-2], [B7-1] to [B7-2-2], and [B8-1].

[B10-4] The pharmaceutical composition according to any of [B10-1] to [B10-3], wherein the cell is a cell according to any of [B9-5] to [B9-9].

[B10-5] The pharmaceutical composition according to any of [B10-1] to [B10-4], wherein the mutated antibody has a prolonged half-life in blood or a high isoelectric point compared with a corresponding non-mutated antibody.

[B10-6] The pharmaceutical composition according to any of [B10-1] to [B10-5], wherein the mutated antibody has a mutation, including substitution, deletion, addition or modification, of at least one amino acid in a CH2 region, and the domain (1) is capable of binding to the mutated antibody via a moiety having the mutation.

[B10-7] The pharmaceutical composition according to [B10-6], wherein the mutation in the CH2 region is a mutation at any of positions 231, 232, 233, 234, 235, 236, 237, 238, 239, 240, 264, 265, 266, 267, 269, 270, 295, 296, 297, 298, 299, 300, 325, 327, 328, 329, 330, 331 and 332 according to the EU numbering.

[B10-8] The pharmaceutical composition according to [B10-6] or [B10-7], wherein the CH2 region of the mutated antibody has a mutation selected from the group of 234A, 235A, and/or 297A, and the mutation positions are numbered according to the EU numbering.

[B10-9] The pharmaceutical composition according to any of [B10-6] or [B10-7], wherein the CH2 region of the mutated antibody has mutations of a combination selected from the group of

    (1) 235R and 239K;
    (2) 235R and 236R;
    (3) 235R, 239K and 297A;
    (4) 235R, 236R and 239K;
    (5) 252Y and 434Y;
    (6) 235R, 239K, 252Y and 434Y;
    (7) 252Y, 434Y and 436V;
    (8) 235R, 239K, 252Y, 434Y and 436V;
    (9) 250V, 252Y, 307Q, 308P, 311A, 434Y and 436V; and
    (10) 235R, 239K, 250V, 252Y, 307Q, 308P, 311A, 434Y and 436V

and the mutation positions are numbered according to the EU numbering.

[B10-10] The pharmaceutical composition according to any of [B10-6] to [B10-9], wherein the CH2 region of the mutated antibody comprises the amino acid sequence of SEQ ID NO: 3.

[B10-11] The pharmaceutical composition according to any of [B10-1] to [B10-5], wherein the mutated antibody has a mutation, including substitution, deletion, addition or modification, of at least one amino acid in a CH1 region, and the domain (1) is capable of binding to the mutated antibody via a moiety having the mutation.

[B10-12] The pharmaceutical composition according to [B10-11], wherein the mutated antibody is a full-length antibody, Fab, or F(ab')$_2$.

[B10-13] The pharmaceutical composition according to any of [B10-10] to [B10-12], wherein the mutation in the CH1 region is a mutation at any of positions 131, 133, 137 and 138 according to the EU numbering.

[B10-14] The pharmaceutical composition according to any of [B10-10] to [B10-13], wherein the CH1 region of the mutated antibody has a mutation selected from the group of 131S, 133K, 220S, 137G and 138G, and the mutation positions are numbered according to the EU numbering.

[B10-16] The pharmaceutical composition according to any of [B10-1] to [B10-5], wherein the mutated antibody has a mutation, including substitution, deletion, addition or modification, of at least one amino acid in a CH3 region, and the domain (1) is capable of binding to the mutated antibody via a moiety having the mutation.

[B10-17] The pharmaceutical composition according to [B10-16], wherein the mutation in the CH3 region is a mutation at any of positions 349, 351, 354, 356, 357, 366, 370, 394, 399, 405, 407, 409, 439, 446 and 447 according to the EU numbering.

[B10-18] The pharmaceutical composition according to [B10-16] or [B10-17], wherein the CH3 region of the mutated antibody has a mutation selected from the group of 397M, F or Y, 392D, E, T, V or I, 356K, 397F or Y, 439E, 366Y, 366W, 394S, 394W, 405A, 405W, 407T, and 407A, and the mutation positions are numbered according to the EU numbering.

[B10-19] The pharmaceutical composition according to [B10-16] or [B10-17], wherein the CH3 region of the mutated antibody has a mutation at any one of positions of a combination selected from the group of

    (1) 356 and 439;
    (2) 357 and 370;
    (3) 399 and 409; and
    (4) 399, 409, 356 and 439,

and the mutation positions are numbered according to the EU numbering.

[B10-20] The pharmaceutical composition according to any of [B10-16] to [B10-19], wherein terminal GK of the CH3 region of the mutated antibody is deleted.

[B10-21] The pharmaceutical composition according to any of [B10-1] to [B10-5], wherein the mutated antibody has a mutation, including substitution, deletion, addition or modification, of at least one amino acid in a CL region, and the extracellular binding domain is capable of binding to the mutated antibody via a moiety having the mutation.

[B10-22] The pharmaceutical composition according to [B10-21], wherein the mutated antibody is a full-length antibody, Fab, or F(ab')$_2$.

[B10-23] The pharmaceutical composition according to [B10-21] or [B10-22], wherein the mutation in the CL region is a mutation at any of positions 123, 131, 160 and 180 according to the EU numbering.

[B10-24] The pharmaceutical composition according to any of [B10-1] to [B10-5], wherein the mutated antibody has a mutation, including substitution, deletion, addition or modification, of at least one amino acid in CH2 and CH3 regions, and the extracellular binding domain is capable of binding to the mutated antibody via a moiety having the mutation.

[B10-25] The pharmaceutical composition according to [B10-24], wherein the CH2 and CH3 regions of the mutated antibody have a mutation selected from the group of 397M, F or Y, 392D, E, T, V or I, 356K, 397F or Y, 439E, 366Y, 366W, 394S, 394W, 405A, 405W, 407T, and 407A, and the mutation positions are numbered according to the EU numbering.

[B10-26] The pharmaceutical composition according to any of [B10-1] to [B10-5], wherein the mutated antibody has a mutation, including substitution, deletion, addition or modification, of at least one amino acid in a framework region, and the domain (1) is capable of binding to the mutated antibody via a moiety having the mutation.

[B10-27] The pharmaceutical composition according to [B10-26], wherein the mutation in the framework region is a mutation at any of positions 10, 12, 23, 39, 43 and 105, each represented by its position according to the Kabat numbering.

[B10-28] The pharmaceutical composition according to any of [B10-1] to [B10-27], wherein the mutated antibody has a mutation in a hinge, and the position of the mutation is any of positions 220, 221, 222, 223, 224, 225, 226, 227, 228, 229, and 230 according to the EU numbering.

[B10-29] The pharmaceutical composition according to any of [B10-1] to [B10-28], wherein the hinge region of the mutated antibody has any mutation represented by 221K or Y, 222F, W, E or Y, 223F, W, E or K, 224F, W, E or Y, 225E, K or W, 227E, G, K or Y, 228E, G, K or Y, or 230A, E, G or Y.

[B10-30] The pharmaceutical composition according to any of [B10-1] to [B10-29], wherein the mutated antibody further has a second mutation other than the mutation involved in binding to the bispecific antibody.

[B10-31] The pharmaceutical composition according to [B10-30], wherein the mutated antibody having the second mutation, compared with a corresponding antibody free of the mutation has a prolonged half-life in blood or a high isoelectric point.

[B10-32] The pharmaceutical composition according to any of [B10-1] to [B10-31] for use in treatment or prevention through the antibody-dependent cellular cytotoxicity (ADCC) of a T lymphocyte or an NK cell or the antibody-dependent cellular phagocytosis (ADCP) of a macrophage in a recipient.

[B10-33] The pharmaceutical composition according to any of [B10-1] to [B10-32], wherein the mutated antibody is capable of binding to a tumor antigen.

[B10-34] The pharmaceutical composition according to any of [B10-1] to [B10-33] for use in the treatment or prevention of a cancer.

[B10-35] The pharmaceutical composition according to [B10-34], wherein the cancer is selected from the group consisting of carcinoma, lymphoma, sarcoma, blastoma and leukemia.

[B10-36] The pharmaceutical composition according to [B10-35], wherein the cancer is selected from the group consisting of B-lineage acute lymphoblastic leukemia, B-cell chronic lymphocytic leukemia, B-cell non-Hodgkin's lymphoma, breast cancer, stomach cancer, neuroblastoma, osteosarcoma, lung cancer, melanoma, prostate cancer, colon cancer, renal cell cancer, ovary cancer, rhabdomyosarcoma, leukemia and Hodgkin's lymphoma.

[B11-1] The pharmaceutical composition according to any of [B10-1] to [B10-31], wherein the domain (1) of the bispecific antibody is the domain (1) whose binding activity against the mutated antibody varies according to a concentration of a compound specific for a target tissue.

[B11-2] The pharmaceutical composition according to [B11-1], wherein the target tissue is a cancer tissue.

[B11-3] The pharmaceutical composition according to [B11-2], wherein the compound specific for the cancer tissue is a cancer cell-specific metabolite, a metabolite specific to immunocytes infiltrating into the cancer tissue, or a metabolite specific to stromal cells of the cancer tissue.

[B11-4] The pharmaceutical composition according to [B11-1], wherein the target tissue is an inflammatory tissue.

[B11-5] The pharmaceutical composition according to [B11-4], wherein the compound specific for the inflammatory tissue is a metabolite specific to immunocytes infiltrating into the inflammatory tissue, or a metabolite specific to normal cells damaged in the inflammatory tissue.

[B11-6] The pharmaceutical composition according to any of [B11-1] to [B11-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[B11-7] The pharmaceutical composition according to any of [B11-1] to [B11-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[B12-1] The bispecific antibody according to any of [B1-1] to [B1-7-2], [B2-1] to [B2-6-2], [B3-1] to [B3-2-3], [B4-1] to [B4-3-1], [B5-1] to [B5-6-1], [B6-1] to [B6-3-2], [B7-1] to [B7-2-2], and [B8-1] wherein the domain (1) of the bispecific antibody is the domain (1) whose binding activity against the mutated antibody varies according to a concentration of a compound specific for a target tissue.

[B12-2] The bispecific antibody according to [B12-1], wherein the target tissue is a cancer tissue.

[B12-3] The bispecific antibody according to [B12-2], wherein the compound specific for the cancer tissue is a cancer cell-specific metabolite, a metabolite specific to immunocytes infiltrating into the cancer tissue, or a metabolite specific to stromal cells of the cancer tissue.

[B12-4] The bispecific antibody according to [B12-1], wherein the target tissue is an inflammatory tissue.

[B 12-5] The bispecific antibody according to [B 12-4], wherein the compound specific for the inflammatory tissue is a metabolite specific to immunocytes infiltrating into the inflammatory tissue, or a metabolite specific to normal cells damaged in the inflammatory tissue.

[B12-6] The bispecific antibody according to any of [B12-1] to [B12-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[B 12-7] The bispecific antibody according to any of [B 12-1] to [B 12-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[B13-1] The pharmaceutical composition according to any of [B10-1] to [B10-31], wherein the mutated antibody is an antibody whose binding activity against an antigen varies according to a concentration of a compound specific for a target tissue.

[B13-2] The pharmaceutical composition according to [B13-1], wherein the target tissue is a cancer tissue.

[B13-3] The pharmaceutical composition according to [B 13-2], wherein the compound specific for the cancer tissue is a cancer cell-specific metabolite, a metabolite specific to immunocytes infiltrating into the cancer tissue, or a metabolite specific to stromal cells of the cancer tissue.

[B13-4] The pharmaceutical composition according to [B13-1], wherein the target tissue is an inflammatory tissue.

[B13-5] The pharmaceutical composition according to [B13-4], wherein the compound specific for the inflammatory tissue is a metabolite specific to immunocytes infiltrating into the inflammatory tissue, or a metabolite specific to normal cells damaged in the inflammatory tissue.

[B13-6] The pharmaceutical composition according to any of [B13-1] to [B13-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[B13-7] The pharmaceutical composition according to any of [B13-1] to [B13-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[B14-1] The bispecific antibody according to any of [B1-1] to [B1-7-2], [B2-1] to [B2-6-2], [B3-1] to [B3-2-3], [B4-1] to [B4-3-1], [B5-1] to [B5-6-1], [B6-1] to [B6-3-2], [B7-1] to [B7-2-2], and [B8-1] wherein the mutated antibody is an antibody whose binding activity against an antigen varies according to a concentration of a compound specific for a target tissue.

[B14-2] The bispecific antibody according to [B14-1], wherein the target tissue is a cancer tissue.

[B14-3] The bispecific antibody according to [B14-2], wherein the compound specific for the cancer tissue is a cancer cell-specific metabolite, a metabolite specific to immunocytes infiltrating into the cancer tissue, or a metabolite specific to stromal cells of the cancer tissue.

[B14-4] The bispecific antibody according to [B14-1], wherein the target tissue is an inflammatory tissue.

[B14-5] The bispecific antibody according to [B14-4], wherein the compound specific for the inflammatory tissue is a metabolite specific to immunocytes infiltrating into the inflammatory tissue, or a metabolite specific to normal cells damaged in the inflammatory tissue.

[B 14-6] The bispecific antibody according to any of [B 14-1] to [B 14-5], wherein the metabolite specific for the target tissue is at least one compound selected from a nucleoside having a purine ring structure, an amino acid and a metabolite thereof, a lipid and a metabolite thereof, a primary metabolite of glycometabolism, and nicotinamide and a metabolite thereof.

[B 14-7] The bispecific antibody according to any of [B 14-1] to [B 14-6], wherein the metabolite specific for the target tissue is at least one compound selected from adenosine, adenosine triphosphate, inosine, alanine, glutamic acid, aspartic acid, kynurenine, prostaglandin E2, succinic acid, citric acid, and 1-methylnicotinamide.

[C1-1] A method for treating a subject in need of treatment, comprising:

administering a therapeutically effective amount of a mutated antibody having a mutation, including substitution, deletion, addition or modification, of at least one amino acid in a CH1 region, a CH2 region, a CH3 region, a CL region, or a framework region to the subject; and administering a therapeutically effective amount of a cell expressing a chimeric receptor to the subject, wherein

the mutated antibody is capable of binding to an extracellular binding domain of the chimeric receptor via a moiety having the mutation, and

the extracellular binding domain does not specifically bind to an antibody free of the mutation.

[C1-2] The method according to [C1-1], wherein a pharmaceutical composition according to any of [A10-1] to [A10-39] is administered to the subject.

[C1-3] A method for treating a subject in need of treatment, comprising:

administering a therapeutically effective amount of a mutated antibody having a mutation, including substitution, deletion, addition or modification, of at least one amino acid in a CH1 region, a CH2 region, a CH3 region, a CL region, or a framework region to the subject; and administering a therapeutically effective amount of a bispecific antibody to the subject, wherein

the bispecific antibody comprises (1) a domain comprising antibody variable regions capable of specifically binding to the mutated antibody via a moiety having the mutation, and (2) a domain comprising antibody variable regions having binding activity against a molecule expressed on T cell surface, and does not specifically bind to an antibody free of the mutation.

[C1-4] The method according to [C1-3], wherein a pharmaceutical composition according to any of [B 10-1] to [B 10-36] is administered to the subject.

[C1-5] The method according to any of [C1-1] to [C1-4] for use in the treatment or prevention of a cancer.

[C1-5] The method according to [C1-4], wherein the cancer is selected from the group consisting of carcinoma, lymphoma, sarcoma, blastoma and leukemia.

[C1-6] The method according to [C1-4], wherein the cancer is selected from the group consisting of B-lineage acute lymphoblastic leukemia, B-cell chronic lymphocytic leukemia, B-cell non-Hodgkin's lymphoma, breast cancer, stomach cancer, neuroblastoma, osteosarcoma, lung cancer, melanoma, prostate cancer, colon cancer, renal cell cancer, ovary cancer, rhabdomyosarcoma, leukemia and Hodgkin's lymphoma.

[C1-7] The method according to [C1-1], wherein a pharmaceutical composition according to any of [A11-1] to [A11-7] and [A13-1] to [A13-7] is administered to the subject.

[C1-8] The method according to [C1-3], wherein a pharmaceutical composition according to any of [B11-1] to [B11-7] and [B13-1] to [B13-7] is administered to the subject.

[C1-9] The method according to [C1-7] or [C1-8] for use in the treatment or prevention of a cancer.

[C1-10] The method according to [C1-9], wherein the cancer is selected from the group consisting of carcinoma, lymphoma, sarcoma, blastoma and leukemia.

[C1-11] The method according to [C1-9], wherein the cancer is selected from the group consisting of B-lineage acute lymphoblastic leukemia, B-cell chronic lymphocytic leukemia, B-cell non-Hodgkin's lymphoma, breast cancer, stomach cancer, neuroblastoma, osteosarcoma, lung cancer, melanoma, prostate cancer, colon cancer, renal cell cancer, ovary cancer, rhabdomyosarcoma, leukemia and Hodgkin's lymphoma.

BRIEF DESCRIPTION OF DRAWINGS

Figure 1 is a diagram showing the forms of universal TRAB using an antibody that specifically binds to engineered Fc, and universal CAR-T having an extracellular binding domain that specifically binds to engineered Fc. x represents alteration that inhibits binding to Fc gamma R as one example of engineered Fc.

Figure 2 is a graph showing results of a binding affinity test on a prepared antibody for an antigen.

Figure 3-1 is a graph showing evaluation test results about the T cell activation of universal TRAB that binds to a silent Fc-antibody. The abscissa depicts the concentration of universal TRAB, and the ordinate depicts the degree of emission of luciferase.

Figure 3-2 is a graph showing evaluation test results about the T cell activation of universal TRAB that binds to an delta GK-Fc antibody. The abscissa depicts the concentration of universal TRAB, and the ordinate depicts the degree of emission of luciferase.

Figure 4 is a schematic view showing a vector construct and the order of arrangement of components in frame units from the 5' end to the 3' end.

Figure 5-1 is a graph showing results of an *in vitro* cytotoxic activity test and shows the percentage of residual cancer cells 48 hours after mixing of GPC3-negative cancer cells SK-Hep1 with CAR-T cells. Each data is indicated by mean ± SD (n = 3).

Figure 5-2 is a graph showing results of an *in vitro* cytotoxic activity test and shows the percentage of residual cancer cells 48 hours after mixing of GPC3-positive cancer cells SK-Pca60 with CAR-T cells. Each data is indicated by mean ± SD (n = 3).

Figure 6 is a graph showing results of an antitumor effect confirmation test in mice. The abscissa depicts the number of days with the date of initial administration of a primary antibody defined as 0.

## DESCRIPTION OF EMBODIMENTS

Other features and advantages of the present disclosure will be evident from the detailed description given below. However, it is obvious to those skilled in the art from this detailed description that various changes or modifications can be made in the present disclosure without departing from the spirit and scope of the present disclosure. Therefore, the detailed description and specific examples showing preferred embodiments of the present disclosure should be construed as being given merely for illustrative purposes.

Hereinafter, embodiments of the present disclosure will be described with reference to the drawings.

The terms "substantially" and "approximately" or "about" mean a reasonable amount of deviation of the modified term such that end results are not significantly changed, i.e., an acceptable error range of a particular value determined by those skilled in the art. For example, the term "approximately" may mean acceptable standard deviation for practice in the art. Alternatively, the term "approximately" may mean up to ±20%, preferably up to +10%, more preferably up to ±5%, further preferably up to ±1%, of a certain value. Alternatively, this term, particularly, in a biological system or process, may mean within a single digit, preferably within twice, from a certain value. When a particular value is described in the present specification and the appended claims, the term "approximately" is implicated therein and means an acceptable error range for the particular value in the context, unless otherwise specified.

As used in the English translated sentences of the present specification and the appended claims, the singular forms "a", "an" and "the" include a plurality of references unless the content clearly dictates otherwise. It should also be noted that the term "or" is generally employed in its sense including "and/or" unless the content clearly dictates otherwise.

The recitation of numerical ranges by endpoints in the present disclosure includes all numbers and fractions subsumed within that range (e.g., 1 to 5 includes 1, 1.5, 2, 2.75, 3, 3.90, 4, and 5). It should also be understood that all numbers and fractions are presumed to be modified by the term "approximately". However, when it is evident that numerical values indicated by a numerical range are integers, the numerical range is construed as reciting integers included in this range in a limited manner. In such a case, for example, 1 to 5, is construed as reciting 1, 2, 3, 4, and 5 in a limited manner.

Further, the definitions and embodiments described in particular sections are intended to be applicable to other embodiments herein described for which they are suitable as would be understood by those skilled in the art. For example, in the following passages, different aspects of the present disclosure are defined in more detail. Each aspect thus defined may be combined with any other aspect or aspects unless clearly indicated to the contrary. In particular, any feature indicated as being preferred or advantageous may be combined with any other feature or features indicated as being preferred or advantageous.

The term "chimeric receptor" refers to a recombinant polypeptide that comprises at least an extracellular binding domain, a transmembrane domain and an intracellular signaling domain and induces specificity and intracellular signal production for target cells, for example, cancer cells, when expressed in immune effector cells. The term "chimeric antigen receptor" or "CAR" means a chimeric receptor whose extracellular binding domain binds to an antigen.

The term "extracellular binding domain" means any proteinous molecule or a portion thereof capable of specifically binding to a predetermined molecule such as an antigen, and includes, for example, a single chain antibody (scFv) in which a light chain variable region (VL) and a heavy chain variable region (VH) of a monoclonal antibody specific for a tumor antigen or the like are linked in series. The extracellular binding domain may be used interchangeably with an extracellular recognition domain.

The term "transmembrane domain" is positioned between the extracellular binding domain and the intracellular signaling domain and comprises a polypeptide having a function of penetrating a cell membrane.

The term "intracellular signaling domain" means any oligopeptide domain or polypeptide domain known to work to transduce signals that cause activation or inhibition of an intracellular biological process, for example, activation of immunocytes such as T cells or NK cells, and comprises at least one "stimulatory molecule signaling domain" derived from a stimulatory molecule of T cells mentioned later, or at least one "costimulatory molecule signaling domain" derived from a costimulatory molecule of T cells mentioned later.

In the present disclosure, an antibody having a mutation in an IgG1, IgG2, IgG3 or IgG4 sequence, at a site other than an antigen recognition site is also referred to as an "adaptor antibody" or a "primary antibody". An extracellular binding domain of a chimeric receptor or a T cell-redirecting antibody that recognizes a mutated site of the adaptor antibody is also referred to as a "secondary antibody".

In one embodiment, an extracellular hinge domain and a transmembrane domain may be contained between the extracellular binding domain and the intracellular signaling domain. The term "extracellular hinge domain" means a domain that links the extracellular binding domain to the transmembrane domain. The extracellular hinge domain is not particularly limited as long as the extracellular hinge domain can link the extracellular binding domain to the transmembrane domain. The extracellular hinge domain may be derived from a natural protein or may be artificially designed. The extracellular hinge domain can be constituted by, for example, approximately 10 to 300 amino acids, preferably approximately 20 to 100 amino acids. The extracellular hinge domain preferably neither hinders the ability of the extracellular binding domain to bind to an Fc region of the Fc region mutated antibody of the present disclosure nor hinders signal transduction mediated by the intracellular signaling domain. The term "transmembrane domain" is not particularly limited as long as the transmembrane domain is positioned between the extracellular binding domain and the intracellular signaling domain and is a polypeptide having a function of penetrating a cell membrane. The transmembrane domain may be derived from a natural protein or may be artificially designed. The transmembrane domain derived from a natural protein can be obtained from any membrane-associated protein or transmembrane protein.

In one embodiment, examples of the extracellular hinge domain include extracellular hinge domains of CD8 alpha, CD8 beta, CD28, CD4, NKp30, NKp44, and NKp46. Alternatively, a hinge region of an immunoglobulin (e.g., IgG4) may be used.

In one embodiment, examples of the protein from which the transmembrane domain is derived can include T cell receptor alpha and beta chains, CD3 zeta, CD28, CD3 epsilon, CD45, CD4, CD5, CD8 alpha, CD8 beta, CD9, CD16, CD22, CD33, CD37, CD64, CD80, CD86, CD134, CD137, ICOS, CD154, GITR, NKp30, NKp44, and NKp46.

In one embodiment, the chimeric receptor is a molecule comprising domains defined below.

In one embodiment, the chimeric receptor comprises a chimeric fusion protein comprising an extracellular binding domain, an extracellular hinge domain, a transmembrane domain, and an intracellular signaling domain comprising a stimulatory molecule signaling domain derived from a stimulatory molecule.

In one embodiment, the chimeric receptor comprises a chimeric fusion protein comprising an extracellular binding domain, an extracellular hinge domain, a transmembrane domain, and an intracellular signaling domain comprising a costimulatory molecule signaling domain derived from a costimulatory molecule and a functional signaling domain derived from a stimulatory molecule.

In one embodiment, the chimeric receptor comprises a chimeric fusion protein comprising an extracellular antigen binding domain, a transmembrane domain, and an intracellular signaling domain comprising at least two functional signaling domains derived from one or more costimulatory molecules and a functional signaling domain derived from a stimulatory molecule.

In one embodiment, the chimeric receptor comprises a chimeric fusion protein comprising an extracellular antigen binding domain, a transmembrane domain, and an intracellular signaling domain comprising at least two costimulatory molecule signaling domains derived from one or more costimulatory molecules, a stimulatory molecule signaling domain derived from a stimulatory molecule, and an additional functional domain and/or motif.

In one embodiment, the chimeric receptor described in the present specification can be used as a chimeric antigen receptor (CAR).

In one embodiment, the chimeric receptor comprises an additional leader sequence at the amino terminus (N terminus) of the chimeric receptor fusion protein.

In one embodiment, the chimeric receptor further comprises a leader sequence at the N terminus of the extracellular antigen recognition domain. In this context, the leader sequence may be cleaved from the antigen recognition domain (e.g., scFv) during cell processing and the localization of the chimeric receptor to a cell membrane.

As used in the present disclosure, the "domain" means, for example, one region in a polypeptide that is folded into a particular structure independently of other regions and/or has a particular function. The domain can be, for example, a cytoplasmic moiety of a molecule or a portion thereof. As used in the present disclosure, the "cytoplasmic domain" of a molecule means a full-length cytoplasmic domain or a portion thereof that transduces intracellular signals when activated.

The term "scFv" means a fusion protein comprising at least one antibody fragment comprising a light chain variable region and at least one antibody fragment comprising a heavy chain variable region. In one embodiment, the scFv means a fusion protein that can be expressed as a single polypeptide chain in which the light chain variable region and the heavy chain variable region are continuously linked via, for example, a synthetic linker, for example, a short flexible polypeptide linker, and the scFv further retains the specificity of its original antibody. In the present disclosure, the scFv may have the light chain variable region (VL) and the heavy chain variable region (VH) in any order, unless otherwise specified. For example, as for the ends of the N terminus and C terminus of the polypeptide, the scFv may comprise VL-linker-VH or may comprise VH-linker-VL. Various methods for preparing scFv are known and include methods described in U.S. Patent No. 4694778, Science, vol. 242, pp. 423-442 (1988), Nature, vol. 334, p. 54454 (1989), and Science, vol. 242, pp. 1038-1041 (1988).

The term "flexible polypeptide linker" or "linker" used regarding scFv refers to a peptide linker consisting of amino acids, such as glycine and/or serine residues, used singly or in combinations for linking a heavy chain variable region and a light chain variable region together. In one embodiment, the flexible polypeptide linker is a Gly/Ser linker and comprises an amino acid sequence (Gly-Gly-Gly-Ser) n wherein n is an integer of 1 or larger. For example, n = 1, n = 2, n = 3, n = 4, n = 5, n = 6, n = 7, n = 8, n = 9 and n = 10. In one embodiment, examples of the flexible polypeptide linker include, but are not limited to $(Gly_4Ser)_5$, $(Gly_4Ser)_4$ and $(Gly_4Ser)_3$. In another embodiment, the linker contains a plurality of repeats of $(Gly_2Ser)$, $(GlySer)$ or $(Gly_3Ser)$. Linkers described in WO2012/138475, which are incorporated herein by reference, are also included in the scope of the present disclosure.

The term "stimulation" refers to primary response that is induced by the binding of a stimulatory molecule (e.g., a TCR/CD3 complex or CAR) to its ligand of the same origin (or a tumor antigen for CAR), and thereby mediates a signal transduction event, for example, but not limited to, signal transduction mediated by a TCR/CD3 complex, or signal transduction mediated by an appropriate NK receptor or signaling domain of CAR. The stimulation may mediate changed expression of a certain molecule.

The term "stimulatory molecule" refers to a molecule that is expressed by immunocytes, for example, T cells, NK cells or B cells, which provide an intracytoplasmic signaling sequence regulating the activation of the immunocytes in the form of stimulation, in at least some aspects of an immunocyte signaling pathway. In one aspect, the signal is a primary signal that is triggered, for example, by the binding between a TCR/CD3 complex and an MHC molecule presenting a peptide, and thereby mediates T cell response including, but not limited to, growth, activation, differentiation, and the like. The primary intracytoplasmic signaling sequence (also referred to as a "primary signaling domain") which acts in the form of stimulation may contain a signaling motif, which is known as an immunoreceptor tyrosine-based activation motif or ITAM In the present disclosure, examples of ITAM containing an intracytoplasmic signaling sequence having particular application include, but are not limited to, those derived from CD3 zeta, common FcR gamma (FCER1G), Fc gamma RIIa, FcR beta (Fc epsilon R1b), CD3 gamma, CD3 delta, CD3 epsilon, CD22, CD79a, CD79b, CD278 ("ICOS"), Fc epsilon RI, CD66d, CD32, DAP10, DAP12, CLEC2, CLEC7A (Dectin 1), CLEC9A, EZRIN, RADIXIN and MOESIN. In any one or plural particular chimeric receptors of the present disclosure, the intracellular signaling domain comprises an intracellular signaling sequence, for example, a primary signaling sequence of CD3 zeta.

As used in the present disclosure, the term "CD3 zeta" means cluster of differentiation 3 (CD3) T cell coreceptor of every mammalian species, preferably a human. In mammals, CD3 comprises a CD3 zeta chain, a CD3 delta chain and two CD3 epsilon chains. The CD3 zeta chain (e.g., NCBI RefSeq: NP_932170.1) comprises an intracellular signaling domain that can be used for engineering the chimeric receptor. In the particular chimeric receptor of the present disclosure, the primary signaling sequence of CD3 zeta is the full-length cytoplasmic region sequence of GenBank NM000734.3 (nucleotides 299 to 634) or a portion thereof, or equivalent residues from a non-human species, for example, a mouse, a rodent, a monkey, an anthropoid and the like.

In one embodiment, the intracellular signaling domain may comprise a cytoplasmic domain of an interleukin receptor chain.

One embodiment discloses a chimeric receptor comprising i) an extracellular domain capable of binding to a predetermined antigen, ii) a transmembrane domain, and iii) an intracellular segment comprising one or more intracellular signaling domains selected from a cytoplasmic costimulatory domain and/or a cytoplasmic domain of an interleukin receptor chain and a CD3 zeta intracellular signaling domain comprising an exogenous STAT3 association motif (wherein the intracellular segment comprises an endogenous or exogenous JAK binding motif and STAT5 association motif). In an embodiment, these domains are optionally fused directly or indirectly in the foregoing order starting from the N terminus. In an embodiment, these domains within the intracellular segment are fused in the inverse order.

The term "signaling domain" refers to a functional moiety of a protein that acts by conveying intracellular information in order to regulate cell activity via a defined signaling pathway through the production of a second messenger or through the functionalization of an effector in response to such a messenger.

As used in the present disclosure, the "intracellular signaling domain" refers to an intracellular moiety of a molecule. The intracellular signaling domain can generate signals that promote an immune effector function of cells containing a chimeric receptor such as CAR, for example, CART cells. Examples of the immune effector function of CART cells include cytolytic activity and helper activity, for example, cytokine secretion. In an embodiment, the intracellular signaling domain is a moiety of a protein that transduces effector function signals and allows cells to carry out a specified function. Although the whole intracellular signaling domain may be adopted, it is not necessarily required to use the whole chain in many cases. A truncated moiety that transduces effector function signals can be used instead of an intact chain as long as a truncated moiety of the intracellular signaling domain is used. Thus, the term "intracellular signaling domain" is meant to include every truncated moiety of the intracellular signaling domain sufficient for transducing effector function signals.

In an embodiment, the intracellular signaling domain may comprise a primary intracellular signaling domain. Typical examples of the primary intracellular signaling domain include those derived from molecules involved in primary stimulation or antigen dependent stimulation. In an embodiment, the intracellular signaling domain may comprise a costimulatory intracellular domain. Typical examples of the costimulatory intracellular signaling domain include those derived from molecules involved in costimulatory signals or antigen independent stimulation. In the case of, for example, CART, the primary intracellular signaling domain may comprise an intracytoplasmic sequence of a T cell receptor, or the costimulatory intracellular signaling domain may comprise an intracytoplasmic sequence of a co-receptor or a costimulatory molecule.

The term "costimulatory molecule" refers to a cognate binding partner on a T cell that specifically binds to a costimulatory ligand and thereby mediates the costimulatory response, for example, but not limited to, growth, of the T cell (secondary signal). The costimulatory molecule is a cell surface molecule other than an antigen receptor or its ligand that is responsible for an efficient immune response. The term "costimulatory intracellular signaling domain" refers to an intracellular moiety of the costimulatory molecule. The intracellular signaling domain may comprise the whole intracellular moiety, or the whole natural intracellular signaling domain of the molecule from which the intracellular moiety is obtained, or a functional fragment or derivative thereof. Examples of the costimulatory molecule include, but are not limited to, ligands that specifically bind to MHC class I molecule, TNF receptor protein, immunoglobulin-like protein, cytokine receptor, integrin, signaling lymphocytic activation molecule (SLAM protein), activating NK cell receptor, BTLA, Toll ligand receptor, OX40 (CD134), CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD5, CD8 alpha, CD8 beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD154 and CD83.

In one embodiment, the costimulatory molecule is selected from, for example, 4-1BB (i.e., CD137), CD27, CD28 and/or OX40, and enhances T cell receptor stimulation.

The term "4-1BB" refers to a member of the TNFR superfamily having an amino acid sequence provided as GenBank accession No. AAA62478.2, or equivalent residues from a non-human species, for example, a mouse, a rodent, a monkey, an anthropoid and the like. The term "4-1BB costimulatory domain" is defined as amino acid residues 214 to 255 of GenBank accession No. AAA62478.2, or equivalent residues from a non-human species, for example, a mouse, a rodent, a monkey, an anthropoid and the like. In one aspect, the "4-1BB costimulatory domain" is the full-length sequence from nucleotides 886 to 1026 of GenBank NM001561.5 or a portion thereof, or equivalent residues from a non-human species, for example, a mouse, a rodent, a monkey, an anthropoid and the like.

The term "T cell-redirecting antibody (TRAB)" is an antibody having an antitumor effect based on a cytotoxic mechanism through which T cells are recruited as effector cells (Nature (1985) 314 (6012), 628-31; Int J Cancer (1988) 41 (4), 609-15; and Proc Natl Acad Sci USA (1986) 83 (5), 1453-7). The T cell-redirecting antibody is a bispecific antibody comprising a binding domain for any constituent subunit of a T cell receptor (TCR) complex on T cells, particularly, a domain that binds to a CD3 epsilon chain in CD3, and a domain that binds to an antigen on targeted cancer cells. The T cell-redirecting antibody binds to the CD3 epsilon chain and the tumor antigen at the same time so that the T cells approach the cancer cells. As a result, it is considered that the cytotoxicity effect of the T cells exerts an antitumor effect on the cancer cells.

The TRAB of the present disclosure is a bispecific antibody comprising a domain that binds to a constituent subunit of a T cell receptor complex, and a domain that binds to a site having a mutation in an antibody.

In the present disclosure, the "domain comprising antibody variable regions having T cell receptor complex binding activity" refers to a moiety of a T cell receptor complex antibody comprising a region that specifically binds to and is complementary to a portion or the whole of a T cell receptor complex. The T cell receptor complex may be a T cell receptor itself or may be an adaptor molecule constituting the T cell receptor complex together with the T cell receptor. The adaptor is preferably CD3.

The "domain comprising antibody variable regions having CD3 binding activity" refers to a moiety of an anti-CD3 antibody comprising a region that specifically binds to and is complementary to a portion or the whole of CD3. Preferably, the domain comprises a light chain variable region (VL) and a heavy chain variable region (VH) of the anti-CD3 antibody. Examples of such a domain preferably include "scFv (single chain Fv)", "single chain antibody", "Fv", "scFv$_2$ (single chain Fv$_2$)", "Fab" and "F(ab')$_2$".

The domain comprising antibody variable regions having CD3 binding activity according to the present disclosure is capable of binding to any epitope as long as the epitope is present in a gamma chain, delta chain or epsilon chain sequence constituting human CD3. In the present disclosure, a domain comprising a light chain variable region (VL) and a heavy chain variable region (VH) of an anti-CD3 antibody that binds to an epitope present in an extracellular region of an epsilon chain of a human CD3 complex is preferably used. A CD3 binding domain comprising a light chain variable region (VL) and a heavy chain variable region (VH) of an anti-CD3 antibody described in Examples as well as a light chain variable region (VL) and a heavy chain variable region (VH) of OKT3 antibody (Proc. Natl. Acad. Sci. USA (1980) 77, 4914-4917) or any of various anti-CD3 antibodies known in the art is preferably used as such a domain. Also, a domain comprising antibody variable regions originating from an anti-CD3 antibody having the desired properties, which is obtained by immunizing the desired animal by the method described above using a gamma chain, a delta chain or an epsilon chain constituting human CD3, may be appropriately used. An appropriately humanized antibody as described above or a human antibody is appropriately used as the anti-CD3 antibody that gives rise to the domain comprising antibody variable regions having CD3 binding activity. As for the structure of the gamma chain, the delta chain or the epsilon chain constituting CD3, their polynucleotide sequences are represented by RefSeq registration Nos. NM_000073.2, NM_000732.4 and M_000733.3, and their polypeptide sequences are represented by RefSeq registration Nos. NP_000064.1, NP_000723.1 and NP_000724.1.

The term "mutation" means the substitution, deletion, addition or modification of one or several or more amino acids, or any combination thereof. An antibody having a mutation can be prepared for the purpose of acquiring the desired characteristics, for example, any of characteristics such as decrease or enhancement in binding to Fc receptor, improvement in the pharmacokinetics of an antibody, reduction in heterogeneity, improvement in commercial productivity, or recognition by the TRAB and/or the chimeric receptor of the present disclosure. A mutant also includes, particularly, a molecule engineered by generally known conservative substitution as long as the molecule substantially retains the same function as that of the original sequence. The deletion and insertion of an amino acid sequence includes amino-terminal and/or carboxyl-terminal deletion and insertion of an amino acid. A particular amino acid mutation is the "mutation" in the present specification. The mutation also includes the substitution of a non-natural amino acid, or naturally occurring amino acid derivatives of 20 standard amino acids. The amino acid mutation can be produced using a gene or a chemical method well known in the art. Examples of the genetic method include site-directed mutagenesis, PCR, and gene synthesis. A chemical modification may be useful as a method other than genetic engineering. The mutation can be at least one or more mutations in regions defined in the present disclosure and may comprise the deletion and/or conservative substitution of up to 50, up to 40, up to 30, up to 20, up to 10, or up to 5 amino acids in other regions. In the present disclosure, the intended introduction of a mutation is referred to as "alteration", irrespective of the presence or absence of acquirement of characteristics.

When a secondary antibody specifically binds to a primary antibody via a moiety having a mutation of the primary antibody, the secondary antibody may recognize and bind to an engineered amino acid and its neighborhood of the primary antibody (mutated antibody) as an epitope in an antigen. In the present disclosure, the phrase "engineered amino acid and its neighborhood" may comprise a sequence of most commonly at least 5, for example, approximately 8 to approximately 10, or 6 to 20 amino acids including the engineered amino acid, as in a linear epitope of an antigen, or may comprise a three-dimensional structure surrounding the engineered amino acid of the primary antibody, which is recognized by the secondary antibody, as in a conformational epitope of an antigen.

The term "antigen" or "Ag" refers to a molecule that initiates immune reaction. This immune reaction may include any of antibody production or activation of specific immunocompetent cells, or both. Every high molecule including substantially all proteins or peptides may be useful as the antigen. The antigen may be derived from recombinant or genomic DNA. Every DNA comprising a nucleotide sequence or a partial nucleotide sequence encoding a protein that initiates immune reaction eventually encodes an "antigen" similar to the term antigen used in the present disclosure. The antigen is not always encoded only by the full-length nucleotide sequence of a gene. It is readily understood that: use of partial nucleotide sequences of more than one gene is included in the present disclosure; and these nucleotide sequences are arranged in various combinations so as to encode polypeptides that initiate more desirable immune reaction, though the present disclosure is not limited thereby. The antigen may not always be encoded by a " gene". The antigen may be produced or synthesized, or may be derived from a biological sample, or may be a high molecule other than a polypeptide. Examples of such a biological sample can include, but are not limited to, tissue samples, tumor samples, cells and fluids containing other biological components. When the chimeric receptor, which is a secondary antibody, of the present disclosure has an amino acid sequence derived from an antibody as a portion of the extracellular binding domain, a molecule to which the extracellular binding domain binds may be referred to as an antigen. A primary antibody to which the bispecific antibody (TRAB), which is a secondary antibody, of the present disclosure binds may be referred to as an antigen.

In one embodiment, examples of the antigen preferably include receptors, tumor antigens, MHC antigens, and differentiation antigens.

Examples of the receptor can include receptors belonging to receptor families such as hematopoietic factor receptor family, cytokine receptor family, tyrosine kinase receptor family, serine/threonine kinase receptor family, TNF receptor family, G protein-coupled receptor family, GPI-anchored receptor family, tyrosine phosphatase receptor family, adhesion factor family, and hormone receptor family. The receptors belonging to these receptor families, and their features are described in many literatures, for example, reviews of Cooke BA., King RJB., van der Molen HJ. ed. New Comprehensive Biochemistry Vol. 18B "Hormones and their Actions Part II" pp. 1-46 (1988) Elsevier Science Publishers BV., or Masayuki Miyasaka ed., Cell Technology suppl. Handbook series "Adhesion Factor Handbook" (1994) (Gakken Medical Shujunsha Co., Ltd., Tokyo, Japan) as well as Patthy (Cell (1990) 61 (1), 13-14), Ullrich et al. (Cell (1990) 61 (2), 203-212), Massague (e with acute accent code) (Cell (1992) 69 (6), 1067-1070), Miyajima et al. (Annu. Rev. Immunol. (1992) 10, 295-331), Taga et al. (FASEB J. (1992) 6, 3387-3396), Fantl et al. (Annu. Rev. Biochem. (1993), 62, 453-481), Smith et al. (Cell (1994) 76 (6) 959-962), and Flower DR. (Biochim. Biophys. Acta (1999) 1422 (3) 207-234).

Specific examples of the receptors belonging to the receptor families preferably include human or mouse erythropoietin (EPO) receptor (Blood (1990) 76 (1), 31-35; and Cell (1989) 57 (2), 277-285), human or mouse granulocyte colony-stimulating factor (G-CSF) receptor (Proc. Natl. Acad. Sci. USA. (1990) 87 (22), 8702-8706; and Cell (1990) 61 (2), 341-350), human or mouse thrombopoietin (TPO) receptor (Proc Natl Acad Sci U S A. (1992) 89 (12), 5640-5644; and EMBO J. (1993) 12 (7), 2645-53), human or mouse insulin receptor (Nature (1985) 313 (6005), 756-761), human or mouse Flt-3 ligand receptor (Proc. Natl. Acad. Sci. USA. (1994) 91 (2), 459-463), human or mouse platelet-derived growth factor (PDGF) receptor (Proc. Natl. Acad. Sci. USA. (1988) 85 (10) 3435-3439), human or mouse interferon (IFN)-alpha, beta receptor (Cell (1990) 60 (2), 225-234; and Cell (1994) 77 (3), 391-400), human or mouse leptin receptor, human or mouse growth hormone (GH) receptor, human or mouse interleukin (IL)-10 receptor, human or mouse insulin-like growth factor (IGF)-I receptor, human or mouse leukemia inhibitory factor (LIF) receptor, and human or mouse ciliary neurotrophic factor (CNTF) receptor.

The term "tumor antigen" refers to an antigen expressed on a cancer cell, and means a biological molecule having antigenicity, the expression of which becomes recognized in association with the malignant alteration of cells. The tumor antigen of the present disclosure includes a tumor-specific antigen (antigen that is present only in tumor cells and is not found in other normal cells), and a tumor-associated antigen (antigen that is also present in other organs and tissues or heterogeneous and allogeneic normal cells, or antigen that is expressed during development and/or differentiation). Also, an aberrant sugar chain that appears on cell surface or a protein molecule during cell canceration is the tumor antigen and is also called cancer sugar chain antigen.

Examples of the tumor antigen preferably include GPC3 which belongs as the receptor described above to the GPI-anchored receptor family and is expressed in some cancers including liver cancer (Int J Cancer. (2003) 103 (4), 455-65), EpCAM which is expressed in a plurality of cancers including lung cancer (Proc Natl Acad Sci U S A. (1989) 86 (1), 27-31) (its polynucleotide sequence and polypeptide sequence are described in RefSeq registration Nos. NM_002354.2 and NP_002345.2, respectively), EGFR, CA19-9, CA15-3, sialyl SSEA-1 (SLX), Her2, prostate stem cell antigen (PSCA), alpha-fetoprotein (AFP), cancer embryonic antigen (CEA), tumor antigen-125 (CA-125), calretinin, MUC-1, MUC-16, epithelial membrane protein (EMA), epithelial tumor antigen (ETA), tyrosinase, melanoma-associated antigen (MAGE), chromogranin, cytokeratin, desmin, glial fibrillary acidic protein (GFAP), gross cystic disease fluid protein (GCDFP-15), HMB-45 antigen, protein melan-A (melanoma antigen that is recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, a dimer form of pyruvate kinase isozyme type M2 (tumor M2-PK), GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of prostate member 1), TROP-2, Claudin 6, RNF43a, aberrant ras protein or aberrant p53 protein, integrin alpha v beta 3 (CD61), galectin, K-Ras (V-Ki-ras2 Kirsten rat sarcoma viral oncogene), and Ral-B.

Further examples thereof include: thyroid-stimulating hormone receptor (TSHR); CD171; CS-1 (CD2 subset 1, CRACC, SLAMF7, CD319 and 19A24); C-type lectin-like molecule-1 (CLL-1); ganglioside GD3 (aNeu5Ac(2-8)aNeu5Ac(2-3)bDGalp(1-4)bDGlcp(1-1)Cer); Tn antigen (Tn Ag); T antigen (T Ag); Fms-like tyrosine kinase 3 (FLT3); CD38; CD44v6; B7H3 (CD276); KIT (CD117); interleukin-13 receptor subunit alpha-2 (IL-13Ra2); interleukin 11 receptor alpha (IL-11Ra); interleukin 2 receptor alpha (IL-2Ra); prostate stem cell antigen (PSCA); protease serine 21 (PRSS21); vascular endothelial cell growth factor receptor 2 (VEGFR2); Lewis (Y) antigen; CD24; platelet-derived growth factor receptor beta (PDGFR- beta); stage-specific embryonic antigen-4 (SSEA-4); neural cell adhesion molecule (NCAM); carbonic anhydrase IX (CAIX); proteasome (prosome, macropain) subunit, beta, 9 (LMP2); ephrin type-A receptor 2 (EphA2); fucosyl GM1; sialyl Lewis adhesion molecule (sLe); ganglioside GM3 (aNeu5Ac(2-3)bDGalp(1-4)bDGlcp(1-1)Cer; TGS5; high-molecular-weight melanoma-associated antigen (HMWMAA); o-acetyl-GD2 ganglioside (OAcGD2); folate receptor beta; tumor endothelial marker 1 (TEM1/CD248); tumor endothelial marker 7 related (TEM7R); claudin 6 (CLDN6); G protein-coupled receptor glass C group 5, member D (GPRC5D); chromosome X open reading frame 61 (CXORF61); CD97; CD179a; anaplastic lymphoma kinase (ALK); polysialic acid; placenta specific 1 (PLAC1); a hexasaccharide moiety of globoH glycoceramide (GloboH); mammary gland differentiation antigen (NY-BR-1); uroplakin 2 (UPK2); hepatitis A virus cellular receptor 1 (HAVCR1); adrenaline receptor beta 3 (ADRB3); pannexin 3 (PANX3); G protein-coupled receptor 20 (GPR20); lymphocyte antigen 6 complex, locus K9 (LY6K); olfactory receptor 51E2 (OR51E2); TCR gamma alternate reading frame protein (TARP); Wilms' tumor protein (WT1); ETS translocation variant gene 6, located on chromosome 12p (ETV6-AML); sperm protein 17 (SPA17); X antigen family, member 1A (XAGE1); angiopoietin binding cell surface receptor 2 (Tie 2); melanoma cancer-testis antigen-1 (MAD-CT-1); melanoma cancer-testis antigen-2 (MAD-CT-2); Fos-related antigen 1; p53 mutants; human telomerase reverse transcriptase (hTERT); sarcoma translocation breakpoint; melanoma inhibitor of apoptosis (ML-IAP); ERG (transmembrane protease, serine 2 (TMPRSS2)-ETS fusion gene); N-acetylglucosaminyl-transferase V (NA17); paired box protein Pax-3 (PAX3); androgen receptor; cyclin B1; v-myc avian myelocytomatosis viral oncogene neuroblastoma-derived homolog (MYCN); Ras homolog family member C (RhoC); cytochrome P450 1B1 (CYP1B1); CCCTC binding factor (zinc finger protein)-like (BORIS); squamous cell carcinoma antigen recognized by T cells 3 (SART3); paired box protein Pax-5 (PAX5); proacrosin binding protein p32 (OY-TES1); lymphocyte-specific protein tyrosine kinase (LCK); A kinase anchoring protein 4 (AKAP-4); synovial sarcoma, X breakpoint 2 (SSX2); CD79a; CD79b; CD72; leukocyte-associated immunoglobulin like receptor 1 (LAIR1); Fc fragment of IgA receptor (FCAR); leukocyte immunoglobulin-like receptor subfamily A member 2 (LILRA2); CD300 molecule-like family member f (CD300LF); C-type lectin domain family 12 member A (CLEC12A); bone marrow stromal antigen 2 (BST2); EGF-like module-containing mucin-like hormone receptor-like 2 (EMR2); lymphocyte antigen 75 (LY75); glypican-3 (GPC3); Fc receptor-like 5 (FCRL5); and immunoglobulin lambda-like polypeptide 1 (IGLL1).

The "MHC antigen" is a gene product of major histocompatibility complex (MHC). Among such antigens, glycoproteins expressed on cell membrane are mainly classified into MHC class I antigens and MHC class II antigens. The MHC class I antigens include HLA-A, -B, -C, -E, -F, -G, and -H, and the MHC class II antigens include HLA-DR, -DQ, and -DP. Tumor antigen-derived peptides presented on these MHC antigens are also included therein. A tumor antigen such as GP100, MART-1, or MAGE-1, or a complex with MHC presenting a mutated site, such as RAS or p53, is also regarded as one of the tumor antigens.

The "differentiation antigen" is a generic name for cell surface molecules that appear or disappear in association with the differentiation of bone marrow stem cells into macrophages, T cells, B cells or the like. The differentiation antigen may include CD1, CD2, CD4, CD5, CD6, CD7, CD8, CD10, CD11a, CD11b, CD11c, CD13, CD14, CD15s, CD16, CD18, CD19, CD20, CD21, CD22, CD23, CD25, CD27, CD28, CD29, CD30, CD32, CD33, CD34, CD35, CD38, CD40, CD41a, CD41b, CD42a, CD42b, CD43, CD44, CD45, CD45RO, CD48, CD49a, CD49b, CD49c, CD49d, CD49e, CD49f, CD51, CD54, CD55, CD56, CD57, CD58, CD61, CD62E, CD62L, CD62P, CD64, CD69, CD70, CD71, CD73, CD95, CD99, CD102, CD106, CD117, CD122, CD126, and CDw130.

In one embodiment, the extracellular binding domain of the chimeric receptor is and/or comprises an antigen binding region of an antibody capable of binding to a predetermined antigen via a particular antibody having a mutation in a CH1, CH2, CH3, CL, or FR region of the antibody.

In one embodiment, the extracellular binding domain of the chimeric receptor binds via a portion of an antibody that binds to a predetermined antigen. Examples of the antigen of the antibody preferably include receptors, tumor antigens, MHC antigens, and differentiation antigens.

In one embodiment, the TRAB ("T cell-redirecting antibody"), which is used as a secondary antibody, of the present disclosure is capable of binding to a predetermined antigen via a particular antibody (primary antibody) having a mutation in a CH1, CH2, CH3, CL, or FR region of the antibody. The TRAB thereby binds strongly to the target antigen through the primary antibody and can exert a strong immunological effect by closely situating cancer cells to T cells, as compared with a bispecific antibody comprising a domain against any constituent subunit of a T cell receptor (TCR) complex on T cells, and a domain that binds to an antigen on the targeted cancer cells.

In one embodiment, the extracellular binding domain of the chimeric receptor of the present disclosure, or the primary antibody binding domain of the TRAB of the present disclosure may be a domain whose binding activity against the antibody (primary antibody) that binds to a predetermined antigen varies according to a concentration of a compound specific for a target tissue. See, for example, WO2013/180200 and US2019/0359704.

In the present specification, the term "core hinge region" is a region flanked by at least two cysteine residues forming an inter-heavy chain disulfide bond in a hinge region. Examples thereof for IgG1 and IgG4 include a region constituted by amino acids at positions 226 to 229 (according to the EU numbering) in antibody heavy chains. As an example, the core hinge region in human IgG1 refers to a region consisting of Cys at position 226, Pro at position 227, Pro at position 228, and Cys at position 229 (all according to the EU numbering) in antibody heavy chains.

Domain that recognizes a site having mutation in an antibody

The TRAB and the chimeric receptor of the present disclosure have a "domain that recognizes a site having a mutation in an antibody". This domain refers to a moiety capable of specifically binding to a moiety comprising a mutated amino acid of a primary antibody. The domain comprising antibody variable regions is provided from variable domains of one or more antibodies. Preferably, the domain comprising antibody variable regions comprises an antibody light chain variable region (VL) and an antibody heavy chain variable region (VH). Examples of such a domain comprising antibody variable regions preferably include "scFv (single chain Fv)", "single chain antibody", "Fv", "scFv$_2$ (single chain Fv$_2$)", "Fab" and "F(ab')$_2$".

The term "specific" refers to a state in which a specifically binding molecule does not exhibit the same binding activity or exhibits drastically reduced binding activity against a molecule other than its one or more binding partner molecules. This term is also used when the domain comprising antibody variable regions is specific for a particular epitope among a plurality of epitopes contained in a certain antigen. When an epitope to which the domain comprising antibody variable regions binds is contained in a plurality of different antigens, an antigen binding molecule having this domain comprising antibody variable regions can bind to various antigens containing the epitope.

In some embodiments, the term "not specifically bind" means, for example, being capable of binding to a site comprising a mutated amino acid, which is present in IgG1 or IgG4 but is absent in their mutants or which is absent in IgG1 or IgG4 but is present in their mutants, as an epitope, at a concentration different (e.g., a high concentration or a low concentration) from that for the site having no mutation. For example, the chimeric receptor or the TRAB of the present disclosure exhibits the same binding activity against a primary antibody having a site comprising a mutated amino acid and a primary antibody having the site having no mutation when there is an increasing concentration difference of at least 10 times, at least 100 times, at least 1000 times, at least 10000 times or more up to infinity. In another embodiment, the term refers to a relationship in which a primary antibody having a site comprising a mutated amino acid and a primary antibody having the site having no mutation do not compete with each other.

The terms "compete" and "cross-compete" are interchangeably used in the present disclosure in order to refer to the ability of an antibody molecule. Interference to binding may be direct or may be indirect (e.g., through an antibody molecule or the allosteric alteration of a target). The extent to which an antibody molecule can interfere with the binding of another antibody molecule to a target and thus, whether it can be regarded as competing can be determined by use of, for example, competitive binding assay as described in the present disclosure. In some embodiments, the competitive binding assay is quantitative competitive assay. In other embodiments, when the binding of a first antibody molecule to a target is reduced by 10% or more, for example, 20% or more, 30% or more, 40% or more, 50% or more, 55% or more, 60% or more, 65% or more, 70% or more, 75% or more, 80% or more, 85% or more, 90% or more, 95% or more, 98% or more, or 99% or more, in the competitive binding assay (e.g., the competitive assay described in the present disclosure), the first antibody molecule is regarded as competing with a second antibody molecule for the binding to the target.

As used in the present disclosure, the term "epitope" refers to a component of an antigen that specifically interacts with an antibody molecule. Such a component typically comprises a factor such as an amino acid side chain or a sugar side chain, or is a portion of such a factor. The epitope can be defined by a method known in the art or disclosed in the present disclosure, for example, by crystallography or hydrogen-deuterium exchange. At least one or some components on an antibody molecule, which specifically interact with the epitope, are typically positioned within CDRs. Typically, the epitope has a feature of a specific three-dimensional structure. Typically, the epitope has a feature of a specific charge. Some epitopes are linear epitopes while other epitopes are conformational epitopes.

The epitope may be defined by the binding activity of an antibody molecule that recognizes the epitope against an antigen. When the antigen is a peptide or a polypeptide, the epitope may be defined by an amino acid residue constituting the epitope. When the epitope is a sugar chain, the epitope may be defined by a particular sugar chain structure. When the chimeric receptor of the present disclosure has an amino acid sequence derived from an antibody as a portion of the extracellular binding domain, a binding site of a molecule to which the extracellular binding domain binds may be referred to as an epitope.

The linear epitope refers to an epitope comprising an epitope that is recognized by its primary sequence of amino acids. The linear epitope contains typically at least 3 and most commonly at least 5, for example, approximately 8 to approximately 10 or 6 to 20 amino acids, in its unique sequence.

In contrast to the linear epitope, the conformational epitope refers to an epitope that is contained in a primary sequence of amino acids containing a component other than the single defined component of the epitope to be recognized (e.g., an epitope whose primary sequence of amino acids may not be recognized by an antibody that determines the epitope). The conformational epitope may contain an increased number of amino acids, as compared with the linear epitope. As for the recognition of the conformational epitope, an antibody recognizes the three-dimensional structure of the peptide or the protein. For example, when a protein molecule is folded to form a three-dimensional structure, certain amino acids and/or polypeptide backbone constituting the conformational epitope are arranged in parallel to allow the antibody to recognize the epitope. Examples of the method for determining the conformation of the epitope include, but are not limited to, X-ray crystallography, two-dimensional nuclear magnetic resonance spectroscopy, and site-specific spin labeling and electron paramagnetic resonance spectroscopy. See, for example, Epitope Mapping Protocols in Methods in Molecular Biology (1996), Vol. 66, Morris ed.

The term "compound specific for a target tissue (target tissue-specific compound)" refers to a compound that is differentially present in the target tissue compared with a non-target tissue. In some embodiments, the target tissue-specific compound can be, for example, a compound that is defined by qualitative target tissue specificity such as its presence in the target tissue but absence in a non-target tissue, or its absence in the target tissue but presence in a non-target tissue.

The term "compound specific for a cancer tissue (cancer tissue-specific compound)" refers to a compound that is differentially present in the cancer tissue compared with a non-cancer tissue. In some embodiments, the cancer tissue-specific compound can be, for example, a compound that is defined by qualitative cancer tissue specificity such as its presence in the cancer tissue but absence in a non-cancer tissue, or its absence in the cancer tissue but presence in a non-cancer tissue.

In other embodiments, the cancer tissue-specific compound can be a compound that is defined by quantitative cancer tissue specificity such as its presence in the cancer tissue at a concentration different (e.g., a high concentration or a low concentration) from that for a non-cancer tissue. The cancer tissue-specific compound is differentially present, for example, with any concentration. However, in general, the cancer tissue-specific compound may be present with an increasing concentration of at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 2 times, at least 5 times, at least 10 times, at least 50 times, at least 100 times, at least 103 times, at least 104 times, at least 105 times, at least 106 times or more up to infinity (i.e., the case of being absent in a non-cancer tissue). Alternatively, in general, the cancer tissue-specific compound may be present with a decreasing concentration of at least 5%, at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95%, or at least 100% (i.e., which indicates absence). The cancer tissue-specific compound is preferably differentially present with a statistically significant concentration (i.e., a p value of less than 0.05 and/or a q value of less than 0.10 as determined by use of any of Welch's t test and Wilcoxon rank sum test). In one non-limiting aspect, examples of the cancer tissue-specific compound can include compounds which are metabolites specific for the cancer tissue (cancer tissue-specific metabolites; cancer cell-specific metabolites, metabolites specific to immunocytes infiltrating into the cancer tissue, and cancer stromal cell-specific metabolite), produced through metabolic activity unique to cancer cells, immunocytes, or stromal cells contained in cancer tissues as described below.

The term "metabolism" refers to chemical change that occurs in tissues of organisms and includes "anabolism" and "catabolism". The anabolism refers to the biosynthesis or accumulation of a molecule, and the catabolism refers to the degradation of a molecule. The "metabolite" is an intermediate or a product attributed to substance metabolism. The "primary metabolite" refers to a metabolite involved directly in the process of growth or proliferation of cells or organisms. In one non-limiting aspect, the cancer tissue-specific compound or the cancer tissue-specific metabolite used in the present disclosure is preferably at least one compound selected from the following compounds:

(1) primary metabolites of the glycolytic system such as lactic acid, succinic acid, and citric acid, or the Krebs cycle,
(2) amino acids, such as alanine, glutamic acid, and aspartic acid, which accumulate with high concentrations in cancer tissues by glutamine degradation or the like,
(3) amino acid metabolite such as kynurenine and its metabolites anthranilic acid, 3-hydroxykynurenine, and kynurenic acid,
(4) arachidonic acid metabolites such as prostaglandin E2 and thromboxane A2 (TXA2),
(5) nucleosides having a purine ring structure, such as adenosine, adenosine triphosphate (ATP), adenosine diphosphate (ADP), adenosine monophosphate (AMP), and methylthioadenosine (MTA; CAS No: 2457-80-9), and their degradation product inosine, which accumulate with high concentrations in cancer tissues by purine nucleotide metabolism,
(6) uric acid,
(7) 1-methylnicotinamide which accumulates with high concentrations in cancer tissues by nicotinamide metabolism, etc.

The term "compound specific for an inflammatory tissue (inflammatory tissue-specific compound)" refers to a compound that is differentially present in the inflammatory tissue compared with a non-inflammatory tissue. In the present specification, examples of the "inflammatory tissue" preferably include

a nerve tissue such as the myelin sheath, and a muscle tissue in multiple sclerosis or myasthenia gravis,
a joint tissue in rheumatoid arthritis or osteoarthritis,
a lung (alveolus) tissue in bronchial asthma or COPD,
a digestive organ tissue in inflammatory bowel disease, Crohn disease, ulcerative colitis or colorectal cancer,
a fibrotic tissue in fibrosis in the liver, the kidney, or the lung,
a tissue under rejection (including graft-versus-host disease) of organ transplantation or skin transplantation,
a vascular vessel or heart (cardiac muscle) tissue in hemophilia A, arteriosclerosis or heart failure, myocarditis, or pericarditis,
a visceral fat tissue in metabolic syndrome,
a skin tissue in atopic dermatitis and other dermatitides,
a spinal nerve tissue in disk herniation or chronic lumbago,
an abdominal lymph node, a connective tissue or a tissue inside an organ with massive invasion of Burkitt's lymphoma, sarcoma or prostate cancer, and
a tissue having an infection such as smallpox.

Examples of the cells that are attacked by the CAR-T cell or the TRAB of the present disclosure preferably include autoantibody-producing B cells, fibrotic cells and myofibroblasts. Examples of the antigen preferably include CD19, CD20, desmoglein 3, MuSK, TNP (2,4,5-trinitrophenol), CEA, MOG (myelin oligodendrocyte glycoprotein), FAP, PDGFR,FVIII, vimentin, integrin, adiponectin, CD26, desmin, and HLA-A2 (see Front Immunol. 2018; 9: 2359).

The term "inflammatory tissue-specific metabolite" is a metabolite that is highly produced by immunocytes infiltrating into the inflammatory tissue, and a metabolite that is highly produced by normal cells damaged in the inflammatory tissue. Examples of the infiltrating immunocytes include effector T cells, mature dendritic cells, neutrophils, granular cells (mast cells), and basophils. The metabolite according to the present disclosure also includes a compound released by cells (immunocytes or normal cells) present in the inflammatory tissue from the inside of the cells to the outside of the cells upon cell death by apoptosis, necrosis or the like. In one non-limiting aspect, the inflammatory tissue-specific compound or the inflammatory tissue-specific metabolite used in the present disclosure is preferably at least one compound selected from the following compounds:

(1) arachidonic acid metabolites such as prostaglandin E2,
(2) nucleosides having a purine ring structure, such as adenosine, adenosine triphosphate (ATP), adenosine diphosphate (ADP), adenosine monophosphate (AMP), and methylthioadenosine (MTA; CAS No: 2457-80-9), and their degradation product inosine, which accumulate with high concentrations in inflammatory tissues by purine nucleotide metabolism, and
(3) uric acid.

In one aspect, examples of the cancer tissue-specific compound, the cancer tissue-specific metabolite, or the compound specific for an inflammatory tissue or the inflammatory tissue-specific compound of the present disclosure include ATP, adenosine, inosine, MTA, prostaglandin E2, succinic acid, lactic acid and kynurenine.

As used in the present disclosure, the term "antibody" refers to a protein or polypeptide sequence derived from an immunoglobulin molecule that specifically binds to an antigen. The antibody includes a monoclonal antibody, a polyclonal antibody, a mouse-human chimeric antibody, a humanized antibody, and a mouse, bovine, rabbit, rat, goat, camel, shark or human antibody and antibodies derived from other organisms. The antibody may be derived from a recombinant source and/or may be produced in a transgenic animal. The antibody may be synthetic.

The term "antibody fragment" refers to at least a portion of an antibody that retains the ability to specifically interact with an epitope of an antigen (e.g., through binding, steric hindrance, stabilization/destabilization, or spatial distribution). As used in the present disclosure, examples thereof include, but are not limited to: Fab, Fab', F(ab')$_2$, scFv, dsFv, ds-scFv, Fd fragments consisting of VH and CH1 domains, linear antibodies, and single domain antibodies, for example, sdAb (either VL or VH); camelized VHH domains; multispecific antibodies formed from antibody fragments such as a divalent fragment comprising two Fab fragments linked by disulfide bridge in a hinge region; and isolated CDRs or other epitope binding fragments of an antibody. The antigen binding fragment may also be incorporated into a single domain antibody, a maxibody, a minibody, a nanobody, an intrabody, a diabody, a triabody, a tetrabody, v-NAR and bis-scFv (see e.g., Hollinger and Hudson, Nature Biotechnology 23: 1126-1136, 2005). The antigen binding fragment may be grafted to a scaffold based on a polypeptide such as fibronectin III (Fn3) (see U.S. Patent No. 6,703,199 which describes a minibody of a fibronectin polypeptide). Fab, Fab' and F(ab')$_2$, scFv, dsFv, ds-scFv, a dimer, a minibody, a diabody, a bispecific antibody fragment and other fragments can also be synthesized by recombination techniques.

The term "antibody heavy chain" refers to the larger one of two types of polypeptide chains present in an antibody molecule having a naturally occurring conformation. Usually, a class to which an antibody belongs is determined on the basis of the antibody heavy chain.

The term "antibody light chain" refers to the smaller one of two types of polypeptide chains present in an antibody molecule having a naturally occurring conformation. Kappa ($\kappa$) and lambda ($\lambda$) light chains refer to two major antibody light chain isotypes.

The term "recombinant antibody" refers to an antibody that is produced by use of a recombinant DNA technique, for example, an antibody expressed by a bacteriophage or a yeast expression system. This term is also interpreted as meaning an antibody that is produced by the synthesis of a DNA molecule encoding the antibody (this DNA molecule causes expression of an antibody protein), or an amino acid sequence designating the antibody. In this case, the DNA or the amino acid sequence is obtained by use of a recombinant DNA or amino acid sequence technique available and well known in the art.

The "humanized" form of a non-human (e.g., mouse) antibody is a chimeric immunoglobulin, an immunoglobulin chain or a fragment thereof [e.g., Fv, Fab, Fab', F(ab')$_2$ or other antigen binding partial sequences of antibodies] containing the minimum sequence derived from a non-human immunoglobulin. In most of the cases, the humanized antibody and an antibody fragment thereof are human immunoglobulins (recipient antibody or antibody fragment) in which residues from complementary determining regions (CDRs) of a recipient are replaced by residues from CDRs of a non-human species (donor antibody), such as a mouse, a rat or a rabbit, having the desired specificity, affinity, and ability. In some cases, Fv framework region (FR) residues of the human immunoglobulin are replaced by corresponding non-human residues. The humanized antibody and/or the antibody fragment may further comprise residues that are found neither in the recipient antibody nor in the introduced CDR or framework sequences. Such alteration may further refine and optimize antibody or antibody fragment performance. In general, the humanized antibody or the antibody fragment thereof presumably comprises substantially all of at least one, and typically two, variable domains, in which all or substantially all of the CDR regions correspond to those of a non-human immunoglobulin and all or a significant portion of the FR regions are those of a human immunoglobulin sequence. The humanized antibody or the antibody fragment may also comprise at least a portion of an immunoglobulin constant region (Fc), typically, an immunoglobulin constant region (Fc) of a human immunoglobulin. For more details, see Jones et al., Nature, 321: 522-525, 1986; Reichmann et al., Nature, 332: 323-329, 1988; and Presta, Curr. Op. Struct. Biol., 2: 593-596, 1992.

The term "fully human" refers to an immunoglobulin, for example, an antibody or an antibody fragment, the whole molecule of which is derived from a human or consists of an amino acid sequence identical to a human form of the antibody or the immunoglobulin.

According to the present disclosure, conventional molecular biological, microbiological and recombinant DNA techniques may be used without departing from the skills possessed by those skilled in the art. Such techniques are fully described in literatures. See, for example, in particular, Sambrook, Fritsch & Maniatis, Molecular Cloning: A Laboratory Manual, Second Edition (1989) Cold Spring Harbor Laboratory Press, Cold Spring Harbor, New York (herein "Sambrook et al., 1989"); DNA Cloning: A practical Approach, Volumes I and II (D.N. Glover ed. 1985); Oligonucleotide Synthesis (MJ. Gait ed. 1984); Nucleic Acid Hybridization (B.D. Hames & S.J. Higgins eds. (1985); Transcription and Translation (B.D. Hames & S.J. Higgins, eds. (1984); Animal Cell Culture (R.I. Freshney, ed. (1986); Immobilized Cells and Enzymes (IRL Press, (1986); B. Perbal, A practical Guide To Molecular Cloning (1984); F.M. Ausubel et al. (eds.), Current Protocols in Molecular Biology, John Wiley & Sons, Inc. (1994).

Methods for preparing antibodies are known in the art. In order to produce a human monoclonal antibody and/or a binding fragment thereof, antibody-producing cells (lymphocytes) can be collected from a human having a cancer and fused with myeloma cells by standard somatic cell fusion procedures for the immortalization of these cells to obtain hybridoma cells. Such a technique is well known in the art (e.g., the hybridoma technique originally developed by Kohler and Milstein (Nature 256: 495-497 (1975)) as well as other techniques such as human B cell hybridoma technique (Kozbor et al., Immunol. Today 4: 72 (1983)), EBV-hybridoma technique for producing human monoclonal antibodies (Cole et al., Methods Enzymol, 121: 140-67 (1986)), and screening of combinatorial antibody libraries (Huse et al., Science 246: 1275 (1989)). The hybridoma cells can be immunochemically screened for the production of antibodies specifically reactive with cancer cells to isolate a monoclonal antibody.

The "variable region" or the "VR" in the present disclosure refers to a region or a domain of an antibody heavy chain or light chain involved in the binding of the antibody to its antigen. Usually, heavy chain and light chain variable domains (VH and VL, respectively) of a natural antibody are structurally similar and each contain 4 conserved framework regions (FRs) and 3 hypervariable regions (HVRs) (see e.g., Kindt et al., Kuby Immunology, 6th ed., W.H. Freeman and Co., page 91 (2007)). One VH or VL domain may suffice for conferring antigen binding specificity. An antibody that binds to a certain antigen may be isolated by using VH or VL domains of antibodies that bind to the antigen, and screening a complementary library of the VL or VH domains. See, for example, Portolano et al., J. Immunol. 150: 880-887 (1993); and Clarkson et al., Nature 352: 624-628 (1991).

The term "hypervariable region" or "HVR" used in the present disclosure is hypervariable ("complementarity determining region" or "CDR") in the sequence, and/or forms a structurally determined loop ("hypervariable loop"), and/or refers to each region of an antibody variable domain comprising antigen contact residues ("antigen contacts"). Usually, an antibody contains 6 HVRs: three in VH (HI, H2, and H3), and three in VL (L1, L2, and L3). In the present disclosure, exemplary HVRs include the following: (a) hypervariable loops formed at amino acid residues 26 to 32 (L1), 50 to 52 (L2), 91 to 96 (L3), 26 to 32 (HI), 53 to 55 (H2), and 96 to 101 (H3) (Chothia and Lesk, J. Mol. Biol. 196: 901-917 (1987)); (b) CDRs formed at amino acid residues 24 to 34 (L1), 50 to 56 (L2), 89 to 97 (L3), 31 to 35b (HI), 50 to 65 (H2), and 95 to 102 (H3) (Kabat et al., Sequences of Proteins of Immunological Interest, 5th Ed. Public Health Service, National Institutes of Health, Bethesda, MD (1991)); (c) antigen contacts formed at amino acid residues 27c to 36 (L1), 46 to 55 (L2), 89 to 96 (L3), 30 to 35b (HI), 47 to 58 (H2), and 93 to 101 (H3) (MacCallum et al., J. Mol. Biol. 262: 732-745 (1996)); and (d) a combination of (a), (b), and/or (c) containing HVR amino acid residues 46 to 56 (L2), 47 to 56 (L2), 48 to 56 (L2), 49 to 56 (L2), 26 to 35 (HI), 26 to 35b (HI), 49 to 65 (H2), 93 to 102 (H3), and 94 to 102 (H3).

The "framework" or the "FR" refers to variable domain residues other than hypervariable region (HVR) residues. FRs in a variable domain usually consist of 4 FR domains: FR1, FR2, FR3, and FR4. Accordingly, the sequences of HVRs and FRs usually appear in VH (or VL) in the following order: FR1-H1 (L1)-FR2-H2 (L2)-FR3-H3 (L3)-FR4.

The "percent (%) amino acid sequence identity" in the present disclosure for a reference polypeptide sequence is defined as the percentage of amino acid residues in a candidate sequence that are identical to the amino acid residues in the reference polypeptide sequence, after the sequences are aligned and gaps are introduced, if necessary, so as to obtain the maximum percent sequence identity, and when none of conservative substitution is considered as a portion of the sequence identity. Alignment for purposes of determining the percent amino acid sequence identity can be achieved by various methods, for example, by using publicly available computer software such as BLAST, BLAST-2, ALIGN or Megalign (DNASTAR) software, without departing from the skill in the art. Those skilled in the art can determine appropriate parameters for taking sequence alignment, including any algorithm necessary for achieving maximum alignment over the full lengths of the sequences to be compared. For purposes of the present disclosure, however, % amino acid sequence identity values are generated using the sequence comparison computer program ALIGN-2. The ALIGN-2 sequence comparison computer program has been authored by Genentech, Inc., and its source code has been filed with user documentation in the U.S. Copyright Office, Washington D.C., 20559 and registered under U.S. Copyright Registration No. TXU510087. The ALIGN-2 program is publicly available from Genentech, Inc., South San Francisco, California or may be compiled from the source code. The ALIGN-2 program should be compiled for use on a UNIX operating system including Digital UNIX(R) V4.0D. All sequence comparison parameters are set by the ALIGN-2 program and do not vary.

In a situation where ALIGN-2 is used for amino acid sequence comparison, the % amino acid sequence identity of given amino acid sequence A to, with, or against given amino acid sequence B (which can alternatively be phrased as given amino acid sequence A having or comprising certain % amino acid sequence identity to, with, or against given amino acid sequence B) is calculated as follows: 100 times the fraction X/Y. In this context, X is the number of amino acid residues scored as identical matches by the sequence alignment program ALIGN-2 in that program's alignment of A and B, and Y is the total number of amino acid residues in B. It will be understood that when the length of amino acid sequence A is not equal to the length of amino acid sequence B, the % amino acid sequence identity of A to B is not equal to the % amino acid sequence identity of B to A. All the % amino acid sequence identity values used in the present disclosure are obtained using the ALIGN-2 computer program, as mentioned in the immediately preceding paragraph, unless otherwise specified.

The "Fc region" in the present disclosure is used for defining the C-terminal region of immunoglobulin heavy chains, including at least a portion of constant regions. This term includes a Fc region having a natural sequence and a mutant Fc region. In one aspect, the heavy chain Fc region of human IgG spans from Cys226 or Pro230 to the carboxyl terminus of the heavy chain. However, the C-terminal lysine (Lys447) of the Fc region may be present or absent. In the present disclosure, amino acid residues in a Fc region or a constant region are numbered according to the EU numbering system (also called EU index) described in Kabat et al., Sequences of Proteins of Immunological Interest, 5th Ed. Public Health Service, National Institutes of Health, Bethesda, MD 1991, unless otherwise specified.

The "mutated Fc region" in the present disclosure comprises an amino acid sequence that differs from that of a natural sequence Fc region by at least one amino acid mutation (alteration), preferably one or more amino acid substitutions or deletions. Preferably, the mutated Fc region has at least one amino acid substitution, for example, approximately 1 to approximately 10 amino acid substitutions, preferably approximately 1 to approximately 5 amino acid substitutions, in a natural sequence Fc region or an Fc region of a parent polypeptide, as compared with the natural sequence Fc region or the Fc region of a parent polypeptide. The mutated Fc region of the present disclosure preferably possess at least approximately 80% homology, more preferably at least approximately 90% homology, further preferably at least approximately 95% homology, to a natural sequence Fc region and/or with an Fc region of a parent polypeptide.

In one aspect of the present disclosure, the mutated Fc region comprises a mutated Fc region that does not increase the occurrence of intercellular bridge with other immunocytes, as compared with a corresponding non-mutated Fc region. Also, the Fc region comprises an Fc region having reduced binding activity against any Fc gamma receptor of FcγRI, FcγRIIA, FcγRIIB, FcγRIIIA and FcγRIIIB. In one aspect of the present disclosure, the mutated Fc region comprises an antibody Fc region lacking any of positions 446 and 447 according to the EU numbering.

In the present disclosure, when the mutation of the mutated antibody decreases its binding activity against every active FcγR, the mutated antibody has decreased binding activity against every active FcγR compared with a non-mutated antibody. Decrease in the activity can be confirmed by conducting assay by a method well known to those skilled in the art. Likewise, change in the binding activity of the mutated antibody in such a way that the mutated antibody is an antibody having enhanced binding activity against FcγRIa as compared with a corresponding non-mutated antibody and the mutated antibody is an antibody having enhanced binding activity against any Fcγ receptor of FcγI, FcγIIA, FcγIIB, FcγIIIA and FcγIIIB as compared with a corresponding non-mutated antibody can be confirmed by conducting assay by a method well known to those skilled in the art, and comparing the results with the corresponding non-mutated antibody.

In the present disclosure, the "binding activity against an antigen at acidic pH" means antigen binding activity at pH 4.0 to pH 6.5. The term preferably means antigen binding activity at pH 5.5 to pH 6.5 and particularly preferably means antigen binding activity at pH 5.8 to pH 6.0 which is close to pH in early endosome *in vivo.* The "binding activity against an antigen at neutral pH" means antigen binding activity at pH 6.7 to pH 10.0. The term preferably means antigen binding activity at pH 7.0 to pH 8.0 and particularly preferably means antigen binding activity at pH 7.4 which is close to pH in plasma *in vivo.*

The "boundary moiety" in the present disclosure comprises one or more "contact" amino acid residues of a first polypeptide that interact with one or more "contact" amino acid residues in the boundary moiety of a second polypeptide.

The boundary moiety is preferably an immunoglobulin region such as a variable region or a constant region (or a portion thereof). The boundary moiety preferably comprises an immunoglobulin CH3 region derived from preferably an IgG antibody, most preferably a human IgG1 antibody.

The "knob" in the present disclosure refers to at least one amino acid side chain that projects from the boundary moiety of a first polypeptide and is thus positionable in a compensatory hole in an adjacent boundary moiety (i.e., the boundary moiety of a second polypeptide) so as to stabilize the heteromultimer and thereby favors, for example, heteromultimer formation over homomultimer formation. The knob may be present in the original boundary moiety or may be synthetically introduced (e.g., by changing a nucleic acid encoding the boundary moiety). Usually, a nucleic acid encoding the boundary moiety of the first polypeptide is changed so as to encode the knob. In order to achieve this, a nucleic acid encoding at least one "original" amino acid residue in the boundary moiety of the first polypeptide is substituted by a nucleic acid encoding at least one "introduced" amino acid residue having a larger side chain volume than that of the original amino acid residue. It will be appreciated that there can be more than one original and corresponding introduced residue. The upper limit for the number of original residues to be substituted is the total number of residues in the boundary moiety of the first polypeptide. The side chain volumes of various amino acid residues are shown in the table given below. The introduced residue preferred for knob formation is generally a naturally occurring amino acid residue and is preferably selected from arginine (R), phenylalanine (F), tyrosine (Y) and tryptophan (W). Tryptophan or tyrosine is most preferred. In a preferred embodiment, the original residue for knob formation is, for example, alanine, asparagine, aspartic acid, glycine, serine, threonine or valine, having a small side chain volume.

The "hole" in the present disclosure refers to at least one amino acid side chain that is recessed from the boundary moiety of the second polypeptide and thus accommodates a corresponding knob of the adjacent boundary moiety of the first polypeptide. The hole may be present in the original boundary moiety or may be synthetically introduced (e.g., by changing a nucleic acid encoding the boundary moiety). Usually, a nucleic acid encoding the boundary moiety of the second polypeptide is changed so as to encode the hole. In order to achieve this, a nucleic acid encoding at least one "original" amino acid residue in the boundary moiety of the second polypeptide is substituted by DNA encoding at least one "introduced" amino acid residue having a smaller side chain volume than that of the original amino acid residue. It will be appreciated that there can be one or more original and introduced residues. The upper limit for the number of original residues to be substituted is the total number of residues in the boundary moiety of the second polypeptide. The side chain volumes of various amino acid residues are shown in Table 1 described above. The introduced residue preferred for hole formation is usually a naturally occurring amino acid residue and is preferably selected from alanine (A), serine (S), threonine (T) and valine (V). Serine, alanine or threonine is most preferred. In a preferred embodiment, the original residue for hole formation is, for example, tyrosine, arginine, phenylalanine or tryptophan, having a large side chain volume.

The "host cell", the "host cell line", and the "host cell cultures" in the present disclosure are interchangeably used and refer to cells harboring a foreign nucleic acid (including the progeny of such cells). The host cell includes a "transformant" and a "transformed cell", which include a primary transformed cell and progeny derived from the cell, regardless of the number of passages. The progeny may not be completely identical in terms of nucleic acid contents to a parent cell, and may have a mutation. Mutant progeny having the same function or biological activity as that used for screening or selecting the original transformed cells for are also included in the present disclosure.

The "vector" in the present disclosure refers to a nucleic acid molecule that can propagate another nucleic acid to which the vector is linked. This term includes a vector as a self-replicating nucleic acid structure, and a vector integrated into the genome of a host cell harboring the vector. A certain vector can bring about the expression of a nucleic acid operatively linked to the vector itself. Such a vector is also referred to as an "expression vector" in the present disclosure.

The monoclonal antibody of the present disclosure can be prepared by use of a hybridoma method which was first described by Kohler, et al., Nature, 256 (1975) 495, or can be prepared by a recombinant DNA method (U.S. Patent No. 4816567).

In the hybridoma method, a mouse or any other appropriate host animal, for example, a hamster, is immunized to induce lymphocytes that produce or are capable of producing antibodies that specifically bind to a protein (antigen) used for immunization. In general, antibodies are raised in animals by subcutaneously (sc) or intraperitoneally (ip) injecting an antigen and an adjuvant a plurality of times. In one embodiment, an animal is immunized with an antigen fused to an Fc site of an immunoglobulin heavy chain. In a preferred embodiment, an animal is immunized with an antigen-IgGl fusion protein. Usually, an animal is immunized against an immunogenic conjugate or derivative of an antigen with monophosphoryl lipid A (MPL)/trehalose dicrynomycolate (TDM) (Ribi Immunochem. Research, Inc., Hamilton, MT), and the solution is subcutaneously injected at a plurality of sites. Two weeks later, the animal is boosted. Seven to 14 days later, blood is collected from the animal, and the serum is assayed for an antibody titer. The animal is boosted until the antibody titer plateaus.

Alternatively, lymphocytes may be immunized *in vitro.* Then, the lymphocytes are fused with myeloma cells using an appropriate fusing agent such as polyethylene glycol to form hybridoma cells (Goding, Monoclonal Antibodies: Principles and Practice, p. 59-103 (Academic Press, 1986)). Also, a method for DNA immunization of animals with antigen mutant-encoding cDNA operably linked to an expression control region is known in the art. The DNA immunization eliminates the need of purifying immunogens.

The hybridoma cells thus prepared are seeded and grown in an appropriate medium preferably containing one or more substances that inhibit the growth or survival of the unfused parent myeloma cells. For example, if the parent myeloma cells lack an enzyme hypoxanthine guanine phosphoribosyl transferase (HGPRT or HPRT), the medium for the hybridomas should typically include hypoxanthine, aminopterin and thymidine (HAT medium), which are substances hindering the growth of HGPRT-deficient cells.

The myeloma cells are preferably cells that are efficiently fused, support stable high-level production of antibodies by the selected antibody-producing cells, and are sensitive to a medium such as HAT medium. Among them, a preferred established line of the myeloma cells is, for example, a mouse myeloma line, for example, a line derived from MOPC-21 or MPC-11 mouse tumor available from the Salk Institute Cell Distribution Center, San Diego, California USA, or SP-2 or X63-Ag8-653 cells available from the American Type Culture Collection, Rockville, Maryland USA. Established lines of human myeloma and mouse-human heteromyeloma cells are also disclosed for the production of human monoclonal antibodies (Kozbor, J. Immunol., 133: 3001 (1984); and Brodeur et al., Monoclonal Antibody Production Techniques and Applications, p. 51-63 (Marcel Dekker, Inc., New York, 1987)).

Alternatively, cDNA encoding an antibody variable region may be obtained directly from antibody-producing cells of an immunized animal. For example, a method of limiting-diluting B cells of an immunized rabbit and culturing the B cells with feeder cells is known in the art. Specificity is compared in advance among antibodies produced in culture solutions, and cDNA encoding an antibody variable region can be recovered by an approach such as PCR from cells producing the antibody of interest. The recovered cDNA can be incorporated into an appropriate expression system to obtain a monoclonal antibody without the aid of hybridomas.

The medium containing the grown hybridoma cells is assayed for production of monoclonal antibodies. Preferably, the binding specificity of monoclonal antibodies produced by the hybridoma cells is measured by immunoprecipitation or by *in vitro* binding assay, for example, radioimmunoassay (RIA) or enzyme-linked immunosorbent assay (ELISA).

The binding affinity of the monoclonal antibody can be measured by, for example, the Scatchard analysis method of Munson et al., Anal. Biochem., 107: 220 (1980).

After identification of hybridoma cells that produce antibodies having the desired specificity, affinity, and/or activity, the clones can be subcloned by a limiting dilution method and grown by a standard method (Goding, Monoclonal Antibodies: Principles and Practice, p. 59-103 (Academic Press, 1986)). For example, D-MEM or RPMI-1640 medium is included in a medium preferred for this purpose. In addition, the hybridoma cells can be grown *in vivo* as ascites tumors in an animal.

Monoclonal antibodies secreted by the subclones are preferably separated from the medium, ascites fluid, or serum by, for example, a conventional immunoglobulin purification method such as protein A-Sepharose, hydroxyapatite chromatography, gel electrophoresis, dialysis, or affinity chromatography.

The antibody include in the present disclosure can be identified using a combinatorial library in order to screen for a synthetic antibody clone having the desired activity. In principle, the synthetic antibody clone is selected by screening a phage library having phages that display various fragments of antibody variable regions (Fvs) fused to phage coat proteins. Such a phage library is panned by affinity chromatography against the desired antigen. Clones expressing Fv fragments capable of binding to the desired antigen are adsorbed to the antigen and thereby separated from the non-binding clones in the library. Subsequently, the binding clones are elutable from the antigen, and can be further enriched by additional cycles of antigen adsorption/elution. Any antibody of the present disclosure can be obtained by designing an appropriate antigen screening approach in order to select the phage clone of interest, followed by the construction of a full-length antibody clone using the Fv sequences from the phage clone of interest and appropriate constant region (Fc) sequences described in Kabat et al., Sequences of Proteins of Immunological Interest, Fifth Edition, NIH Publication 91-3242, Bethesda Md. (1991), vols. 1-3.

The antigen-binding domain of an antibody is formed from two variable (V) regions, i.e., light (VL) and heavy (VH) chains, of approximately 110 amino acids, both of which have three hypervariable loops or complementarity determining regions (CDRs). Variable domains can be functionally displayed on phages, either as single chain Fv (scFv) fragments in which VH and VL are covalently linked via a short flexible peptide or as Fab fragments in which VH and VL are each fused to a constant domain and interact non-covalently, as described in Winter et al., Ann. Rev. Immunol., 12: 433-455 (1994). As used herein, scFv-encoding phage clones and Fab-encoding phage clones are collectively referred to as "Fv phage clones" or "Fv clones".

Repertoires of VH and VL genes may be separated and cloned by polymerase chain reaction (PCR) and randomly recombined in phage libraries, which can then be searched for antigen binding clones, as described in Winter et al., Ann. Rev. Immunol., 12: 433-455 (1994). Libraries from immunized sources provide high-affinity antibodies to immunogens without the need of constructing hybridomas. Alternatively, natural repertoires may be cloned to provide a single source of human antibodies against a wide range of non-self and also self-antigens without any immunization, as described by Griffiths et al., EMBO J, 12: 725-734 (1993). Finally, the natural library can also be synthetically prepared by cloning the unrearranged V gene segments from stem cells, and using PCR primers containing a random sequence so that highly variable CDR3 regions are encoded to accomplish rearrangement *in vitro,* as described by Hoogenboom and Winter, J. Mol. Biol., 227: 381-388 (1992).

Filamentous phages are used to display antibody fragments by fusion to minor coat protein pIII. The antibody fragments can be displayed as single chain Fv fragments, VH and VL domains of which are linked on the same polypeptide chain through a flexible polypeptide spacer, as in Fab fragments in which one chain is fused to pIII and the other chain is secreted into bacterial host cell periplasm where assembly of a Fab-coat protein structure which becomes displayed on the phage surface by displacing some wild type coat proteins is present, for example, as described by Marks et al., J. Mol. Biol., 222: 581-597 (1991), or, for example, as described in Hoogenboom et al., Nucl. Acids Res., 19: 4133-4137 (1991).

In general, nucleic acids encoding antibody gene fragments are obtained from immunocytes harvested from humans or animals. If a library biased in favor of the desired clone is desired, an individual is immunized with an antigen to generate antibody response. Then, spleen cells and/or circulating B cells which are other peripheral blood lymphocytes (PBLs) are recovered for library construction.

Further enrichment of antigen-reactive cell populations can be obtained by isolating B cells expressing antigen-specific membrane-bound antibody by use of an appropriate screening approach, for example, by cell separation by affinity chromatography using the antigen, fluorescent dye labeling, or the adsorption of cells to the antigen followed by fluorescence-activated cell sorting (FACS).

Alternatively, use of spleen cells and/or B cells or other PBLs from a non-immunized donor provides better display of possible antibody repertoires, and also permits construction of an antibody library using any animal (human or non-human) species having a different immunogen. For a library incorporating *in vitro* antibody gene constructs, stem cells are harvested from an individual to provide nucleic acids encoding non-rearranged antibody gene segments. The immunocytes of interest can be obtained from various animal species, for example, human, mouse, rat, *Lagomorpha,* lupine, canine, feline, pig, bovine, horse, and avian species.

Nucleic acid encoding antibody variable gene segments (including VH and VL segments) were recovered from the cells of interest and amplified. In the case of rearranged VH and VL gene libraries, the desired DNA can be obtained by isolating genomic DNA or mRNA from lymphocytes and performing polymerase chain reaction (PCR) with primers matching the 5' and 3' ends of rearranged VH and VL genes, as described in Orlandi et al., Proc. Natl. Acad. Sci. (USA), 86: 3833-3837 (1989). Diverse V gene repertoires for expression can thereby be prepared. The V genes can be amplified from cDNA and genomic DNA using back primers at the 5' ends of the exons encoding mature V-domains and forward primers based on the J-segment, as described in Orlandi et al., (1989) and Ward et al., Nature, 341: 544-546 (1989). However, for amplification from cDNA, the back primers can also be based in leader exons, as described in Jones et al., Biotechnol., 9: 88-89 (1991), and the forward primers can be based within constant regions, as described in Sastry et al., Proc. Natl. Acad. Sci. (USA), 86: 5728-5732 (1989). In order to maximize complementarity, degeneracy can be incorporated in the primers, as described in Orlandi et al. (1989) or Sastry et al. (1989). Preferably, the library diversity is maximized using PCR primers targeting each V gene family in order to amplify all available VH and VL sequences present in nucleic acid samples of immunocytes, for example, as described in the method of Marks et al., J. Mol. Biol., 222: 581-597 (1991) or as described in the method of Orum et al., Nucleic Acids Res., 21: 4491-4498 (1993). For the cloning of the amplified DNA into expression vectors, a rare restriction site can be introduced as a tag to one end within the PCR primer by further PCR amplification with a tagged primer, as described in Orlandi et al. (1989) or as described in Clackson et al., Nature, 352: 624-628 (1991).

Repertoires of synthetically rearranged V genes can be derived *in vivo* from V gene segments. Most of the human VH gene segments have been cloned and sequenced (reported in Tomlinson et al., J. Mol. Biol., 227: 776-798 (1992)), and mapped (reported in Matsuda et al., Nature Genet., 3: 88-94 (1993); these cloned segments (including all major conformations of the H1 and H2 loop) are used to prepare diverse VH gene repertoires with PCR primers encoding H3 loops having diverse sequences and lengths, as described in Hoogenboom and Winter, J. Mol. Biol., 227: 381-388 (1992). The VH repertoires can also be prepared with every sequence diversity focused on a long H3 loop of a single length, as described in Barbas et al., Proc. Natl. Acad. Sci. USA, 89: 4457-4461 (1992). Human Vκ and Vλ segments have been cloned and sequenced (reported in Williams and Winter, Eur. J. Immunol., 23: 1456-1461 (1993)), and can be used to prepare synthetic light chain repertoires. Synthetic V gene repertoires based on the ranges of VH and VL folds and the lengths of L3 and H3 encode antibodies having considerable structural diversity. Following amplification of V gene encoding DNA, germline V gene segments can be rearranged *in vitro* according to the method of Hoogenboom and Winter, J. Mol. Biol., 227: 381-388 (1992).

Repertoires of antibody fragments can be constructed by combining VH and VL gene repertoires together by some methods. Each repertoire is prepared in different vectors, and the vectors can be prepared *in vitro,* for example, as described in Hogrefe et al., Gene, 128: 119-126 (1993), or *in vivo* by combinatorial infection, for example, a loxP system described in Waterhouse et al., Nucl. Acids Res., 21: 2265-2266 (1993). Such an *in vivo* recombination approach exploits the two-chain species of Fab fragments in order to overcome the limit on library size imposed by *E. coli* transformation efficiency. Naive VH and VL repertoires are cloned separately, one into a phagemid and the other into a phage vector. These two libraries are then combined by phage infection of phagemid-containing bacteria such that cells have different combinations and the library size is limited only by the number of cells present (approximately $10^{12}$ clones). Both the vectors have *in vivo* recombination signals such that the VH and VL genes are recombined into single replicons and co-packaged into phage virions. These huge libraries provide many diverse antibodies of favorable affinity (Kd$^{-1}$ of approximately $10^{-8}$ M).

Alternatively, the repertoires can be assembled by sequential cloning into the same vector, for example, as described in Barbas et al., Proc. Natl. Acad. Sci. USA, 88: 7978-7982 (1991), or by cloning after PCR, as described in Clackson et al., Nature, 352: 624-628 (1991). The PCR assembly can also be used to form single chain Fv (scFv) repertoires by linking VH and VL DNAs to DNA encoding a flexible peptide spacer. In yet another technique, the "intracellular PCR assembly" is used to combine VH and VL genes within lymphocytes by PCR and then clone repertoires of the linked genes, as described in Embleton et al., Nucl. Acids Res., 20: 3831-3837 (1992).

Although antibodies produced by naive libraries (either natural or synthetic) may have moderate affinity (Kd$^{-1}$ of approximately $10^6$ to $10^7$ M$^{-1}$), even affinity maturation can be mimicked *in vitro* by construction and release from secondary libraries, as described in Winter et al. (1994), supra. For example, mutations can be randomly introduced *in vitro* using error-prone polymerase (reported in Leung et al., Technique, 1: 11-15 (1989)) in the method of Hawkins et al., J. Mol. Biol., 226: 889-896 (1992) or the method of Gram et al., Proc. Natl. Acad. Sci USA, 89: 3576-3580 (1992). Furthermore, affinity maturation can be performed by randomly mutating one or more CDRs, for example, by using PCR with primers having a random sequence spanning the CDR of interest in selected individual Fv clones, and screening for higher-affinity clones. International Publication No. WO 9607754 (published on March 14, 1996) describes a method for preparing a library of light chain genes by inducing mutagenesis in a complementarity determining region of an immunoglobulin light chain. Other effective approaches are to recombine VH or VL domains selected by phage display with repertoires of naturally occurring V domain mutants obtained from non-immunized donors, and to screen for higher affinity in several rounds of chain shuffling, as described in Marks et al., Biotechnol., 10: 779-783 (1992). This technique permits the production of antibodies and antibody fragments having affinity in the $10^{-9}$ M range.

The nucleic acid and amino acid sequences of the antigen, such as a tumor antigen, included in the present disclosure are known in the art. The nucleic acid sequence encoding the targeted antigen may be designed using the amino acid sequence of the desired region of the antigen.

The nucleic acid encoding the targeted antigen can be prepared by various methods known in the art. These methods include, but are not limited to, chemical synthesis by any method described in Engels et al., Agnew. Chem. Int. Ed. Engl., 28: 716-734 (1989), for example, triester, phosphite, phosphoramidite and H-phosphonate methods. In one embodiment, codons preferred for expression host cells are used in the design of antigen-encoding DNA. Alternatively, DNA encoding the antigen can be isolated from a genomic or cDNA library.

Following construction of the DNA molecule encoding the antigen, this DNA molecule is operably linked to an expression control sequence of an expression vector such as a plasmid. The control sequence is recognized by host cells transformed with the vector. In general, plasmid vectors contain replication and control sequences derived from species compatible with the host cells. This vector usually has not only a sequence encoding a protein capable of providing phenotypic selection in transformed cells but a replication site. Vectors preferred for expression in prokaryotic and eukaryotic host cells are known in the art, some of which are further described in the present specification. Cells derived from eukaryotic organisms such as yeasts, or multicellular organisms such as mammals may be used.

Optionally, the DNA encoding the antigen is operably linked to a secretory leader sequence resulting in the secretion of an expression product by host cells into a medium. Examples of the secretory leader sequence include stII, ecotin, lamB, herpes GD, lpp, alkaline phosphatase, invertase, and alpha factor. In this context, a 36-amino acid leader sequence of protein A (Abrahmsen et al., EMBO J., 4: 3901 (1985)) is suitable for use.

Host cells are transfected, preferably transformed, with the expression or cloning vector mentioned above according to this invention, and cultured in a general culture solution modified so as to be suitable for inducing a promoter, selecting transformants, or amplifying a gene encoding the desired sequence.

The transfection means the uptake of an expression vector by a host cell, which does not necessarily actually provide the expression of any coding sequence. Many transfection methods are known to the ordinarily skilled artisan, for example, CaPO$_4$ precipitation and electroporation. In general, successful transfection is recognized when a sign of the operation of the vector appears within the host cell.

The transformation means the introduction of DNA into an organism such that the DNA is replicable either as an extrachromosomal component or by chromosomal integration. Depending on the host cells used, the transformation is performed by use of a standard technique suitable for the cells. Transformation methods are known in the art, some of which are further described in the present specification.

The prokaryotic host cells for use in producing the antigen can generally be cultured as described in Sambrook et al., supra.

The mammalian host cells for use in producing the antigen can be cultured in various media. The media are well known in the art, some of which are described in the present specification.

The host cells mentioned in this disclosure include not only cells within a host animal but cells in *in vitro* cultures.

The purification of the antigen is carried out by use of a method recognized in the art. Some of such methods are described in the present specification.

Affinity chromatographic separation of phage display clone

For use in the affinity chromatographic separation of phage display clones, the purified antigen protein can be attached to an appropriate matrix, for example, agarose beads, acrylamide beads, glass beads, cellulose, various acrylic copolymers, hydroxyl methacrylate gels, polyacrylic and polymethacrylic copolymers, nylon, neutral and ionic carriers. The attachment of the antigen protein to the matrix can be accomplished by a method described in Methods in Enzymology, vol. 44 (1976). A technique widely used for attaching a protein ligand to a polysaccharide matrix such as agarose, dextran or cellulose involves the activation of the carrier with cyanogen halide followed by the coupling of primary aliphatic or aromatic amine of the peptide ligand to the activated matrix.

Alternatively, the antigen can be used to coat wells of an adsorption plate, expressed on host cells attached to an adsorption plate, used in cell sorting, conjugated with biotin for capture with beads coated with streptavidin, or used in any other method of the art for panning phage display libraries.

Phage library samples are contacted with an immobilized antigen under conditions suitable for the binding of at least a portion of phage particles with an adsorbent. Usually, the conditions, including pH, ionic strength, temperature, etc. are selected to mimic physiological conditions. Phages bound to the solid phase are washed and then eluted with an acid, for example, as described in Barbas et al., Proc. Natl. Acad. Sci USA, 88: 7978-7982 (1991), or with an alkali, for example, as described in Marks et al., J. Mol. Biol., 222: 581-597 (1991), or by an approach similar to, for example, the antigen competition method of Clackson et al., Nature, 352: 624-628 (1991). The phages can be enriched 20 to 1,000-fold in a single round of selection. The enriched phages can be further grown in a bacterial culture solution and subjected to further rounds of selection.

The efficiency of selection depends on many factors, which include the kinetics of dissociation during washing, and whether a plurality of antibody fragments on a single phage can be engaged simultaneously with the antigen. Antibodies having a primary dissociation constant (and weak binding affinity) can be retained by use of short washing, multivalent phage display and a high coating density of the antigen in a solid phase. The high density not only stabilizes phages via multivalent interaction but favors the rebinding of dissociated phages. The selection of antibodies having slow dissociation kinetics (and good binding affinity) can be promoted by use of long washing and monovalent phage display as described in Bass et al., Proteins, 8: 309-314 (1990) and in International Publication No. WO 92/09690, and by a low coating density of the antigen as described in Marks et al., Biotechnol., 10: 779-783 (1992).

The selection may be made between phage antibodies differing in affinity for the antigen, even if the affinity differs slightly. However, a random mutation (e.g., as performed by some of the affinity maturation techniques described above) of a selected antibody easily give rises to many mutants, most of which bind to the antigen and a few of which have higher affinity. Limitation on the antigen allows rare high-affinity phages to be competed out. In order to retain all higher-affinity mutants, the phages may be incubated with an excess of biotinylated antigens, but can be incubated with a biotinylated antigen with a lower molar concentration than a target molar concentration affinity constant for the antigen. Subsequently the high-affinity binding phages can be captured with paramagnetic beads coated with streptavidin. Such "equilibrium capture" allows antibodies to be selected according to binding affinity with sensitivity that permits isolation of mutant clones with only two-fold higher affinity from an excess of phages with low affinity. Conditions for use in washing phages bound to a solid phase may be manipulated to discriminate on the basis of dissociation constants.

DNA encoding a monoclonal antibody derived from the hybridoma or the phage display Fv clone of the present disclosure is readily separated and sequenced by use of a routine method (e.g., by using oligonucleotide primers designed so as to specifically amplify heavy and light chain DNA templates encoding the region of interest in the hybridoma, or a phage DNA template). Once separated, the DNA can be placed into an expression vector, with which host cells, such as *E. coli* cells, simian COS cells, Chinese hamster ovary (CHO) cells, or myeloma cells, which do not otherwise produce the antibody protein, are transfected to achieve the synthesis of the monoclonal antibodies in recombinant host cells. Review articles regarding the recombinant expression, in bacteria, of antibody-encoding DNA include Skerra et al., Curr. Opinion in Immunol., 5: 256 (1993) and Pluckthun, Immunol. Revs, 130:151-188 (1992).

DNA encoding the Fv clone of the present disclosure can be combined with a DNA sequence known in the art to encode a heavy chain and/or light chain constant region (e.g., a preferred DNA sequence can be obtained from Kabat et al., supra) to form a clone encoding a full- or partial-length heavy chain and/or light chain. It will be understood that constant regions of any isotype, for example, IgG, IgM, IgA, IgD and IgE constant regions, can be used for this purpose. Such constant regions can be obtained from any human or animal species. A Fv clone obtained from the variable domain DNA of a certain animal (e.g., human) species and subsequently fused to constant region DNA of another animal species in order to form a coding sequence of a "hybrid" full-length heavy chain and/or light chain is included in the definition of the "chimeric" and "hybrid" antibody used in the present specification. In a preferred embodiment, a Fv clone obtained from human variable DNA is fused to human constant region DNA to form coding sequences of all human full- or partial-length heavy chains and/or light chains.

DNA encoding the antibody derived from the hybridoma of the present disclosure can be modified, for example, by substituting the coding sequences of human heavy chain and light chain constant domains in place of homologous mouse sequences derived from the hybridoma clone (e.g., the method of Morrison et al., Proc. Natl. Acad. Sci. USA, 81: 6851 (1984)). DNA encoding the antibody or the antibody fragment derived from the hybridoma or the Fv clone can be further modified by covalently linking the whole or a portion of the coding sequence of a non-immunoglobulin polypeptide to the immunoglobulin coding sequence. The "chimeric" or "hybrid" antibody thus prepared has the binding specificity of the antibody derived from the Fv clone or the hybridoma clone of the present disclosure.

The present disclosure encompasses an antibody fragment. In particular cases, use of the antibody fragment is more advantageous than that of a whole antibody. A smaller size of the fragment accelerates clearance and may improve access to solid tumor.

Various techniques have been developed in order to produce antibody fragments. Traditionally, these fragments were derived via the proteolytic digestion of intact antibodies (see, e.g., Morimoto et al., Journal of Biochemical and Biophysical Methods 24: 107-117 (1992); and Brennan et al., Science, 229: 81 (1985)). However, these fragments can now be produced directly by recombinant host cells. For example, all Fab, Fv and ScFv antibody fragments are expressed in and secreted from *E. coli,* therefore facilitating the large-scale production of these fragments. The antibody fragment can be separated from the antibody phage library mentioned above. Alternatively, Fab'-SH fragments can be recovered directly from *E. coli* and can be chemically coupled to form a $F(ab')_2$ fragment (Carter et al., Bio/Technology 10: 163-167 (1992)). According to another approach, the $F(ab')_2$ fragment can be separated directly from recombinant host cell culture. Fab and $F(ab')_2$ fragments having an increased *in vivo* half-life and containing salvage receptor binding epitope residues are described in U.S. Patent No. 5869046. Other methods for the production of the antibody fragment will be apparent to the skilled practitioner. In other embodiments, the selected antibody is a single chain Fv fragment (scFv). See International Publication No. WO93/16185; U.S. Patent No. 5571894; and U.S. Patent No. 5587458. Fv and sFv are the only species having an intact binding moiety that is devoid of constant regions; thus, they are suitable for reducing nonspecific binding during *in vivo* use. sFv fusion proteins may be constructed in order to obtain a fusion product of an effector protein at either the amino or the carboxy terminus of sFv. See Antibody Engineering, ed. Borrebaeck, supra. The antibody fragment may also be, for example, a "linear antibody" as described in U.S. Patent No. 5641870. Such a linear fragment may be monospecific or bispecific.

The present disclosure encompasses a humanized antibody. Various methods for humanizing non-human antibodies have heretofore been well known. For example, the humanized antibody harbors one or more amino acid residues of non-human origin. These non-human amino acid residues are often referred to as "introduced" residues which are typically obtained from an "introduced" variable domain. The humanization can essentially be carried out by use of the method of Winter and co-researchers (Jones et al., Nature, 321: 522-525 (1986); Riechmann et al., Nature, 332: 323-327 (1988); and Verhoeyen et al., Science, 239: 1534-1536 (1988)) by substituting the corresponding hypervariable region sequences of a human antibody. Accordingly, such a "humanized" antibody is a chimeric antibody (U.S. Patent No. 4816567) in which substantially less than an intact human variable domain is substituted by the corresponding sequence derived from a non-human species. In actuality, the humanized antibody is typically a human antibody in which some hypervariable region residues and optionally some FR residues are substituted by residues from analogous sites of a rodent antibody.

The selection of human variable domains of both light and heavy chains for use in producing the humanized antibody is very important for reducing antigenicity. According to the "best-fit" method, the sequence of a variable domain of a rodent antibody is screened against the whole library of known human variable-domain sequences. Then, a human sequence closest to that of the rodent is accepted as a human framework region of the humanized antibody (Sims et al, J. Immunol., 151: 2296 (1993); and Chothia et al., J. Mol. Biol., 196: 901 (1987)). Another method employs a particular framework region derived from the consensus sequence of all human antibodies of a particular subgroup of light chains or heavy chains. The same framework may be used for several different humanized antibodies (Carter et al., Proc. Natl. Acad. Sci. USA, 89: 4285 (1992); and Presta et al., J. Immunol., 151: 2623 (1993)).

It is further important to humanize an antibody while retaining high affinity for an antigen and other favorable biological properties. In order to achieve this goal, the humanized antibody is prepared through the step of analyzing parent sequences and various conceptual humanized products using three-dimensional models of the parent and humanized sequences according to a certain method. Three-dimensional immunoglobulin models are generally available and are well known to those skilled in the art. Computer programs are purchasable which illustrate and display putative three-dimensional conformational structures of selected candidate immunoglobulin sequences. The inspection of their display permits analysis of likely roles of residues in the functions of the candidate immunoglobulin sequences, i.e., analysis of residues that influence the ability of the candidate immunoglobulins to bind their antigens. In this way, for example, FR residues can be selected and combined from recipient and introduced sequences so as to achieve the desired antibody characteristic, such as enhanced affinity for a target antigen. In general, hypervariable region residues directly and most substantially influence antigen binding activity.

The antibody included in the present disclosure can be constructed by linking Fv clone variable domain sequences selected from human-derived phage display libraries with human constant domain sequences known in the art, as described above. Alternatively, the human monoclonal antibody of the present disclosure can be prepared by a hybridoma method. Human myeloma and mouse-human heteromyeloma cell lines for the production of human monoclonal antibodies are described, for example, by Kozbor, J. Immunol. 133, 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).

It is now possible to produce transgenic animals (e.g., mice) capable of producing complete repertoires of human antibodies, without the production of endogenous immunoglobulins, by immunization. For example, the homozygous deletion of an antibody heavy chain joining region (JH) gene in chimeric and germline mutant mice has been reported to bring about the complete inhibition of production of endogenous antibodies. The transfer of a human germline immunoglobulin gene sequence to such germline mutant mice causes production of human antibodies by antigen challenge. See, for example, Jakobovits et al., Proc. Natl. Acad. Sci. USA 90, 2551-255 (1993); and Jakobovits et al., Nature 362, 255-258 (1993).

Gene shuffling may be used to obtain a human antibody from a non-human (e.g., rodent) antibody when the human antibody has similar affinity and characteristics to those of the starting non-human (e.g., rodent) antibody. According to this method, also called "epitope imprinting", either a heavy chain or light chain variable region gene of a non-human antibody fragment obtained by the phage display technique described above is substituted by a repertoire of human V domain genes to prepare a population of non-human chain/human chain scFv or Fab chimeras. Antigenic selection isolates non-human chain/human chain chimeric scFv or Fab in which the human chain restores an antigen binding site destroyed by the removal of the corresponding non-human chain in the initial phage display clone, i.e., the epitope governs (imprints) the selection of the human chain partner. When this step is repeated in order to replace the remaining non-human chain, a human antibody is obtained (see PCT Patent Application WO 93/06213 published on April 1, 1993). Unlike the traditional humanization of non-human antibodies by CDR grafting, this technique produces a completely human antibody having no FR or CDR residue of non-human origin.

Bispecific antibodies are monoclonal antibodies, preferably human or humanized antibodies, having binding specificities for at least two different epitopes. In this case, one of the binding specificities is for a particular antigen and the other is for any other antigen. Exemplary bispecific antibodies are capable of binding to two different epitopes of one antigen. The bispecific antibodies may also be used to localize cytotoxic activity to cells expressing an antigen. These antibodies possess an antigen binding arm and an arm that binds to CD3 for exerting T cell-dependent cytotoxic activity. The bispecific antibodies can be prepared as full-length antibodies or antibody fragments (e.g., $F(ab')_2$ bispecific antibodies).

In the present disclosure, the "domain comprising antibody variable regions having T cell receptor complex binding activity" refers to a moiety of an anti-T cell receptor complex antibody comprising a region that specifically binds to and is complementary to a portion or the whole of a T cell receptor complex. The T cell receptor complex may be a T cell receptor itself or may be an adaptor molecule constituting the T cell receptor complex together with the T cell receptor. The adaptor is preferably CD3.

In the present disclosure, the "domain comprising antibody variable regions having T cell receptor binding activity" refers to a moiety of an anti-T cell receptor antibody comprising a region that specifically binds to and is complementary to a portion or the whole of a T cell receptor.

The moiety of the T cell receptor to which the domain of the present disclosure binds may be a variable region or may be a constant region, and is preferably an epitope present in a constant region. Examples of the sequence of the constant region can include the sequences of a T cell receptor $\alpha$ chain (SEQ ID NO: 64) of RefSeq registration No. CAA26636.1, a T cell receptor $\beta$ chain (SEQ ID NO: 65) of RefSeq registration No. C25777, a T cell receptor $\gamma$1 chain (SEQ ID NO: 66) of RefSeq registration No. A26659, a T cell receptor $\gamma$2 chain (SEQ ID NO: 67) of RefSeq registration No. AAB63312.1, and a T cell receptor $\delta$ chain (SEQ ID NO: 68) of RefSeq registration No. AAA61033.1.

In the present disclosure, the "domain comprising antibody variable regions having CD3 binding activity" refers to a moiety of an anti-CD3 antibody comprising a region that specifically binds to and is complementary to a portion or the whole of CD3. Preferably, the domain comprises a light chain variable region (VL) and a heavy chain variable region (VH) of the anti-CD3 antibody. Examples of such a domain preferably include "scFv (single chain Fv)", "single chain antibody", "Fv", "scFv$_2$ (single chain Fv$_2$)", "Fab" and "F(ab')$_2$".

The domain comprising antibody variable regions having CD3 binding activity according to the present disclosure is capable of binding to any epitope as long as the epitope is present in a gamma chain, delta chain or epsilon chain sequence constituting human CD3. In the present disclosure, a domain comprising a light chain variable region (VL) and a heavy chain variable region (VH) of an anti-CD3 antibody that binds to an epitope present in an extracellular region of an epsilon chain of a human CD3 complex is preferably used. A CD3 binding domain comprising a light chain variable region (VL) and a heavy chain variable region (VH) of an anti-CD3 antibody described in Examples as well as a light chain variable region (VL) and a heavy chain variable region (VH) of OKT3 antibody (Proc. Natl. Acad. Sci. USA (1980) 77, 4914-4917) or any of various anti-CD3 antibodies known in the art is preferably used as such a domain. Also, a domain comprising antibody variable regions originating from an anti-CD3 antibody having the desired properties, which is obtained by immunizing the desired animal by the method described above using a γ chain, a δ chain or an ε chain constituting human CD3, may be appropriately used. An appropriately humanized antibody as described above or a human antibody is appropriately used as the anti-CD3 antibody that gives rise to the domain comprising antibody variable regions having CD3 binding activity. As for the structure of the gamma chain, the delta chain or the epsilon chain constituting CD3, their polynucleotide sequences are registered as RefSeq registration Nos. NM_000073.2, NM_000732.4 and NM_000733.3, and their polypeptide sequences are registered as RefSeq registration Nos. NP_000064.1, NP_000723.1 and NP_000724.1.

Methods for preparing bispecific antibodies are known in the art. The traditional recombinant production of bispecific antibodies is based on the coexpression of two immunoglobulin heavy chain-light chain pairs. In this context, the two heavy chains have different specificities (Millstein et al., Nature, 305: 537-539 (1983)). Since immunoglobulin heavy and light chains are randomly assorted, these hybridomas (quadromas) produce a potential mixture of 10 different antibody molecules, only one of which has a correct bispecific structure. The purification of the correct molecule, which is usually performed by an affinity chromatography process, is considerably cumbersome with low product yields. Similar methods are disclosed in International Publication No. WO93/08829 published on May 13, 1993, and Traunecker et al., EMBO J., 10: 3655-3659 (1991).

According to a different and more preferred approach, antibody variable domains with the desired binding specificity (antibody-antigen binding sites) are fused to immunoglobulin constant domain sequences. The fusion is preferably the one with an immunoglobulin heavy chain constant domain comprising at least a portion of a hinge, CH2, and CH3 regions. The first heavy chain constant region (CH1) comprising a site necessary for light chain binding is desirably present in at least one fusion. DNAs encoding the immunoglobulin heavy chain fusions and, if desired, the immunoglobulin light chain, are inserted into separate expression vectors, with which an appropriate host organism is cotransfected. This imparts great flexibility to the adjustment of the mutual proportions of three polypeptide fragments in an aspect in which unequal ratios of the three polypeptide chains for use in a construct bring about the optimum yields. However, coding sequences for two of or all the three polypeptide chains may be inserted to one expression vector when the expression of at least two polypeptide chains at equal ratios brings about high yields or when the ratios are of no particular significance.

In a preferred embodiment of this approach, the bispecific antibody consists of a hybrid immunoglobulin heavy chain of one arm having a first binding specificity and a hybrid immunoglobulin heavy chain-light chain pair (which provides a second binding specificity) of the other arm. This asymmetric structure has been found to facilitate the separation of the desired bispecific compound from unnecessary immunoglobulin chain combinations, because the presence of an immunoglobulin light chain in only one half of the bispecific molecule provides an easy separation method. This approach is disclosed in International Publication No. WO 94/04690. For further details of bispecific antibody production, see, for example, Suresh et al., Methods in Enzymology, 121: 210 (1986).

According to another approach, the interface between a pair of antibody molecules can be engineered to maximize the percentage of heterodimers which are recovered from recombinant cell culture. The interface preferably comprises at least a portion of the CH3 domain of an antibody constant domain. In this method, one or more small amino acid side chains from the interface of a first antibody molecule are replaced with larger side chains (e.g., tyrosine or tryptophan). Complementary "cavities" having the same size as, or a size similar to, that of the large side chains are created on the interface of a second antibody molecule by replacing large amino acid side chains with smaller ones (e.g., alanine or threonine). This provides a mechanism to increase the yield of heterodimers over other unnecessary end products such as homodimers.

In the present disclosure, the "interface" usually refers to a face at which two regions associate or interact with each other. Amino acid residues forming the interface are usually one or more amino acid residues contained in each polypeptide region subjected to the association and more preferably refer to amino acid residues that approach each other upon association and participate in interaction. Specifically, the interaction includes a hydrogen bond, electrostatic interaction, or salt bridge formation between the amino acid residues approaching each other upon association.

In the present disclosure, the "amino acid residues forming the interface" specifically refers to amino acid residues contained in polypeptide regions constituting the interface. As one example, the polypeptide regions constituting the interface refer to polypeptide regions responsible for intermolecular selective binding in antibodies, ligands, receptors, substrates, etc. Specific examples of such polypeptide regions in antibodies can include a heavy chain constant region, a heavy chain variable region, a light chain constant region, and a light chain variable region.

In the present disclosure, the phrase "control association" or "association is controlled" refers to controlling so as to attain the desired associated state, and more specifically refers to controlling so as not to form undesired association between a heavy chain and a light chain.

The bispecific antibody includes a cross-linked antibody and a "heteroconjugate antibody". For example, one of the antibodies in the heteroconjugate may be bound to avidin, and the other antibody may be bound to biotin. Such an antibody has been proposed, for example, for the purposes of targeting immune system cells to unnecessary cells (U.S. Patent No. 4676980) and treating HIV infection (International Publication No. WO 91/00360, International Publication No. WO 92/00373 and EP Patent No. 03089). The heteroconjugate antibody can be produced by an appropriate cross-linking method. Appropriate cross-linking agents are well known in the art, and described in U.S. Patent No. 4676980, etc., together with a plurality of cross-linking methods.

The chimeric receptor of the present disclosure can be engineered so as to comprise an extracellular domain having an antigen binding domain fused with an intracellular signaling domain of a T cell antigen receptor complex zeta chain (e.g., CD3 zeta). The CAR of the present disclosure can reinduce antigen recognition based on antigen binding specificity, when expressed in T cells.

As for the transmembrane domain, the chimeric receptor can be designed so as to comprise a transmembrane domain fused with its extracellular domain. In one aspect, a transmembrane domain naturally associated with one of the domains in the chimeric receptor is used. In some cases, the transmembrane domain can be selected, or selected or engineered by amino acid substitution, so as to avoid the binding of such domains to the transmembrane domains of the same or different surface membrane proteins, in order to minimize interaction with other members of a receptor complex.

The transmembrane domain may be derived from either a natural source or a synthetic source. When the source is natural, the domain may be derived from any membrane-associated protein or transmembrane protein. A transmembrane region particularly useful in the present disclosure may be derived from an alpha chain, a beta chain, or a zeta chain of a T cell receptor, CD28, CD3 epsilon, CD45, CD4, CD5, CD8, CD9, CD16, CD22, CD33, CD37, CD64, CD80, CD86, CD134, CD137, or CD154 (i.e., comprises at least a transmembrane region thereof). Alternatively, the transmembrane domain may be synthetic. In this case, the transmembrane domain is considered to predominantly comprise hydrophobic residues such as leucine and valine. Preferably, a triplet of phenylalanine, tryptophan and valine may be found at each end of the synthetic transmembrane domain. Optionally, a short oligopeptide or polypeptide linker preferably 2 to 10 amino acids in length may form the linkage between the transmembrane domain and the cytoplasmic signaling domain of the chimeric receptor. A glycine-serine doublet serves as a particularly suitable linker.

The cytoplasmic domain, or in other words, the intracellular signaling domain, of the chimeric receptor of the present disclosure is responsible for the activation of at least one of the normal effector functions of an immunocyte expressing the chimeric receptor. The term "effector function" refers to a specialized function of a cell. The effector function of, for example, T cells, may be cytolytic activity or helper activity including the secretion of cytokines. Thus, the term "intracellular signaling domain" refers to a moiety of a protein that transduces effector function signals and directs the cell to perform a specialized function. As the whole intracellular signaling domain may usually be used, it is not necessary to use the whole chain thereof in many cases. Within the scope where a truncated portion of the intracellular signaling domain is used, such a truncated portion can be used instead of the intact chain as long as the truncated portion transduces effector function signals. Hence, the term "intracellular signaling domain" includes any truncated portion of the intracellular signaling domain sufficient for transducing effector function signals.

Preferred examples of the intracellular signaling domain for use in the chimeric receptor of the present disclosure include the cytoplasmic sequences of a T cell receptor (TCR) and co-receptors that act cooperatively so as to initiate signal transduction after engagement between an antigen and the receptor, and any derivative or mutant of these sequences and any synthetic sequence having the same functional ability.

It is known in the art that: signals generated through TCR alone are insufficient for the complete activation of T cells; and a secondary signal or a costimulatory signal is also required. For this reason, it can be said that T cell activation is mediated by two separate classes of cytoplasmic signaling sequences: a sequence that initiates antigen-dependent primary activation through TCR (primary cytoplasmic signaling sequence), and a sequence that acts in an antigen-independent manner to bring about a secondary signal or a costimulatory signal (secondary cytoplasmic signaling sequence).

Prior to expansion and genetic alteration of T cells according to the present disclosure, a source of the T cells is obtained from a subject. The T cells can be obtained from many sources including peripheral blood mononuclear cells, bone marrow, lymph node tissues, cord blood, thymus tissues, tissues derived from infection sites, ascites fluid, pleural effusion, spleen tissues, and tumor. In a certain aspect of the present disclosure, any of various T cell lines available in the art can be used. In a certain aspect of the present disclosure, the T cells are obtained from a blood unit collected from a subject by use of any of various approaches known to those skilled in the art, such as Ficoll(TM) separation. In a preferred aspect, cells from the circulating blood of an individual are obtained by apheresis. The apheresis product typically contains lymphocytes including T cells, monocytes, granulocytes, and B cells, other nucleated white blood cells, red blood cells, and platelet. In one aspect, the cells collected by apheresis can be washed to remove a plasma fraction, and the resulting cells can be placed in an appropriate buffer solution or medium for a subsequent treatment stage. In one aspect of the present disclosure, the cells are washed with phosphate buffered saline (PBS). In an alternative aspect, the washing solution is free from calcium and free from magnesium, or is free from many, albeit not all, divalent cations. In this case as well, surprisingly, an initial activation stage in the absence of calcium enhances activation. Those skilled in the art would readily understand that the washing stage can be achieved by a method known in the art, for example, by using a semi-automated "flow-through" centrifuge (e.g., Cobe 2991 cell processor, Baxter CytoMate, or Haemonetics Cell Saver 5) according to the manufacturer's instructions. The cells thus washed can be resuspended in, for example, various biocompatible buffer solutions, such as $Ca^{2+}$-free, $Mg^{2+}$-free PBS, PlasmaLyte A, or other saline solution with or without a buffer. Alternatively, the undesired components of the apheresis sample may be removed, and the cells can be resuspended directly in a culture medium.

In general, T cells for expressing the desired chimeric receptor can be activated and thereby expanded, either before or after genetic alteration of the T cells, by use of methods described in, for example, U.S. Patent Nos. 6,352,694, 6,534,055, 6,905,680, 6,692,964, 5,858,358, 6,887,466, 6,905,681, 7,144,575, 7,067,318, 7,172,869, 7,232,566, 7,175,843, 5,883,223, 6,905,874, 6,797,514, and 6,867,041; and U.S. Patent Application Publication No. 20060121005.

The T cells of the present disclosure are generally expanded by contact with a surface attached to an agent that stimulates CD3/TCR complex-associated signals, and a ligand that stimulates a costimulatory molecule on the surface of the T cells. Particularly, a T cell population can be stimulated, as described in the present specification, for example, by contact with an anti-CD3 antibody or antigen binding fragment thereof, or an anti-CD2 antibody immobilized on surface, or by contact with a protein kinase C activator (e.g., bryostatin) involving a calcium ionophore. For the co-stimulation of an accessory molecule on the surface of the T cells, a ligand that binds to the accessory molecule is used. For example, a population of T cells can be contacted with an anti-CD3 antibody and an anti-CD28 antibody, under conditions suitable for stimulating the growth of the T cells. In order to stimulate the growth of either $CD4^+$ T cells or $CD8^+$ T cells, an anti-CD3 antibody and an anti-CD28 antibody can be used. Examples of the anti-CD28 antibody include 9.3, B-T3, XR-CD28 (Diaclone, Besancon, France), which can be used in the same way as in other methods generally known in the art (Berg et al., Transplant Proc. 30 (8): 3975-3977, 1998; Haanen et al., J. Exp. Med. 190 (9): 13191328, 1999; and Garland et al., J. Immunol Meth. 227 (1-2): 53-63, 1999).

The scope of the present disclosure includes a cell (e.g., a T cell) transduced with a lentivirus vector (LV). For example, the LV encodes a chimeric receptor comprising an antigen recognition domain of a specific antibody combined with an intracellular domain of CD3-ζ, CD28, or 4-1BB or any combination thereof. Optionally, the transduced T cell can therefore induce T cell response mediated by the chimeric receptor.

The present disclosure provides use of CAR for reineducing the specificity of primary T cells toward a tumor antigen. Thus, the present disclosure also provides a method for stimulating T cell-mediated immune response to a target cell population or tissue in a mammal, comprising the step of administering T cells expressing CAR to the mammal, wherein the CAR comprises a binding moiety that specifically interacts with the predetermined target, for example, a ζ chain moiety comprising an intracellular domain of human CD3ζ, and a costimulatory signaling region.

In one aspect, the present disclosure includes a type of cell therapy which involves genetically altering T cells so as to express a chimeric receptor, and transfusing the CAR-T cells to a recipient in need thereof. The transfused cells can kill tumor cells in the recipient. Unlike antibody therapy, the CAR-T cells can replicate *in vivo* to bring about long-term viability probably leading to sustained tumor control.

Techniques for producing bispecific antibodies from antibody fragments are also described in literatures. The bispecific antibody can be prepared using, for example, a chemical bond. Brennan et al., Science, 229: 81 (1985) describe procedures of proteolytically cleaving intact antibodies to produce F(ab')$_2$ fragments. These fragments are reduced in the presence of a dithiol complexing agent sodium arsenite to stabilize vicinal dithiol and prevent intermolecular disulfide formation. The produced Fab' fragments are subsequently converted to thionitrobenzoate (TNB) derivatives. One of the Fab'-TNB derivatives is subsequently reconverted to Fab'-thiol by reduction with mercaptoethylamine and mixed with an equimolar amount of the other Fab'-TNB derivative to form a bispecific antibody. The prepared bispecific antibody can be used as an agent for the selective immobilization of enzymes.

In recent years, Fab'-SH fragments have been easily recovered directly from *E. coli,* and thereby are chemically coupled to form a bispecific antibody. Shalaby et al., J. Exp. Med., 175: 217-225 (1992) describe the production of a fully humanized bispecific antibody F(ab')$_2$ molecule. Respective Fab' fragments are separately secreted from *E. coli* and chemically coupled *in vitro* to form a bispecific antibody. Thus, the formed bispecific antibody was able not only to bind to cells overexpressing the HER2 receptor and normal human T cells but to cause the lytic activity of human cytotoxic lymphocytes against human breast tumor targets.

Various methods for preparing and separating bispecific antibody fragments directly from recombinant cell culture are also described. For example, the bispecific antibody has been produced using leucine zipper (Kostelny et al., J. Immunol., 148 (5): 1547-1553 (1992)). Leucine zipper peptides from the Fos and Jun proteins were linked to Fab' moieties of two different antibodies by gene fusion. The antibody homodimer is reduced at the hinge region to form monomers, which are then reoxidized to form an antibody heterodimer. This method can also be used for the production of antibody homodimers. "Diabody" technology described by Hollinger et al., Proc. Natl. Acad. Sci. USA, 90: 6444-6448 (1993) has provided another mechanism for preparing bispecific antibody fragments. The fragments comprise a heavy chain variable domain (VH) linked to a light chain variable domain (VL) by a linker which is too short to allow pairing between the two domains on the same chain. Thus, the VH and VL domains of one fragment are forced to pair with the complementary VL and VH domains of another fragment to form two antigen binding sites. Another strategy for producing bispecific antibody fragments using single chain Fv (sFv) dimers has also been reported. See Gruber et al., J. Immunol., 152: 5368 (1994).

Antibodies with more than bivalence are also possible. For example, trispecific antibodies can be prepared (Tutt et al., J. Immunol. 147: 60 (1991)).

A multivalent antibody may be internalized (and/or catabolized) faster than a bivalent antibody by cells expressing an antigen to which these antibodies bind. The antibody of the present disclosure may be a multivalent antibody (of class other than IgM) having three or more antigen binding sites (e.g., a tetravalent antibody), and can be easily produced by the recombinant expression of a nucleic acid encoding the polypeptide chain of the antibody. The multivalent antibody has a dimerization domain and three or more antigen binding sites. The dimerization domain preferably has (or consists of) an Fc region or a hinge region. In this scenario, the antibody may have an Fc region and three or more antigen binding sites at the amino terminus of the Fc region. In this context, the multivalent antibody preferably has (or consists of) three to eight, preferably four, antigen binding sites. The multivalent antibody has at least one polypeptide chain (preferably two polypeptide chains), and the polypeptide chain(s) comprises two or more variable domains. The polypeptide chain(s) may have, for example, VD1- $(X1)_n$-VD2- $(X2)_n$-Fc wherein VD1 is a first variable domain, VD2 is a second variable domain, Fc is one polypeptide chain of an Fc region, X1 and X2 each represent an amino acid or a polypeptide, and n is 0 or 1. The polypeptide chain(s) may have, for example: a VH-CH1-flexible linker-VH-CH1-Fc region chain; or a VH-CH1-VH-CH1-Fc region chain. In this context, the multivalent antibody preferably further has at least two (preferably four) light chain variable domain polypeptides. In this context, the multivalent antibody may have, for instance, approximately 2 to approximately 8 light chain variable domain polypeptides. The light chain variable domain polypeptides contemplated here have a light chain variable domain and, optionally, further have a CL domain.

In some embodiments, the amino acid sequence modification of the antibody disclosed herein is contemplated. For example, it may be desirable to be able to improve the binding affinity and/or other biological characteristics of the antibody. An amino acid sequence mutant of the antibody is prepared by introducing appropriate nucleotide change into a nucleic acid of the antibody, or by peptide synthesis. Such a modification includes, for example, deletion of, insertion into, or substitution of, residues within the amino acid sequence of the antibody. Any combination of deletion, insertion, and substitution is made as long as a final construct has the desired feature. The amino acid change may be introduced in a subject antibody amino acid sequence when the sequence is formed.

A method useful for identification of particular residues or regions of the antibody that are positions preferred for mutagenesis is called "alanine scanning mutagenesis" as disclosed by Cunningham and Wells, Science, 244: 1081-1085 (1989). In this context, a targeted residue or group of residues are identified (e.g., charged residues such as arg, asp, his, lys, and glu) and substituted by a neutral or negatively charged amino acid (most preferably alanine or polyalanine) to influence the interaction of amino acids with an antigen. Subsequently, the amino acid positions that exhibit functional sensitivity to the substitution are refined by introducing further or other mutants at or for the sites of the substitution. Although the site where an amino acid sequence mutant is to be introduced is predetermined in this way, the properties of the mutation itself do not have to be predetermined. For example, in order to analyze the function of a mutation at any site, ala scanning or random mutagenesis is carried out at the target codon or region, and the expressed immunoglobulins are screened for the desired activity.

The amino acid sequence insertion includes amino-terminal fusion and/or carboxy-terminal fusion over lengths from one residue to a polypeptide having 100 or more residues, and the intrasequence insertion of single or multiple amino acid residues. Examples of the terminal insertion include an antibody having an N-terminal methionyl residue, and an antibody fused to a cytotoxic polypeptide. Other insertion mutants of the antibody molecule include the fusion of a polypeptide that increases the serum half-life of the antibody, or an enzyme (e.g., for ADEPT) to the N terminus or C terminus of the antibody.

The glycosylation of polypeptides is typically either N-linked or O-linked. The N-linked glycosylation means the attachment of a carbohydrate moiety to the side chain of an asparagine residue. Tripeptide sequences asparagine-X-serine and asparagine-X-threonine (wherein X is any amino acid except for proline) are recognition sequences for the enzymatic attachment of the carbohydrate moiety to the asparagine side chain. Thus, the presence of either of these tripeptide sequences in a polypeptide creates a potential glycosylation site. The O-linked glycosylation means the attachment of one of the sugars N-acetylgalactosamine, galactose, and xylose to a hydroxyamino acid, most generally serine or threonine, though 5-hydroxyproline or 5-hydroxylysine is also used.

The addition of a glycosylation site to the antibody is conveniently achieved by changing the amino acid sequence such that the amino acid sequence comprises one or more of the tripeptide sequences (for N-linked glycosylation sites) mentioned above. The change is also made by the addition of or substitution by one or more serine or threonine residues to the sequence of the original antibody (for O-linked glycosylation sites).

When the antibody contains an Fc region, carbohydrate attached thereto may be changed. For example, an antibody having a mature carbohydrate structure devoid of fucose attached to an Fc region of the antibody is described in U.S. Patent Publication No. 2003/0157108 (Presta, L.). See also U.S. Patent Publication No. 2004/0093621 (Kyowa Hakko Kogyo Co., Ltd.). An antibody having bisecting N-acetylglucosamine (GlcNAc) in carbohydrate attached to an Fc region of the antibody is referenced in International Publication No. WO 03/011878, Jean-Mairet et al., and U.S. Patent No. 6602684, Umana et al. An antibody having at least one galactose residue in oligosaccharide attached to an Fc region of the antibody has been reported in International Publication No. WO 97/30087, Patel et al. For an antibody having changed carbohydrate attached to the Fc region of the antibody, see also International Publication No. WO 98/58964 (Raju, S.) and International Publication No. WO 99/22764 (Raju, S.). For an antigen binding molecule having modified glycosylation, see U.S. Patent Publication No. 2005/0123546 (Umana et al.).

A preferred glycosylation mutant in the present disclosure contains an Fc region, and a carbohydrate structure attached to the Fc region lacks fucose. Such a mutant has an improved ADCC function. Optionally, the Fc region further has one or more amino acid substitutions to further improve ADCC, for example, substitutions at positions 298, 333 and/or 334 of the Fc region (Eu numbering of residues). Examples of the literature regarding "afucosylated" or "fucose-deficient" antibodies include the following: U.S. Patent Publication No. 2003/0157108; International Publication No. WO 2000/61739; International Publication No. WO 2001/29246; U.S. Patent Publication No. 2003/0115614; U.S. Patent Publication No. 2002/0164328; U.S. Patent Publication No. 2004/0093621; U.S. Patent Publication No. 2004/0132140; U.S. Patent Publication No. 2004/0110704; U.S. Patent Publication No. 2004/0110282; U.S. Patent Publication No. 2004/0109865; International Publication No. WO 2003/085119; International Publication No. WO 2003/084570; International Publication No. WO 2005/035586; International Publication No. WO 2005/035778; International Publication No. WO 2005/053742; Okazaki et al., J. Mol. Biol. 336: 1239-1249 (2004); and Yamane-Ohnuki et al., Biotech. Bioeng. 87: 614 (2004). Examples of the cell line producing afucosylated antibodies include Lec 13 CHO cells deficient in protein fucosylation (Ripka et al., Arch. Biochem. Biophys. 249: 533-545 (1986); U.S. Patent Publication No. 2003/0157108, Presta, L; and International Publication No. WO 2004/056312, Adams et al., particularly, Example 11), and knockout cell lines such as $\alpha$-1,6-fucosyltransferase gene (FUT8)-knockout CHO cells (Yamane-Ohnuki et al., Biotech. Bioeng. 87: 614 (2004)).

Other forms of the mutant are amino acid substitution mutants. These mutants have the insertion of a different residue to at least one amino acid residue (at least 2, at least 3, or at least 4 or more residues) in the antibody molecule. A site of the greatest interest for substitution mutation includes a hypervariable region, though FR alternating change is also taken into consideration.

Substantial modifications in the biological properties of the antibody are achieved by selecting substitutions that differ significantly in their effect on maintaining (a) the structure of a polypeptide backbone in a substitution region, for example, a sheet or a helical conformation, (b) the charge or hydrophobicity of the molecule at a target site, or (c) the bulk of the side chain. Naturally occurring residues are divided into groups on the basis of common side chain characteristics: (1) hydrophobicity: norleucine, met, ala, val, leu, and ile; (2) neutral hydrophilicity: cys, ser, thr, asn, and gln; (3) acidity: asp and glu; (4) basicity: his, lys, and arg; (5) residues that influence chain orientation: gly and pro; and (6) aromaticity: trp, tyr, and phe.

Non-conservative substitution may require exchanging a member of one of these classes for a member of another class.

Charged amino acids are known among amino acids. In general, lysine (K), arginine (R), and histidine (H) are known as positively charged amino acids (positive-charge amino acids). Aspartic acid (D), glutamic acid (E), and the like are known as negatively charged amino acids (negative-charge amino acids). Alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), and the like are known as uncharged amino acids or nonpolar amino acids. Thus, in the present disclosure, amino acids having charges that repel each other (having the same charge) mean

(1) amino acids, one of which has positive charge and the other amino acid of which also has positive charge, and
(2) amino acids, one of which has negative charge and the other amino acid of which also has negative charge.

A certain form of the substitution mutant has the substitution of one or more hypervariable region residues of a parent antibody (e.g., a humanized or human antibody). In general, the obtained mutant selected for further development has improved biological characteristics as compared with the parent antibody from which the mutant is prepared. A convenient method for preparing such a substitution mutant includes affinity mutation using phage display. Briefly, several hypervariable region sites (e.g., 6 or 7 sites) are mutated to generate all possible amino acid substitutions at each site. The multivalent antibodies thus produced are displayed from filamentous phage particles as fusion products to a gene III product of M13 packaged within each particle. The phage-displayed mutants are subsequently screened for their biological activity (e.g., binding affinity) as disclosed herein. In order to identify candidate hypervariable region sites for modification, alanine scanning mutagenesis can be carried out to identify hypervariable region residues that significantly contribute to antigen binding. Alternatively or additionally, it may be beneficial to analyze a crystal structure of an antigen-antibody complex to identify contact points between the antibody and antigen. Such contact residues and adjacent residues are candidates for substitution according to the techniques mentioned herein. Once such mutants are produced, the panel of the mutants is screened as described herein to select an antibody having excellent characteristics in one or more relevant assays for further development.

A nucleic acid molecule encoding the amino acid sequence mutant of the antibody is prepared by various methods known in the art. These methods include, but are not limited to, isolation from a natural source (for naturally occurring amino acid sequence mutants) or preparation by oligonucleotide-mediated (or site-directed) mutagenesis, PCR mutagenesis, and cassette mutagenesis of an initially prepared mutant or non-mutant of the antibody.

It is desirable to introduce one or more amino acid modifications into an Fc region of the immunoglobulin polypeptide of the present disclosure to produce an Fc region mutant. The Fc region mutant may comprise a human Fc region sequence (e.g., a human IgG1, IgG2, IgG3 or IgG4 Fc region) having an amino acid modification (e.g., substitution) at one or more amino acid positions, including hinge cysteine modification.

In an embodiment, according to the description or teachings of the art, it is contemplated that an antibody used in the method of the present disclosure has one or more mutations, for example, in an Fc region, as compared with a corresponding wild-type antibody. Nonetheless, this antibody maintains substantially the same feature that exhibits therapeutic usefulness as that of its wild type counterpart. It is possible that a particular mutation is caused in the Fc region, resulting in change (i.e., improvement or decrease) in C1q binding and/or complement-dependent cytotoxicity (CDC), for example, as described in International Publication No. WO 99/51642. For other examples of the Fc region mutant, see Duncan & Winter Nature 322: 738-40 (1988); U.S. Patent No. 5648260; U.S. Patent No. 5,624,821; and International Publication No. WO 94/29351. International Publication Nos. WO 00/42072 (Presta) and WO 2004/056312 (Lowman) disclose an antibody mutant having improved or diminished binding to FcR. The contents of these patent literatures are specifically incorporated herein by reference. See also Shields et al., J. Biol. Chem. 9 (2): 6591-6604 (2001). An antibody having an increased half-life and improved binding to neonatal Fc receptor (FcRn), which is responsible for the transfer of maternal IgG to a fetus (Guyer et al., J. Immunol. 117: 587 (1976); and Kim et al., J. Immunol. 24: 249 (1994)), is disclosed in US2005/0014934A1 (Hinton et al.). These antibodies comprise an Fc region having one or more substitutions that improve the binding of the Fc region to FcRn. A polypeptide mutant having the increased or decreased ability to bind to C1q by changing an Fc region amino acid sequence is disclosed in U.S. Patent No. 6194551B1 and International Publication No. WO 99/51642. The contents of these patent literatures are specifically incorporated herein by reference. See also Idusogie et al., J. Immunol. 164: 4178-4184 (2000).

The antibody of the present disclosure can be further modified so as to comprise a further non-protein moiety that is known in the art and is readily available. Preferably, the moiety suitable for the derivatization of the antibody is a water-soluble polymer. Non-limiting examples of the water-soluble polymer include, but are not limited to, polyethylene glycol (PEG), ethylene glycol/propylene glycol copolymers, carboxymethylcellulose, dextran, polyvinyl alcohol, polyvinylpyrrolidone, poly-1,3-dioxolane, poly-1,3,6-trioxane, ethylene/maleic anhydride copolymers, polyamino acids (homopolymers or random copolymers), and dextran or poly(n-vinylpyrrolidone) polyethylene glycol, propylene glycol homopolymers, prolypropylene oxide/ethylene oxide copolymers, polyoxyethylenated polyol (e.g., glycerol), and mixtures thereof. Polyethylene glycol propionaldehyde may be advantageous in production because of its stability in water. The polymer may have any molecular weight, and may be branched or non-branched. The number of polymers bound to the antibody may vary. More than one polymer, when bound thereto, may be the same or different molecules. In general, the number and/or type of polymers for use in derivatization can be determined on the basis of considerations including, but are not limited to, whether to use the antibody derivative in treatment under defined conditions, and the particular properties or function of the antibody to be improved.

Purified antibodies can be further characterized by a series of assays including, but are not limited to, N-terminal sequencing, amino acid analysis, non-denaturing size exclusion high-performance liquid chromatography (HPLC), mass spectrometry, ion-exchange chromatography and papain digestion.

In a particular embodiment of the present disclosure, the secondary antibody produced herein is analyzed for its biological activity. In an embodiment, the secondary antibody of the present disclosure is tested for its antigen binding activity. Antigen binding assay that is known in the art and is available herein includes, but is not limited to, any direct or competitive binding assay using techniques such as Western blot, radioimmunoassay, ELISA (enzyme-linked immunosorbent assay), "sandwich" immunoassay, immunoprecipitation assay, fluorescent immunoassay, and protein A immunoassay. The antigen binding assay and other assays will be described in the section of Examples given below.

In the ELISA format, the binding activity of a test antigen binding molecule against an antigen is quantitatively evaluated by comparing the levels of signals generated through enzymatic reaction. Specifically, a test polypeptide in an associated form is added to an ELISA plate with a protein or the like comprising the antigen, immobilized thereon. Then, the test antigen binding molecule bound with the antigen is detected through the use of an enzyme-labeled antibody that recognizes the test antigen binding molecule. Alternatively, in FACS, a dilution series of a test antigen binding molecule is prepared, and the antibody binding titer for the antigen can be determined to compare the binding activity of the test antigen binding molecule against the antigen.

The value of antigen binding activity that may be used can be kd (dissociation rate constant) when the antigen is a soluble molecule, and apparent kd (apparent dissociation rate constant) when the antigen is a membrane molecule. The kd (dissociation rate constant) and the apparent kd (apparent dissociation rate constant) can be measured by methods known to those skilled in the art. For example, Biacore (GE Healthcare Japan Corp.) or a flow cytometer can be used. When the binding activity of an antigen binding domain (or an antigen binding molecule comprising the domain) against an antigen is measured at a certain concentration of a compound specific for a target tissue in the present invention, it is preferred that conditions other than the concentration of the compound be the same.

Antigen binding domain dependent on target tissue-specific compound

For example, the approaches described in the preceding section about binding activity are appropriately applicable to obtain an antigen binding domain (or an antigen binding molecule comprising the domain) whose binding activity against an antigen varies according to a concentration of a compound specific for a target tissue, i.e., an antigen binding domain (or an antigen binding molecule comprising the domain) dependent on a compound specific for a target tissue. In one non-limiting aspect, some specific examples thereof will be illustrated below. For example, in order to confirm that the binding activity of an antigen binding domain (or an antigen binding molecule comprising the domain) against an antigen varies to be higher in the presence of a compound specific for a target tissue than that of the antigen binding domain (or the antigen binding molecule comprising the domain) against the antigen in the absence of the compound, the binding activity of the antigen binding domain (or the antigen binding molecule comprising the domain) against the antigen is compared between in the absence and the presence of the compound specific for a target tissue, or in the presence of a low concentration and in the presence of a high concentration. In another non-limiting aspect, in order to confirm that the binding activity of an antigen binding domain (or an antigen binding molecule comprising the domain) against an antigen varies to be higher in the presence of a high concentration of a compound specific for a target tissue than that of the antigen binding domain (or the antigen binding molecule comprising the domain) against the antigen in the presence of a low concentration of the compound, the binding activity of the antigen binding domain (or the antigen binding molecule comprising the domain) against the antigen is compared between in the presence of the low concentration of the compound specific for a target tissue and in the presence of the high concentration thereof. See, for example, WO2013180200 and US20190359704.

For example, the antigen binding domain (or the antigen binding molecule comprising the domain) whose binding activity against an antigen in the absence of a compound specific for a target tissue is lower than that against the antigen in the presence of the compound according to one aspect provided by the present invention can be obtained by the screening of antigen binding domains (or antigen binding molecules), comprising the following steps (a) to (c):

(a) obtaining the antigen binding activity of the antigen binding domains (or the antigen binding molecules) in the absence of the compound specific for a target tissue;
(b) obtaining the antigen binding activity of the antigen binding domains (or the antigen binding molecules) in the presence of the compound specific for a target tissue; and
(c) selecting an antigen binding domain (or an antigen binding molecule) whose antigen binding activity in the absence of the compound specific for a target tissue is lower than that in the presence of the compound.

For example, the antigen binding domain (or the antigen binding molecule comprising the domain) whose binding activity against an antigen in the presence of a low concentration of a compound specific for a target tissue is lower than that against the antigen in the presence of a high concentration of the compound according to one aspect provided by the present invention can be obtained by the screening of antigen binding domains (or antigen binding molecules), comprising the following steps (a) to (c):

(a) obtaining the antigen binding activity of the antigen binding domains (or the antigen binding molecules) in the presence of the low concentration of the compound specific for a target tissue;
(b) obtaining the antigen binding activity of the antigen binding domains (or the antigen binding molecules) in the presence of the high concentration of the compound specific for a target tissue; and
(c) selecting an antigen binding domain (or an antigen binding molecule) whose antigen binding activity in the presence of the low concentration of the compound specific for a target tissue is lower than that in the presence of the high concentration of the compound.

If the inhibition of cell growth is desired, a test can be conducted by *in vitro* and/or *in vivo* assay to measure the inhibition of cell growth. If it is desirable to or not to promote apoptosis, a test can be conducted by assay to measure apoptosis. Methods for examining the growth and/or proliferation of cancer cells or for determining the apoptosis of cancer cells are well known in the art, some of which are described herein for illustration. Exemplary methods for determining cell growth and/or proliferation or apoptosis include, for example, BrdU uptake assay, MTT, [3H]-thymidine uptake (e.g., TopCount assay (PerkinElmer, Inc.)), cell survival rate assay (e.g., CellTiter-Glo (Promega Corp.)), DNA fragmentation assay, caspase activation assay, trypan blue removal, and chromatin morphology assay.

In one embodiment, the present disclosure is directed to an antibody that possesses an effector function. In an embodiment, the Fc activity of the antibody is measured. *In vitro* and/or *in vivo* cytotoxicity assay can be conducted to confirm the decrease and/or depletion of CDC and/or ADCC activity. For example, Fc receptor (FcR) binding assay can be conducted to confirm that the antibody lacks FcγR binding (i.e., almost lacks ADCC activity) but maintains the ability to bind to FcRn. NK cells which are primary cells involved in ADCC express only FcγRIII, whereas mononuclear cells express FcγRI, FcγRII and FcγRIII. FcR expression in hematopoietic cells is summarized in Table 3 in page 464 of Ravetch and Kinet, Annu. Rev. Immunol 9: 457-92 (1991). Examples of *in vitro* assay for evaluating the ADCC activity of the molecule of interest are described in U.S. Patent No. 5500362 or 5821337. Assay for detecting ADCC activity is also illustrated in the present specification. Effector cells useful for such assay include peripheral blood mononuclear cells (PBMCs) and natural killer (NK) cells. Alternatively or additionally, the ADCC activity of the molecule of interest can be evaluated *in vivo* in animal models, for example, as disclosed in Clynes et al., PNAS (USA) 95: 652-656 (1998). C1q binding assays may be conducted to confirm that the antibody cannot bind to C1q, i.e., lacks CDC activity. In order to evaluate complement activation, CDC assay may be conducted, for example, as described in Gazzano-Santoro et al., J. Immunol. Methods 202: 163 (1996). FcRn binding and *in vivo* clearance and/or half-life can also be measured by use of methods known in the art.

For the recombinant production of the antibody of the present disclosure, a nucleic acid encoding the antibody is isolated and inserted into a replicable vector for further cloning (amplification of DNA) or expression. DNA encoding the antibody is easily isolated and sequenced by conventional procedures (e.g., using oligonucleotide probes that can specifically bind to genes encoding the heavy chain and light chain of the antibody). Many vectors are available. The vector is selected depending to some extent on the host cells used. In general, preferred host cells are cells derived from a prokaryote or a eukaryote (generally a mammal). Constant regions of any isotype, including IgG, IgM, IgA, IgD and IgE constant regions, may be used for this purpose. It will be understood that such constant regions can be obtained from any of human and animal species.

A polynucleotide sequence encoding a polypeptide component of the antibody of the present disclosure can be obtained by use of a standard recombination technique. The desired polynucleotide sequence can be isolated and sequenced from antibody-producing cells such as hybridoma cells. Alternatively, the polynucleotide can be synthesized using nucleotide synthesizer or PCR. Once obtained, the sequence encoding the polypeptide is inserted into a recombinant vector that permits replication and expression of heterologous polynucleotides in prokaryotic hosts. Many vectors that are available and known in the art can be used for the purpose of the present disclosure. The selection of an appropriate vector depends mainly on the size of the nucleic acid to be inserted into the vector and a particular host to be transformed with the vector. Each vector contains various components according to a function (amplification or expression of heterologous polynucleotides, or both) and compatibility with particular host cells in which the vector resides. In general, vector components include, but are not limited to a replication origin, a selection marker gene, a promoter, a ribosome binding site (RBS), a signal sequence, a heterologous nucleic acid insert and a transcription termination sequence.

In general, a plasmid vector containing a replicon and a control sequence derived from a species compatible with host cells is used in associated with the host cells. The vector usually has a replication origin and a marking sequence capable of providing phenotypic selection in transformed cells. For example, *E. coli* is generally transformed with pBR322, a plasmid derived from an *E. coli* species. Since the pBR322 contains genes encoding ampicillin (Amp) and tetracycline (Tet) resistance, transformed cells can be easily identified. The pBR322, its derivative, or other microbial plasmids or bacteriophages also contain or are changed to contain a promoter that can be used by a microbe expressing a foreign protein. Examples of the pBR322 derivative for use in the expression of a particular antibody are described in detail in Carter et al., U.S. Patent No. 5648237.

Also, a phage vector containing a replicon and a control sequence compatible with host microbes can be used as a transforming vector in association with these hosts. For example, a bacteriophage such as □GEM(TM)-11 can be used in preparing a recombinant vector that can be used to transform sensitive host cells such as *E. coli* LE392.

The expression vector of the present disclosure may comprise two or more promoter-cistron (translation unit) pairs encoding each polypeptide component. The promoter is an untranslated sequence positioned upstream (5') of the cistron that regulates its expression. Prokaryotic promoters are typically of two classes, inducible and constitutive. The inducible promoter is a promoter that inductively increases the transcription level of the cistron under its control in response to change in culture conditions, for example, the presence or absence of a nutrient or change in temperature.

An enormous number of promoters that are recognized by various potential host cells are known in the art. The selected promoter can be operably linked to cistron DNA encoding a light chain or a heavy chain by removing the promoter from source DNA by restriction enzyme digestion and inserting the isolated promoter into the vector of the present disclosure. Both natural promoter sequences and many heterologous promoters can be used to amplify and/or express target genes. In an embodiment, a heterologous promoter is useful because the heterologous promoter generally permits greater transcription and higher efficiency of an expressed target gene as compared with the natural promoter of the target polypeptide.

Promoters preferred for use in prokaryotic hosts include PhoA promoter, β galactamase and lactose promoter systems, a tryptophan (trp) promoter system and hybrid promoters, for example, tac or trc promoter. However, other promoters that are functional in bacteria (e.g., other known bacterial or phage promoters) are also preferred. Their nucleotide sequences have been published. Accordingly, those skilled in the art can operably link these promoters to cistrons encoding a target light chain and heavy chain using linkers or adaptors that supply any necessary restriction site (Siebenlist et al., (1980) Cell 20: 269).

In one aspect of the present disclosure, each cistron within the recombinant vector comprises a secretion signal sequence component that induces the transcription of a polypeptide expressed across a membrane. In general, the signal sequence may be a component of the vector or may be a portion of target polypeptide DNA inserted into the vector. The signal sequence selected for the purpose of this invention must be recognized and processed (i.e., cleaved by signal peptidase) by host cells. For prokaryotic host cells that do not recognize but process signal sequences native to heterologous polypeptides, the signal sequence is substituted by a prokaryotic signal sequence selected from the group consisting of, for example, the alkaline phosphatase, penicillinase, Ipp, heat-stable enterotoxin II (STII) leader, LamB, PhoE, PelB, OmpA, and MBP. In one embodiment, the signal sequence for use in both cistrons of the expression system is STII signal sequence or a mutant thereof.

In another aspect, the immunoglobulin according to the present disclosure is intracytoplasmically produced by host cells, and therefore does not require the presence of a secretion signal sequence within each cistron. In this respect, an immunoglobulin light chain and heavy chain are expressed, folded and assembled to intracytoplasmically form a functional immunoglobulin. There exists a host system (e.g., *E. coli* trxB system) that exhibits cytoplasm conditions preferred for disulfide bond formation, and can preferably fold and assemble expressed protein subunits (Proba and Pluckthun Gene, 159: 203 (1995)).

Prokaryotic host cells suitable for expressing the antibody of the present disclosure include archaebacteria and eubacteria, for example, gram-negative or gram-positive organisms. Examples of the useful bacterium include the genus *Escherichia* (e.g., *E. coli),* the genus *Bacillus* (e.g., *Bacillus subtilis*), the genus *Enterobacteria, Pseudomonas* species (e.g., *Pseudomonas aeruginosa), Salmonella typhimurium, Serratia marcescens,* the genus *Klebsiella,* the genus *Proteus, Shigella, Rhizobia, Vitreoscilla,* and *Paracoccus.* In one embodiment, a gram-negative bacterium is used. In one embodiment, *E. coli* cells are used as the host of the present disclosure. Examples of the *E. coli* strain include W3110 strain (Bachmann, Cellular and Molecular Biology, vol. 2 (Washington, D.C.: American Society for Microbiology, 1987), p. 1190-1219; ATCC deposition No. 27,325) and derivatives thereof, including 33D3 strain having genotype W3110 ΔfhuA (ΔtonA) ptr3 lac Iq lacL8 ΔompTΔ (nmpc-fepE) degP41 kanR (U.S. Patent No. 5,639,635). Other strains and derivatives thereof, such as *E. coli* B, *E. coli* λ 1776 (ATCC 31,537) and *E. coli* RV308 (ATCC 31,608) are also preferred. These examples are illustrative, not limiting. Methods for constructing derivatives of any of the bacteria described above having a defined genotype are known to those skilled in the art and described in, for example, Bass et al., Proteins, 8: 309-314 (1990). It is generally necessary to select a suitable bacterium by taking into consideration the replicability of a replicon in cells of the bacterium. In the case of supplying a replicon using a well-known plasmid such as pBR322, pBR325, pACYC177, or pKN410, for example, *E. coli,* the genus *Serratia,* or a *Salmonella* species can be preferably used as the host. Typically, the host cells must secrete the minimum amount of a proteolytic enzyme, and desirably, a further protease inhibitor can be introduced during cell culture.

Host cells are transformed or transfected with the expression vector described above and cultured in a usual nutrient medium modified as suitable for inducing the promoter, selecting transformants, or amplifying the gene encoding the desired sequence. The transformation means the introduction of DNA into a prokaryotic host such that the DNA is replicable either as an extrachromosomal factor or by chromosomal integration. Depending on the host cells used, the transformation is performed by use of a standard technique suitable for such cells. Calcium treatment with calcium chloride is generally used for bacterial cells containing a substantial cell wall barrier. Another method for the transformation employs polyethylene glycol/DMSO. Yet another method is electroporation.

The prokaryotic cells for use in producing the polypeptide of the present disclosure can be grown in a medium that is known in the art and is suitable for the culture of the selected host cells. Preferred examples of the medium include Luria broth (LB) plus essential nutrient supplements. In an embodiment, the medium also contains a selective agent that is selected on the basis of the configuration of the expression vector in order to selectively permit growth of the prokaryotic cells containing the expression vector. For example, ampicillin is added to a medium for the growth of cells expressing ampicillin resistant gene.

Carbon, nitrogen, and inorganic phosphate sources as well as any necessary supplement may be contained, at appropriate concentrations to be introduced, alone or as a mixture with another supplement or medium such as a complex nitrogen source. Optionally, the culture medium may contain one or more reducing agents selected from the group consisting of glutathione, cysteine, cystamine, thioglycollate, dithioerythritol and dithiothreitol.

The prokaryotic host cells are cultured at an appropriate temperature. For example, for *E. coli* growth, the temperature preferably ranges from approximately 20°C to approximately 39°C, more preferably from approximately 25°C to approximately 37°C and is still more preferably approximately 30°C. The pH of the medium can be any pH ranging from approximately 5 to approximately 9 depending mainly on the host organism. For *E. coli,* the pH is preferably from approximately 6.8 to approximately 7.4, more preferably approximately 7.0.

In the case of using an inducible promoter in the expression vector of the present disclosure, protein expression is induced under conditions suitable for the activity of the promoter. In one aspect of the present disclosure, PhoA promoter is used for the transcriptional control of a polypeptide. Thus, the transformed host cells are cultured in a phosphate-limiting medium for induction. Preferably, the phosphate-limiting medium is C.R.A.P medium (see, e.g., Simmons et al., J. Immunol. Methods (2002), 263: 133-147). Other various inducers may be used according to the vector construct used, as known by those skilled in the art.

In one embodiment, the expressed polypeptide of the present disclosure is secreted into the periplasm of the host cells and recovered therefrom. The recovery of proteins typically involves disrupting microbes, generally by an approach such as osmotic shock, sonication or lysis. Once cells are disrupted, cell debris or whole cells can be removed by centrifugation or filtration. The proteins can be further purified, for example, by affinity resin chromatography. Alternatively, the proteins can be transported to the culture media and isolated therein. The cells can be removed from the culture, and the culture supernatant is filtered and concentrated for the further purification of the produced proteins. The expressed polypeptide can be further isolated and identified by use of generally known methods such as polyacrylamide gel electrophoresis (PAGE) and Western blot assay.

In one aspect of the present disclosure, antibody production is performed at a large scale by a fermentation method. Various large-scale fed-batch fermentation methods can be used for the production of recombinant proteins. The large-scale fermentation has a capacity of at least 1000 liters, preferably a capacity of approximately 1000 to 100000 liters. Such a fermenter employs a stirring impeller that disperses oxygen and nutrients, particularly, glucose (preferred carbon/energy source). Small-scale fermentation generally means fermentation in a fermenter having a volumetric capacity of about 100 liters or less which can range from approximately 1 liter to approximately 100 liters.

In the fermentation process, the induction of protein expression is typically started after the cells are grown under appropriate conditions to the desired density, for example, OD550 of approximately 180 to 220, at a stage where the cells are in the early stationary phase. Various inducers can be used according to the vector construct used, as known in the art and mentioned above. The cells may be grown for a short time before induction. The cells are usually induced for approximately 12 to 50 hours, which may however be a longer or shorter induction time.

In order to improve the production yield and quality of the polypeptide of the present disclosure, fermentation conditions can be variously changed. For example, in order to improve the correct assembly and folding of a secreted antibody polypeptide, the host prokaryotic cells can be cotransformed with a further vector for the overexpression of, for example, chaperone protein such as Dsb protein (DsbA, DsbB, DsbC, DsbD and/or DsbG) or FkpA (peptidylprolyl cis,trans-isomerase having chaperone activity). The chaperone protein has been demonstrated to facilitate the appropriate folding and solubility of heterologous proteins produced in bacterial host cells (Chen et al., (1999) J Bio Chem 274: 19601-19605; Georgiou et al., U.S. Patent No. 6083715; Georgiou et al., U.S. Patent No. 6027888; Bothmann and Pluckthun (2000) J. Biol. Chem. 275: 17100-17105; Ramm and Pluckthun (2000) J. Biol. Chem. 275: 17106-17113; and Arie et al. (2001) Mol. Microbiol. 39: 199-210).

In order to minimize the proteolysis of the expressed heterologous protein (particularly, susceptible to proteolysis), a certain host strain devoid of proteolytic enzymes can be used in the present disclosure. For example, the prokaryotic host cell line can be engineered to cause a gene mutation in a gene encoding known bacterial protease such as protease III, OmpT, DegP, Tsp, protease I, protease Mi, protease V, protease VI, and combinations thereof. Some *E. coli* strains that lack protease are available and are described in, for example, Joly et al., (1998), supra; Georgiou et al., U.S. Patent No. 5264365; Georgiou et al., U.S. Patent No. 5508192; and Hara et al., (1996) Microbial Drug Resistance 2: 63-72.

In an embodiment, an *E. coli* strain that lacks proteolytic enzymes and is transformed with a plasmid for the overexpression of one or more chaperone proteins is used as host cells in the expression system of the present disclosure.

Standard protein purification methods known in the art can be used. The following methods are preferred examples of purification procedures: fractionation through immunoaffinity or ion-exchange columns, ethanol precipitation, reverse-phase HPLC, chromatography through silica or on a cation-exchange resin such as DEAE, chromatofocusing, SDS-PAGE, ammonium sulfate precipitation, and gel filtration using, for example, Sephadex G-75.

In one aspect, protein A immobilized on a solid phase is used for the immunoaffinity purification of the full-length antibody product of the present disclosure. The protein A is a 41 kD cell wall protein isolated from *Staphylococcus aureus,* which binds with a high affinity to the Fc regions of antibodies (Lindmark et al., (1983) J. Immunol. Meth. 62: 1-13). The solid phase with protein A immobilized thereon is preferably glass or silica surface, more preferably column including a controlled pore glass column or a silicic acid column. In a certain method, the column is coated with a reagent such as glycerol in order to prevent the nonspecific attachment of contaminants.

In an initial step of purification, a preparation from cell cultures as described above is applied to the protein A immobilized solid phase so that the antibody of interest specifically binds to the protein A. Subsequently, the solid phase is washed to remove contaminants nonspecifically bound to the solid phase. Finally, the antibody of interest is eluted off from the solid phase.

In general, vectors comprise, but are not limited to, one or more of the following components: a signal sequence, a replication origin, one or more marker genes, an enhancer element, a promoter, and a transcription termination sequence.

The vector for use in eukaryotic host cells may contain a signal sequence or a gene of another polypeptide having a specific cleavage site at the N terminus of a mature protein or the polypeptide of interest. A preferably selected heterologous signal sequence is recognized and processed (i.e., cleaved by signal peptidase) by host cells. In expression in mammalian cells, a mammalian signal sequence and a viral secretory leader, for example, herpes simplex gD signals, can be used.

DNA of such a precursor region is ligated in reading frame to DNA encoding a multivalent antibody.

In general, replication origin components are not necessary for mammalian expression vectors. For example, an SV40 origin is typically used only because of having an early promoter.

The expression and cloning vectors contain a selection gene, also called selectable marker. The selection gene typically encodes a protein that (a) confers resistance to antibiotics or other toxins, such as ampicillin, neomycin, methotrexate or tetracycline, (b) complements auxotrophic deficiencies, if necessary, or (c) supplies important nutrients that are not obtained from complex media.

An exemplary selection method employs a drug arresting the growth of host cells. Cells successfully transformed with a heterologous gene produce a protein that confers drug resistance and accordingly survive the selection process. Examples of such dominant selection employ drugs neomycin, mycophenolic acid and hygromycin.

Other example of the selectable marker appropriate for mammalian cells include markers that permit identification of cell components capable of capturing antibody nucleic acids, for example, DHFR, thymidine kinase, metallothionein I and II, preferably primate metallothionein genes, adenosine deaminase, and ornithine decarboxylase. For example, cells transformed with DHFR selection gene are first identified by culturing all of the transformants in a medium containing methotrexate (Mtx), a competitive antagonist of DHFR. Host cells preferred for use of wild-type DHFR are an established line of Chinese hamster ovary (CHO) cells deficient in DHFR activity (e.g., ATCC CRL-9096).

Alternatively, host cells (particularly, wild-type hosts containing endogenous DHFR) transformed or cotransformed with a DNA sequence encoding an antibody, wild-type DHFR protein, and another selectable marker such as aminoglycoside 3'-phosphotransferase (APH) can be selected by cell growth in a medium containing a selective agent for a selectable marker such as an aminoglycoside antibiotic, for example, kanamycin, neomycin or G418. See U.S. Patent No. 4965199.

The expression and cloning vectors usually contain a promoter that is recognized by a host organism and is operably linked to a nucleic acid of an antibody polypeptide. Eukaryotic promoter sequences are known. Substantially all eukaryotic genes have an AT-rich region found about 25 to 30 bases upstream from a transcription initiation site. Another sequence found 70 to 80 bases upstream from the transcription initiation positions of many genes is a CNCAAT region wherein N is any nucleotide. The 3' ends of most eukaryotic genes have an AATAAA sequence serving as signals for the addition of a poly A tail to the 3' end of a coding sequence. All of these sequences are properly inserted into eukaryotic expression vectors.

The transcription of antibody polypeptides from vectors in mammalian host cells is regulated, for example, by promoters obtained from the genomes of viruses such as polyoma virus, fowlpox virus, adenovirus (e.g., adenovirus 2), bovine papilloma virus, avian sarcoma virus, cytomegalovirus, retrovirus, hepatitis B virus and simian virus 40 (SV40), by heterologous mammalian promoters, for example, actin promoter or an immunoglobulin promoter, or by heat shock promoter, as long as such promoters are compatible with the host cell system.

The early and late promoters of SV40 virus are conveniently obtained as an SV40 restriction fragment further containing a replication origin of the SV40 virus. The immediate early promoter of human cytomegalovirus is conveniently obtained as a HindIII E restriction fragment. A system that expresses DNA in mammalian hosts using bovine papilloma virus as a vector is disclosed in U.S. Patent No. 4419446. A modification of this system is disclosed in U.S. Patent No. 4601978. Alternatively, a Rous sarcoma virus long terminal repeat can be used as the promoter.

The transcription of DNA encoding the antibody polypeptide of this invention by higher eukaryotes is often enhanced by inserting an enhancer sequence into the vector. Many enhancer sequences derived from mammalian genes are currently known (globin, elastase, albumin, $\alpha$-fetoprotein and insulin). Typically, however, enhancers derived from eukaryotic cell viruses may be used. Examples thereof include SV40 enhancer on the late side of a replication origin (100 to 270 base pairs), cytomegalovirus early promoter enhancer, polyoma enhancer on the late side of a replication origin, and adenovirus enhancer. For enhancing elements for the activation of eukaryotic promoters, see also Yaniv, Nature 297: 17-18 (1982). The enhancer may be spliced into the vector at position 5' or 3' to the coding sequence of the antibody polypeptide, and is preferably positioned at a site 5' from the promoter.

The expression vector for use in eukaryotic host cells typically contains sequences necessary for the termination of transcription and the stabilization of mRNA. Such sequences can generally be obtained from 5' and, occasionally 3', untranslated regions of eukaryotic or viral DNA or cDNA. These regions contain nucleotide segments that are transcribed as polyadenylated fragments in the untranslated moiety of mRNA encoding the antibody. One useful transcription termination component is a bovine growth hormone polyadenylation region. See International Publication No. WO 94/11026 and expression vectors disclosed therein.

The host cells appropriate for cloning or expressing DNA in the vector described herein include higher eukaryote cells described in the present specification, including vertebrate host cells. The vertebrate cells are grown in culture (tissue culture) by routine procedures. Examples of the useful mammalian host cell line include: monkey kidney CV1 line transformed by SV40 (COS-7, ATCC CRL1651); human embryonic kidney line (293 or 293 cells subcloned for growth in suspension culture; Graham et al., J. Gen. Virol. 36: 59 (1977)); baby hamster kidney cells (BHK, ATCC CCL10); Chinese hamster ovary cells/- DHFR (CHO; Urlaub et al., Proc. Natl. Acad. Sci. USA 77: 4216 (1980)); mouse Sertoli cells (TM4; Mather, Biol. Reprod. 23: 243-251 (1980)); monkey kidney cells (CV1, ATCC CCL70); African green monkey kidney cells (VERO-76, ATCC CRL-1587); human cervical carcinoma cells (HELA, ATCC CCL2); canine kidney cells (MDCK, ATCC CCL34); buffalo rat liver cells (BRL3A, ATCC CRL1442); human lung cells (W138, ATCC CCL75); human liver cells (Hep G2, HB8065); mouse mammary tumor (MMT060562, ATCC CCL51); TRI cells (Mather et al., Annals N.Y. Acad. Sci. 383: 44-68 (1982)); MRC5 cells; FS4 cells; and human liver cancer line (HepG2).

Host cells are transformed with the expression or cloning vector mentioned above for antibody production and cultured in a conventional nutrient medium modified as suitable for inducing the promoter, selecting transformants, or amplifying the gene encoding the desired sequence.

The host cells for use in producing the antibody of the present disclosure can be cultured in various media. Examples of the commercially available medium include Ham's F10 (Sigma-Aldrich Co. LLC), Minimal Essential Medium ((MEM) (Sigma-Aldrich Co. LLC), RPMI-1640 (Sigma-Aldrich Co. LLC) and Dulbecco's Modified Eagle's Medium (DMEM) (Sigma-Aldrich Co. LLC), which are preferred for the culture of the host cells. Also, any of media described in Ham et al., Meth. Enz. 58: 44 (1979), Barnes et al., Anal. Biochem. 102: 255 (1980), U.S. Patent No. 4767704, 4657866, 4927762, 4560655 or 5122469, International Publication No. WO 90/03430, International Publication No. WO 87/00195, or U.S. Reissue Patent No. 30985 can be used as the medium for the host cells. Any of these media can be supplemented, if necessary, with hormone and/or other growth factors (e.g., insulin, transferrin, or epidermal growth factor), salts (e.g., sodium chloride, calcium salt, magnesium salt, and phosphate), buffers (e.g., HEPES), nucleotides (e.g., adenosine and thymidine), antibiotics (e.g., GENTAMYCIN(TM) drug), trace elements (which are defined as inorganic compounds usually present at final concentrations in a micromolar range), and glucose or an equivalent energy source. Any other necessary supplement may be contained at appropriate concentrations known to those skilled in the art. The culture conditions, for example, temperature and pH, are those previously used as to the host cells selected for expression, and will be apparent to those skilled in the art.

In the case of using a recombination technique, the antibody is intracellularly produced or secreted directly into the medium. When the antibody is intracellularly produced, particulate debris, either host cells or lysed fragments, are removed, for example, by centrifugation or ultrafiltration as a first step. When the antibody is secreted into the medium, a supernatant from such an expression system is generally first concentrated using a commercially available protein concentration filter, for example, Amicon or Pellicon ultrafiltration apparatus. A protease inhibitor such as PMSF may be included in any of the steps described above to inhibit proteolysis. Also, antibiotics may be included to prevent the growth of foreign contaminants.

An antibody composition prepared from the cells can be purified by use of, for example, hydroxyapatite chromatography, gel electrophoresis, dialysis, and affinity chromatography. Affinity chromatography is a preferred purification technique. The compatibility of protein A as an affinity ligand depends on the species and isotype of an immunoglobulin Fc region present in the antibody. The protein A can be used to purify antibodies based on human $\gamma1$, $\gamma2$, or $\gamma4$ heavy chains (Lindmark et al., J. Immunol. Meth. 62: 1-13 (1983)). Protein G is recommended for all mouse isotypes and human $\gamma3$ (Guss et al., EMBO J. 5: 1567-1575 (1986)). The matrix to which the affinity ligand is attached is most often agarose, and other matrices may also be used. Mechanically stable matrices such as controlled pore glass or poly(styrene divinyl)benzene permit faster flow rates and shorter treatment times than those achievable with agarose. When the antibody comprises a CH3 domain, Bakerbond ABX(TM) resin (J.T. Baker, Phillipsburg, N.J.), polyaspartic acid columns, chromatofocusing, SDS-PAGE, and ammonium sulfate precipitation may be used according to the multivalent antibody to be recovered.

Following a preliminary purification step, the mixed solution containing the antibody of interest and contaminants is subjected to low-pH hydrophobic interaction chromatography using an elution buffer solution having a pH of approximately 2.5 to 4.5 and preferably a low salt concentration (e.g., approximately 0 to 0.25 M salt).

The tumor may be solid tumor or may be non-solid tumor or soft tissue tumor. Examples of the soft tissue tumor include leukemia (e.g., chronic myelogenous leukemia, acute myelogenous leukemia, adult acute lymphocytic leukemia, acute myelogenous leukemia, mature B cell acute lymphocytic leukemia, chronic lymphocytic leukemia, prolymphocytic leukemia, and hairy cell leukemia) and lymphoma (e.g., non-Hodgkin's lymphoma, cutaneous T cell lymphoma, and Hodgkin's disease). The solid tumor includes every cancer of body tissues other than blood, bone marrow, or the lymphatic system. The solid tumor can be further divided into those originating from epithelial cells and those originating from non-epithelial cells. Examples of the epithelial cell solid tumor include tumors of the gastrointestinal tract, the large intestine, the breast, the prostate, the lung, the kidney, the liver, the pancreas, the ovary, the head and neck, oral cavity, the stomach, the duodenum, the small intestine, the anus, the gallbladder, the labium, the nasopharynx, the skin, the uterus, the male genital organ, the urinary organ, and the bladder. The solid tumor of non-epithelial origin includes sarcomas, brain tumor, and bone tumor. Other examples of the tumor are also described in other sections of the present disclosure.

In some embodiments, the patient of the present disclosure is subjected to a diagnostic test, for example, before and/or during treatment and after treatment. In general, in the case of carrying out a diagnostic test, a sample can be collected from a patient in need of treatment. Where the subject has a cancer, the sample for diagnosis thereof can usually be a tumor sample or other biological samples. Examples thereof specifically include, but are not limited to, biological fluids including blood, urine, saliva, ascites fluid, and derivatives such as serum and plasma.

In the present disclosure, the biological sample is a fixed sample, for example, a formalin-fixed paraffin-embedded (FFPE) sample, or is a frozen sample.

Various methods for determining mRNA or protein expression include, but are not limited to, gene expression profiling, polymerase chain reaction (PCR) including quantitative real-time PCR (qRT-PCR), microarray analysis, SAGE, MassARRAY, gene expression analysis by massively parallel signature sequencing (MPSS), proteomics, immunohistochemistry (IHC), and the like. Preferably, mRNA is quantified. Such mRNA analysis is preferably conducted by use of the technique of polymerase chain reaction (PCR), or by microarray analysis. In the case of using PCR, a preferred form of PCR is quantitative real-time PCR (qRT-PCR). In one embodiment, the expression of one or more of the genes described above is regarded as positive expression when its level is equal to or more than a median, for example, as compared with other samples of the same tumor type. The median expression level can basically be determined at the same time with the measurement of gene expression or can be determined beforehand.

The steps of a typical protocol gene expression profiling using fixed paraffin-embedded tissues as an RNA source include mRNA isolation, purification, primer extension and amplification and are described in the articles of various publications (e.g., Godfrey et al., J. Molec. Diagnostics 2: 84-91 (2000); and Specht et al., Am. J. Pathol. 158: 419-29 (2001)). Briefly, the typical process is started at the cutting of paraffin-embedded tumor tissues into approximately 10 microgram thick samples. Subsequently, RNA is extracted, and protein and DNA are removed. After analysis of the RNA concentration, RNA repair and/or amplification steps are included, if necessary, and the RNA is reverse-transcribed using a promoter specific for the gene, followed by PCR. Finally, the data is analyzed to identify the best treatment option available to the patient on the basis of a characteristic gene expression pattern identified in the tested tumor samples.

The detection of gene or protein expression can be determined directly or indirectly.

The expression, translocation or amplification of a tumor antigen in a cancer can be determined (directly or indirectly). Various diagnostic and/or prognostic assays can be used for this purpose. In one embodiment, antigen overexpression can be analyzed by IHC. Paraffin-embedded tissue sections derived from tumor biopsy may be subjected to IHC assay to accord the samples with the following antigen protein staining intensity criteria:

Score 0: No staining is observed, or membrane staining is observed in less than 10% of tumor cells.
Score 1+: Slightly or weakly perceptible membrane staining is detected in more than 10% of tumor cells. The cells are stained only at a portion of their membrane.
Score 2+: Weak to moderate complete membrane staining is observed in more than 10% of tumor cells.
Score 3+: Moderate to strong complete membrane staining is observed in more than 10% of tumor cells.

In some embodiments, tumor that manifests 0 or 1+ score for antigen overexpression may be characterized by not overexpressing the antigen, whereas tumor that manifests 2+ or 3+ score may be characterized by overexpressing the antigen.

In some embodiments, the tumor overexpressing the antigen may be rated by an immunohistochemical score corresponding to the number of copies of antigen molecules expressed per cell, and can be biochemically determined:

$$0 = 0 \text{ to } 90 \text{ copies/cell}$$

$$1+ = \text{at least approximately } 100 \text{ copies/cell}$$

2+ = at least approximately 1000 copies/cell

3+ = at least approximately 10000 copies/cell

Alternatively or additionally, FISH assay can be carried out on formalin-fixed paraffin-embedded tumor tissues to determine the presence and/or extent of antigen amplification or translocation (if any) in the tumor.

The treatment method included in the present disclosure can be performed as combination therapy. The combination therapy can further comprise one or more chemotherapeutic agents. The combined administration includes coadministration or concurrent administration using separate formulations or a single pharmaceutical formulation, and continuous administration in any order which preferably provides a period for which both (or all) the active agents exert their biological activity at the same time.

The chemotherapeutic agent, when administered, is commonly administered at a dosage known for such an agent, or optionally at a dosage lowered due to effects from combined use of the drugs or harmful adverse reactions attributed to the administration of an antimetabolite chemotherapeutic agent. The preparation and dosing schedule of such a chemotherapeutic agent can be used according to manufacturers' instruction or by the empirical determination of those skilled in the art.

Various chemotherapeutic agents that can be used in combination will be illustrated below.

In some embodiments, the chemotherapeutic agents to be combined are preferably selected from the group consisting of REVLIMID, a proteasome inhibitor (e.g., bortezomib (VELCADE) and PS342), a plant-derived agent (vincristine, vinblastine, etoposide, irinotecan, nogitecan, paclitaxel, and docetaxel), taxoid (including docetaxel and paclitaxel), vinca (e.g., vinorelbine or vinblastine), a platinum compound (e.g., carboplatin, cisplatin or nedaplatin), an aromatase inhibitor (e.g., letrozole, anastrozole, or exemestane), anti-estrogen (e.g. fulvestrant or tamoxifen), etoposide, thiotepa, an alkylating agent (e.g., cyclophosphamide or ifosfamide), methotrexate, liposomal doxorubicin, PEGylated liposomal doxorubicin, capecitabine, gemcitabine, vinorelbine, melthalin, an anticancer antibiotic (e.g., bleomycin, peplomycin, mitomycin C, doxorubicin, epirubicin, or pirarubicin), vincristine, irinotecan, a COX-2 inhibitor (e.g., celecoxib) or steroid (e.g., dexamethasone and prednisone), an antimetabolite (e.g., 5-fluorouracil, UFT (tegafur-uracil), doxifluridine, TS-1 (also abbreviated to S-1) (tegafur-gimeracil-oteracil potassium), and cytarabine). In some embodiments (e.g., an embodiment associated with the treatment of t(4;14)$^+$ multiple myeloma), dexamethasone and lenalidomide, or dexamethasone, or bortezomib, or vincristine, doxorubicin and dexamethason, or thalidomide and dexamethasone, or liposomal doxorubicin, vincristine and dexamethasone, or lenalidomide and dexamethasone, or bortezomib and dexamethasone, or bortezomib, doxorubicin, and dexamethasone are combined. In some embodiments (e.g., an embodiment associated with bladder cancer), taxane (e.g., paclitaxel or docetaxel), or pemetrexed, or methotrexate, vinblastine, doxorubicin and cisplatin, or carboplatin, or mitomycin C combined with 5-fluorouracil, or cisplatin, or cisplatin and 5-fluorouracil are combined.

The formulation, dosage, and administration of the therapeutic agents described above are determined in conformity with good medical practice. Factors that should be taken into consideration in this context include a particular disorder to be treated, a particular subject to be treated, the clinical condition of each individual patient, the cause of the disorder, a drug delivery site, an administration method, a dosing schedule, the interaction between the drugs to be combined, and other factors known to medical practitioners.

Therapeutic formulations are prepared by use of a standard method known in the art by mixing the active ingredient having the desired purity with any physiologically acceptable carrier, excipient or stabilizer (Remington's Pharmaceutical Sciences (20th edition), ed. A. Gennaro, 2000, Lippincott, Williams & Wilkins, Philadelphia, Pa.).

The formulation according to the present specification may contain two or more active compounds necessary for a particular sign to be treated, preferably compounds having complementary activities that do not adversely affect each other. Such molecules are properly used in combination in amounts effective for the intended purpose.

The therapeutic drug of the present disclosure can be administered to a human patient according to a known method such as intravenous administration as a bolus or continuous infusion over a given time, intramuscular administration, administration into oral cavity, intracerobrospinal administration, subcutaneous administration, intraarticular administration, intrasynovial administration, intrathecal administration, oral, local administration, or inhalation. An ex vivo strategy can also be used for therapeutic application.

The term "nucleic acid" or "polynucleotide" refers to deoxyribonucleic acid (DNA) or ribonucleic acid (RNA), and a polymer thereof in either single- or double-stranded form. The term "nucleic acid" includes a gene, cDNA, or mRNA. In one embodiment, the nucleic acid molecule is a synthetic (e.g., chemically synthesized) nucleic acid molecule or a recombinant nucleic acid molecule. This term encompasses a nucleic acid containing analogs or derivatives of natural nucleotides that have binding characteristics similar to those of a reference nucleic acid and are metabolized in a manner similar to that of naturally occurring nucleotides, unless particularly limited. A particular nucleic acid sequence also virtually includes conservatively engineered mutants thereof (e.g., by degenerate codon substitution), alleles, orthologs, SNPs, and complementary sequences as well as explicitly presented sequences, unless otherwise specified. Specifically, the degenerate codon substitution may be achieved by producing a sequence in which the third position of one or more selected (or all) codons is substituted with mixed base and/or deoxyinosine residues [Batzer et al., Nucleic Acid Res. 19: 5081 (1991); Ohtsuka et al., J. Biol. Chem. 260: 2605-2608 (1985); and Rossolini et al., Mol. Cell. Probes 8: 91-98 (1994)].

As used in the present disclosure, the term "nucleic acid sequence" refers to a sequence of nucleoside or nucleotide monomers consisting of natural bases, sugars and intersugar (backbone) bonds. This term also includes a modified or substituted sequence comprising a non-natural monomer or a portion thereof. The nucleic acid sequence of the present application can be a deoxyribonucleic acid sequence (DNA) or a ribonucleic acid sequence (RNA) and contains natural bases including adenine, guanine, cytosine, thymidine and uracil. These sequences may also contain modified bases. Examples of such a modified base include aza and deaza adenine, guanine, cytosine, thymidine and uracil; and xanthine and hypoxanthine.

As used in the present disclosure, the term "isolated nucleic acid" refers to a nucleic acid substantially free of cellular materials or culture media when produced by a recombinant DNA technique, or free of chemical precursors or other chemicals when chemically synthesized. The isolated nucleic acid is also substantially free of sequences naturally flanking the nucleic acid (i.e., sequences positioned at the 5' and 3' ends of the nucleic acid) from which the nucleic acid is derived. The term "nucleic acid" includes DNA and RNA, can be either double-stranded or single-stranded, and corresponds to a sense or antisense strand. The term "nucleic acid" further includes a complementary nucleic acid sequence, for example, cDNA.

The term "coding (encoding)" refers to the inherent characteristics of a specific sequence of nucleotides in a polynucleotide, such as a gene, a cDNA, or an mRNA, useful as a template for the synthesis of other polymers and high molecules in biological processes having either a defined sequence of nucleotides (e.g., rRNA, tRNA and mRNA) or a defined sequence of amino acids, and biological characteristics resulting therefrom. Thus, a gene, cDNA, or RNA encodes a protein when the transcription and translation of mRNA corresponding to the gene produce the protein in cells or other biological systems. Both a coding strand, the nucleotide sequence of which is identical to a mRNA sequence and is usually shown in a sequence listing, and a non-coding strand which is used as a template for the transcription of a gene or cDNA, can be regarded as encoding a protein or other products of the gene or the cDNA.

The term "nucleotide sequence encoding an amino acid sequence" encompasses all of nucleotide sequences that are degenerate forms of each other and nucleotide sequences encoding the same amino acid sequence, unless otherwise specified. The phrase "nucleotide sequence encoding a protein or RNA" may encompasses an intron, in some cases, to the extent that the nucleotide sequence encoding the protein is capable of containing the intron. The nucleic acid molecule is operably linkable to at least one regulatory element for the expression of the chimeric receptor.

The terms "peptide", "polypeptide", and "protein" are interchangeably used and refer to a compound constituted by amino acid residues covalently linked through peptide bonds. The protein or the peptide must contain at least two amino acids, with no limitation on the maximum number of amino acids capable of constituting the sequence of the protein or the peptide. The polypeptide includes every peptide or protein comprising two or more amino acids linked to each other through peptide bonds. As used in the present disclosure, this term refers to both a short chain also generally called peptide, oligopeptide and oligomer in the art, and a longer chain generally called protein in the art, of which there are many types. Examples of the "polypeptide" particularly include biologically active fragments, substantially homologous polypeptides, oligopeptides, homodimers, heterodimers, mutants of polypeptides, engineered polypeptides, derivatives, analogs, and fusion protein. Examples of the peptide include natural peptides, recombinant peptides, and combinations thereof.

The term "isolated polypeptide", also called "isolated protein", refers to a polypeptide substantially free of cellular materials or culture media when produced by a recombinant DNA technique, or free of chemical precursors or other chemicals when chemically synthesized.

The term "amino acid" includes all of natural amino acids and modified amino acids.

As used in the present disclosure, the term "conservative amino acid variation" is the substitution of an amino acid residue by another amino acid residue without impairing the desired characteristics of the protein.

The term "conservative sequence alteration" refers to amino acid alteration that does not significantly influence or change the binding characteristics of an antibody or an antibody fragment containing the amino acid sequence. Examples of such conservative alteration include amino acid substitution, addition and deletion. The alteration can be introduced into the antibody or the antibody fragment of the present disclosure by standard techniques known in the art, such as site-directed mutagenesis and PCR-mediated mutagenesis. The conservative amino acid substitution is substitution that replaces an amino acid residue with an amino acid residue having a similar side chain. Families of amino acid residues having similar side chains are defined in the art. These families include amino acids having a basic side chain (e.g., lysine, arginine, and histidine), amino acids having an acidic side chain (e.g., aspartic acid and glutamic acid), amino acids having an uncharged polar side chain (e.g., glycine, asparagine, glutamine, serine, threonine, tyrosine, cysteine, and tryptophan), amino acids having a nonpolar side chain (e.g., alanine, valine, leucine, isoleucine, proline, phenylalanine, and methionine), amino acids having a beta-branched side chain (e.g., threonine, valine, and isoleucine) and amino acids having an aromatic side chain (e.g., tyrosine, phenylalanine, tryptophan, and histidine). Thus, one or more amino acid residues within the chimeric receptor of the present disclosure may be replaced with other amino acid residues of the same side chain family, and the chimeric receptor thus changed can be tested by use of functional assay described in the present disclosure.

The term "expression" refers to the transcription and/or translation of a particular nucleotide sequence driven by a promoter.

The term "transfer vector" refers to a composition that comprises an isolated nucleic acid and can be used to deliver the isolated nucleic acid to the inside of a cell. Many vectors are known in the art. Examples thereof include, but are not limited to, linear polynucleotides, polynucleotides bound to ionic or amphiphilic compounds, plasmids, and viruses. Thus, the term "transfer vector" encompasses an autonomously replicating plasmid or a virus. This term is also construed to further include non-plasmid and non-viral compounds that facilitate the transfer of nucleic acids into cells, for example, a polylysine compound, a liposome, and the like. Examples of the viral transfer vector include, but are not limited to, adenovirus vectors, adeno-associated virus vectors, retrovirus vectors, lentivirus vectors, and the like.

The term "vector" refers to a polynucleotide that permits amplification of a nucleic acid molecule inserted (linked) thereto and transport thereof to host cells. This term includes a vector as a self-replicating nucleic acid structure, and a vector integrated into the genomes of host cells harboring the vector. A certain vector can bring about the expression of a nucleic acid operatively link to the vector itself. Such a vector is also referred to as an "expression vector" in the present disclosure. The expression vector can be introduced to host cells by a method using a virus, electroporation, or the like. The introduction of the expression vector is not limited to *ex vivo* introduction, and the vector may be administered directly to a living body and introduced to host cells *in vivo.* The expression vector includes all those known in the art. Examples thereof include cosmids, plasmids (e.g., naked or contained in liposomes) and viruses (e.g., lentivirus, retrovirus, adenovirus, and adeno-associated virus) in which a recombinant polynucleotide is incorporated.

The term "transfection", "transformation", or "transduction" refers to a process of transferring or introducing an exogenous nucleic acid to host cells. "Transfected", "transformed", or "transduced" cells are cells transfected, transformed, or transduced with an exogenous nucleic acid. The cells include the primary cells of interest and progeny thereof.

The term "promoter" refers to a DNA sequence that is recognized by the synthetic mechanism of cells, or an introduced synthetic mechanism, necessary for starting the specific transcription of a polynucleotide sequence. The function of a regulatory region including a promoter is essential for the transcription of DNA information into mRNA. In some cases, this region comprises a core promoter sequence and in other cases, this region may also include an enhancer sequence and other regulatory factors necessary for expression of a gene product. The region may be, for example, a sequence that causes a gene product to be expressed in a tissue-specific manner.

The term "constitutive" promoter refers to a nucleotide sequence that, when operably linked to a polynucleotide encoding or designating a gene product, causes the gene product to be produced in cells under most or all physiological conditions of the cells.

The term "inducible" promoter refers to a nucleotide sequence that, when operably linked to a polynucleotide encoding or designating a gene product, causes the gene product to be produced in cells substantially only when an inducer corresponding to the promoter is present in the cells.

The term "tissues-specific" promoter refers to a nucleotide sequence that, when operably linked to a polynucleotide encoding a gene or designated by a gene, causes the gene product to be produced in cells substantially only when the cells are cells of tissue type corresponding to the promoter.

The term "lentivirus" refers to a genus of the family *Retroviridae.* The lentivirus is unique, among the retroviruses, in being able to infect non-dividing cells. The lentivirus can deliver a significant amount of genetic information into DNA of host cells and is therefore one of the most efficient methods of a gene delivery vector. HIV, SIV, and FIV are all examples of the lentivirus.

The term "lentivirus vector" refers to a vector derived from at least a portion of the lentivirus genome. Examples thereof particularly include self-inactivating lentivirus vectors as disclosed in Milone et al., Mol. Ther. 17 (8): 1453-1464 (2009). Other examples of the lentivirus vector that may be used in clinics include, but are not limited to, LENTIVECTOR(R) gene delivery technology manufactured by Oxford BioMedica plc, LENTIMAX(TM) vector system manufactured by Lentigen Technology, Inc., and the like. Nonclinical types of lentivirus vectors are also available and would be known to those skilled in the art.

The term "antigen presenting cell" or "APC" refers to an immune system cell, such as an accessory cell (e.g., a B-cell, a dendritic cell, and the like), which presents a foreign antigen complexed with major histocompatibility complex (MHC) on its surface. T cells may recognize the complex using their T cell receptors (TCRs). APC processes the antigen and presents the antigen to T cells.

As used in the present disclosure, the term "immune effector cell" refers to a cell involved in the promotion of immune response, for example, immune effector response. Examples of the immune effector cell include T cells, for example, alpha/beta T cells and gamma/delta T cells, B cells, natural killer (NK) cells, natural killer T (NK-T) cells, mast cells, and myeloid series-derived phagocytes.

Examples of the T cells can include T cells derived from humans, and T cells derived from non-human mammals such as dogs, cats, pigs and mice. The T cells can also be obtained by isolation and purification from immunocytes infiltrating into a body fluid such as blood or bone marrow fluid, a tissue of the spleen, the thymus gland, lymph node, or the like, or a cancer tissue such as primary tumor, metastatic tumor, or cancerous ascites. Examples of such T cells can include alpha/beta T cells, gamma/delta T cells, CD8+ T cells, CD4+ T cells, tumor infiltrating T cells, memory T cells, naive T cells, and NKT cells.

As used in the present disclosure, the term "immune effector function or immune effector response" refers to, for example, a function or response of immune effector cells that enhances or promotes the immune attack of target cells. The immune effector function or response refers to, for example, the characteristics of T or NK cells that promote the killing or inhibition of growth or proliferation of target cells. In the case of T cells, primary stimulation and co-stimulation are examples of the immune effector function or response.

The term "effector function" refers to a specified function of cells. The effector function of T cells can be, for example, cytolytic activity or helper activity such as cytokine secretion.

The term "autologous" refers to every material derived from the same individual as an individual to which the material is later to be re-introduced.

The term "allogeneic" refers to every material derived from a different animal of the same species as that of an individual to which the material is introduced. Two or more individuals are said to be allogeneic to each other when genes at one or more loci are not identical. In some aspects, allogeneic material from individuals of the same species may differ genetically to an extent sufficient for interacting antigenically.

The term "heterologous" refers to a graft derived from an animal of a different species.

As used in the present disclosure, the term "subject" means all members of the animal kingdom including humans. In a preferred aspect, the subject is a human.

In the context of the present disclosure, the terms "treatment", "treat", and the like mean the relief or alleviation of at least one symptom associated with a disease condition, or the delay or reversal of progression of such a condition, as long as the terms relate to any of disease conditions recited herein. Within the scope of this meaning of the present disclosure, the term "treatment" also means arrest, the delay of onset (i.e., the time to the clinical manifestation of a disease) and/or reduction in the risk of developing or worsening a disease. For example, in connection with a cancer, the term "treatment" may mean the elimination or reduction of a patient's tumor burden, or the prevention, delay or blocking of metastasis, etc.

As used in the present disclosure and as well understood in the art, the "therapy" is an approach for obtaining beneficial or desired results, including clinical results. The beneficial or desired clinical results can include, but are not limited to, the alleviation or amelioration of one or more symptoms or pathological conditions, the diminishment of the extent of a disease, the stabilization (i.e., not worsening) of a disease state, the prevention of spread of a disease, the delay or slowing of progression of a disease, the amelioration or alleviation of a disease condition, and remission (partial or complete), regardless of whether to be detectable or undetectable. The "therapy" may also mean the prolongation of a survival period compared with an expected survival period without the treatment. The term "alleviate" a disease or a disorder means that the extent and/or undesirable clinical manifestation of a disorder or a disease condition is lessened and/or the time course of progression is slowed or lengthened, as compared with the case of not treating the disorder.

In general, the term "tumor" is a generic name for masses that develop on the surface of the body or in the inside of the body and can be touched or have a distinctively colored area. The tumor includes malignant tumor having three features, autonomous growth, infiltration and metastasis, and cachexia, and benign tumor characterized only by autonomous growth. The malignant tumor "cancer" refers to a disease characterized by the uncontrollable growth of aberrant cells. Cancer cells can spread locally or via bloodstream and the lymphatic system to other parts of the body. Examples of various cancers are described in the present disclosure and include, but are not limited to, breast cancer, prostate cancer, ovary cancer, cervical cancer, skin cancer, pancreatic cancer, colorectal cancer, kidney cancer, liver cancer, brain cancer, lymphoma, leukemia, lung cancer and the like. The terms "tumor" and "cancer" are interchangeably used in the present disclosure. Both the terms encompass, for example, solid and liquid, for example, diffuse or circulating, tumors. As used in the present disclosure, the term "cancer" or "tumor" encompasses premalignant as well as malignant cancers and tumors.

Examples of the cancer for the anticancer agent or the method for treating a cancer as mentioned later according to the present disclosure can include cancers such as adenocarcinoma, squamous cell carcinoma, adenosquamous cancer, undifferentiated cancer, large-cell cancer, small-cell cancer, skin cancer, breast cancer, prostate cancer, bladder cancer, vaginal cancer, neck cancer, uterus cancer, liver cancer, kidney cancer, pancreatic cancer, spleen cancer, lung cancer, tracheal cancer, bronchial cancer, colon cancer, small intestine cancer, stomach cancer, esophageal cancer, gallbladder cancer, testis cancer, and ovary cancer, cancers of bone tissues, cartilage tissues, fat tissues, muscle tissues, vascular tissues and hematopoietic tissues as well as sarcoma such as chondrosarcoma, Ewing's sarcoma, malignant hemangioendothelioma, malignant schwannoma, osteosarcoma, and soft tissue sarcoma, blastoma such as hepatoblastoma, medulloblastoma, nephroblastoma, neuroblastoma, pancreatoblastoma, pleuropulmonary blastoma, and retinoblastoma, germ cell tumor, lymphoma, and leukemia.

In one embodiment, the tumor antigen in association with a cancer type is a marker expressed by both normal cells and cancer cells, for example, a lineage marker such as CD19 on B cells. In a certain aspect, the tumor antigen of the present disclosure is derived from a cancer including, but is not limited to, primary or metastatic melanoma, thymoma, lymphoma, sarcoma, lung cancer, liver cancer, non-Hodgkin's lymphoma, Hodgkin's lymphoma, leukemias, uterus cancer, cervical cancer, bladder cancer, kidney cancer and adenocarcinoma, for example, breast cancer, prostate cancer, ovary cancer, pancreatic cancer, and the like. In one embodiment, the tumor antigen is an antigen common in particular proliferative disorders. In one embodiment, the tumor antigen is a cell surface molecule that is overexpressed in cancer cells compared with normal cells, for example, by 1-fold overexpression, 2-fold overexpression, 3-fold overexpression or more as compared with normal cells. In some embodiments, the tumor antigen is a cell surface molecule that is inappropriately synthesized in cancer cells, for example, a molecule containing deletion, addition or mutation as compared with a molecule expressed in normal cells. In one embodiment, the tumor antigen is expressed exclusively on the cell surface of cancer cells, as a whole or as a fragment (e.g., MHC/peptide), and neither synthesized nor expressed on the surface of normal cells. In some embodiments, the chimeric receptor and the TRAB of the present disclosure include CAR and TRAB comprising an antigen binding domain (e.g., an antibody or an antibody fragment) that binds to a peptide presented by MHC. Usually, peptides derived from endogenous proteins fill the pockets of major histocompatibility complex (MHC) class I molecules, and are recognized by T cell receptors (TCRs) on CD8[+] T lymphocytes. The MHC class I complex is constitutively expressed by all nucleated cells. In cancers, virus-specific and/or tumor-specific peptide/MHC complexes are representative of a unique class of cell surface targets for immunotherapy. TCR-like antibodies targeting virus- or tumor antigen-derived peptides in the context of human leukocyte antigen (HLA)-A1 or HLA-A2 are described [see e.g., Sastry et al., J Virol.2011 85(5): 1935-1942; Sergeeva et al., Bood, 2011 117(16): 4262-4272; Verma et al., J Immunol 2010 184(4): 2156-2165; Willemsen et al., Gene Ther 2001 8(21): 1601-1608; Dao et al., Sci Transl Med 2013 5(176): 176ra33;Tassev et al., Cancer Gene Ther 2012 19(2): 84-100]. The TCR-like antibody can be identified, for example, by screening a library such as a human scFv phage display library.

As used in the present disclosure, the phrase "treating or preventing a cancer" means the inhibition of cancer cell replication, the provision of antitumor immunity, the inhibition of cancer spread (metastasis), the inhibition of tumor growth, reduction in the number of cancer cells or tumor growth, or the amelioration of cancer-related symptoms.

As used in the present disclosure, the terms "prevent," "preventing" and "prevention" refer to an action that is performed before a subject begins to suffer from the condition or before relapse of the condition, unless otherwise specified. The prevention does not have to bring about the complete prevention of the condition. This term encompasses the partial prevention or alleviation of the condition or a symptom of the condition, or reduction in the risk of developing the condition.

The term "anticancer effect" refers to a biological effect that can be demonstrated by various approaches including but are not limited to, for example, decrease in tumor volume, decrease in the number of cancer cells, decrease in the number of metastases, increase in life expectancy, decrease in cancer cell growth, decrease in cancer cell survival, and amelioration of various physiological symptoms associated with a cancerous condition. The "anticancer effect" can also be demonstrated by the ability of the peptide, the polynucleotide, the cell and the antibody described in the present disclosure in the prevention of the occurrence of a cancer at a primary location. The term "antitumor effect" refers to a biological effect that can be demonstrated by various approaches including but are not limited to, for example, decrease in tumor volume, decrease in the number of tumor cells, decrease in tumor cell growth, and decrease in tumor cell survival.

As used herein, the term "therapeutically effective" applied to a dosage or an amount refers to an amount of a compound or a pharmaceutical composition (e.g., a composition comprising T lymphocytes (and/or NK cells) comprising the chimeric receptor of the present disclosure, and if desired, further comprising a tumor-specific cytotoxic monoclonal antibody or another antitumor molecule comprising an Fc moiety (e.g., a fusion molecule consisting of a ligand (e.g., a cytokine or an immune cell receptor) bound to a tumor surface receptor combined with an immunoglobulin Fc moiety or Fc-containing DNA or RNA), or a composition comprising the primary antibody of the present disclosure) sufficient for causing the desired activity when the compound or the pharmaceutical composition is administered to a subject in need of treatment. The term "therapeutically effective" according to the present disclosure refers to an amount of a compound or a pharmaceutical composition effective for delaying the manifestation of, arresting the progression of, or relieving or alleviating at least one symptom of a disorder to be treated according to the method of the present disclosure.

It should be noted that when a combination of active ingredients is administered, the effective amount of the combination may or may not include the respective amounts of the ingredients that would be effective if administered individually.

As used in the present disclosure, the term "administer" means that, for example, cells expressing the chimeric receptor or the composition of the present application is administered to a patient, in a therapeutically effective amount for reducing and/or inhibiting the diffusion of cells expressing a predetermined antigen.

In the present disclosure, the term "pharmaceutically acceptable" refers to a molecular entity and other ingredients of such a composition that are physiologically tolerable and do not typically produce undesirable reactions when the composition is administered to a mammal (e.g., a human). Preferably, as used herein, the term "pharmaceutically acceptable" means approved by a regulatory agency of the Federal or a state government or listed in the U.S. Pharmacopeia or other generally recognized pharmacopeia for use in mammals, more specifically, humans.

As used in the present disclosure, the term "comprise" and its derivatives are openended terms that specify the presence of the stated features, elements, components, groups, integers, and/or steps, but do not exclude the presence of other unstated features, elements, components, groups, integers and/or steps. The foregoing also applies to words having similar meanings such as the terms "include" and "have" and their derivatives.

Definitions and embodiments described in particular sections are intended to be applicable to other embodiments of the present specification described for which they are suitable as understood by those skilled in the art.

(1) Chimeric receptor of present disclosure

In one aspect of the present disclosure, the chimeric receptor of the present disclosure comprises two signaling domains described herein, i.e., CD3 zeta and 4-1BB/CD137, or CD3 zeta as well as one or more signaling domains. In a particular aspect, several signaling domains are fused to each other for additive or synergistic effects. Non-limiting examples of the useful additional signaling domain include a portion or the whole of one or more of TCR zeta chain, CD27, CD28, OX40/CD134, 4-1BB/CD137, Fc epsilon RIy, ICOS/CD278, ILRB/CD122, IL-2RG/CD132, DAP-10 and CD40.

The present disclosure discloses a chimeric receptor comprising i) an extracellular domain capable of binding to a predetermined antigen via a mutated antibody, ii) a transmembrane domain, and iii) an intracellular segment comprising one or more intracellular signaling domains selected from a cytoplasmic costimulatory domain and/or a cytoplasmic domain of an interleukin receptor chain and a CD3 zeta intracellular signaling domain comprising an exogenous STAT3 association motif (wherein the intracellular segment comprises an endogenous or exogenous JAK binding motif and STAT5 association motif). In an embodiment, these domains are optionally fused directly or indirectly in the foregoing order starting from the N terminus. In an embodiment, these domains within the intracellular segment are fused in the inverse order.

The present disclosure also includes a chimeric receptor comprising i) an extracellular domain capable of binding to a predetermined antigen via a mutated antibody, ii) a transmembrane domain, and iii) an intracellular segment comprising one or more intracellular signaling domains including a cytoplasmic domain of an IL receptor chain and optionally at least one supplementary cytoplasmic domain. In an embodiment, these domains are optionally fused directly or indirectly in the foregoing order starting from the N terminus. In one embodiment, these domains within the intracellular segment are fused in the inverse order.

In some embodiments, the IL receptor chain is proximal to the transmembrane domain and/or is near or forms the N terminus of the intracellular segment of the chimeric receptor. In other embodiments, the IL receptor chain is near or forms the C terminus of the intracellular segment of the chimeric receptor. In some embodiments, the IL receptor chain is upstream or N-terminal from a CD3 zeta intracellular signaling domain comprising an exogenous STAT3 association motif YXXQ.

In an embodiment where the intracellular segment comprises only a signaling domain of the IL receptor chain, cells expressing the chimeric receptor can be activated by a predetermined antigen presented in an MHC complex via endogenous TCR and/or by a CD80/86 molecule via endogenous CD28, for example by B cells.

The present disclosure also provides a cell expressing a chimeric receptor. Such a cell can have, for example, a high growth rate and/or a high survival rate, produces high amounts of cytokines, and/or can have high cytotoxic activity against a cell having, on its surface, a predetermined/preselected antigen to which the chimeric receptor binds, as compared with a parent cell that does not express the chimeric receptor. For example, as shown in Examples, T cells transduced with the chimeric receptor of the present disclosure have high ability to divide and grow in primary antibody-dependent and tumor antigen-dependent manners, and provide an antitumor effect and improve an overall survival, in mice receiving the cells as treatment. Accordingly, an antitumor effect in humans is also expected.

(a) Extracellular domain

The extracellular domain used for the chimeric receptor of the present disclosure is a domain comprising a proteinous molecule or a portion thereof capable of binding to a moiety having a mutation of a targeted mutated antibody, and includes, for example, an antigen binding domain of an antibody. This domain binds to a moiety having a mutation of a targeted mutated antibody so that an antigen recognition domain of the mutated antibody interacts with a target antigen, for example, a surface antigen of target cells such as cancer cells, and thereby bridges immune effector cells expressing the chimeric receptor to the target cells so as to exert an immune effector function. The extracellular domain used for the chimeric receptor of the present disclosure comprises variable regions of an antibody (e.g., an H chain and an L chain), a single chain or a binding fragment thereof, or TCR (TCR alpha, TCR beta, TCR gamma, or TCR delta). For example, an antibody Fab fragment, antibody variable regions [H chain V region (VH) and L chain V region (VL)] or an extracellular ligand binding domain of a receptor can be used. Particularly, in an embodiment, a single chain variable fragment (scFv) can be used.

The extracellular domain of the chimeric receptor of the present disclosure may bind to only one moiety having a mutation of a targeted mutated antibody, or may bind to two or more moieties having a mutation of a targeted mutated antibody. In addition, the present disclosure includes both a chimeric receptor comprising one extracellular domain and a chimeric receptor comprising two or more extracellular domains.

The mutated antibody (also referred to as a "primary antibody") that is recognized by the extracellular domain of the chimeric receptor of the present disclosure comprises a portion of an antibody that is recognized by a target antigen, optionally a cell surface antigen or a soluble antigen, or an antibody that interacts with an antigen. Examples of the antigen for the mutated antibody include viral antigens, bacterial (particularly, infectious bacteria) antigens, parasite antigens, cell surface markers on target cells related to particular pathological conditions (e.g., tumor antigens), and surface molecules of immunocytes causing autoimmunity.

One aspect of the present disclosure provides a chimeric receptor capable of binding to an antigen derived from the family *Retroviridae* (e.g., human immunodeficiency virus, for example, HIV-1 and HIV-LP), the family *Picornaviridae* (e.g., poliovirus, hepatitis A virus, enterovirus, human coxsackievirus, rhinovirus, and echovirus), rubella virus, coronavirus, vesicular stomatitis virus, rabies virus, Ebola virus, parainfluenza virus, mumps virus, measles virus, respiratory syncytial virus, influenza virus, hepatitis B virus, parvovirus, the family *Adenoviridae,* the family *Herpesviridae* [e.g., type 1 and type 2 herpes simplex virus (HSV), varicella-zoster virus, cytomegalovirus (CMV), and herpes virus], the family *Poxviridae* (e.g. smallpox virus, vaccinia virus, and pox virus), or hepatitis C virus, via a mutated antibody that binds to the extracellular binding domain of the chimeric receptor.

One aspect of the present disclosure provides a chimeric receptor capable of binding to an antigen derived from a bacterial strain of the genus *Staphylococci,* the genus *Streptococcus, Escherichia coli, Pseudomonas,* or the genus *Salmonella,* via a mutated antibody that binds to the extracellular binding domain of the chimeric receptor. Particularly, the present disclosure provides a chimeric receptor capable of binding to an antigen derived from an infectious bacterium, for example, *Helicobacter pyloris, Legionella pneumophilia,* a bacterial strain of *Mycobacteria* sps. (e.g., *M. tuberculosis, M. avium, M. intracellulare, M. kansasii,* or *M. gordonae*), *Staphylococcus aureus, Neisseria gonorrhoeae, Neisseria meningitides, Listeria monocytogenes, Streptococcus pyogenes,* Group A *Streptococcus,* Group B *Streptococcus* (*Streptococcus agalactiae*), *Streptococcus pneumoniae,* or *Clostridium tetani,* via the mutated antibody.

In one aspect of the present disclosure, the mutated antibody that binds to the extracellular binding domain of the chimeric receptor is capable of binding to a tumor antigen such as 5T4, alpha 5 beta 1-integrin, 707-AP, AFP, ART-4, B7H4, BAGE, beta-catenin/m, Bcr-abl, MN/C IX antibody, CA125, CAMEL, CAP-1, CASP-8, CD4, CD19, CD20, CD22, CD25, CDC27/m, CD30, CD33, CD52, CD56, CD80, CDK4/m, CEA, CT, Cyp-B, DAM, EGFR, ErbB3, ELF2M, EMMPRIN, EpCam, ETV6-AML1, G250, GAGE, GnT-V, Gp100, HAGE, HER-2/new, HLA-A*0201-R170I, HPV-E7, HSP70-2M, HST-2, hTERT (or hTRT), iCE, IGF-1R, IL-2R, IL-5, KIAA0205, LAGE, LDLR/FUT, MAGE, MART-1/melan-A, MART-2/Ski, MC1R, myosin/m, MUC1, MUC-16, MUM-1, MUM-2, MUM-3, NA88-A, PAP, proteinase-3, p190 minor bcr-abl, Pml/RAR alpha, PRAME, PSA, PSM, PSMA, RAGE, RU1 or RU2, SAGE, SART-1 or SART-3, survivin, TEL/AML1, TGF beta, TPI/m, TRP-1, TRP-2, TRP-2/INT2, VEGF, WT1, NY-Eso-1 or NY-Eso-B. In the present disclosure, the antibody is also capable of binding to a cell surface adhesion molecule, a surface molecule of inflammatory cells found in autoimmune diseases, or a TCR causing autoimmunity.

(b) Intracellular segment

The intracellular segment of the chimeric receptor according to the present disclosure is a proteinous molecule that can comprise one or more intracellular signaling domains and is capable of transducing signals into cells when the extracellular domain present in the same molecule binds to (interacts with) its cognate antigen/ligand.

In an aspect, the intracellular segment of the chimeric receptor comprises a CD3 zeta intracellular signaling domain comprising an exogenous STAT3 association motif. In addition, the intracellular segment of the chimeric receptor comprises one or more intracellular signaling domains selected from a cytoplasmic domain of an IL receptor chain and/or a cytoplasmic costimulatory domain. The intracellular segment comprises an endogenous or exogenous JAK binding motif and a STAT5 association motif.

A primary cytoplasmic signaling sequence regulates the primary activation of a TCR complex. For example, the CD3 zeta intracellular signaling domain provides a primary cytoplasmic signal. The primary cytoplasmic signaling sequence may comprise a signaling motif known as an immunoreceptor tyrosine-based activation motif (ITAM) [Nature, vol. 338, pp. 383-384 (1989)]. On the other hand, a primary cytoplasmic signaling sequence that acts in an inhibitory manner may comprise a signaling motif known as an immunoreceptor tyrosine-based inhibition motif (ITIM) [J Immunol.,

vol. 162, No. 2, pp. 897-902 (1999)]. In the present disclosure, an intracellular signaling domain having ITAM and/or ITIM can be used.

In one embodiment, the ITAM motif within the CD3 zeta intracellular domain of the chimeric receptor is maintained. In one embodiment, examples of the intracellular signaling domain having ITAM that can be used instead of or to replace CD3 zeta include intracellular signaling domains having ITAM derived from FcR gamma, FcR beta, CD3 gamma, CD3 delta, CD3 epsilon, CD5, CD22, CD79a, CD79b, and CD66d. Specifically, examples of the intracellular domain comprising one or more ITAM motifs include peptides having sequences from amino acids 52 to 164 of CD3 zeta (NCBI RefSeq: NP_932170.1), from amino acids 45 to 86 of Fc epsilon RI gamma (NCBI RefSeq: NP_004097.1), from amino acids 201 to 244 of Fc epsilon RI beta (NCBI RefSeq: NP_000130.1), from amino acids 139 to 182 of CD3 gamma (NCBI RefSeq: NP_000064.1), from amino acids 128 to 171 of CD3 delta (NCBI RefSeq: NP_000723.1), from amino acids 153 to 207 of CD3 epsilon (NCBI RefSeq: NP_000724.1), from amino acids 402 to 495 of CD5 (NCBI RefSeq: NP_055022.2), from amino acids 707 to 847 of CD22 (NCBI RefSeq: NP_001762.2), from amino acids 166 to 226 of CD79a (NCBI RefSeq: NP_001774.1), from amino acids 182 to 229 of CD79b (NCBI RefSeq: NP_000617.1), and from amino acids 177 to 252 of CD66d (NCBI RefSeq: NP_001806.2), and mutants having the same functions as those of these peptides. The amino acids based on amino acid sequence information of NCBI RefSeq ID or GenBank described in the present disclosure are numbered on the basis of the full length of a precursor (comprising a signal peptide sequence, etc.) of each protein.

In an embodiment, the amino acid residue represented by "X" in the STAT3 association motif YXXQ can be any natural amino acid including any modified natural amino acid that retains STAT3 binding. In one embodiment, the amino acid X is independently selected from leucine, arginine, histidine, phenylalanine, lysine, proline, methionine, valine, glutamine, threonine, and aspartic acid. The amino acid X is, for example, arginine. The amino acid X is, for example, histidine.

In an embodiment, the two amino acid residues flanking the tyrosine residue in the STAT3 association motif YXXQ are arginine-histidine. In yet another embodiment, the exogenous STAT3 association motif is YRHQ.

The exogenous STAT3 association motif YXXQ may be introduced in any moiety of the intracellular domain of CD3 zeta. In an embodiment, the YXXQ association motif is inserted near a C-terminal region. Without wishing to be bound by a particular theory, many endogenous YXXQ motifs are probably located near or within 100 aa from the C terminus. Also, the YXXQ motif located near the C-terminal region has been shown to be more functional at a more proximal site in GP130 and LIFR studies (Schmitz J et al., J Immunol. 2000; 164: 848-54; and Tomida M et al., Blood. 1999; 93: 1934-41).

In an embodiment, the exogenous STAT3 association motif YXXQ is introduced in any moiety of the intracellular domain of CD3 zeta located within 200 amino acid residues from the C terminus of the chimeric receptor. For example, the STAT3 association motif is introduced within 200 amino acid residues, within 150 amino acid residues, within 100 amino acid residues, within 90 amino acid residues, within 80 amino acid residues, within 70 amino acid residues, within 60 amino acid residues, within 50 amino acid residues, within 40 amino acid residues, within 30 amino acid residues, within 20 amino acid residues or within 10 amino acid residues from the C terminus of the chimeric receptor. In one embodiment, the exogenous STAT3 association motif is introduced at a location other than ITAM

In an embodiment, the CD3 zeta intracellular domain comprising an exogenous STAT3 association motif comprises at least one ITAM motif. In one embodiment, the CD3 zeta intracellular domain comprising an exogenous STAT3 association motif comprises two ITAM motifs. In a further embodiment, the CD3 zeta intracellular domain comprising an exogenous STAT3 association motif comprises three ITAM motifs.

Those skilled in the art will appreciate that several methods may be used to introduce a STAT3 association motif into the intracellular signaling domain of CD3 zeta. For example, the exogenous STAT3 association motif can be introduced by substituting amino acid residues Leu-His-Met at amino acid residues 104 to 106 of the intracellular signaling domain of CD3 zeta by tyrosine at residue 104 and any other two amino acid residues at positions 105 and 106 flanking the tyrosine residue. The amino acid residues 104-105-106 of the intracellular signaling domain of CD3 zeta correspond to amino acid residues 156-157-158 of the full-length CD3 zeta (e.g., NCBI RefSeq: NP_932170.1).

As described, the chimeric receptor according to an embodiment comprises an intracellular segment comprising one or more intracellular signaling domains selected from a cytoplasmic domain of an IL receptor chain and a cytoplasmic costimulatory domain.

In the present disclosure, the cytoplasmic domain of an IL receptor chain can be selected from any chain of the IL receptor. For example, a cytoplasmic domain comprising amino acids 266 to 551 of an IL-2 receptor beta chain (NCBI REFSEQ: NP_000869.1) (amino acids 256 to 538 of an IL-21 receptor alpha chain (NCBI REFSEQ: NP_068570.1)), amino acids 284 to 369 of a common IL-2 receptor gamma chain (NCBI REFSEQ: NP_000197.1), amino acids 265 to 459 of IL-7R alpha (NCBI REFSEQ: NP_002176.2), amino acids 292 to 521 of IL-9R alpha (NCBI REFSEQ: NP_002177.2) or amino acids 257 to 825 of IL-4R alpha (NCBI REFSEQ: NP_000409.1) may be used. The whole region of the cytoplasmic domain of the IL receptor chain may be used.

Alternatively, a truncated fragment of the cytoplasmic domain of the IL receptor chain may also be used. The truncated fragment comprises, for example, up to 250 amino acids of the ILR cytoplasmic domain, or is 50 to 200 amino acids or 80 to 150 amino acids of the ILR cytoplasmic domain.

In an embodiment, the cytoplasmic domain of the IL receptor chain, optionally the truncated fragment of the cytoplasmic domain of the IL receptor chain, comprises at least a STAT association motif, optionally a STAT5 association motif, and a JAK binding motif (also known as a box-1 motif). In an embodiment, the cytoplasmic domain of the IL receptor chain or the truncated fragment thereof comprises a STAT5 association motif and a JAK binding motif.

In an embodiment, the cytoplasmic domain and/or the truncated fragment of the IL receptor chain includes mutants having the same function, for example, mutants that induce STAT signal transduction, optionally STAT5 signal transduction and/or JAK signal transduction.

In one aspect of the present disclosure, a cytoplasmic domain of an IL-2 receptor (IL-2R) beta chain may be used. Examples of the cytoplasmic domain of an IL-2R beta chain that may be used in the present disclosure include amino acids 266 to 551 of an IL-2R beta chain (NCBI RefSeq: NP_000869.1). In one embodiment, peptides having sequences from amino acids 266 to 337 and 530 to 551 are included therein. In one aspect of the present disclosure, a truncated fragment of the cytoplasmic domain of the IL-2R beta chain may be used. This truncated fragment may comprise i) a JAK binding motif (e.g., amino acids 278 to 286 of NCBI RefSeq: NP_000869.1), also called BOX-1 motif, which permits association with tyrosine kinase JAK1, and ii) a STAT association motif, optionally a STAT5 or STAT3 association motif. Other moieties of the IL receptor chain can be changed, for example, by conservative amino acid variation.

In an embodiment, the intracellular segment may comprise an exogenous JAK binding motif, or a signaling molecule comprising a JAK binding motif. The JAK binding motif is derived from, for example, IL2R gamma (IL2RG), erythropoietin receptor (EpoR), thrombopoietin receptor (TpoR), granulocyte macrophage colony stimulating factor receptor (GM-CSFR), and growth hormone receptor (GHR).

The IL-2R beta chain comprises three functional STAT5 binding motifs, YFFF, YCTF and YLSL, for use in STAT5 association. Mutations of these tyrosine residues can abolish the IL-2 reactivity of the IL-2R beta chain (Friedmann et al., 1996). The erythropoietin receptor (EpoR) comprises two tyrosine residues that mediate STAT5 activation, i.e., Y343 and Y401 both of which have a YXXL motif as described above (Klingmuller et al., 1996). Thus, YXXL can be a preferred motif for STAT5 recruitment. Other amino acid residues are also functional, for example, as shown with the IL-2R beta chain STAT5 binding motif. In one embodiment, the STAT5 association motif is an IL-2R beta chain STAT5 association motif and comprises tyrosine residue-510 (tyrosine residue 510 is amino acid 536 of NCBI RefSeq: NP_000869.1).

In an embodiment, the STAT5 association motif can be derived from IL2R gamma, EpoR, TpoR, GM-CSFR and GHR

In an embodiment, the STAT5 association motif of the IL-2R beta chain comprises amino acid residues YXXL. In an embodiment, the amino acid residue represented by "X" in the STAT5 association motif can be any natural amino acid including any modified natural amino acid that retains STAT5 binding.

Likewise, the intracellular segment comprises one or more JAK binding motifs that may be located or introduced in any of the intracellular signaling domains.

In one aspect of the present disclosure, a cytoplasmic domain of an IL-21 receptor (IL-21R) alpha chain may be used. Examples of the cytoplasmic domain of an IL-21R alpha chain that may be used in the present disclosure include intracellular signaling domains comprising amino acids 256 to 538 of an IL-21R alpha chain (NCBI RefSeq: NP_068570.1). In one aspect of the present disclosure, a truncated fragment of the cytoplasmic domain of the IL-21R alpha chain may be used. The truncated fragment comprises a box-1 motif (amino acids 266 to 274 of NCBI RefSeq: NP_068570.1) necessary for association with tyrosine kinase JAK1, and comprises a STAT association motif. In an embodiment, the STAT association motif comprises tyrosine residue-500 (amino acid 519 of NCBI RefSeq: NP_000869.1) and three residues flanking at the C-terminal side of tyrosine residue 500, i.e., YLRQ, necessary for STAT1/3 association.

Other examples of the intracellular signaling domain include cytoplasmic regions derived from a TCR complex and/or a costimulatory molecule, and any mutant having the same functions as those of these sequences. Other examples thereof include cytoplasmic signaling domains listed in Table 2 of Sadelain et al., 2009, which is incorporated herein by reference.

The activation of natural T cells is transduced by two different types of intracellular signaling domains, i.e., a domain for inducing antigen-dependent primary activation via a TCR complex (e.g., a primary cytoplasmic signal provided by, for example, CD3 zeta) and a domain that acts in an antigen-independent manner to provide a secondary or costimulatory signal (secondary cytoplasmic signal).

In one aspect, the intracellular segment of the chimeric receptor of the present disclosure comprises a CD3 zeta intracellular cytoplasmic signaling domain comprising an exogenous STAT3 association motif and optionally a secondary cytoplasmic signaling sequence.

Examples of the intracellular domain comprising the secondary or costimulatory cytoplasmic signaling domain that may be used in the present disclosure include sequences derived from CD2, CD4, CD5, CD8 alpha, CD8 beta, CD28, CD134, CD137 (4-1BB), ICOS, and CD154, for example, truncated fragments thereof comprising a signaling motif. Specific examples thereof include peptides having sequences from amino acids 236 to 351 of CD2 (NCBI RefSeq: NP_001758.2), from amino acids 421 to 458 of CD4 (NCBI RefSeq: NP_000607.1), from amino acids 402 to 495 of CD5 (NCBI RefSeq: NP_055022.2), from amino acids 207 to 235 of CD8 alpha (NCBI RefSeq: NP_001759.3), from amino acids 196 to 210 of CD8 beta (GenBank: AAA35664.1), from amino acids 180 to 220 of CD28 (NCBI RefSeq: NP_006130.1), from amino acids 214 to 255 of CD137 (4-1BB, NCBI RefSeq: NP_001552.2), from amino acids 241 to 277 of CD134 (OX40, NCBI RefSeq: NP_003318.1), and from amino acids 166 to 199 of ICOS (NCBI RefSeq: NP_036224.1), and mutants having the same functions as those of these peptides.

In one aspect, the disclosure preferably includes a chimeric receptor comprising an intracellular segment having one or more, for example, 2 or 3 intracellular signaling domains in addition to the intracellular signaling domain of CD3 zeta comprising an exogenous STAT3 association motif.

The present disclosure also includes a chimeric receptor comprising an intracellular segment having two or more same intracellular signaling domains linked in series. In one aspect, the present disclosure provides a chimeric receptor having a cytoplasmic domain of an IL receptor located on the N-terminal side of an intracellular signaling domain of CD3 zeta, i.e., a chimeric receptor comprising a cytoplasmic domain of an IL receptor and an intracellular signaling domain of CD3 zeta linked in this order from the N-terminal side. The present disclosure also includes a chimeric receptor obtained by further adding an intracellular domain of CD28 (e.g., a cytoplasmic costimulatory domain of CD28) to the chimeric receptor mentioned above, i.e., a chimeric receptor comprising an intracellular signaling domain of CD28, a cytoplasmic domain of an IL receptor, and an intracellular signaling domain of CD3 zeta comprising an exogenous STAT3 motif, linked in this order from the N-terminal side.

In an embodiment, the chimeric receptor comprises an intracellular segment comprising a CD3 zeta intracellular signaling domain comprising an exogenous STAT3 association motif and an intracellular signaling domain selected from a cytoplasmic domain of an interleukin receptor chain and a cytoplasmic costimulatory domain, wherein at least one intracellular signaling domain comprises an endogenous or exogenous JAK binding motif and a STAT5 association motif.

In one embodiment, the chimeric receptor comprises a CD3 zeta intracellular signaling domain having an exogenous STAT3 association motif, a cytoplasmic domain of an IL receptor chain fragment comprising an endogenous or exogenous JAK binding motif and a STAT5 association motif, and a cytoplasmic costimulatory domain of CD28.

In the chimeric receptor of the present disclosure, an oligopeptide linker or a polypeptide linker can be inserted between the domains of the intracellular segment so as to link the domains therein and/or to link these domains to other domains. For example, a linker having a length of 2 to 10 amino acids can be used. Particularly, a linker having a glycine-serine continuous sequence can be used. For example, a linker IDGGGGSGGGGSGGGGS can be inserted between the CD28 cytoplasmic domain and the partial cytoplasmic IL-2 receptor beta domain. For example, a linker KLGGSGP can be inserted between the partial cytoplasmic IL-2 receptor beta domain and the intracellular domain of the CD3 zeta chain.

Another aspect provides a chimeric receptor comprising i) an extracellular domain capable of binding to a predetermined antigen, ii) a transmembrane domain, and iii) an intracellular segment comprising one or more intracellular signaling domains including a cytoplasmic domain of an interleukin receptor chain and optionally a supplementary cytoplasmic domain.

The cytoplasmic domain of an IL receptor chain may be selected from any chain of the IL receptor described in the present specification. The whole region of the cytoplasmic domain of the IL receptor chain may be used. Alternatively, a truncated fragment of the cytoplasmic domain of the IL receptor chain may also be used. Examples of the full length and the truncated fragment thereof are given in the present specification.

In an embodiment, the truncated fragment may comprise at least one tyrosine kinase association motif (also known as a box-1 motif) and a STAT (signal transducer and activator of transcription) association motif described in the present specification. The truncated fragment comprises, for example, up to 250 amino acids of the ILR cytoplasmic domain, or is 50 to 200 amino acids or 80 to 150 amino acids of the ILR cytoplasmic domain.

The STAT association motif of the IL-2R beta chain comprises tyrosine residue-510 (tyrosine residue 510 is amino acid 536 of NCBI RefSeq: NP_000869.1). In an embodiment, the STAT association motif comprises tyrosine residue 510 and 4 residues flanking at the C-terminal side of tyrosine residue 510, i.e., YLSLQ.

Other STAT association motifs are also known and include, for example, YXXQ, optionally YXPQ, of IL-6, YXXQ of IL-10, YLPSNID of IL-12, YLSLQ, YCTFP and YFFFH of IL-2, YVTMS of IL-7, YLPQE of IL-9, and YKAFS and YKPFQ of IL-4. Any of the STAT signaling domains may be used and/or can be introduced into the ILR chain.

In an embodiment, the intracellular segment of the chimeric receptor comprises at least one supplementary signaling domain other than those present in the IL receptor, in addition to the cytoplasmic domain of the IL receptor. Examples of the intracellular signaling domain include a cytoplasmic region derived from a TCR complex and/or a costimulatory molecule, and any mutant having the same functions as those of these sequences.

The present disclosure includes a chimeric receptor comprising an intracellular segment comprising one or more, for example, 2 or 3 intracellular signaling domains in addition to the cytoplasmic domain of the IL receptor. The chimeric receptor comprises, for example, a cytoplasmic domain of an IL receptor and an intracellular signaling domain of CD3 zeta. The chimeric receptor comprises, for example, a cytoplasmic domain of an IL receptor, an intracellular signaling domain of CD3 zeta and a cytoplasmic costimulatory domain of CD28.

In an embodiment, the chimeric receptor comprises an intracellular segment comprising a CD3 zeta intracellular signaling domain, and one or more cytoplasmic costimulatory domains, wherein the intracellular segment comprises a JAK binding motif, a STAT5 and/or STAT3 association motif.

(c) Transmembrane domain and spacer domain

The chimeric receptor of the present disclosure comprises a transmembrane domain. The transmembrane domain may be derived from a natural polypeptide or may be artificially designed. The transmembrane domain derived from a natural polypeptide can be obtained from a membrane-associated or transmembrane protein. For example, a transmembrane domain of a T cell receptor alpha or beta chain, a CD3 zeta chain, CD28, CD3 epsilon, CD45, CD4, CD5, CD8, CD9, CD16, CD22, CD33, CD37, CD64, CD80, CD86, CD134, CD137, ICOS, CD154, or GITR can be used. The artificially designed transmembrane domain is a polypeptide mainly comprising hydrophobic residues such as leucine and valine. For example, a triplet of phenylalanine, tryptophan and valine can be found at each end of the synthetic transmembrane domain. Optionally, a short-chain oligopeptide linker or a polypeptide linker, for example, a linker having a length of 2 to 10 amino acids can be arranged between the transmembrane domain and the intracellular segment as described in the present specification. Particularly, a linker sequence having a glycine-serine continuous sequence can be used.

The transmembrane domain used can be, for example, a transmembrane domain having a sequence from amino acids 153 to 179 of CD28 (NCBI RefSeq: NP_006130.1).

In the chimeric receptor of the present disclosure, a spacer domain can be arranged between the extracellular domain and the transmembrane domain, or between the intracellular segment and the transmembrane domain. The spacer domain means any oligopeptide or polypeptide that works to link the transmembrane domain to the extracellular domain and/or the transmembrane domain to the intracellular segment. The spacer domain comprises up to 300 amino acids, for example, approximately 10 to 100 amino acids, or approximately 25 to 50 amino acids.

The spacer domain preferably has a sequence that promotes the binding of the chimeric receptor to an antigen via a mutated antibody and enhances signal transduction into cells. Examples of the amino acid that is expected to promote the binding include cysteine, charged amino acids, and serine and threonine within a potential glycosylation site. These amino acids can be used as amino acids constituting the spacer domain.

In an embodiment, the spacer domain is a polypeptide comprising or consisting of amino acids 118 to 178 of CD8 alpha (NCBI RefSeq: NP_001759.3), i.e., a hinge region of CD8 alpha, amino acids 135 to 195 of CD8 beta (GenBank: AAA35664.1), amino acids 315 to 396 of CD4 (NCBI RefSeq: NP_000607.1), amino acids 114 to 152 of CD28 (NCBI RefSeq: NP_006130.1), or a portion thereof. Further, the spacer domain may be an artificially synthesized sequence.

The chimeric receptor of the present disclosure can be designed so as to form a polymer, particularly, a dimer. For example, in order to polymerize (dimerize) the chimeric receptor, for example, via a disulfide bond, cysteine is inserted into the spacer domain and/or the transmembrane domain.

Further, in the chimeric receptor of the present disclosure, a signal peptide sequence can be linked to the N terminus. The signal peptide sequence resides at the N termini of many secretory proteins and membrane proteins, and has a length of 15 to 30 amino acids. Most protein molecules having an intracellular domain described in the present specification are membrane proteins, and have a signal peptide sequence. The signal peptide derived from such a secretory protein or a membrane protein can be used as the signal peptide for the chimeric receptor of the present disclosure. Any signal peptide can be used. The signal peptide can be, for example, an oncostatin M. signal peptide. The signal peptide can be derived from a human and may be derived from a non-human source, for example, insect cells or a virus. In an embodiment, the signal peptide is a human signal peptide.

(2) Nucleic acid encoding chimeric receptor

The present disclosure provides a nucleic acid encoding the chimeric receptor described in the present specification. The nucleic acid encoding the chimeric receptor can be easily prepared from the amino acid sequence of the defined chimeric receptor by a routine method. A nucleotide sequence encoding an amino acid sequence can be obtained from NCBI RefSeq ID or GenBank accession number mentioned above for the amino acid sequence of each domain, and the nucleic acid of the present disclosure can be prepared by use of standard molecular biological and/or chemical procedures. For example, a nucleic acid can be synthesized on the basis of the nucleotide sequence, and the nucleic acid of the present disclosure can be prepared by combining DNA fragments obtained from a cDNA library through polymerase chain reaction (PCR).

The nucleic acid of the present disclosure can be linked to another nucleic acid so as to be expressed under the control of a preferred promoter. Examples of the promoter include promoters that constitutively promote the expression of a gene or an operably linked construct, and promoters that induce the expression of a gene or an operably linked construct by the action of a drug or the like (e.g., tetracycline or doxorubicin). The nucleic acid of the present disclosure can be also linked to a nucleic acid comprising other regulatory elements, for example, an enhancer sequence or a terminator sequence, which cooperate with a promoter or a transcription initiation site, in order to obtain the efficient transcription of the nucleic acid. In addition to the nucleic acid of the present disclosure, a gene capable of serving as a marker for confirming expression of the nucleic acid (e.g., a drug resistance gene, a gene encoding a reporter enzyme, or a gene encoding a fluorescent protein) may be incorporated.

In an embodiment, the nucleic acid is a codon-optimized nucleic acid for expression in a particular host.

Composition comprising nucleic acid of the present disclosure together with pharmaceutically acceptable excipient

The present disclosure provides a composition comprising the nucleic acid of the present disclosure as an active ingredient, together with a pharmaceutically acceptable excipient. Preferred pharmaceutically acceptable excipients are well known to those skilled in the art. Examples of the pharmaceutically acceptable excipient include phosphate buffered saline (e.g., 0.01 M phosphate, 0.138 M NaCl, 0.0027 M KCl, pH 7.4), aqueous solutions containing inorganic acid salts such as hydrochloride, hydrobromide, phosphate, or sulfate, saline, solutions of glycol or ethanol, and salts of organic acids such as acetate, propionate, malonate and benzoate. Adjuvants such as a wetting agent or an emulsifier, and a pH buffering agent may be used. Excipients described in Remington's Pharmaceutical Sciences (Mack Pub. Co., N.J. 1991) (which is incorporated herein by reference) can be properly used as the pharmaceutically acceptable excipient. The composition of the present disclosure can be formulated into a known form preferred for parenteral administration, for example, injection or infusion. Further, the composition of the present disclosure may contain formulation additives such as a suspending agent, a preservative, a stabilizer and/or a dispersant, and a preservative for prolonging efficacy during preservation. The composition may be in a dry form for reconstitution with an appropriate sterile liquid before use. For administration mediated by fine particles, particles such as gold particles of a microscopic size can be coated with DNA.

In the case of introducing the nucleic acid of the present disclosure into cells *ex vivo,* the nucleic acid of the present disclosure may be combined with a substance that promotes the migration of the nucleic acid into cells, for example, a reagent for introducing a nucleic acid, such as a liposome or a cationic lipid, in addition to the excipient mentioned above. Alternatively, a vector carrying the nucleic acid of the present disclosure is also useful, as mentioned later. Particularly, a composition in a form preferred for administration to a living body, containing the nucleic acid of the present disclosure carried by a preferred vector is suitable for *in vivo* gene therapy.

The composition comprising the nucleic acid of the present disclosure as an active ingredient can be administered for the treatment of, for example, a disease such as a cancer [blood cancer (leukemia), solid tumor etc.], an inflammatory disease/autoimmune disease (e.g., asthma and eczema), hepatitis, and an infectious disease, the cause of which is a virus such as influenza and HIV, a bacterium, or a fungus, for example, tuberculosis, MRSA, VRE, or deep mycosis, depending on an antigen to which the chimeric receptor encoded by the nucleic acid binds via a mutated antibody. The composition comprising the nucleic acid of the present disclosure as an active ingredient can be administered intradermally, intramuscularly, subcutaneously, intraperitoneally, intranasally, intraarterially, intravenously, intratumorally, or into an afferent lymph vessel, by parenteral administration, for example, by injection or infusion, or can be appropriately formulated for such administration, though the administration route is not particularly limited.

(3) Method for producing cell expressing chimeric receptor

A method for producing a cell expressing the chimeric receptor of the present disclosure comprises the step of introducing a nucleic acid encoding the chimeric receptor described in the present specification into a cell. This step is performed *ex vivo.* For example, a cell can be transformed *ex vivo* with a virus vector or a non-virus vector carrying the nucleic acid of the present disclosure so as to produce a cell expressing the chimeric receptor of the present disclosure.

In the method of the present disclosure, a cell derived from a mammal, for example, a human cell, or a cell derived from a non-human mammal such as a monkey, a mouse, a rat, a pig, a horse, or a dog can be used.

In one embodiment, the mammal is a human.

The present disclosure provides a chimeric antigen receptor, a nucleic acid encoding the chimeric antigen receptor, a cell expressing the chimeric antigen receptor, and a composition comprising any of the foregoing. In an embodiment, one or more of the foregoing may be used in the field of adoptive gene immunotherapy targeting antigens such as tumor antigens, and/or in screening or other *in vitro* assays. The chimeric antigen receptor of the present disclosure can be introduced into cells to obtain, for example, enhancement or elevation in the expression level of the chimeric antigen receptor in the cells. Such cells are capable of exerting cytotoxic activity against cells expressing a target antigen.

One aspect provides a method for preparing a cell expressing the chimeric receptor disclosed in the present specification, comprising:

a) isolating an immunocyte from a mammal (optionally, the immunocyte is a T cell);
b) transfecting or transducing the isolated immunocyte (optionally, T cell) with a nucleic acid encoding the chimeric receptor described in the present specification; and
c) optionally isolating and/or culturing and expanding a chimeric receptor-expressing cell (optionally, a chimeric receptor-expressing T cell) after the transfection or the transduction.

In one embodiment, autologous T lymphocytes, autologous NK cells, or autologous macrophages are activated and/or grown *ex vivo* before re-introduction to a subject. In one embodiment, the T lymphocytes or the NK cells are allogeneic T lymphocytes or allogeneic NK cells. In one embodiment, the allogeneic T lymphocytes are T lymphocytes whose expression of an endogenous T cell receptor is blocked or eliminated. In one embodiment, allogeneic T lymphocytes or allogeneic NK cells are activated and/or grown *ex vivo* before introduction to a subject. In one embodiment, the chimeric receptor is introduced to T lymphocytes, NK cells or macrophages by a method selected from the group consisting of retroviral transduction, lentiviral transduction, DNA electroporation and RNA electroporation, DNA or RNA transfection, and genetic alteration by gene editing.

The NK cells used in the method of the present disclosure are devoid of or rarely express a major histocompatibility complex I and/or II molecule. Such a cell line includes, but is not necessarily limited to, K562 [ATCC, CCL 243; Lozzio et al., Blood 45 (3): 321-334 (1975); and Klein et al., Int. J. Cancer 18: 421-431 (1976)]. Preferably, the cell line used is devoid of or rarely expresses both the MHC I and II molecules, as in K562. Alternatively, a solid support may be used instead of the cell line. Such a support preferably is attached on its surface to at least one molecule capable of binding to NK cells and inducing an initial activation event and/or proliferative response or capable of binding a molecule having such an affect, thereby acting as a scaffold. The support may be attached on its surface to CD137 ligand protein, an anti-CD137 antibody, IL-15 protein or an anti-IL-15 receptor antibody. Preferably, the support has an anti-IL-15 receptor antibody and an anti-CD137 antibody attached on its surface.

In one embodiment, in any method of the present disclosure involving T lymphocyte activation, T lymphocytes can be activated in the presence of one or more agents selected from the group consisting of anti-CD3/CD28, IL-2 and phytohemagglutinin. In any method of the present disclosure involving NK cell activation, NK cells can be activated in the presence of one or more agents selected from the group consisting of CD137 ligand protein, anti-CD137 antibody, IL-15 protein, an anti-IL-15 receptor antibody, IL-2 protein, IL-12 protein, IL-21 protein and a K562 cell line.

The cell used in the method of the present disclosure is not particularly limited, and any cell can be used. For example, a cell collected, isolated, or purified from a body fluid, a tissue or an organ, for example, blood (peripheral blood, umbilical cord blood etc.) or bone marrow, or a cell obtained by differentiating or reprogramming (in order to prepare induce pluripotent stem cells (iPSCs)) the cell mentioned above can be used (see e.g., Themeli et al., 2013). A peripheral blood mononuclear cell (PBMC), an immune cell [including, for example, a T cell, a dendritic cell, a B cell, a hematopoietic stem cell, a macrophage, a monocyte, a NK cell or a hematopoietic cell (a neutrophil or a basophil)], an umbilical cord blood mononuclear cell, a fibroblast, a precursor adipocyte, a hepatocyte, a skin keratinocyte, a mesenchymal stem cell, an adipose stem cell, various cancer cell lines, or a neural stem cell can be used. For example, a NK cell or a T cell, a precursor cell of a T cell (a hematopoietic stem cell, a lymphocyte precursor cell etc.) or a cell population containing these cells can be used. Examples of the T cell include CD8-positive T cells, CD4-positive T cells, regulatory T cells, cytotoxic T cells, and tumor infiltrating lymphocytes. The cell population containing a T cell and a precursor cell of a T cell includes PBMCs. These cells may be collected from a living body, may be obtained by culturing and expanding cells collected from a living body, or may be established as a cell line. If the transplantation of the produced chimeric receptor-expressing cell or a cell differentiated from the produced chimeric receptor-expressing cell into a living body is desired, the nucleic acid can be introduced into a cell collected from the living body itself or a conspecific living body thereof.

In conjunction with the polynucleotide, the present disclosure also provides a vector comprising such a polynucleotide (including a vector in which such a polynucleotide is operably linked to at least one regulatory element for expression of the chimeric receptor). Non-limiting examples of the useful vector of the present disclosure include virus vectors such as retrovirus vectors and lentivirus vectors.

In a particular aspect, such a vector also comprises a suicide gene. The term "suicide gene" used herein refers to a gene that causes a cell expressing the suicide gene to die. The suicide gene can be a gene that imparts sensitivity to an agent, e.g., a drug, to a cell in which the gene is expressed, and causes the cell to die when the cell is contacted with or exposed to the agent. The suicide gene is known in the art (see e.g., Suicide Gene Therapy: Methods and Reviews, Springer, Caroline J. (Cancer Research UK Centre for Cancer Therapeutics at the Institute of Cancer Research, Sutton, Surrey, UK), Humana Press, 2004) and includes, for example, herpes simplex virus (HSV) thymidine kinase (TK) gene, and genes of cytosine deaminase, purine nucleoside phosphorylase, nitroreductase and caspases such as caspase 8.

The nucleic acid encoding the chimeric receptor of the present disclosure can be introduced to a vector, and the vector can be introduced into cells. For example, a virus vector such as a retrovirus vector (including an oncoretrovirus vector, a lentivirus vector, and a pseudotyped vector), an adenovirus vector, an adeno-associated virus (AAV) vector, a simian virus vector, a vaccinia virus vector or a Sendai virus vector, an Epstein-Barr virus (EBV) vector, and a HSV vector can be used. For example, a virus vector devoid of replicating ability so as not to self-replicate in infected cells can be used.

In addition, a non-virus vector can also be used in the present disclosure in combination with a liposome or a condensing agent such as a cationic lipid, as described in WO96/10038, WO97/18185, WO97/25329, WO97/30170 and WO97/31934 (which are incorporated herein by reference). The nucleic acid of the present disclosure may be introduced into cells by calcium phosphate transduction, DEAE-dextran, electroporation, or particle bombardment.

In the case of using, for example, a retrovirus vector, the method of the present disclosure can be performed by selecting a suitable packaging cell based on a LTR sequence and a packaging signal sequence that is possessed by the vector, and preparing a retrovirus particle using the packaging cell. Examples of the packaging cell include PG13 (ATCC CRL-10686), PA317 (ATCC CRL-9078), GP+E-86 and GP+envAm-12 (U.S. Patent No. 5,278,056), and Psi-Crip [Proceedings of the National Academy of Sciences of the United States of America, vol. 85, pp. 6460-6464 (1988)]. The retrovirus particle may be prepared using a 293 cell or a 293T cell having high transfection efficiency. Many types of retrovirus vectors produced on the basis of retrovirus and packaging cells that can be used for packaging of retrovirus vectors are widely commercially available from many companies.

The present disclosure also provides a host cell comprising the chimeric receptor. Non-limiting examples of the useful host cell include T lymphocytes and NK cells, which may be autologous or allogeneic (whose endogenous T cell receptor is removed or maintained). In a certain aspect, the host cell is an autologous T lymphocyte isolated from a patient having a cancer. In a particular aspect, such an autologous T lymphocyte is activated and grown *ex vivo.*

The chimeric receptor of the present disclosure can be introduced into the host cell by any method known in the art. Non-limiting examples of the particularly useful method include retroviral transduction, lentiviral transduction and DNA and mRNA electroporation. As shown in Examples below, mRNA electroporation causes effective expression of the chimeric receptor of the present disclosure in T lymphocytes. Examples of references describing the retroviral transduction include Anderson et al., U.S. Patent No. 5,399,346; Mann et al., Cell 33: 153 (1983); Temin et al., U.S. Pat. No. 4,650,764; Temin et al., U.S. Patent No. 5,124,263; Dougherty et al., International Publication No. WO 95/07358 (published on March 16, 1995); and Kuo et al., Blood 82: 845 (1993). International Publication No. WO 95/07358 describes high efficiency transduction of primary B lymphocytes. For example, for specific techniques of retroviral transduction and mRNA electroporation that can be used, see also the Examples section below.

Host cell activation and growth can usually be used to attain integration of a virus vector into the genome and expression of the gene encoding the chimeric receptor of the present disclosure. However, provided that mRNA electroporation is used, neither activation nor growth is required (though electroporation is more effective when carried out on activated cells). As a result of viral transduction, the host cell (T lymphocyte or NKT cell) expresses the chimeric receptor of the present disclosure for a long period while potentially producing a stronger effect than that upon mRNA electroporation when the receptor is transiently expressed (typically for 3 to 5 days). However, the viral transduction is complicated, expensive and difficult to carry out, whereas the mRNA electroporation is much simpler and much easier to carry out. Further, the transient expression is useful if there is potential toxicity and should be helpful in the initial phases of clinical trials for possible adverse reactions.

One aspect is a method of preparing the cell disclosed in the present specification, comprising transfecting or transducing a cell with the nucleic acid or the vector described in the present specification.

In one embodiment, isolated immune cells are isolated T cells.

In an embodiment, isolated cells are CD3$^+$ and are optionally stimulated with an anti-CD3 antibody, optionally in a soluble or membrane-bound form, for example, OKT3 or mOKT3, and/or with APC before transduction or transfection. In one embodiment, the APC are artificial APC (aAPC). In another embodiment, the APC expresses a membrane form of an anti-CD3 monoclonal antibody.

In one embodiment, the transfection or transduction step is repeated. For example, the transfection or transduction step can be performed twice, or three times, or four times, or until, for example, an adequate level of expression is achieved. The transfection or transduction step can be performed, for example, five times.

In one embodiment, cells are transfected or transduced for two or more consecutive days. Cells are transfected or transduced, for example, for two consecutive days, three consecutive days, or four consecutive days.

In one embodiment, the chimeric receptor-transduced cells are stimulated with irradiated cells expressing a predetermined antigen. The chimeric receptor-transduced cells are stimulated with irradiated cells, for example, at an effector:target ratio of 100:1, 75:1, 50:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:50 or 1:100.

(4) Cell expressing chimeric receptor and use thereof

The cell expressing the chimeric receptor of the present disclosure is a cell allowed to harbor or express a nucleic acid encoding the chimeric receptor described in the present specification by the production method described in the present specification.

The cell of the present disclosure binds to a particular antigen via the chimeric receptor. Signals are thereby transduced into the cell, and as a result, the cell is activated. The activation of the cell expressing the chimeric receptor differs depending on the type of host cells and the intracellular domains of the chimeric receptor, and can be confirmed on the basis of, for example, cytokine release, improvement in cell growth rate, change in cell surface molecule, as an index. For example, the release of a cytotoxic cytokine (a tumor necrosis factor, lymphotoxin, etc.) from the activated cell causes destruction of target cells expressing an antigen. In addition, the cytokine release or the change in cell surface molecule stimulates other immunocytes, for example, B cells, dendritic cells, NK cells, and macrophages.

The T lymphocytes used in the method of the present disclosure are most preferably patient's own cells (i.e., autologous cells) that are isolated in advance from a blood sample and preferably activated and grown *ex vivo* (e.g., for 3 to 5 days) by use of a standard method, for example, anti-CD3/CD28 beads, IL-2 or phytohemagglutinin. Alternatively, allogeneic T lymphocytes can be used (preferably allogeneic T lymphocytes whose expression of an endogenous T cell receptor is blocked or eliminated). See Torikai et al., Blood, 2012 119: 5697-5705. T lymphocytes and NK cells thus isolated (and, if desired, activated and/or grown) from a patient are transduced (or electroporated) with a polynucleotide encoding the chimeric receptor of the present disclosure (or a vector comprising such a polynucleotide) so that the chimeric receptor is expressed on the cell surface of the T cells or the NK cells. The modified cells can then be administered to the patient (e.g., 1 day after drip infusion of a therapeutic antibody).

One aspect provides use of the chimeric receptor-expressing cell, the nucleic acid, the vector, the cell or the composition of the present disclosure for treating a disease.

Another aspect is a method for treating or preventing a disease in a mammal, comprising administering an effective amount of the cell or the composition of the present disclosure to the mammal in need thereof.

A further aspect is a method for providing antitumor immunity in a mammal, comprising administering an effective amount of the cell or the composition of the present disclosure to the mammal in need thereof.

A therapeutic drug comprising the chimeric receptor-expressing cell as an active ingredient can be administered intradermally, intramuscularly, subcutaneously, intraperitoneally, intranasally, intraarterially, intravenously, intratumorally, or into an afferent lymph vessel, by parenteral administration, for example, by injection or infusion, though the administration route is not limited.

According to the present disclosure, patients can be treated by infusing a therapeutically effective dose of T lymphocytes or NK cells comprising the chimeric receptor of the present disclosure in the range of approximately $10^5$ to $10^{10}$ or more cells per kilogram body weight (cells/kg). The infusion can be repeated as frequently and as many times as possible as long as the patients can tolerate until the desired response is achieved. The appropriate infusion dosage and schedule may differ among patients, but can be determined by a physician who treats a particular patient. Typically, an initial dose of approximately $10^6$ cells/kg is infused and increased to $10^8$ or more cells/kg. IL-2 can also be used in combination therewith for the post-infusion growth of the infused cells. The amount of IL-2 can be approximately 1 to 5 $\square$ $10^6$ international units per square meter of body surface.

A further aspect is a method comprising integrating a nucleic acid encoding the chimeric receptor of the present disclosure into a living body using a virus vector or the like, and directly expressing the chimeric receptor. Examples of the virus vector include, but are not limited to, adenovirus vectors. Alternatively, the nucleic acid encoding the chimeric receptor may be integrated directly into a living body by electroporation, an approach of directly administering the nucleic acid, or the like without the use of the virus vector, and the chimeric receptor may be intermittently secreted in a living body by administering cells genetically engineered so as to secrete and express the chimeric receptor to the living body.

The therapeutic drug comprises the cell expressing the chimeric receptor as an active ingredient and may further comprise a preferred excipient. Examples of the excipient include the pharmaceutically acceptable excipients mentioned above for the composition comprising the nucleic acid of the present disclosure as an active ingredient, various cell culture media, and isotonic sodium chloride.

Pharmaceutical composition of present disclosure

A further aspect of the present disclosure provides a pharmaceutical composition. In one aspect, the present disclosure provides a pharmaceutical composition comprising (i) a polynucleotide encoding the chimeric receptor of the present disclosure or a vector comprising such a polynucleotide and (ii) a pharmaceutically acceptable carrier or additive.

Appropriate additives for use in the pharmaceutical composition of the present disclosure are well known to those skilled in the art and can include, for example, a tissue culture medium (e.g., for the *ex vivo* survival of cells) or an aqueous salt solution (e.g., upon injection of cells to patients). Detailed description on the pharmaceutically acceptable additives is available from Remington's Pharmaceutical Sciences (Mack Pub. Co., N.J. 1991).

The disease against to which the cell expressing the chimeric receptor is administered is not particularly limited as long as the disease exhibits sensitivity to therapy using the chimeric receptor-expressing cell. In one embodiment, the disease is a cancer.

The cancer is, for example, blood cancer or solid tumor. The blood cancer is, for example, leukemia, lymphoma or myeloma. The solid tumor is, for example, cancers such as adenocarcinoma, squamous cell carcinoma, adenosquamous cancer, undifferentiated cancer, large-cell cancer, small-cell cancer, skin cancer, breast cancer, prostate cancer, bladder cancer, vaginal cancer, neck cancer, uterus cancer, liver cancer, kidney cancer, pancreatic cancer, spleen cancer, lung cancer, tracheal cancer, bronchial cancer, colon cancer, small intestine cancer, stomach cancer, esophageal cancer, gallbladder cancer, testis cancer, and ovary cancer, cancers of bone tissues, cartilage tissues, fat tissues, muscle tissues, vascular tissues and hematopoietic tissues as well as sarcoma such as chondrosarcoma, Ewing's sarcoma, malignant hemangioendothelioma, malignant schwannoma, osteosarcoma, and soft tissue sarcoma, blastoma such as hepatoblastoma, medulloblastoma, nephroblastoma, neuroblastoma, pancreatoblastoma, pleuropulmonary blastoma, and retinoblastoma, and germ cell tumor.

In an embodiment, the disease is an inflammatory disease/autoimmune disease (e.g., asthma and eczema), hepatitis, and an infectious disease, the cause of which is a virus such as influenza and HIV, a bacterium, or a fungus, for example, tuberculosis, MRSA, VRE, or deep mycosis.

The cell expressing the chimeric receptor of the present disclosure that binds to an antigen processed by cells desired to be reduced or eliminated for the treatment of the diseases mentioned above, i.e., a tumor antigen, a viral antigen, a bacterial antigen, or the like, is administered for the treatment of these diseases.

Accordingly, one aspect includes a method for decreasing the number of cells expressing a predetermined antigen in a subject, comprising administering an effective amount of the chimeric receptor-expressing cell of the present disclosure to the subject in need thereof, wherein the chimeric receptor-expressing cell specifically binds to the predetermined antigen via a mutated antibody that binds to the extracellular binding domain.

The cell of the present disclosure can also be utilized for the prevention of an infectious disease after bone marrow transplantation or exposure to radiation, donor lymphocyte transfusion for the purpose of remission of recurrent leukemia, and the like.

In one embodiment, the subject is suspected of having a cancer or has a cancer. In one embodiment, the subject is suspected of having an inflammatory disease or has an inflammatory disease.

Treatment method of present disclosure

The chimeric receptor of the present disclosure confers antibody-dependent cytotoxic activity to T lymphocytes and enhances antibody-dependent cellular cytotoxicity (ADCC) in NK cells. The binding of the receptor through an antibody (or another antitumor molecule comprising an Fc moiety) that binds to tumor cells triggers T cell activation, sustained proliferation and specific cytotoxicity against cancer cells targeted by the antibody (or such another antitumor molecule comprising an Fc moiety).

The chimeric receptor of the present disclosure promotes T cell therapy that allows a single chimeric receptor to be used for diverse cancer cell types. This can be eventually advantageous when an immune escape mechanism exploited by tumor is taken into consideration. At the same time, diverse antigens can be targeted. Since T cells expressing the chimeric receptor of the present disclosure are activated only by an antibody bound to target cells, unbound immunoglobulins do not exert any stimulation into the infused T cells. Clinical safety can be further enhanced by using mRNA electroporation for the transient expression of the chimeric receptor in order to limit any potential autoimmune reactivity.

Hence, in one aspect, the present disclosure provides a method for enhancing an effect of antibody-based immunotherapy for a cancer in a subject in need thereof, comprising administering a therapeutically effective amount of T lymphocytes or NK cells (the T lymphocytes or the NK cells comprise the chimeric receptor of the present disclosure) to the subject.

In one aspect of the method, the T lymphocytes or the NK cells are autologous T lymphocytes or NK cells isolated from the subject. In a particular aspect, the autologous T lymphocytes or NK cells are activated and/or grown *ex vivo* before re-introduction to the subject. In another aspect, the T lymphocytes or the NK cells are allogeneic T lymphocytes or NK cells. In a certain aspect, the T lymphocytes are allogeneic T lymphocytes whose expression of an endogenous T cell receptor is blocked or eliminated. In a particular aspect, the allogeneic T lymphocytes are activated and/or grown *ex vivo* before introduction to the subject. The T lymphocytes can be activated by any method known in the art in the presence of, for example, anti-CD3/CD28, IL-2 and/or phytohemagglutinin. The NK cells can be activated by any method known in the art in the presence of one or more agents selected from the group consisting of, for example, CD137 ligand protein, anti-CD137 antibody, IL-15 protein, an anti-IL-15 receptor antibody, IL-2 protein, IL-12 protein, IL-21 protein and a K562 cell line. For example, for description on useful methods for growing NK cells, see U.S. Patent Nos. 7,435,596 and 8,026,097.

In one aspect of the method, following introduction (or re-introduction) of the T lymphocytes or the NK cells to the subject, a therapeutically effective amount of a PD-1/PD-L1 signal inhibitor and/or a VEGF signal inhibitor is administered to the subject.

Combination treatment of present disclosure

The composition and the method described in the present specification may be used in combination with another type of treatment for a cancer, such as chemotherapy, surgery, radiation, or gene therapy. Such treatment can be applied concurrently or sequentially (in any order) with the immunotherapy according to the present disclosure.

In combined use with an additional therapeutic agent, an appropriate therapeutically effective dose of each agent can be decreased owing to additive or synergistic effects.

The treatment of the present disclosure can be combined with, for example, another immunomodulatory treatment such as a therapeutic vaccine (including, but not limited to, GVAX, DC vaccines, etc.), a checkpoint inhibitor (including, but not limited to, agents blocking CTLA4, PD1, LAG3, TIM3, etc.) or an activator (including, but not limited to, agents enhancing 41BB, OX40, etc.).

The pharmaceutical composition of the present disclosure may also comprise one or more additional active compounds, preferably compounds having complementary activity without adversely affecting each other, if necessary for a particular indication to be treated. Non-limiting examples of the possible additional active compounds include PD-1/PD-L1 signal inhibitors and VEGF signal inhibitors.

In another aspect, the present disclosure provides a pharmaceutical composition comprising (i) a host cell comprising the chimeric receptor of the present disclosure and (ii) a pharmaceutically acceptable carrier or additive. In a particular aspect, such a pharmaceutical composition further comprises a monoclonal antibody (e.g., rituximab, trastuzumab, or hul4.18K322A) that can exert cytotoxicity against cancer cells, or another antitumor molecule comprising an Fc moiety (e.g., a composite molecule comprising a ligand (e.g., a cytokine or an immune cell receptor) that binds to a tumor surface receptor combined with an immunoglobulin Fc moiety or Fc-containing DNA or RNA).

An appropriate dose of the antibody used depends on the type of cancer to be treated, the severity and course of the disease, previous treatment, patient's clinical history and response to the antibody, and the discretion of the treating physician. The antibody can be administered to the patient at once or over a series of treatments. The progress of the treatment according to the present disclosure can be easily monitored by a conventional technique and assay.

The administration of the antibody can be carried out by any appropriate route including systemic administration and direct administration to a site of the disease (e.g., to primary tumor).

Non-limiting examples of the additional therapeutic agent useful for combination with the immunotherapy of the present disclosure include the following:

(i) anti-angiogenic agents (e.g., TNP-470, platelet factor 4, thrombospondin-1, tissue inhibitors of metalloproteases (TIMP 1 and TIMP2), prolactin (16 kD fragment), angiostatin (38 kD fragment of plasminogen), endostatin, bFGF soluble receptor, transforming growth factor beta, interferon alpha, soluble KDR and FLT-1 receptor, placental proliferin-related protein, and those described in Carmeliet and Jain (2000));

(ii) VEGF antagonists or VEGF receptor antagonists such as anti-VEGF antibodies, VEGF variants, soluble VEGF receptor fragments, aptamers capable of blocking VEGF or VEGFR, neutralizing anti-VEGFR antibodies, inhibitors of VEGFR tyrosine kinase and any combination thereof;

(iii) chemotherapeutic compounds such as pyrimidine analogs (e.g., 5-fluorouracil, floxuridine, capecitabine, gemcitabine and cytarabine), purine analogs, folate antagonists and related inhibitors (e.g., mercaptopurine, thioguanine, pentostatin and 2-chlorodeoxyadenosine (cladribine)); antiproliferative/antimitotic agents including natural products such as vinca alkaloids (e.g., vinblastine, vincristine, and vinorelbine), microtubule disruptors such as taxane (e.g., paclitaxel and docetaxel), vincristine, vinblastine, nocodazole, epothilones and navelbine, epipodophyllotoxin (e.g., etoposide and teniposide), and DNA damaging agents (e.g., actinomycin, amsacrine, anthracyclines, bleomycin, busulfan, camptothecin, carboplatin, chlorambucil, cisplatin, cyclophosphamide, Cytoxan, dactinomycin, daunorubicin, doxorubicin, epirubicin, hexamethylmelamine, oxaliplatin, ifosfamide, melphalan, mechlorethamine, mitomycin, mitoxantrone, nitrosourea, plicamycin, procarbazine, taxol, taxotere, teniposide, triethylene thiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (e.g., L-asparaginase which systemically metabolizes L-asparagine and removes cells lacking the ability to synthesize their own asparagine); antiplatelet agents; antiproliferative/antimitotic alkylating agents such as nitrogen mustards (e.g., mechlorethamine, cyclophosphamide and analogs, melphalan, and chlorambucil), ethylenimines and methylmelamines (e.g., hexamethylmelamine and thiotepa), alkyl sulfonates-busulfan, nitrosoureas (e.g., carmustine (BCNU) and analogs, and streptozocin), and trazenes-dacarbazinine (DTIC); antiproliferative/antimitotic antimetabolites such as folic acid analogs (e.g., methotrexate); platinum coordination complexes (e.g., cisplatin and carboplatin), procarbazine, hydroxyurea, mitotane, and aminoglutethimide; hormones, hormone analogs (e.g., estrogen, tamoxifen, goserelin, bicalutamide, and nilutamide) and aromatase inhibitors (e.g., letrozole and anastrozole); anticoagulants (e.g., heparin, synthetic heparin salts and other thrombin inhibitors); fibrinolytic agents (e.g., tissue plasminogen activator, streptokinase and urokinase), aspirin, dipyridamole, ticlopidine, clopidogrel, and abciximab; antimigratory agents; antisecretory agents (e.g., brefeldin); immunosuppressive agents (e.g., cyclosporine, tacrolimus (FK-506), sirolimus (rapamycin), azathioprine, and mycophenolate mofetil); anti-angiogenic compounds (e.g., TNP-470, genistein, and bevacizumab) and growth factor inhibitors (e.g., fibroblast growth factor (FGF) inhibitors); angiotensin receptor blockers; nitric oxide donors; anti-sense oligonucleotides; antibodies (e.g., trastuzumab); cell cycle inhibitors and differentiation inducers (e.g., tretinoin); mTOR inhibitors, topoisomerase inhibitors (e.g., doxorubicin (adriamycin), amsacrine, camptothecin, daunorubicin, dactinomycin, teniposide, epirubicin, etoposide, idarubicin and mitoxantrone, topotecan, and irinotecan), corticosteroids (e.g., cortisone, dexamethasone, hydrocortisone, methylprednisolone, prednisone, and prednisolone); growth factor signaling kinase inhibitors; mitochondrial dysfunction inducers and caspase activators; and chromatin disruptors.

For further examples of useful agents, see also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J.

Further, the definitions and embodiments described in particular sections are intended to be applicable to other embodiments herein described for which they are suitable as would be understood by those skilled in the art. For example, in the following passages, different aspects of the present disclosure are defined in more detail. Each aspect thus defined may be combined with any other aspect or aspects unless clearly indicated to the contrary. In particular, any feature indicated as being preferred or advantageous may be combined with any other feature or features indicated as being preferred or advantageous.

The above disclosure generally describes the present application. More complete understanding can be obtained by reference to specific examples given below. These examples are described merely for the purpose of illustration and are not intended to limit the scope of the present application. Change in form and substitution of equivalents are also contemplated as circumstances might suggest or render expedient. Although particular terms are used in the present disclosure, such terms are intended in a descriptive sense and not for purposes of limitation.

EXAMPLES

The present disclosure will be described with reference to the following Examples.

Example 1 Universal T cell-redirecting antibody (TRAB) and universal CAR that binds to engineered Fc

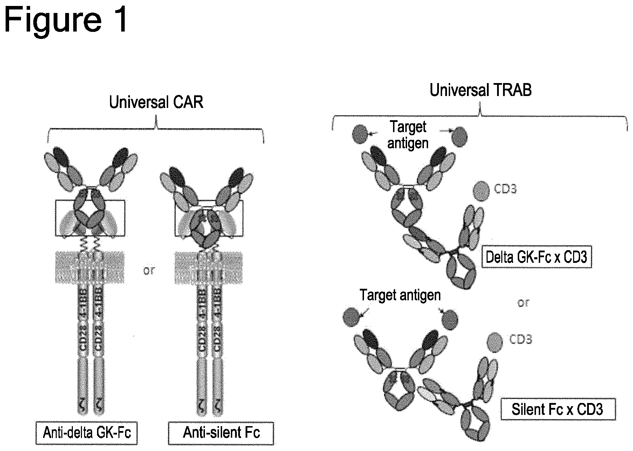

Figure 1 shows the forms of universal TRAB that specifically bind to an antibody having a mutation at a site other than an antigen recognition domain, and universal CAR having an extracellular binding domain that specifically binds to an antibody having a mutation at a site other than an antigen recognition domain. Possible examples of the mutation at a site other than an antigen recognition domain are delta GK-Fc in which C-terminal two amino acids (Gly and Lys) of an H chain are removed, and silent Fc with binding activity against Fc gamma receptor removed or ADCC/ADCP-enhancing Fc with binding to Fc gamma R enhanced by adding a mutation to an Fc gamma R recognition domain of CH2. Antibodies that specifically recognize these engineered moieties, for example, an anti-delta GK-Fc antibody or an anti-silent Fc antibody, is used as a secondary antibody. TRAB which is a bispecific antibody having a domain that specifically binds to an antibody having a mutation at a site other than an antigen recognition domain, and an anti-CD3 domain is referred to as "universal" TRAB Also, CAR-T expressing a chimeric receptor having scFv, Fab or scFab as an engineered Fc binding domain is referred to as "universal (universal)" CAR for the engineered Fc.

For these forms, a formulation comprising one or more types of proteins comprising a mutated site in an antibody which binds to an antigen specifically or selectively expressed in target cells in a test subject in need of treatment is administered as a primary formulation. Mere preparation of one type of universal TRAB and universal CAR that recognizes the mutated site in the antibody can cause cytotoxic activity against any target antigen by changing the primary formulation. Such universal CAR-T is more versatile and useful than CAR-T that directly recognizes an antigen specifically or selectively expressed in target cells.

Example 2 Preparation of anti-silent Fc antibody and anti-delta GK-Fc antibody and measurement of affinity for each antigen

2-1 Preparation of anti-GPC3 antibody having constant region of SG115 which is silent Fc and is delta GK-Fc

Antibody having variable regions against a tumor antigen (GPC3) and constant regions of SG115 disclosed in WO 2016/098356 was prepared. C-terminal Gly and Lys of the heavy chain of H0000-SG115/GL4-k0a were deleted by genetic engineering. Such Fc deprived of C-terminal Gly and Lys of the heavy chain is referred to as delta GK-Fc. Anti-GPC3 antibody H0000 (SEQ ID NO: 1)/GL4 (SEQ ID NO: 2) was used as a binding domain for GPC3. SG115 (SEQ ID NO: 3) was used as a heavy chain constant region, and k0a (SEQ ID NO: 4) was used as a light chain constant region.
Antibodies in the present specification were designated according to the following rule: (heavy chain variable region)-(heavy chain constant region)/(light chain variable region)-(light chain constant region).
For example, antibody name H0000-SG115/GL4-k0a means that this antibody has heavy chain variable region H0000, heavy chain constant region SG115, light chain variable region GL4, and light chain constant region k0a.
Likewise, variable regions of antibodies may be represented according to the following rule:

(heavy chain variable region)/(light chain variable region).
2-2 Preparation of anti-GPC3 antibody having IgG1 constant region having C-terminal GK
Human IgG1 constant region G1T3GK (SEQ ID NO: 5) having C-terminal GK as endogenous human IgG was prepared. Anti-GPC3 antibody H0000/GL4 was used as variable regions.

The antibodies shown in Table 1 were expressed and purified according to the method of Reference Example 1. [Table 1]

Table 1: Structure of antibody

| Antibody name | Variable region | | Constant region | |
| --- | --- | --- | --- | --- |
| | SEQ ID NO of heavy chain | SEQ ID NO of light chain | SEQ ID NO of heavy chain | SEQ ID NO of light chain |
| H0000-SG115/GL4-k0a | 1 | 2 | 3 | 4 |
| H0000-G1T3GK/GL4-k0a | 1 | 2 | 5 | 4 |

2-3 Preparation of bispecific antibody of anti-silent Fc antibody or anti-delta GK-Fc antibody and anti-CD3 epsilon antibody

Each bispecific antibody was prepared in a form where IgG of an anti-silent Fc antibody against silent Fc SG115 or an anti-delta GK-Fc antibody against delta GK-Fc was used as a backbone and one of the variable regions was replaced with an antibody against CD3 epsilon. SKA0009H (SEQ ID NO: 6)/SKA0009Ls (SEQ ID NO: 7) was used as a binding domain for SG115. Also, anti-CD3 antibody TR01H113 (SEQ ID NO: 8)/L0011 (SEQ ID NO: 9) was used as a binding domain for CD3 epsilon. Further, YG55H (SEQ ID NO: 10)/YG55L (SEQ ID NO: 11) was used as a binding domain for delta GK-Fc. F760mnN17 (SEQ ID NO: 12) was used as a heavy chain constant region for the variable regions SKA0009H/SKA0009Ls, and F760mnN17GK (SEQ ID NO: 13) was used as a heavy chain constant region for the variable regions YG55H/YG55L. Also, F760mnP17 (SEQ ID NO: 14) or F760mnP17GK (SEQ ID NO: 15) was used as a heavy chain constant region for the variable regions TR01H113/L0011. k0 (SEQ ID NO: 4), k0a (SEQ ID NO: 4), or k0MT (SEQ ID NO: 4) was used as a light chain constant region. k0, k0a, and k0MT have the same amino acid sequence, though their names differ due to their different nucleotide sequences.
Bispecific antibodies in the present specification are designated according to the following rule: AA (first antibody heavy chain)/XX (first antibody light chain) // BB (second antibody heavy chain)/YY (second antibody light chain).
The antibodies shown in Table 2 were expressed and purified according to the method of Reference Example 1. Subsequently, two types of homomers thus obtained were mixed according to the combinations and the reaction conditions given below to prepare the bispecific antibodies shown in Table 3. The reaction products were evaluated according to the method of Reference Example 2.

(1) SKA0009H-F760mnN17/SKA0009Ls-kOMT and TR01H113-F760mnP17/L0011-k0a
(2) YG55H-F760mnN17GK/YG55L-k0 and TR01H113-F760mnP17GK/L0011-k0a

Reaction conditions: in PBS (pH 7.4), [each mAb] = 1.0 mg/ml, [2-MEA (Sigma-Aldrich Co. LLC)] = 25 mM, 37□C, overnight reaction [Table 2]

Table 2: Structure of antibody

| Antibody name | Variable region | | Constant region | |
|---|---|---|---|---|
| | SEQ ID NO of heavy chain | SEQ ID NO of light chain | SEQ ID NO of heavy chain | SEQ ID NO of light chain |
| SKA0009H-F760mnN17/SKA0009Ls-k0MT | 6 | 7 | 12 | 4 |
| YG55H-F760mnN17GK/YG55L-k0 | 10 | 11 | 13 | 4 |
| TR01H113-F760mnP17/L0011-k0a | 8 | 9 | 14 | 4 |
| TR01H113-F760mnP17GK/L0011-k0a | 8 | 9 | 15 | 4 |

[Table 3]

Table 3: Structure of antibody

| Antibody name | Anti-silent Fc antibody, anti-delta GK-Fc antibody | | | | Anti-CD3 epsilon antibody | | | |
|---|---|---|---|---|---|---|---|---|
| | Variable region | | Constant region | | Variable region | | Constant region | |
| | SEQ ID NO of heavy chain | SEQ ID NO of light chain | SEQ ID NO of heavy chain | SEQ ID NO of light chain | SEQ ID NO of heavy chain | SEQ ID NO of light chain | SEQ ID NO of heavy chain | SEQ ID NO of light chain |
| SKAG009H-F760mnN17/SKA0009Ls-k0MT//TR01H113-F760mnP17/L0011-k0a | 6 | 7 | 12 | 4 | 8 | 9 | 14 | 4 |
| YG55H-F760mnN17GK/YG55L-k0//TR01H113-F760mnP17GK/L0011-k0a | 10 | 11 | 13 | 4 | 8 | 9 | 15 | 4 |

2-4 Evaluation of affinity of anti-silent Fc antibody and anti-delta GK-Fc antibody for each antigen

Binding activity against each antigen was evaluated when SKA0009H (SEQ ID NO: 6)/SKA0009Ls (SEQ ID NO: 7) and YG55H (SEQ ID NO: 10)/YG55L (SEQ ID NO: 11) were used as variable regions. This evaluation was conducted on the bispecific antibodies shown in Table 3 prepared in the section 2-3.

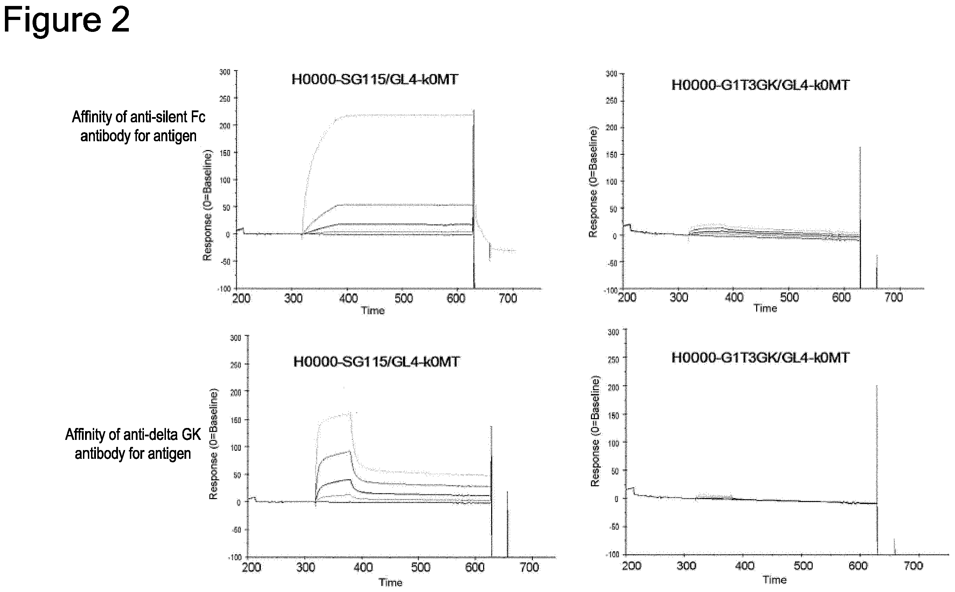

The affinity for an antigen and kinetic parameters of the anti-silent Fc antibody or the anti-delta GK-Fc antibody were measured by the multi-cycle kinetics of surface plasmon resonance assay using Biacore™T200 (GE Healthcare Japan Corp.). HBS-EP+ (GE Healthcare Japan Corp.) was used as a running buffer, and engineered GPC3 (SEQ ID NO: 16) was covalently bound to CM4 chip using Amine Coupling Kit (GE Healthcare Japan Corp.). The anti-GPC3 silent Fc and anti-delta GK-Fc antibody H0000-SG115/GL4-k0a or the anti-GPC3 antibody H0000-G1T3GK/GL4-k0a with C-terminal addition of Gly-Lys, shown in Table 1, were captured by the chip bound with engineered GPC3. A solution of the anti-silent Fc antibody SKA0009H-F760mnN17/SKA0009Ls-k0a//TR01H113-F760mnP17/L0011-k0a or the anti-delta GK-Fc antibody YG55H-F760mnN17GK/YG55L-k0//TR01H113-F760mnP17GK/L0011-k0a shown in Table 3 for use as an analyte were prepared at 15.6, 62.5, 250, and 1000 nM using HBS-EP+. For the measurement, first, the anti-GPC3 antibody solution was captured by engineered GPC3, and further, the anti-silent Fc antibody solution or the anti-delta GK-Fc antibody solution were injected at a flow rate of 10 µL/min for 1 minute and thereby reacted. Then, the solution was shifted to HBS-EP+, and a dissociation phase was measured for 3 minutes. After the completion of measurement of the dissociation phase, the sensor chip was washed with 10 mM Gly-HCl, pH 1.5 for regeneration. For measurement at a concentration of 0, the anti-GPC3 antibody solution was similarly captured by engineered GPC3, and HBS-EP+ was injected for 1 minute and thereby reacted. Then, the solution was shifted to HBS-EP+, and a dissociation phase was measured for 3 minutes. After the completion of measurement of the dissociation phase, the sensor chip was washed with 10 mM Gly-HCl, pH 1.5 for regeneration. From the obtained sensorgrams, kinetic analysis was conducted using Biacore-dedicated data analysis software Biacore T200 Evaluation Software Version 2.0 to calculate an association rate constant (ka), a dissociation rate constant (kd) and a rate constant ratio. The results are shown in Table 4 and Figure 2. [Table 4]

Table 4: Affinity test results

| Antigen | | Affinity of anti-silent Fc antibody for antigen | | | Affinity of anti-delta GK-Fc antibody for antigen | | |
|---|---|---|---|---|---|---|---|
| Antibody name | SEQ ID NO of heavy chain constant region | KD(M) | ka(1/Ms) | kd(1/s) | KD(M) | ka (1/Ms) | kd(1/s) |
| H0000-SG115/GL4-k0a | 3 | $9.82 \times 10^{-13}$. | $3.89 \times 10^4$ | $3.82 \times 10^{-6}$. | $4.17 \times 10^{-6}$ | $8.35 \times 10^4$ | $3.48 \times 10^{-3}$ |
| H0000-G1T3GK/GL4-k0a | 5 | $4.40 \times 10^{-5}$. | $2.61 \times 10^5$ | $1.15 \times 10^{-3}$. | ND | ND | ND |
| *1: No reliable value was obtained due to too low a dissociation constant. ND: Excluded from the analysis results because reliable fitting was not attained due to a low response value. | | | | | | | |

[Reference Example 1] Preparation of antibody expression vector and expression and purification of antibody

Full-length genes having nucleotide sequences encoding a heavy chain and a light chain of each antibody were prepared by a method known to those skilled in the art using assemble PCR or the like. Amino acid substitution, deletion, addition or modification was introduced by a method known to those skilled in the art using PCR or the like. The obtained plasmid fragments were inserted to expression vectors for animal cells to prepare a heavy chain expression vector and a light chain expression vector. The nucleotide sequences of the obtained expression vectors were determined by a method known to those skilled in the art. The prepared plasmids were transiently introduced into A human embryonic kidney cancer cell-derived HEK293H line (Invitrogen Corp.), FreeStyle293 cells (Invitrogen Corp.), or Expi293 cells (Invitrogen Corp.) so that the antibody was expressed. The obtained culture supernatant was recovered and then passed through 0.22 µm filter MILLEX(R)-GV (Merck Millipore) or 0.45 µm filter MILLEX(R)-GV (Merck Millipore) to obtain a culture supernatant. From the obtained culture supernatant, the antibody was purified by a method known to those skilled in the art using rProtein A Sepharose Fast Flow (GE Healthcare Japan Corp.) or Protein G Sepharose 4 Fast Flow (GE Healthcare Japan Corp.). The concentration of the purified antibody was measured as absorbance at 280 nm using a spectrophotometer, and the antibody concentration was calculated using an absorption coefficient calculated by a method such as PACE from the obtained value (Protein Science 1995; 4: 2411-2423).

[Reference Example 2] Evaluation of bispecific antibody production rate by ion-exchange chromatography

The separation of each specimen was evaluated by ion-exchange chromatography purification using Prominence UFLC (Shimadzu Corp.). A Tris buffer solution (pH 5.0) containing 9.6 mM Tris, 6.0 mM piperazine, and 11.0 mM imidazole, and a Tris buffer solution (pH 10.1) containing 9.6 mM Tris containing 150 mM sodium chloride, 6.0 mM piperazine, and 11.0 mM imidazole were used in a mobile phase, and ProPac WCX-10 (Thermo Fisher Scientific Inc.) was used as a column. Each bispecific antibody was separated by the two-liquid mixed gradient method. Data was obtained at a wavelength of 280 nm, and elution results were evaluated using Empower 2 (Waters Corp.).
The bispecific antibody production rate (%) used was a value obtained by dividing the peak area value of the bispecific antibody by the total peak area value of all antibodies present in the system, followed by multiplication by 100. If the recovery rate of one of the homomers was poor, a two-fold value of the area of the other homomer was combined with the peak area value of the bispecific antibody, and this value was calculated as the total peak area value of all antibodies.
Example 3 Evaluation of activity of universal TRAB against engineered Fc

(3-1) Object and approach of evaluation of activity of TRAB against engineered Fc

The cytotoxic activity of CAR-T correlates with the cytotoxic activity of TRAB to some extent. Furthermore, their *in vitro* cytotoxic activity is known to also correlate with their *in vivo* antitumor effects. Thus, for the purpose of confirming the cytotoxic activity of universal CAR-T, the cytotoxic activity of universal TRAB was first evaluated by *in vitro* assay. Here, the ability to activate T cells, which reportedly indicates the cytotoxic activity of the T cells, is given as an index. Specifically, the ability to activate T cells was evaluated by use of Reporter Gene Assay using luciferase, developed and sold by Promega Corp.

(3-2) *In vitro* evaluation of T cell activation of TRAB against engineered Fc using Jurkat reporter gene assay

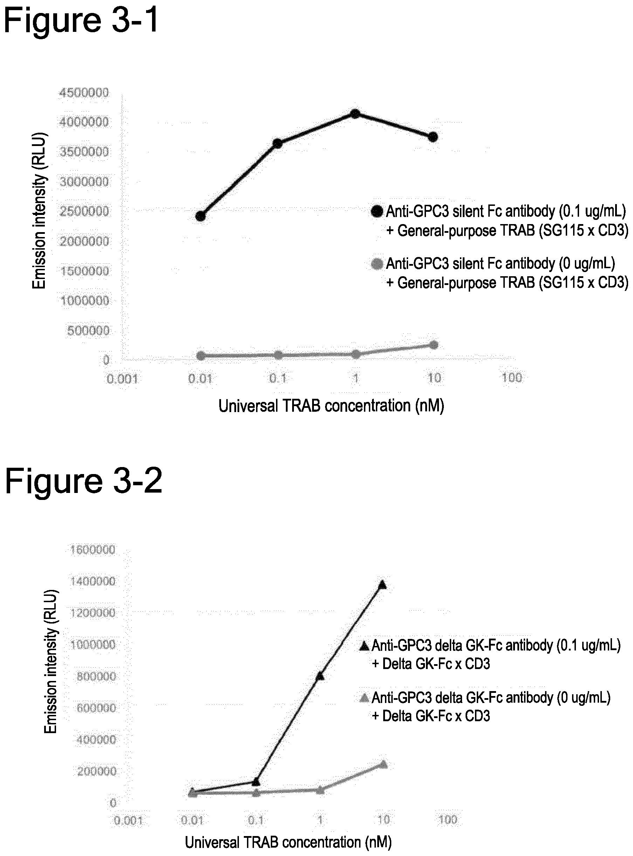

The bispecific antibody silent Fc × CD3 (SKA0009H-F760mnN17/SKA0009Ls-k0MT//TR01H113-F760mnP17/L0011-k0a) consisting of the anti-silent Fc antibody and the anti-CD3 antibody and the bispecific antibody delta GK-Fc × CD3 (YG55H-F760mnN17GK/YG55L-k0//TR01H113-F760mnP17GK/L0011-k0a) consisting of the anti-delta GK-Fc antibody and the anti-CD3 antibody, which were prepared in Example 2-3, were evaluated for T cell activation by use of T Cell Activation Bioassay (NFAT) using Propagation Model (Promega Corp., J1601). 25 µL of a mixed antibody solution of each bispecific antibody and the primary antibody anti-GPC3 silent Fc or anti-GPC3 delta GK-Fc (0.1 µg/mL or 0 µg/mL) was added to 50 µL of a mixed cell solution of SK-pca-60 cells ($1.25 \times 10^4$ cells per well; see EP3296395) obtained by forced expression of human GPC3 into human liver cancer cell line SK-HEP1 cells (purchased from ATCC), and Jurkat-NFAT-RE-luc2 cells ($7.5 \times 10^4$ cells per well; purchased from Promega Corp.), and 24 hours later, luciferase activity was measured using Bio-Glo Luciferase assay system (Promega Corp., G7941). The luciferase activity was measured as a luminescence value using 2104 EnVision (PerkinElmer, Inc.). The results are shown in Figures 3-1 and 3-2.

The universal TRAB using the anti-silent Fc antibody activated T cells in manners specific for cancer cells expressing the tumor antigen and dependent on the antibody concentration, in the presence of the silent Fc-antibody, which is a primary antibody and recognizes the tumor antigen. Likewise, the universal TRAB using the anti-delta GK-Fc antibody activated T cells in manners specific for cancer cells expressing the tumor antigen and dependent on the antibody concentration, in the presence of the delta GK-Fc-antibody, which is a primary antibody and recognizes the tumor antigen. These results demonstrated that the universal TRAB using the anti-silent Fc antibody and the universal TRAB using the anti-delta GK-Fc antibody activate T cells in a manner dependent on the primary antibody that recognizes a tumor antigen and damage cells expressing the tumor antigen. Further, it was predicted that: universal CAR-T using the anti-silent Fc antibody and universal CAR-T using the anti-delta GK-Fc antibody would activate T cells in a manner dependent on the primary antibody that recognizes a tumor antigen and damage cells expressing the tumor antigen; and their cytotoxic activity would induce an antitumor effect.

Example 4 Preparation of universal CAR-T that binds to engineered Fc

(4-1) Construction of retrovirus vector for expression of chimeric receptor that binds to engineered Fc

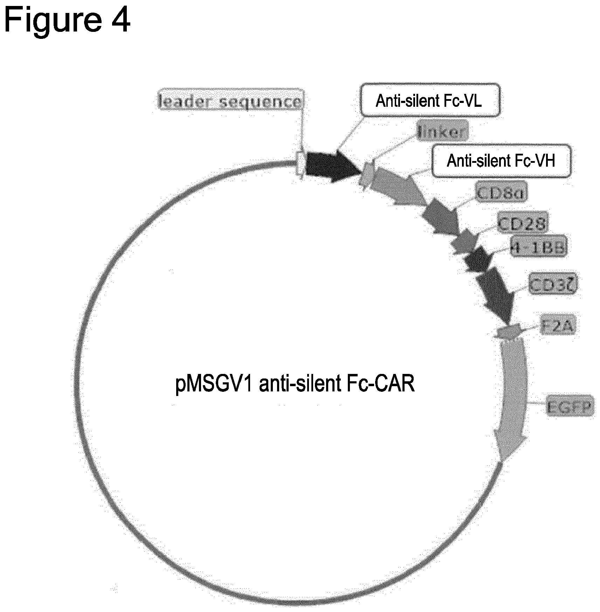

A retrovirus vector for a chimeric receptor was prepared for the expression of scFv of an anti-CH2 antibody that recognizes engineered Fc. pMSGV1-CD8-28BBZ (Hughes M.S. et al., Hum Gene Ther 2005 Apr; 16 (4): 457-72; obtained from Dr. Richard Morgan (National Cancer Institute)) was used as a retrovirus vector backbone, and pMSGV (Tamada k et al., Clin Cancer Res 18: 6436-6445 (2002)) was used as a mouse stem cell virus-based splice-gag vector. Figure 4 is a schematic view showing the vector construct and the order of arrangement of components in frame units from the 5' end to the 3' end. Silent Fc was used as an engineered CH2 region, and human scFv against this region is referred to as anti-silent Fc-scFv and consists of a light chain variable region followed by a 25-amino acid GS linker having 5 repeats of Gly-Gly-Gly-Gly-Ser, and a heavy chain variable region. The anti-silent Fc-scFv was linked to a hinge region and a transmembrane region of a human CD8 alpha chain (nucleotides 1271 to 1519, GenBank NM001768.6), and a cytoplasmic region of a human CD28 molecule (nucleotides 760 to 882, GenBank NM006139.2), a 4-1BB molecule (nucleotides 886 to 1026, GenBank NM001561.5), and a CD3 zeta molecule (nucleotides 299 to 634, GenBank NM000734.3), followed by 2A peptide (F2A) (derived from foot and mouth disease virus) and an eGFP molecule (nucleotides 5521 to 6237, GenBank KF957646.1). A gene encoding anti-silent Fc-CAR (SEQ ID NO: 17) was synthesized by a method known to those skilled in the art. This sequence was ligated with pMSGV1 to produce an anti-silent Fc-CD28-4-1BB-CD3 zeta-eGFP-CAR retrovirus vector (referred to as anti-silent Fc-CAR).

(4-2) Preparation of universal CAR-T

The retrovirus vector constructed in Example 4-1 was used in the retroviral transduction method of human T cells to prepare universal CAR-T expressing scFv of an anti-silent Fc antibody that recognizes engineered Fc.

Specifically, first, GP2-293 packaging cell line (Takara Bio Inc.) was transfected with the anti-silent Fc-CAR vector mentioned above and pAmpho plasmid (Takara Bio Inc.) using Lipofectamine 2000 or 3000 (Life Technologies Corp.) to prepare retrovirus harboring the anti-silent Fc-CAR vector. 48 hours after the transfection, a supernatant containing the retrovirus was recovered and adsorbed onto two 24-well plates to prepare plates for transductions.

Subsequently, for the transduction of human T cells, $2 \times 10^6$ human peripheral blood mononuclear cells per well were cultured for 72 hours in the presence of IL-2 on a 6-well plate with an anti-CD3 monoclonal antibody and RetroNectin(R) (Takara Bio Inc.) immobilized thereon. The cells thus cultured were recovered, and cultured overnight on one of the plates for transductions with the anti-silent Fc-CAR retrovirus adsorbed thereon, which were prepared as mentioned above, in the presence of IL-2. On the next day, the cells were transferred to the other plate for transduction and further cultured overnight to obtain human T cells harboring the anti-silent Fc-CAR vector (anti-silent Fc-CAR-expressing T cells).

(4-3) Confirmation of CAR protein expression rate

The cells thus transduced were maintained in the presence of human IL-2 and used in an experiment 7 to 8 days after the start of culture of the transduced peripheral blood mononuclear cells. The surface expression of the anti-silent Fc-CAR on the transduced human T cells was determined by the staining of the cells with CD3 or CD8 and biotin-labeled anti-GPC3 silent Fc antibody H0000-SG115/GL4-k0a, followed by flow cytometry. 60% on average of all T cells expressed the anti-silent Fc-CAR.

Example 5 Evaluation of *in vitro* cytotoxic activity of universal CAR-T against engineered Fc

The cytotoxic activity of the universal CAR-T cells against engineered Fc (anti-silent Fc-CAR) prepared in Example 4-1 was evaluated by BD FACSVerse ™ (BD Biosciences). SK-Hep1 was provided as a negative control, and SK-pca60 obtained by allowing SK-Hep1 to stably express human GPC3 was provided as target cells. The negative control and the target cells were inoculated at $1 \times 10^5$ cells and $3 \times 10^5$ cells, respectively, to 6-well plates. The anti-silent Fc-CAR was mixed at $1 \times 10^5$ cells with CAR-expressing cells as effector cells so as to attain an effector cell:target cell (E:T) ratio of 1:1 or 1:3. Subsequently, the anti-GPC3 silent Fc antibody H0000-SG115/GL4-k0a prepared in Example 2-1 was added at 10 μg per well. 48 hours after the addition, the CAR-T cells and the target cells were recovered. The recovered cells were applied to Zombie Aqua™ Fixable Viability Kit (BioLegend, Inc., 423102) to stain dead cells, and the CAR-T cells were stained with an anti-human CD45 antibody (BioLegend, Inc., 304039).

The cytotoxic activity was evaluated by the percentage of residual cancer cells. The percentage of residual cancer cells was calculated from the percentage of CD45⁻ fraction cells in live cells.

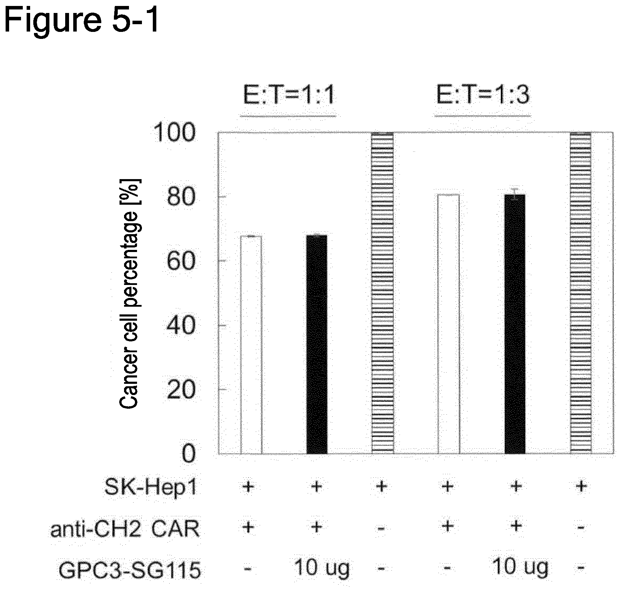

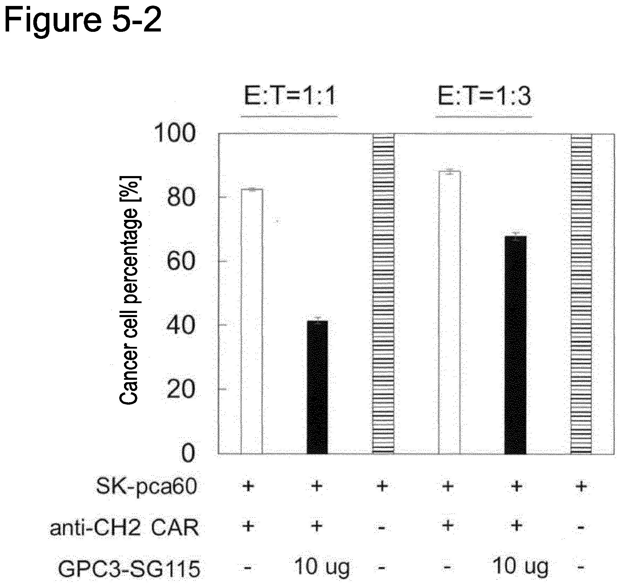

The results are shown in Figures 5-1 and 5-2.

The obtained results demonstrated the cytotoxic activity of the universal CAR-T cells that bind to engineered Fc in vitro.

Example 6 Confirmation of drug efficacy of universal CAR-T cell against engineered Fc in heterologous tumor cell-transplanted mouse model

(6-1) Preparation of cell line and heterologous tumor line-transplanted mouse model

SK-pca60 cells were used as target cells. The SK-pca60 cells were maintained in DMEM medium (Sigma-Aldrich Co. LLC, D5796) supplemented with 10% fetal bovine serum (Sigma-Aldrich Co. LLC, Lot. 14L368) and geneticin (Gibco, 15140-122) at a final concentration of 0.4 mg/mL. The mice used were NOD/Shi-scid,IL-2R gamma Kojic mice (NOG mice, 5-week-old, female) purchased from CLEA Japan, Inc. The SK-pca60 cells were subcutaneously transplanted at $5 \times 10^6$ cells/mouse in the abdominal regions of the mice, which were in turn established as models when the volume of the transplanted tumor became about 60 mm³ to 100 mm³.

The volume of the transplanted tumor was calculated according to the following expression.

$$\text{Tumor volume} = \text{Major axis} \times \text{Minor axis} \times \text{Minor axis} / 2$$

(6-2) Preparation of administration agent and CAR-T cell

The anti-GPC3 silent Fc antibody H0000-SG115/GL4-k0a prepared in Example 2-1 was used as an administration agent (primary antibody) for the SK-pca60 cell-transplanted models. H0000-SG115/GL4-k0a was prepared into a 10 mg/mL solution using a histidine buffer (20 mM His-HCL, 150 mM NaCl, pH 6.0) as a vehicle.

The universal CAR-T cells against engineered Fc (anti-silent Fc-CAR) prepared in Example 4-1 were prepared with Hanks' Balanced Salt solution (Sigma-Aldrich Co. LLC, H9269) such that the CAR-expressing cells were $1.6 \times 10^7$ cells/mL.

(6-3) Administration of agent and CAR-T cell for antitumor effect measurement

On 6 days after transplantation, H0000-SG1 15/GL4-k0a was intraperitoneally administered at a dosage of 1 mg/kg. The prepared dosing solution and the vehicle histidine buffer were administered at a dose of 10 mL/kg. Then, this antibody was continuously administered to each mouse once a week.

On 7 days after transplantation, the CAR-expressing T cells of each type were administered at $5 \times 10^6$ cells per mouse through the tail vein. The prepared CAR-T cell solution and the histidine buffer were administered at 300 µL/mouse.

Details of the treatment with the agents for antitumor effect measurement are shown in Table 5. [Table 5]

Table 5: Antitumor effect measurement in SK-pca60 cell transplantation model

|  | Group name | n | Administered antibody (i. p) | Administered cell (i. v.) |
|---|---|---|---|---|
| Group 1 | Vehicle group | 5 | Histidine buffer | Histidine buffer |
| Group 2 | Anti-GPC3 silent Fc antibody/- | 5 | H0000-SG115/GL4-k0Ma | Histidine buffer |
| Group 3 | -/Anti-Silent Fc-CAR-T | 5 | Histidine buffer | Anti-silent Fc-CAR-T |
| Group 4 | Anti-GPC3 silent Fc antibody /anti-silent Fc-CAR-T | 5 | H0000-SG115/GL4-k0a | Anti-silent Fc-CAR-T |

(6-4) Evaluation of antitumor effect

The antitumor effect was evaluated by the tumor volume calculated according to the expression described in (6-1).

A value obtained 25 days after the initial administration of the antibody was used as a tumor growth inhibition (TGI) value, which was calculated according to the following expression: TGI (%) = (1 - (Average tumor volume of the group of interest at the time of measurement - Average tumor volume of the group of interest at the time of initial administration) / (Average tumor volume of the control group at the time of measurement - Average tumor volume of the control group at the time of initial administration)) □ 100.

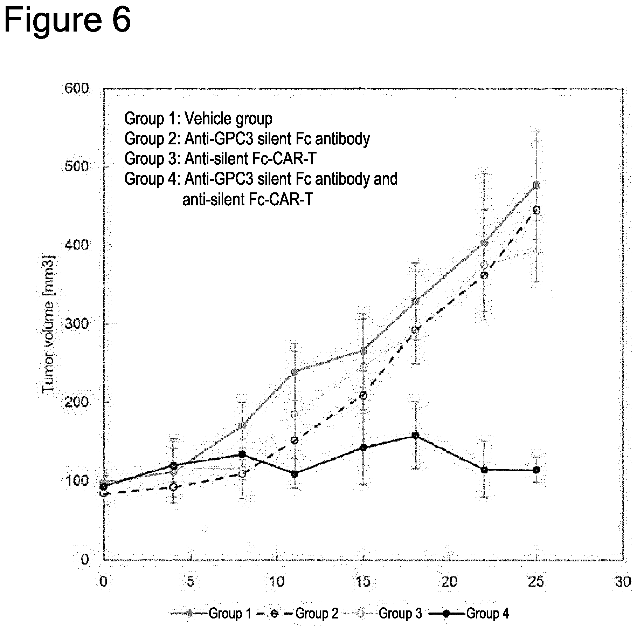

As a result, it was confirmed that the anti-silent Fc-CAR-T cells produce antitumor activity only in the presence of H0000-SG115/GL4-k0a and thus are able to exert antitumor activity in a manner dependent on the primary antibody (TGI = 94 on 25 days after CAR-T cell transplantation) (Figure 6).

[Table 6-1]

| SEQ ID NO | Temporary number | Name | Full-length sequence |
|---|---|---|---|
| 1 | TN1 | H0000 | QVQLVQSGAEVKKPGASVKVSCKASGYTFTDYEMHWIRQPPGQGLEWIGAIDPKTGD TAYSQKFKGRVTLTADKSTSTAYMELSSLTSEDTAVYYCTRFYSYTYWGQGTLVTVS S |
| 2 | TN2 | GL4 | DIVMTQSPLSLPVTPGEPASISCRSSQSLVHSNRNTYLHWYQQKPGQAPRLLIYKVSN RFSGVPDRFSGSGSGTDFTLKISRVEAEDVGVYYCSQNTHVPPTFGQGTKLEIK |
| 3 | TN3 | SG115 | ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPA PELRRGPKVFLFPPKPKDTLMISRTPEVTCVVVDVSHEDPEVKFNWYVDGVEVHNAK TKPREEQYNSTYRVVSVLTVLHQDWLNGKEYKCKVSNKGLPSSIEKTISKAKGQPREP QVYTLPPSREEMTKNQVSLTCLVKGFYPSDIAVEWESNGQPENNYKTTPPVLDSDGS FFLYSKLTVDKSRWQQGNVFSCSVLHEALHAHYTRKELSLSP |
| 4 | TN5 | k0, k0a, k0MT | RTVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGEC |
| 5 | YK1 | G1T3GK | ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKRVEPKSCDKTHTCPPCPA PELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQ VYTLPPSREEMTKNQVSLTCLVKGFYPSDIAVEWESNGQPENNYKTTPPVLDSDGSFF LYSKLTVDKSRWQEGNVFSCSVMHEALHNHYTQKSLSLSPGK |
| 6 | TN6 | SKA0009H | QSVEESGGRLVTPGTSLTLTCTVSGIDLSSYGMGWVRQAPGMGLEYIGIIYAAGRTYY ASWVKGRFIISKTSTTVDLEMTSLTTEDTATYFCARHGSWYAGMDLWGPGTLVTVSS |
| 7 | TN7 | SKA0009Ls | AIEMTQTPSPVSAAVGGTVSINCQASEDIENYFAWYQQKPGQPPKLLIYDASELASGV PSRFSGSGSGTDFTLTISGVQSDDAATYYCQHADYAASSENTFGGGTEVVVK |
| 8 | TN8 | TR01H113 | QVQLVESGGGVVQPGGSLRLSCAASGFTFSNAWMHWVRQAPGKGLEWVAQIKDKS QNYATYVAESVKGRFTISRADSKNSIYLQMNSLKTEDTAVYYCRYVHYAAGYGVDIWG QGTTVTVSS |
| 9 | TN9 | L0011 | DIVMTQSPLSLPVTPGEPASISCRSSQPLVHSNRNTYLHWYQQKPGQAPRLLIYKVSN RFSGVPDRFSGSGSGTDFTLKISRVEAEDVGVYYCGQGTQVPYTFGQGTKLEIK |
| 10 | TN10 | YG55H | QSLEESGGRLVTPGGSLTLTCTVSGIDLSNYAVGWVRQAPGKGLEYIGIIYASGSAYYA SWAKGRFTISKTSSTTVDLKVTSLTTEDTATYFCARGYGRAFRIWGPGTLVTVSS |
| 11 | TN11 | YG55L | AVLTQTPSPVSAAVGGTVTISCQASQSVYNNNFLSWYQQKPGQRPKLLIYDASTLES GVPSRFSGSGSGTQFTLTISSVQCDDAATYYCLGGYDDHDNAFGGGTEVVVK |
| 12 | TN12 | F760mnN17 | ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPA PELRGGPKVFLFPPKPKDTLMISRTPEVTCVVVDVSHEDPEVKFNWYVDGVEVHNAK TKPREEQYASTYRVVSVLTVLHQDWLNGKEYKCKVSNKALPAPIEKTISKAKGQPREP QVYTLPPSREEMTKNQVSLTCLVKGFYPSDIAVEWESNGQPENNYKTTPPYLDSDGS FFLYSKLTVDKSRWQQGNVFSCSVMHEALHNHYTQESLSLSP |
| 13 | TN13 | F760mnN17 GK | ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPA PELRGGPKVFLFPPKPKDTLMISRTPEVTCVVVDVSHEDPEVKFNWYVDGVEVHNAK TKPREEQYASTYRVVSVLTVLHQDWLNGKEYKCKVSNKALPAPIEKTISKAKGQPREP QVYTLPPSREEMTKNQVSLTCLVKGFYPSDIAVEWESNGQPENNYKTTPPYLDSDGS FFLYSKLTVDKSRWQQGNVFSCSVMHEALHNHYTQESLSLSPGK |
| 14 | TN14 | F760mnP17 | ASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPA PELRGGPKVFLFPPKPKDTLMISRTPEVTCVVVDVSHEDPEVKFNWYVDGVEVHNAK TKPREEQYASTYRVVSVLTVLHQDWLNGKEYKCKVSNKALPAPIEKTISKAKGQPREP QVYTLPPSRKEMTKNQVSLTCLVKGFYPSDIAVEWESNGQPENNYKTTPPYLDSDGS FFLYSKLTVDKSRWQQGNVFSCSVMHEALHNHYTQKSLSLSP |

[Table 6-2]

| | | | |
|---|---|---|---|
| 15 | TN15 | F760mnP17GK | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELRGGPKVFLFPPKPKDTLMISRTPEVTCVVVDVSHEDPEVKFNWYVDGVEVHNAKTKPREEQYASTYRVVSVLTVLHQDWLNGKEYKCKVSNKALPAPIEKTISKAKGQPREPQVYTLPPSRKEMTKNQVSLTCLVKGFYPSDIAVEWESNGQPENNYKTTPPYLDSDGSFFLYSKLTVDKSRWQQGNVFSCSVMHEALHNHYTQKSLSLSPGK |
| 16 | YK2 | 改変 GPC3 | QPPPPPPDATCHQVRSFFQRLQPGLKWVPETPVPGSDLQVCLPKGPTCCSRKMEEKYQLTARLNMEQLLQSASMELKFLIQNAAVFQEAFEIFVRHAKNYTNAMFKNNYPSLTPQAFEFVGEFFTDVSLYILGSDINVDDMVNELFDSLFPVIYTQLMNPGLPDSALDINECLRGARRDLKVFGNFPKLIMTQVSKSLQVTRIFLQALNLGIEVINTTDHLKFSKDCGRMLTRMWYCSYCQGLMMVKPCGGYCNVVMQGCMAGVVEIDKYWREYILSLEELVNGMYRIYDMENVLLGLFSTIHDSIQYVQKNAGKLTTTIGKLCAHSQQRQYRSAYYPEDLFIDKKVLKVAHVEHEETLSSRRRELIQKLKSFISFYSALPGYICSHSPVAENDTLCWNGQELVERYSQKAARNGMKNQFNLHELKMKGPEPVVSQIIDKLKHINQLLRTMSMPTGRVLDKNLDEEGFEAGDCGDDEDECIGGAGDGMIKVKNQLRFLAELAYDLDVDDAPGNSQQATPKDNEISTFHNLGNVHSPLKHHHHHH |
| 17 | | Anti-CH2 CAR construct | MDWTWRILFLVAAATGAHSAIEMTQTPSPVSAAVGGTVSINCQASEDIENYFAWYQQKPGQPPKLLIYDASELASGVPSRFSGSGSGTDFTLTISGVQSDDAATYYCQHADYAASSENTFGGGTEVVVKSSADDAKKDAAKKDDAKKDDAKKDGQSVEESGGRLVTPGTSLTLTCTVSGIDLSSYGMGWVRQAPGMGLEYIGIIYAAGRTYYASWVKGRFIISKTSTTVDLEMTSLTTEDTATYFCARHGSWYAGMDLWGPGTLVTVSSAAAFVPVFLPAKPTTTPAPRPPTPAPTIASQPLSLRPEACRPAAGGAVHTRGLDFACDIYIWAPLAGTCGVLLLSLVITLYCNHRNRSKRSRLLHSDYMNMTPRRPGPTRKHYQPYAPPRDFAAYRSRFSVVKRGRKKLLYIFKQPFMRPVQTTQEEDGCSCRFPEEEEGGCELRVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPREFGSGVKQTLNFDLLKLAGDVESNPGPCMVSKGEELFTGVVPILVELDGDVNGHKFSVSGEGEGDATYGKLTLKFICTTGKLPVPWPTLVTTLTYGVQCFSRYPDHMKQHDFFKSAMPEGYVQERTIFFKDDGNYKTRAEVKFEGDTLVNRIELKGIDFKEDGNILGHKLEYNYNSHNVYIMADKQKNGIKVNFKIRHNIEDGSVQLADHYQQNTPIGDGPVLLPDNHYLSTQSALSKDPNEKRDHMVLLEFVTAAGITLGMDELYK |
| 18 | | leader sequence | MDWTWRILFLVAAATGAHS |
| 19 | | anti CH2 scFv VL | AIEMTQTPSPVSAAVGGTVSINCQASEDIENYFAWYQQKPGQPPKLLIYDASELASGVPSRFSGSGSGTDFTLTISGVQSDDAATYYCQHADYAASSENTFGGGTEVVVK |
| 20 | | linker (25a.a.) | SSADDAKKDAAKKDDAKKDDAKKDG |
| 21 | | anti CH2 scFv VH | QSVEESGGRLVTPGTSLTLTCTVSGIDLSSYGMGWVRQAPGMGLEYIGIIYAAGRTYYASWVKGRFIISKTSTTVDLEMTSLTTEDTATYFCARHGSWYAGMDLWGPGTLVTVSS |
| 22 | | CD8 | FVPVFLPAKPTTTPAPRPPTPAPTIASQPLSLRPEACRPAAGGAVHTRGLDFACDIYIWAPLAGTCGVLLLSLVITLYCNHRN |
| 23 | | CD28 | RSKRSRLLHSDYMNMTPRRPGPTRKHYQPYAPPRDFAAYRS |
| 24 | | 4-1BB | RFSVVKRGRKKLLYIFKQPFMRPVQTTQEEDGCSCRFPEEEEGGCEL |
| 25 | | CD3ζ | RVKFSRSADAPAYQQGQNQLYNELNLGRREEYDVLDKRRGRDPEMGGKPRRKNPQEGLYNELQKDKMAEAYSEIGMKGERRRGKGHDGLYQGLSTATKDTYDALHMQALPPR |
| 26 | | F2A | VKQTLNFDLLKLAGDVESNPGP |
| 27 | | eGFP | MVSKGEELFTGVVPILVELDGDVNGHKFSVSGEGEGDATYGKLTLKFICTTGKLPVPWPTLVTTLTYGVQCFSRYPDHMKQHDFFKSAMPEGYVQERTIFFKDDGNYKTRAEVKFEGDTLVNRIELKGIDFKEDGNILGHKLEYNYNSHNVYIMADKQKNGIKVNFKIRHNIEDGSVQLADHYQQNTPIGDGPVLLPDNHYLSTQSALSKDPNEKRDHMVLLEFVTAAGITLGMDELYK |

While the present application has been described with reference to what are presently considered to be the preferred examples, it should be understood that the present application is not limited to the disclosed examples. To the contrary, the present application is intended to cover various modifications and equivalent arrangements included within the spirit and scope of the appended claims.

All publications, patents and patent applications are incorporated herein by reference in their entirety to the same extent as if each individual publication, patent or patent application was specifically and individually indicated to be incorporated by reference in its entirety. Specifically, the sequences associated with each accession numbers provided herein including, for example, accession numbers and/or biomarker sequences (e.g., proteins and/or nucleic acids) shown in the tables or elsewhere, are incorporated by reference in their entirety.

## Claims

1. A pharmaceutical composition for use in combination with administration of a mutated antibody having a mutation, including substitution, deletion, addition or modification, of at least one amino acid in a CH1 region, a CH2 region, a CH3 region, a CL region, or a framework region, wherein

   the pharmaceutical composition comprises a cell expressing a chimeric receptor,
   the chimeric receptor comprises an extracellular binding domain, a transmembrane domain and an intracellular signaling domain,
   the mutated antibody is capable of binding to the extracellular binding domain of the chimeric receptor via a moiety having the mutation, and
the extracellular binding domain does not specifically bind to an antibody free of the mutation.

2. A pharmaceutical composition for use in combination with administration of a cell expressing a chimeric receptor, wherein

the pharmaceutical composition comprises a mutated antibody having a mutation, including substitution, deletion, addition or modification, of at least one amino acid in a CH1 region, a CH2 region, a CH3 region, a CL region, or a framework region,
the chimeric receptor comprises an extracellular binding domain, a transmembrane domain and an intracellular signaling domain,
the mutated antibody is capable of binding to the extracellular binding domain of the chimeric receptor via a moiety having the mutation, and
the extracellular binding domain does not specifically bind to an antibody free of the mutation.

3. A pharmaceutical composition for use in combination with administration of a mutated antibody having a mutation, including substitution, deletion, addition or modification, of at least one amino acid in a CH1 region, a CH2 region, a CH3 region, a CL region, or a framework region, wherein

the pharmaceutical composition comprises a bispecific antibody, and
the bispecific antibody comprises (1) a domain comprising antibody variable regions that specifically bind to the mutated antibody via a moiety having the mutation, and (2) a domain comprising antibody variable regions having binding activity against a molecule expressed on T cell surface, and does not specifically bind to an antibody free of the mutation.

4. A pharmaceutical composition for use in combination with administration of a bispecific antibody, wherein

the pharmaceutical composition comprises a mutated antibody having a mutation, including substitution, deletion, addition or modification, of at least one amino acid in a CH1 region, a CH2 region, a CH3 region, a CL region, or a framework region, and
the bispecific antibody comprises (1) a domain comprising antibody variable regions that specifically bind to the mutated antibody via a moiety having the mutation, and (2) a domain comprising antibody variable regions having binding activity against a molecule expressed on T cell surface, and does not specifically bind to an antibody free of the mutation.

5. The pharmaceutical composition according to any one of claims 1 to 4, wherein the mutated antibody has the mutation in a CH2 region, and the mutated antibody has reduced binding activity against Fc gamma receptor and C1q compared with a corresponding non-mutated antibody.

6. The pharmaceutical composition according to any one of claims 1 to 5, wherein the mutated antibody has a CH2 region mutation at any of positions 234, 235, 236, 237, 238, 265, 266, 267, 268, 269, 270, 271, 295, 296, 298, 300, 324, 325, 326, 327, 328, 329, 330, 331, 332, 333, 334, 335, 336, and 337 according to the EU numbering, and the mutated antibody binds to the extracellular binding domain via a moiety having the mutation.

7. The pharmaceutical composition according to any one of claims 1 to 6, wherein the CH2 region of the mutated antibody has a mutation selected from the group of

a mutation of an amino acid at position 235 to arginine,
a mutation of an amino acid at position 236 to arginine,
a mutation of an amino acid at position 239 to lysine,
a mutation of an amino acid at position 250 to valine,
a mutation of an amino acid at position 252 to tyrosine,
a mutation of an amino acid at position 297 to alanine,
a mutation of an amino acid at position 307 to glutamine,
a mutation of an amino acid at position 308 to proline,
a mutation of an amino acid at position 311 to alanine,
a mutation of an amino acid at position 434 to tyrosine, and
a mutation of an amino acid at position 436 to valine,

according to the EU numbering, and
the mutated antibody binds to the extracellular binding domain via a moiety having the mutation.

8. An isolated nucleic acid encoding a chimeric receptor or a bispecific antibody contained in a pharmaceutical composition according to any one of claims 1 to 7.

9. A vector comprising an isolated nucleic acid according to claim 8.

10. The vector according to claim 9, wherein the vector is operably linkable to at least one regulatory element for the expression of the chimeric receptor or the bispecific antibody.

11. A cell transformed or transduced with an isolated nucleic acid according to claim 8 or a vector according to claim 9 or 10.

# Figure 1

# Figure 2

Affinity of anti-silent Fc
antibody for antigen

H0000-SG115/GL4-k0MT

H0000-G1T3GK/GL4-k0MT

Affinity of anti-delta GK
antibody for antigen

H0000-SG115/GL4-k0MT

H0000-G1T3GK/GL4-k0MT

# Figure 3-1

Anti-GPC3 silent Fc antibody (0.1 ug/mL)
+ General-purpose TRAB (SG115 x CD3)

Anti-GPC3 silent Fc antibody (0 ug/mL)
+ General-purpose TRAB (SG115 x CD3)

# Figure 3-2

Anti-GPC3 delta GK-Fc antibody (0.1 ug/mL)
+ Delta GK-Fc x CD3

Anti-GPC3 delta GK-Fc antibody (0 ug/mL)
+ Delta GK-Fc x CD3

# Figure 4

pMSGV1 anti-silent Fc-CAR

leader sequence · Anti-silent Fc-VL · linker · Anti-silent Fc-VH · CD8α · CD28 · 4-1BB · CD3ζ · F2A · EGFP

# Figure 5-1

# Figure 5-2

# Figure 6

Group 1: Vehicle group
Group 2: Anti-GPC3 silent Fc antibody
Group 3: Anti-silent Fc-CAR-T
Group 4: Anti-GPC3 silent Fc antibody and
anti-silent Fc-CAR-T

**INTERNATIONAL SEARCH REPORT**

| International application No. |
| --- |
| PCT/JP2020/016915 |

A. CLASSIFICATION OF SUBJECT MATTER
A61K 39/395(2006.01)i; A61P 35/00(2006.01)i; C12N 5/10(2006.01)i; C12N
1/15(2006.01)i; C12N 1/19(2006.01)i; C12N 1/21 (2006.01)i; C12N
15/13(2006.01)i; C12N 15/62(2006.01)i; C12N 15/63(2006.01)i; A61K
38/16(2006.01)i; A61K 35/17(2015.01)i
FI:      A61K35/17 Z ZNA; C12N15/13; C12N15/62 Z; C12N15/63 Z; C12N1/15;
         C12N1/19; C12N1/21; C12N5/10; A61P35/00; A61K39/395 T; A61K38/16
According to International Patent Classification (IPC) or to both national classification and IPC

B. FIELDS SEARCHED

Minimum documentation searched (classification system followed by classification symbols)
A61K39/395; A61P35/00; C12N5/10; C12N1/15; C12N1/19; C12N1/21; C12N15/13;
C12N15/62; C12N15/63; A61K38/16; A61K35/17

Documentation searched other than minimum documentation to the extent that such documents are included in the fields searched
Published examined utility model applications of Japan          1922–1996
Published unexamined utility model applications of Japan        1971–2020
Registered utility model specifications of Japan                1996–2020
Published registered utility model applications of Japan        1994–2020

Electronic data base consulted during the international search (name of data base and, where practicable, search terms used)
JSTPlus/JMEDPlus/JST7580 (JDreamIII); CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y | WO 2018/177966 A1 (F. HOFFMANN–LA ROCHE AG)<br>04.10.2018 (2018-10-04) claims, page 75, paragraph<br>[0002] | 1, 2, 5–11<br>3–11 |
| Y | WO 2012/073985 A1 (CHUGAI PHARMACEUTICAL CO.,<br>LTD.) 07.06.2012 (2012-06-07) claims, paragraphs<br>[0068], [0071], [0203] | 3–11 |

| ☐ | Further documents are listed in the continuation of Box C. | ☒ | See patent family annex. |
| --- | --- | --- | --- |

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>16 June 2020 (16.06.2020) | Date of mailing of the international search report<br>30 June 2020 (30.06.2020) |
| --- | --- |

| Name and mailing address of the ISA/<br>Japan Patent Office<br>3-4-3, Kasumigaseki, Chiyoda-ku,<br>Tokyo 100-8915, Japan | Authorized officer |
| --- | --- |
| | Telephone No. |

Form PCT/ISA/210 (second sheet) (January 2015)

130

INTERNATIONAL SEARCH REPORT
Information on patent family members

International application No.

PCT/JP2020/016915

| Patent Documents referred in the Report | Publication Date | Patent Family | Publication Date |
|---|---|---|---|
| WO 2018/177966 A1 | 04 Oct. 2018 | JP 2020-515256 A<br>US 2020/093860 A1<br>EP 3600407 A1<br>CN 110662560 A<br>KR 10-2019-0133017 A | |
| WO 2012/073985 A1 | 07 Jun. 2012 | US 2014/0112914 A1<br>claims, paragraphs<br>[0114]-[0117], [0269]<br>EP 2647707 A1<br>CN 103429737 A<br>KR 10-2019-0028811 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012082841 A
- WO 2015058081 A
- WO 2016040441 A
- WO 2017161333 A
- WO 2018177966 A
- WO 2018189611 A
- WO 2012073985 A
- WO 2013180200 A
- US 4694778 A
- WO 2012138475 A
- US 20190359704 A
- US 6703199 B
- US 4816567 A
- WO 9607754 A
- WO 9209690 A
- US 5869046 A
- WO 9316185 A
- US 5571894 A
- US 5587458 A
- US 5641870 A
- WO 9306213 A
- WO 9308829 A
- WO 9404690 A
- US 4676980 A
- WO 9100360 A
- WO 9200373 A
- EP 03089 A
- US 6352694 B
- US 6534055 B
- US 6905680 B
- US 6692964 B
- US 5858358 A
- US 6887466 B
- US 6905681 B
- US 7144575 B
- US 7067318 B
- US 7172869 B
- US 7232566 B
- US 7175843 B
- US 5883223 A
- US 6905874 B
- US 6797514 B
- US 6867041 B
- US 20060121005
- US 20030157108 A, Presta, L.
- US 20040093621 A
- WO 03011878 A, Jean-Mairet
- US 6602684 B, Umana
- WO 9730087 A, Patel
- WO 9858964 A, Raju, S.
- WO 9922764 A, Raju, S.
- US 20050123546 A, Umana
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2004056312 A, Adams
- WO 9951642 A
- US 5648260 A
- US 5624821 A
- WO 9429351 A
- WO 0042072 A, Presta
- US 20050014934 A1, Hinton
- US 6194551 B1
- US 5500362 A
- US 5821337 A
- US 5648237 A, Carter
- US 5639635 A
- US 6083715 A, Georgiou
- US 6027888 A, Georgiou
- US 5264365 A, Georgiou
- US 5508192 A, Georgiou
- US 4965199 A
- US 4419446 A
- US 4601978 A
- WO 9411026 A
- US 4767704 A
- US 4657866 A
- US 4927762 A
- US 4560655 A
- US 5122469 A
- WO 9003430 A
- WO 8700195 A
- US 30985 A
- WO 9610038 A
- WO 9718185 A
- WO 9725329 A
- WO 9730170 A
- WO 9731934 A

- US 5278056 A
- US 5399346 A, Anderson
- US 4650764 A, Temin
- US 5124263 A, Temin
- WO 9507358 A, Dougherty

- US 7435596 B
- US 8026097 B
- WO 2016098356 A
- EP 3296395 A

**Non-patent literature cited in the description**

- **GRUPP et al.** *N Engl J Med,* 2013, vol. 368 (16), 1509-18
- **MAUDE et al.** *2014 N Engl J Med,* 2014, vol. 371 (16), 1507-17
- **KIM et al.** *J Am Chem Soc,* 2015, vol. 137, 2832-2835
- **TAMADA et al.** *Clin Cancer Res,* 2012, vol. 18 (23), 6436-6445
- **KONTERMANN.** *mAbs,* 2012, vol. 4, 182-197
- **MEZZANZANICA et al.** *International journal of cancer,* 1988, vol. 41, 609-615
- **STAERZ ; BEVAN.** *Proceedings of the National Academy of Sciences of the United States of America,* 1986, vol. 83, 1453-1457
- **STAERZ et al.** *Nature,* 1985, vol. 314, 628-631
- *Science,* 1988, vol. 242, 423-442
- *Nature,* 1989, vol. 334, 54454
- *Science,* 1988, vol. 242, 1038-1041
- *Nature,* 1985, vol. 314 (6012), 628-31
- *Int J Cancer,* 1988, vol. 41 (4), 609-15
- *Proc Natl Acad Sci USA,* 1986, vol. 83 (5), 1453-7
- *Proc. Natl. Acad. Sci. USA,* 1980, vol. 77, 4914-4917
- Hormones and their Actions Part II. New Comprehensive Biochemistry. Elsevier Science Publishers BV, 1988, vol. 18B, 1-46
- Adhesion Factor Handbook. Cell Technology suppl. Handbook series. Gakken Medical Shujunsha Co., Ltd, 1994
- **PATTHY.** *Cell,* 1990, vol. 61 (1), 13-14
- **ULLRICH et al.** *Cell,* 1990, vol. 61 (2), 203-212
- **MASSAGUE.** *Cell,* 1992, vol. 69 (6), 1067-1070
- **MIYAJIMA et al.** *Annu. Rev. Immunol.,* 1992, vol. 10, 295-331
- **TAGA et al.** *FASEB J,* 1992, vol. 6, 3387-3396
- **FANTL et al.** *Annu. Rev. Biochem.,* 1993, vol. 62, 453-481
- **SMITH et al.** *Cell,* 1994, vol. 76 (6), 959-962
- **FLOWER DR.** *Biochim. Biophys. Acta,* 1999, vol. 1422 (3), 207-234
- *Blood,* 1990, vol. 76 (1), 31-35
- *Cell,* 1989, vol. 57 (2), 277-285
- *Proc. Natl. Acad. Sci. USA.,* 1990, vol. 87 (22), 8702-8706
- *Cell,* 1990, vol. 61 (2), 341-350

- *Proc Natl Acad Sci U S A,* 1992, vol. 89 (12), 5640-5644
- *EMBO J,* 1993, vol. 12 (7), 2645-53
- *Nature,* 1985, vol. 313 (6005), 756-761
- *Proc. Natl. Acad. Sci. USA.,* 1994, vol. 91 (2), 459-463
- *Proc. Natl. Acad. Sci. USA.,* 1988, vol. 85 (10), 3435-3439
- *Cell,* 1990, vol. 60 (2), 225-234
- *Cell,* 1994, vol. 77 (3), 391-400
- *Int J Cancer,* 2003, vol. 103 (4), 455-65
- *Proc Natl Acad Sci U S A,* 1989, vol. 86 (1), 27-31
- Epitope Mapping Protocols. Methods in Molecular Biology. 1996, vol. 66
- *CHEMICAL ABSTRACTS,* 2457-80-9
- *Front Immunol,* 2018, vol. 9, 2359
- **HOLLINGER ; HUDSON.** *Nature Biotechnology,* 2005, vol. 23, 1126-1136
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **SAMBROOK ; FRITSCH &AMP ; MANIATIS.** Molecular Cloning: A Laboratory Manual. Cold Spring Harbor Laboratory Press, 1989
- DNA Cloning: A practical Approach. 1985, vol. I,II
- Oligonucleotide Synthesis. 1984
- Nucleic Acid Hybridization. 1985
- Transcription and Translation. 1984
- Animal Cell Culture. 1986
- Immobilized Cells and Enzymes. IRL Press, 1986
- **B. PERBAL.** *A practical Guide To Molecular Cloning,* 1984
- Current Protocols in Molecular Biology. John Wiley & Sons, Inc, 1994
- **KOHLER ; MILSTEIN.** *Nature,* 1975, vol. 256, 495-497
- **KOZBOR et al.** *Immunol. Today,* 1983, vol. 4, 72
- **COLE et al.** *Methods Enzymol,* 1986, vol. 121, 140-67
- **HUSE et al.** *Science,* 1989, vol. 246, 1275
- **KINDT et al.** Kuby Immunology. W.H. Freeman and Co, 2007, 91

- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **KOHLER et al.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON et al.** *Anal. Biochem.,* 1980, vol. 107, 220
- **KABAT et al.** Sequences of Proteins of Immunological Interest. 1991, vol. 1-3
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MARKS et al.** *J. Mol. Biol.,* 1991, vol. 222, 581-597
- **HOOGENBOOM et al.** *Nucl. Acids Res.,* 1991, vol. 19, 4133-4137
- **ORLANDI et al.** *Proc. Natl. Acad. Sci. (USA),* 1989, vol. 86, 3833-3837
- **WARD et al.** *Nature,* 1989, vol. 341, 544-546
- **JONES et al.** *Biotechnol.,* 1991, vol. 9, 88-89
- **SASTRY et al.** *Proc. Natl. Acad. Sci. (USA),* 1989, vol. 86, 5728-5732
- **ORUM et al.** *Nucleic Acids Res.,* 1993, vol. 21, 4491-4498
- **CLACKSON et al.** *Nature,* 1991, vol. 352, 624-628
- **TOMLINSON et al.** *J. Mol. Biol.,* 1992, vol. 227, 776-798
- **MATSUDA et al.** *Nature Genet.,* 1993, vol. 3, 88-94
- **BARBAS et al.** *Proc. Natl. Acad. Sci. USA,* 1992, vol. 89, 4457-4461
- **WILLIAMS ; WINTER.** *Eur. J. Immunol.,* 1993, vol. 23, 1456-1461
- **HOGREFE et al.** *Gene,* 1993, vol. 128, 119-126
- **WATERHOUSE et al.** *Nucl. Acids Res.,* 1993, vol. 21, 2265-2266
- **BARBAS et al.** *Proc. Natl. Acad. Sci. USA,* 1991, vol. 88, 7978-7982
- **EMBLETON et al.** *Nucl. Acids Res.,* 1992, vol. 20, 3831-3837
- **LEUNG et al.** *Technique,* 1989, vol. 1, 11-15
- **HAWKINS et al.** *J. Mol. Biol.,* 1992, vol. 226, 889-896
- **GRAM et al.** *Proc. Natl. Acad. Sci USA,* 1992, vol. 89, 3576-3580
- **MARKS et al.** *Biotechnol.,* 1992, vol. 10, 779-783
- **ENGELS et al.** *Agnew. Chem. Int. Ed. Engl.,* 1989, vol. 28, 716-734
- **ABRAHMSEN et al.** *EMBO J.,* 1985, vol. 4, 3901
- *Methods in Enzymology,* 1976, vol. 44
- **BARBAS et al.** *Proc. Natl. Acad. Sci USA,* 1991, vol. 88, 7978-7982
- **BASS et al.** *Proteins,* 1990, vol. 8, 309-314
- **SKERRA et al.** *Curr. Opinion in Immunol.,* 1993, vol. 5, 256
- **PLUCKTHUN.** *Immunol. Revs,* 1992, vol. 130, 151-188
- **MORRISON et al.** *Proc. Natl. Acad. Sci. USA,* 1984, vol. 81, 6851
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **RIECHMANN et al.** *Nature,* 1988, vol. 332, 323-327
- **VERHOEYEN et al.** *Science,* 1988, vol. 239, 1534-1536
- **SIMS et al.** *J. Immunol.,* 1993, vol. 151, 2296
- **CHOTHIA et al.** *J. Mol. Biol.,* 1987, vol. 196, 901
- **CARTER et al.** *Proc. Natl. Acad. Sci. USA,* 1992, vol. 89, 4285
- **PRESTA et al.** *J. Immunol.,* 1993, vol. 151, 2623
- **BOERNER et al.** *J. Immunol.,* 1991, vol. 147, 86
- **JAKOBOVITS et al.** *Proc. Natl. Acad. Sci. USA,* 1993, vol. 90, 2551-255
- **JAKOBOVITS et al.** *Nature,* 1993, vol. 362, 255-258
- **MILLSTEIN et al.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **BERG et al.** *Transplant Proc,* 1998, vol. 30 (8), 3975-3977
- **HAANEN et al.** *J. Exp. Med.,* 1999, vol. 190 (9), 13191328

- GARLAND et al. *J. Immunol Meth.,* 1999, vol. 227 (1-2), 53-63
- SHALABY et al. *J. Exp. Med.,* 1992, vol. 175, 217-225
- KOSTELNY et al. *J. Immunol.,* 1992, vol. 148 (5), 1547-1553
- HOLLINGER et al. *Proc. Natl. Acad. Sci. USA,* 1993, vol. 90, 6444-6448
- GRUBER et al. *J. Immunol.,* 1994, vol. 152, 5368
- TUTT et al. 147. *J. Immunol.,* 1991, 60
- CUNNINGHAM ; WELLS. *Science,* 1989, vol. 244, 1081-1085
- OKAZAKI et al. *J. Mol. Biol.,* 2004, vol. 336, 1239-1249
- YAMANE-OHNUKI et al. *Biotech. Bioeng.,* 2004, vol. 87, 614
- RIPKA et al. *Arch. Biochem. Biophys.,* 1986, vol. 249, 533-545
- DUNCAN ; WINTER. *Nature,* 1988, vol. 322, 738-40
- SHIELDS et al. *J. Biol. Chem.,* 2001, vol. 9 (2), 6591-6604
- GUYER et al. *J. Immunol.,* 1976, vol. 117, 587
- KIM et al. *J. Immunol.,* 1994, vol. 24, 249
- IDUSOGIE et al. *J. Immunol.,* 2000, vol. 164, 4178-4184
- RAVETCH ; KINET. *Annu. Rev. Immunol,* 1991, vol. 9, 457-92
- CLYNES et al. *PNAS (USA),* 1998, vol. 95, 652-656
- GAZZANO-SANTORO et al. *J. Immunol. Methods,* 1996, vol. 202, 163
- SIEBENLIST et al. *Cell,* 1980, vol. 20, 269
- PROBA ; PLUCKTHUN. *Gene,* 1995, vol. 159, 203
- BACHMANN. Cellular and Molecular Biology. American Society for Microbiology, 1987, vol. 2, 1190-1219
- SIMMONS et al. *J. Immunol. Methods,* 2002, vol. 263, 133-147
- CHEN et al. *J Bio Chem,* 1999, vol. 274, 19601-19605
- BOTHMANN ; PLUCKTHUN. *J. Biol. Chem.,* 2000, vol. 275, 17100-17105
- RAMM ; PLUCKTHUN. *J. Biol. Chem.,* 2000, vol. 275, 17106-17113
- ARIE et al. *Mol. Microbiol.,* 2001, vol. 39, 199-210
- HARA et al. *Microbial Drug Resistance,* 1996, vol. 2, 63-72
- LINDMARK et al. *J. Immunol. Meth.,* 1983, vol. 62, 1-13
- YANIV. *Nature,* 1982, vol. 297, 17-18
- GRAHAM et al. *J. Gen. Virol.,* 1977, vol. 36, 59
- URLAUB et al. *Proc. Natl. Acad. Sci. USA,* 1980, vol. 77, 4216
- MATHER. *Biol. Reprod.,* 1980, vol. 23, 243-251
- MATHER et al. *Annals N.Y. Acad. Sci.,* 1982, vol. 383, 44-68
- HAM et al. *Meth. Enz.,* 1979, vol. 58, 44
- BARNES et al. *Anal. Biochem.,* 1980, vol. 102, 255
- GUSS et al. *EMBO J,* 1986, vol. 5, 1567-1575
- GODFREY et al. *J. Molec. Diagnostics,* 2000, vol. 2, 84-91
- SPECHT et al. *Am. J. Pathol.,* 2001, vol. 158, 419-29
- Remington's Pharmaceutical Sciences. Lippincott, Williams & Wilkins, 2000
- BATZER et al. *Nucleic Acid Res.,* 1991, vol. 19, 5081
- OHTSUKA et al. *J. Biol. Chem.,* 1985, vol. 260, 2605-2608
- ROSSOLINI et al. *Mol. Cell. Probes,* 1994, vol. 8, 91-98
- MILONE et al. *Mol. Ther.,* 2009, vol. 17 (8), 1453-1464
- SASTRY et al. *J Virol,* 2011, vol. 85 (5), 1935-1942
- SERGEEVA et al. *Bood,* 2011, vol. 117 (16), 4262-4272
- VERMA et al. *J Immunol,* 2010, vol. 184 (4), 2156-2165
- WILLEMSEN et al. *Gene Ther,* 2001, vol. 8 (21), 1601-1608
- DAO et al. *Sci Transl Med,* 2013, vol. 5 (176), 176-33
- TASSEV et al. *Cancer Gene Ther,* 2012, vol. 19 (2), 84-100
- *Nature,* 1989, vol. 338, 383-384
- *J Immunol.,* 1999, vol. 162 (2), 897-902
- SCHMITZ J et al. *J Immunol,* 2000, vol. 164, 848-54
- TOMIDA M et al. *Blood,* 1999, vol. 93, 1934-41
- Remington's Pharmaceutical Sciences. Mack Pub. Co, 1991
- LOZZIO et al. *Blood,* 1975, vol. 45 (3), 321-334
- KLEIN et al. *Int. J. Cancer,* 1976, vol. 18, 421-431
- CAROLINE J. Suicide Gene Therapy: Methods and Reviews. Springer, Humana Press, 2004
- *Proceedings of the National Academy of Sciences of the United States of America,* 1988, vol. 85, 6460-6464
- MANN et al. *Cell,* 1983, vol. 33, 153
- KUO et al. *Blood,* 1993, vol. 82, 845
- TORIKAI et al. *Blood,* 2012, vol. 119, 5697-5705
- THOMSON P D R ; MONTVALE N.J. Physician's Desk Reference. 2005

- Remington's The Science and Practice of Pharmacy. Lippincott Williams and Wilkins, 2000
- Harrison's Principles of Internal Medicine. McGraw Hill, 2001
- The Merck Manual of Diagnosis and Therapy. Merck Research Laboratories, 1992
- *Protein Science,* 1995, vol. 4, 2411-2423
- **HUGHES M.S. et al.** *Hum Gene Ther,* April 2005, vol. 16 (4), 457-72
- **TAMADA K et al.** *Clin Cancer Res,* 2002, vol. 18, 6436-6445